# EUROPEAN PATENT APPLICATION

(11) **EP 2 808 402 A2**
(43) Date of publication of application: **03.12.2014**
(21) Application number: 14179548.4
(22) Date of filing: 29.12.2009
(51) Int. Cl.: C12Q 1/68, G01N 33/50

(54) **Methods of predicting responsiveness to interferon treatment**

(30) Priority: 29.12.2008 US 141048 P; 21.01.2009 US 146150 P; 08.06.2009 US 184910 P; 21.10.2009 US 253703 P
(62) Divisional of application: 09808964.2
(71) Applicant: Yissum Research Development Company of the Hebrew University of Jerusalem Ltd., 91390 Jerusalem (IL)
(72) Inventor: Smith, Yoav, 96956 Jerusalem (IL)
(74) Representative: Bentham, Andrew

(57) **Abstract**

A method of predicting responsiveness to interferon treatment of a subject diagnosed with multiple sclerosis or infected with HCV type 2, 3 or 4, comprising comparing a level of expression in a cell of the subject of IFI6, OAS2, ISG15, OAS3 and IFIT1 genes to a reference expression data of said genes obtained from at least one interferon responder subject and/or at least one interferon non-responder subject, thereby predicting the responsiveness of the subject to interferon treatment.

## Description

### FIELD AND BACKGROUND OF THE INVENTION

The present invention, in some embodiments thereof, relates to methods of predicting responsiveness to interferon treatment in subjects infected with hepatitis C virus types 1, 2, 3 or 4 or patients diagnosed with multiple sclerosis, and methods of treating hepatitis c infection.

Interferon is widely used to treat a variety of diseases, in particular hepatitis C virus infection (HCV) and multiple sclerosis (MS). Interestingly, in both type 1 HCV and MS the success of the treatment is only about 50 %, meaning that half of the population can not benefit from the treatment, while still suffering from its side effects. In HCV types 2, 3 and 4 the chances of interferon treatment success are about 80 %. One approach of trying to understand the genetic scenario behind this reality is to look at the gene expression of people in these two groups, before and after the treatment using microarray technology.

Chen et al., 2005 compared the gene expression levels in liver specimens taken before treatment with interferon of 15 non-responders and 16 responders to Pegylated interferon (IFN-alpha) and identified 18 genes whose expression differed significantly between all responders and all non responders and concluded that up-regulation of a specific set of IFN-responsive genes predicts non response to exogenous therapy.

WO2007039906 discloses a method for selecting a set of genes whose expression is different in a first group (for example responders to a specific treatment) as compared to a second group (for example non-responders to a specific treatment) from a pre-determined set of genes such as the full genome.

Taylor M., et al., 2007 found that the induced levels of the OAS1 and 2, MX1, IRF-7 and TLR-7 is lower in poor-interferon response HCV patients than in marked or intermediate interferon response HCV patients.

Van Baarsen et al., 2008 show that the expression level of IFN response genes in the peripheral blood of multiple sclerosis patients prior to treatment can serve a role as a biomarker for the differential clinical response to interferon beta.

Zeremski M, et al., 2007 (J Acquir Immune Defic Syndr. 2007 Jul 1;45(3):262-8) showed that PEG-IFN-induced elevations in IP-10 are greater in virological responders than in nonresponders after the first PEG-IFN dose.

Tarantino et al., 2008, disclosed that serum levels of B-Lymphocytes stimulator (BLyS) have a potential role as a predictor of outcome in patients with acute hepatitis C.

Additional background art includes US Pat Appl. 20060177837 (Borozan Ivan et al.), Lopez- Vazquez et al., 2005 (JID 192: 162-165), Ahmad A and Alvarez F. 2004 (J. of Leukocyte Biology, 76:743-759), Parham P, 2004 (Science 305:786-787), Parham P, 2005 (Nature Reviews, Immunology 5:201-214), Zuniga J., et al., 2009 (Molecular Immunology 46:2723-2727), Rauch A., et al. 2007 (Tissue Antigens 69 Suppl 1:237-40), Paladino N., et al., 2007 (Tissue Antigens 69 Suppl 1:109-111); Giannini C, et al., 2008, (Blood. 112:4353-4); Querec TD et al., 2009 (Nat Immunol. 10:116-25. Epub 2008 Nov 23), Khakoo SI., et al., 2004; Vidal-Casrineira JR., 2009; Rajagopalan S and Long EO. 2005; Gonzalez S, et al., 2005; Shah N., et al., 2009; Vitale M., et al. 2004.

### SUMMARY OF THE INVENTION

According to an aspect of some embodiments of the present invention there is provided a method of predicting responsiveness of a subject to interferon treatment, comprising comparing a level of expression in a cell of the subject of at least one gene selected from the group consisting KIR3DL3, KIR3DL2, KIR3DL1, KIR2DL1, KIR2DL2, KIR2DL3, KLRG1, KIR3DS1, CD160, HLA-A, HLA-B, HLA-C, HLA-F, HLA-G and IFI27 to a reference expression data of the at least one gene obtained from at least one interferon responder subject and/or at least one interferon non-responder subject, thereby predicting the responsiveness of the subject to interferon treatment.

According to an aspect of some embodiments of the present invention there is provided a method of predicting responsiveness of a subject to interferon treatment, comprising comparing a ratio determined between an expression level of TNFRSF17 gene in a cell of the subject following interferon treatment and an expression level of the gene in the cell of the subject prior to interferon treatment, or visa versa, to a reference ratio determined in a cell of at least one interferon responder subject and/or at least one interferon non-responder subject, the reference ratio is determined between an expression level of the gene following interferon treatment and an expression level of the gene prior to interferon treatment, or visa versa, thereby predicting the responsiveness to interferon treatment of a subject.

According to an aspect of some embodiments of the present invention there is provided a method of predicting responsiveness to interferon treatment of a subject diagnosed with multiple sclerosis or infected with HCV type 2, 3 or 4, comprising comparing a level of expression in a cell of the subject of IFI6, OAS2, ISG15, OAS3 and IFIT1 genes to a reference expression data of the genes obtained from at least one interferon responder subject and/or at least one interferon non-responder subject, thereby predicting the responsiveness of the subject to interferon treatment.

According to an aspect of some embodiments of the present invention there is provided a method of predicting responsiveness of a subject to interferon treatment, comprising comparing a ratio determined between an expression level of ISG15, IFI6, IFIT1, OAS2 and OAS3 genes in a cell of the subject following interferon treatment and an expression level of the genes in the cell of the subject prior to interferon treatment, or visa versa, to a reference ratio determined in a cell of at least one interferon responder subject and/or at least one interferon non-responder subject, the reference ratio is determined between an expression level of the genes following interferon treatment and an expression level of the genes prior to interferon treatment, or visa versa, thereby predicting the responsiveness to interferon treatment of a subject.

According to an aspect of some embodiments of the present invention there is provided a method of predicting responsiveness of a subject to interferon treatment, comprising comparing a ratio determined between an expression level of at least one gene selected from the group consisting of: TICAM1, MYD88, TLR7, TRAFD1 and IRF7 in a cell of the subject following interferon treatment and an expression level of the at least one gene in the cell of the subject prior to interferon treatment, or visa versa, to a reference ratio determined in a cell of at least one interferon responder subject and/or at least one interferon non-responder subject, the reference ratio is determined between an expression level of the gene following interferon treatment and an expression level of the gene prior to interferon treatment, or visa versa, thereby predicting the responsiveness to interferon treatment of a subject.

According to an aspect of some embodiments of the present invention there is provided a method of predicting responsiveness of a subject to interferon treatment, comprising comparing a ratio determined between an expression level of at least one gene selected from the group consisting of HERC5 and UBE2L6 in a liver cell of the subject following interferon treatment and an expression level of the at least one gene in the liver cell of the subject prior to interferon treatment, or visa versa, to a reference ratio determined in a cell of at least one interferon responder subject and/or at least one interferon non-responder subject, the reference ratio is determined between an expression level of the at least one gene following interferon treatment and an expression level of the at least one gene prior to interferon treatment, or visa versa, thereby predicting the responsiveness to interferon treatment of a subject.

According to an aspect of some embodiments of the present invention there is provided a method of predicting responsiveness of a subject to interferon treatment, comprising comparing a ratio determined between an expression level of at least one gene selected from the group consisting ISG15, IFI6, IFIT1, OAS2 and OAS3 in a liver cell of the subject following interferon treatment and an expression level of the at least one gene in the liver cell of the subject prior to interferon treatment, or visa versa, to a reference ratio determined in a liver cell of at least one interferon responder subject and/or at least one interferon non-responder subject, the reference ratio is determined between an expression level of the at least one gene following interferon treatment and an expression level of the at least one gene prior to interferon treatment, or visa versa, thereby predicting the responsiveness to interferon treatment of a subject.

According to an aspect of some embodiments of the present invention there is provided a method of treating of a subject in need of interferon treatment, the method comprising: (a) predicting the responsiveness of the subject to interferon treatment according to the method of the invention, and (b) selecting a treatment regimen based on the responsiveness; thereby treating the subject in need of interferon treatment.

According to an aspect of some embodiments of the present invention there is provided a method of treating a subject in need of interferon therapy, comprising co-administering to the subject interferon and an agent capable of downregulating HLA or KIR inhibitory receptor, thereby treating the subject in need of interferon therapy.

According to an aspect of some embodiments of the present invention there is provided a pharmaceutical composition comprising interferon, an agent capable of downregulating HLA or KIR inhibitory receptor, and a pharmaceutically acceptable carrier.

According to some embodiments of the invention, a decrease above a predetermined threshold in the level of expression of the at least one gene in the cell of the subject relative to the reference expression data of the at least one gene obtained from the at least one interferon non-responder subject predicts responsiveness of the subject to interferon treatment of the subject.

According to some embodiments of the invention, an increase above a predetermined threshold in the level of expression of the at least one gene in the cell of the subject relative to the reference expression data of the at least one gene obtained from the at least one interferon responder subject predicts lack of responsiveness of the subject to interferon treatment of the subject.

According to some embodiments of the invention, when a level of expression of the at least one gene in the cell of the subject is identical or changed below a predetermined threshold as compared to the reference expression data of the at least one gene obtained from the at least one interferon responder subject, then the subject is classified as responsive to interferon.

According to some embodiments of the invention, when a level of expression of the at least one gene in the cell of the subject is identical or changed below a predetermined threshold as compared to the reference expression data of the at least one gene obtained from the at least one interferon non-responder subject, then the subject is classified as a non-responsive to interferon.

According to some embodiments of the invention, an increase above a predetermined threshold in the ratio of the subject relative to the reference ratio of the at least one interferon non-responder subject predicts responsiveness of the subject to interferon treatment of the subject.

According to some embodiments of the invention, a decrease above a predetermined threshold in the ratio of the subject relative to the reference ratio of the at least one interferon responder subject predicts lack of responsiveness of the subject to interferon treatment of the subject.

According to some embodiments of the invention, when the ratio of the subject is identical or changed below a predetermined threshold as compared to the reference ratio of the at least one interferon responder subject, then the subject is classified as responsive to interferon.

According to some embodiments of the invention, when the ratio of the subject is identical or changed below a predetermined threshold as compared to the reference ratio of the at least one interferon non-responder subject, then the subject is classified as non-responsive to interferon.

According to some embodiments of the invention, the level of expression is determined prior to interferon treatment.

According to some embodiments of the invention, the cell is a blood cell.

According to some embodiments of the invention, the cell is a liver cell.

According to some embodiments of the invention, following interferon treatment is effected about 4 hours after interferon treatment.

According to some embodiments of the invention, following interferon treatment is effected about 24 hours after interferon treatment.

According to some embodiments of the invention, the subject is diagnosed with HCV infection type 1, 2, 3 or 4.

According to some embodiments of the invention, the method further comprising comparing a ratio determined between an expression level of at least one gene selected from the group consisting of CXCL10 and CD24 in a cell of the subject following interferon treatment and an expression level of the gene in the cell of the subject prior to interferon treatment, or visa versa, to a reference ratio determined in a cell of at least one interferon responder subject and/or at least one interferon non-responder subject, the reference ratio is determined between an expression level of the at least one gene following interferon treatment and an expression level of the at least one gene prior to interferon treatment, or visa versa, thereby predicting the responsiveness to interferon treatment of a subject.

According to some embodiments of the invention, the subject is diagnosed with multiple sclerosis the cell of the subject is a blood cell.

According to some embodiments of the invention, the subject is infected with HCV type 2, 3 or 4 the cell of the subject is a liver cell.

According to some embodiments of the invention, the cell is a blood cell.

According to some embodiments of the invention, further comprising comparing a ratio determined between an expression level of at least one gene selected from the group consisting of ISG15 and USP18 in a liver cell of the subject following interferon treatment and an expression level of the at least one gene in the liver cell of the subject prior to interferon treatment, or visa versa, to a reference ratio determined in a cell of at least one interferon responder subject and/or at least one interferon non-responder subject, the reference ratio is determined between an expression level of the at least one gene following interferon treatment and an expression level of the at least one gene prior to interferon treatment, or visa versa, thereby predicting the responsiveness to interferon treatment of a subject.

According to some embodiments of the invention, the co-administering is effected so as to enable a pharmacokinetic overlap between the interferon and the agent.

According to some embodiments of the invention, the subject is infected with HCV type 1.

According to some embodiments of the invention, the subject is infected with HCV type 2, 3 or 4

According to some embodiments of the invention, the subject is diagnosed with multiple sclerosis.

According to some embodiments of the invention, the level of expression is determined using an RNA detection method.

According to some embodiments of the invention, the level of expression is determined using a protein detection method.

According to some embodiments of the invention, the agent is selected from the group consisting of an antibody, an RNA silencing molecule, a ribozyme and a DNAzyme.

According to some embodiments of the invention, the antibody is an anti-KIR inhibitory receptor antibody.

According to some embodiments of the invention, the RNA silencing molecule is an siRNA directed against a KIR inhibitory receptor or a HLA.

According to some embodiments of the invention, the subject is a non-responder to interferon treatment.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

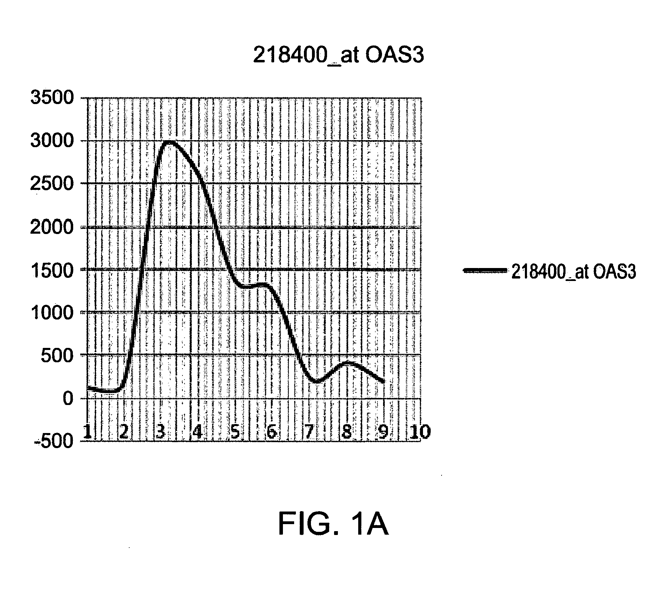

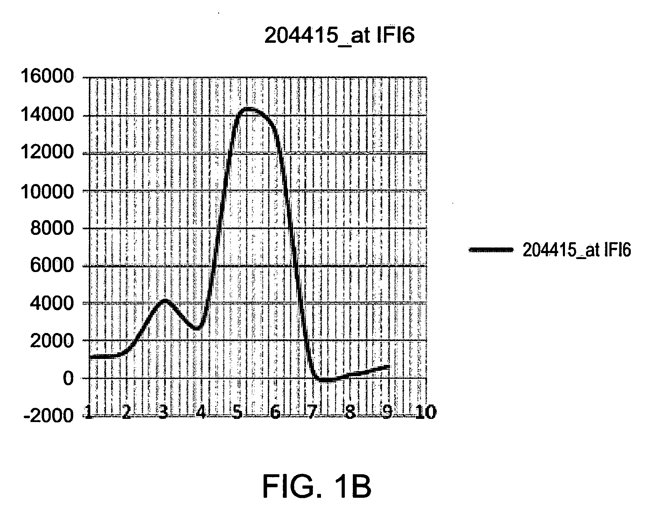

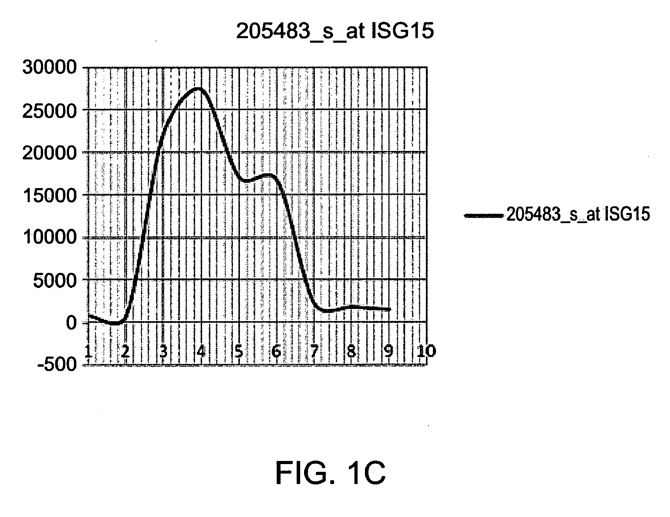

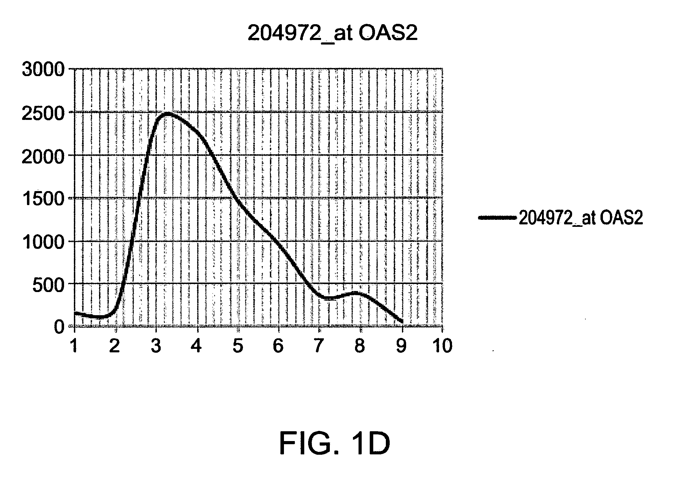

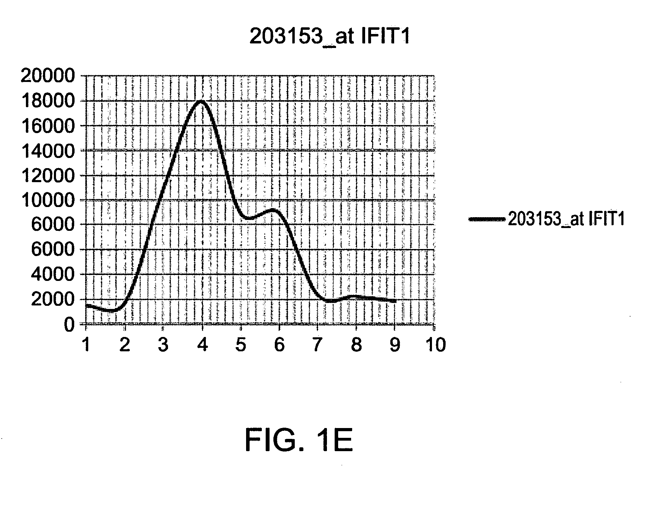

FIGs. 1A-E depict signature gene expression in liver tissue of HCV type 1 patients prior to interferon injection. Expression data was downloaded from the Gene Expression Omnibus Accession No. GSE11190. Figure 1A- 218400_at OAS3; Figure 1B - 204415_at IF1 6; Figure 1C - 205483_s_at ISG15; Figure 1D - 204972_at OAS2; Figure 1E - 203153_at IFIT1. Sequences of probes are provided in Table 2 (Example 2 of the Examples section which follows). Numbers on the "X" axis refer to subjects as follows: Subjects 1-2 = healthy controls; subjects 3-6 - non-responders to interferon; subjects 7-9 - responders to interferon. Note that non responders (subjects 3-6) exhibit high expression of the tested probes (genes) while responders (subjects 7-9) and healthy controls (subjects 1-2) exhibit low expression of the tested probes (genes). Raw data is provided in Table 3 (Examples section which follows).

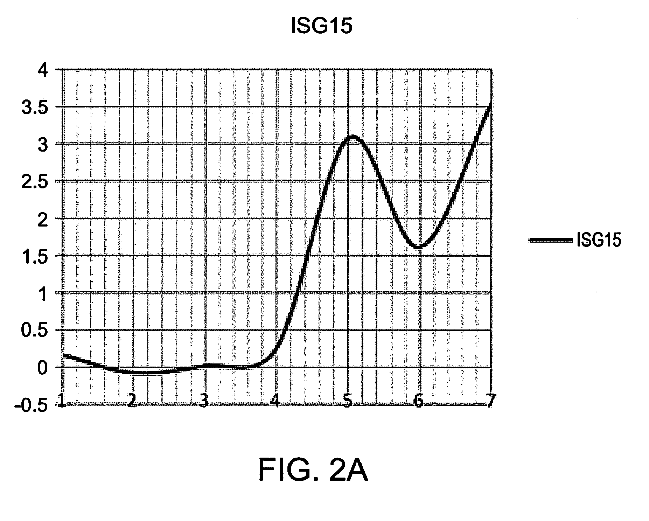

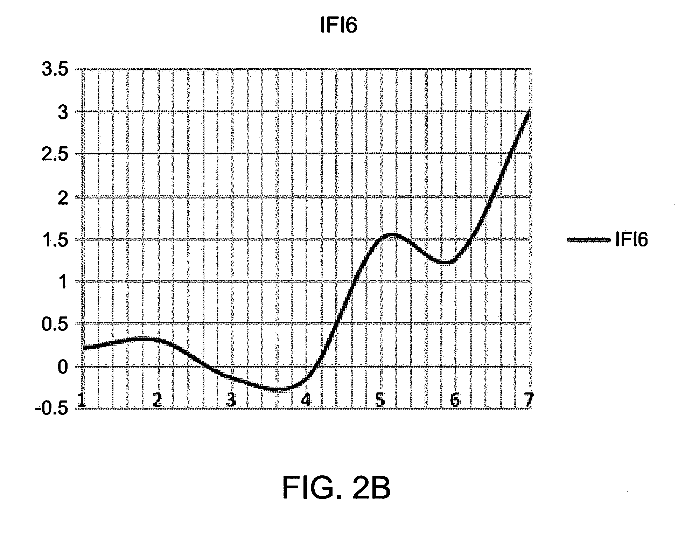

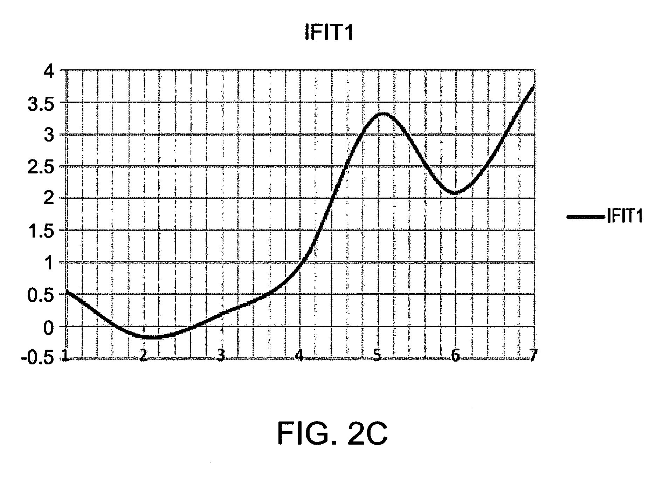

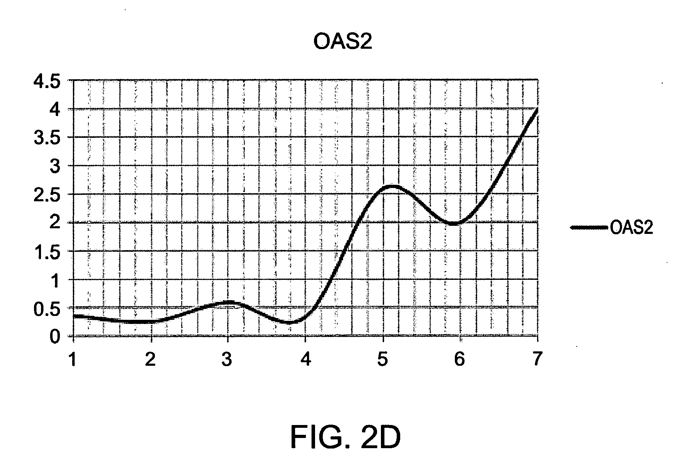

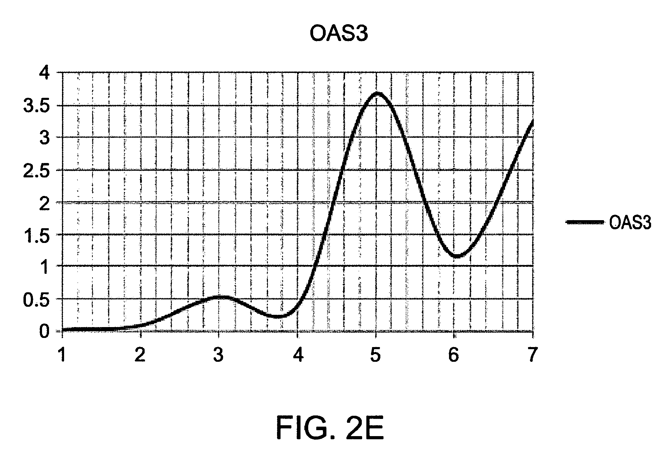

FIGs. 2A-E depict the logarithmic ratio between the expression level of the tested genes measured in liver tissues of type 1 HCV 4 hours after interferon treatment as compared to the expression level measured in the same tissues prior to interferon treatment. Expression data was downloaded from the Gene Expression Omnibus Accession No. GSE11190. Figure 2A - ISG15; Figure 2B - IFI6; Figure 2C - IFIT1; Figure 2D - OAS2; Figure 2E - OAS3. Numbers on the "X" axis refer to subjects as follows: Subjects 1-4 = non-responders to interferon; subjects 5-7 - responders to interferon. Note that while in Type 1 HCV non responders (subjects 1-4) there is no change in the expression level of the tested genes 4 hours after *in vivo* injection of interferon as compared to the level prior to interferon injection, in interferon responders (subjects 5-7) there is a significant log2 up-regulation in the expression level of the tested genes 4 hours after *in vivo* injection of interferon as compared to before injection. Raw data is provided in Table 4 (Examples section which follows).

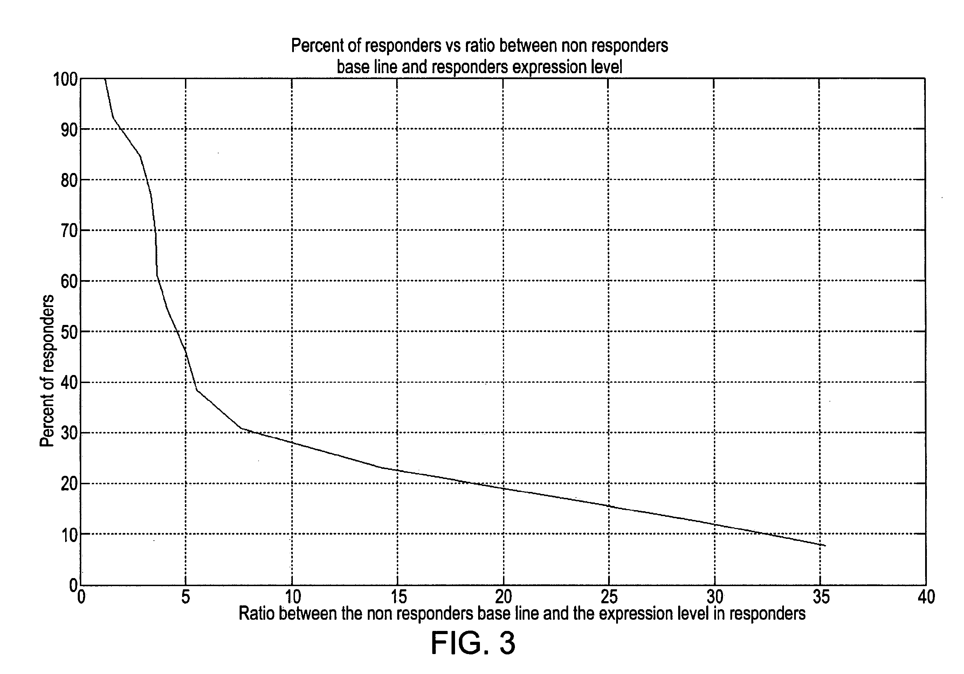

FIG. 3 is a graph depicting the distribution of the ratio between the non-responders HCV type 1 base line and the expression level in responders of the 5-signature genes (ISG15, IFI6, IFIT1, OAS2, OAS3) among interferon responders as measured in liver tissues of naive patients *(i.e.,* time 0, before the first interferon injection). Each point in the graph depicts the percentage of interferon responders having the specific ratio between the non-responders base line and the level in responders. For example, while in 100% of the interferon responders the expression level is 1/1.17 than the non-responders baseline, in 7.6% of the responders the expression level is 1/35 than the non-responders baseline.

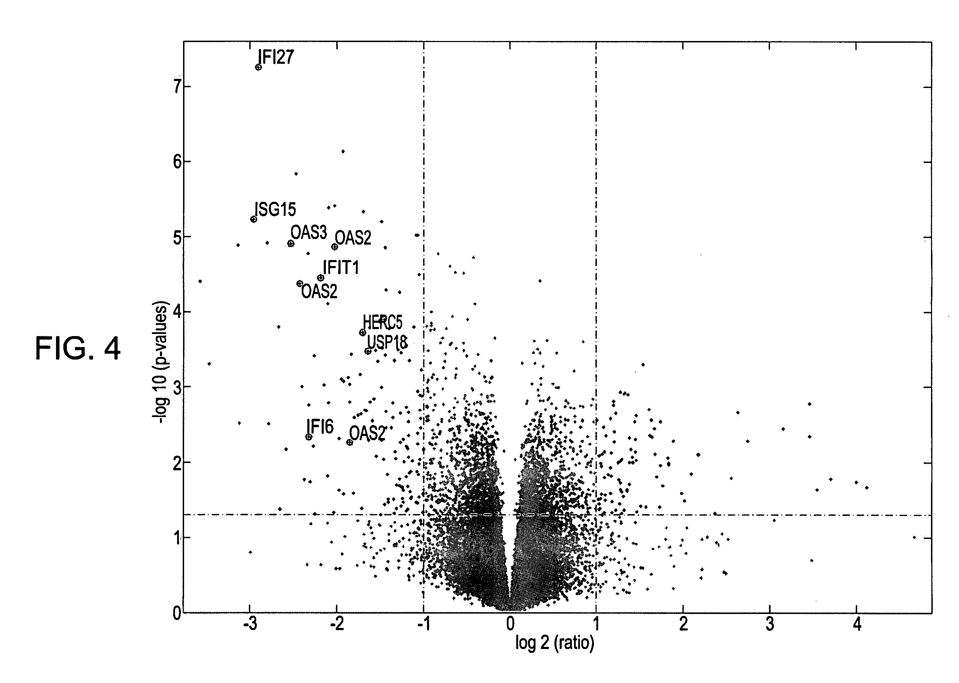

FIG. 4 is a volcano plot depicting the significance of changes in the expression levels of various genes in liver of HCV types 1-4 between interferon responders and non-responders as measured prior to interferon injection. Expression data was downloaded from the Gene Expression Omnibus Accession No. GSE11190. The "X" axis represents log2 of ratio between responders to non responders where the vertical red lines on the right and left represent fold change of 2, meaning that points marked on the left of the left vertical line are up-regulated in non responders and points marked on the right of the right vertical red line are up-regulated more than 2 folds in the responders. The "Y" axis represents the p value assigned to the points. The horizontal red line corresponds to a p value of 0.05. Points appearing above the red horizontal line corresponds to p values lower than 0.05 (i.e., more significant). Note that in liver tissues of HCV types 1-4 interferon responders the 5 genes *i.e.,* ISG15, IFIT1, IFI6, OAS2, OAS3 are down regulated as compared to the non-responders, *i.e.,* the level in non-responders is significantly higher than in the responders.

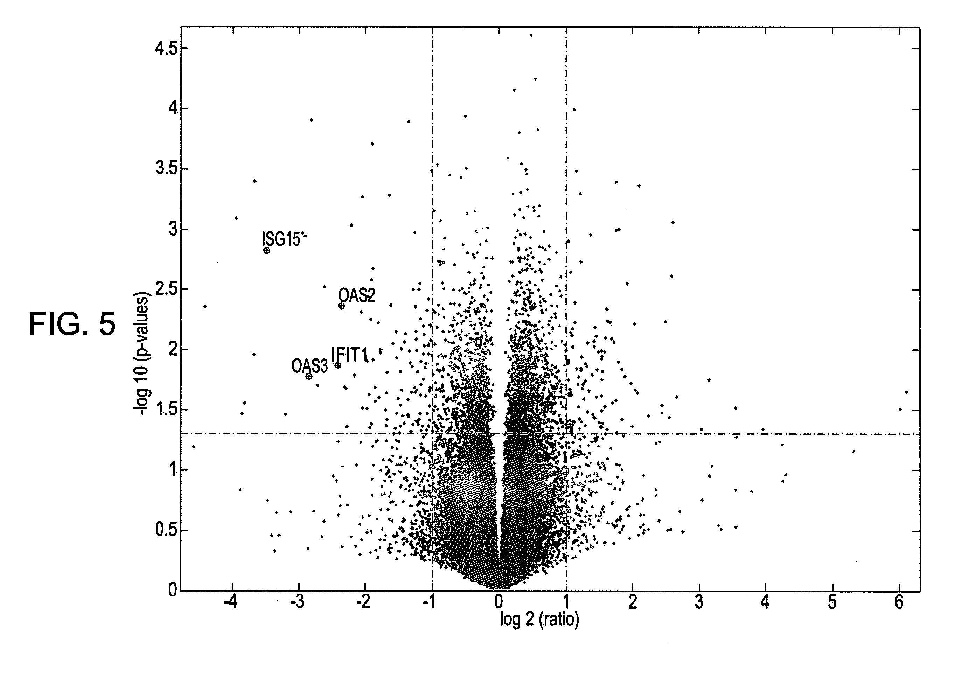

FIG. 5 is a volcano plot depicting the significance of changes in expression levels of various genes in liver of HCV type 1 measured prior to interferon injection between interferon responders and non-responders. Expression data was downloaded from the Gene Expression Omnibus Accession No. GSE11190. The "X" and "Y" axes as well as the vertical and horizontal red lines are as described above with respect to Figure 4. Note that in tissues of HCV type 1 the 5 genes *i.e.,* ISG15, IFIT1, IFI6, OAS2, OAS3 (4 of them are marked) are down regulated in responders HCV type 1 before treatment as compared to non-responders.

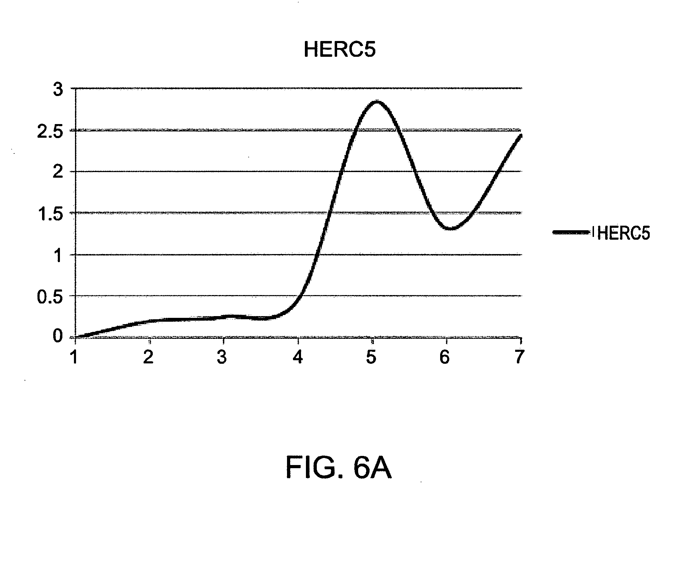

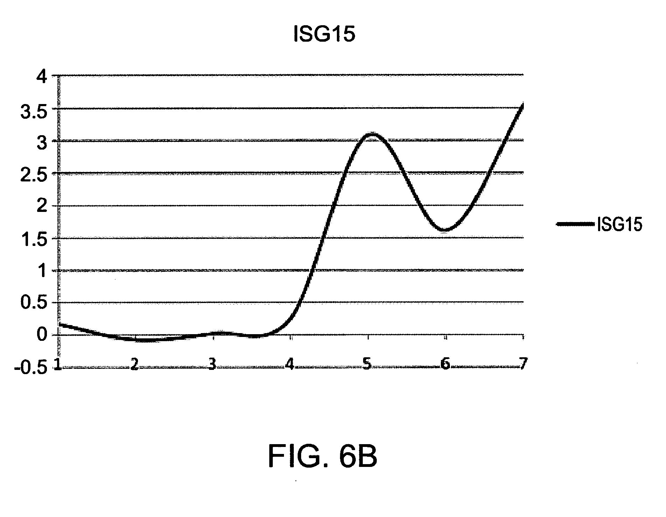

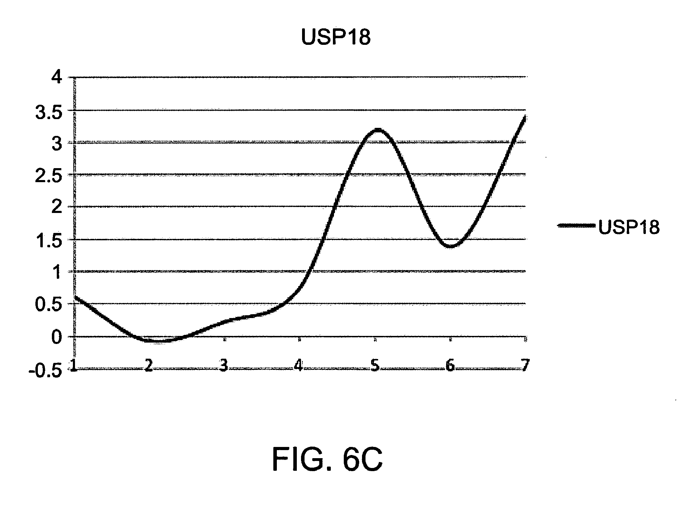

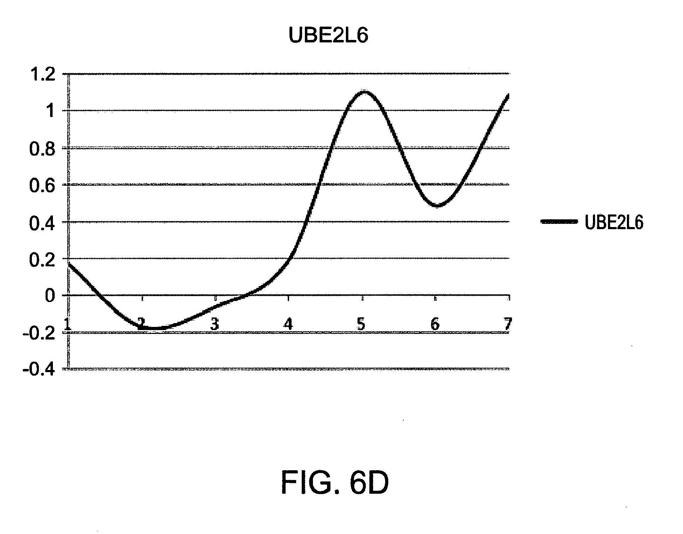

FIGs. 6A-D depict the ratio on a logarithmic scale between the expression level of the HERC5, ISG15, USP18 and UBE2L6 genes involved in the ISGylated process in liver HCV type 1 biopsies before injection of interferon and 4 hours after *in vivo* injection of interferon. Expression data was downloaded from the Gene Expression Omnibus Accession No. GSE11190. Figure 6A - HERC5; Figure 6B - ISG15; Figure 6C - USP18; Figure 6D - UBE216; Numbers on the "x" axis refer to subjects as follows: Subjects 1-4 = non-responders to interferon; subjects 5-7 - responders to interferon. Sequences of probes are provided in Table 5 in the Examples section which follows. Note that while in tissues (liver biopsies) obtained from Type 1 HCV responders to interferon (subjects 5-7) there is a significant up-regulation of the genes involved in the ISGylated process 4 hours after interferon injection as compared to the level prior to interferon injection, in non-responders (subjects 1-4) there is no change in the expression level of these genes following interferon treatment. Raw data is provided in Table 6 in the Examples section which follows.

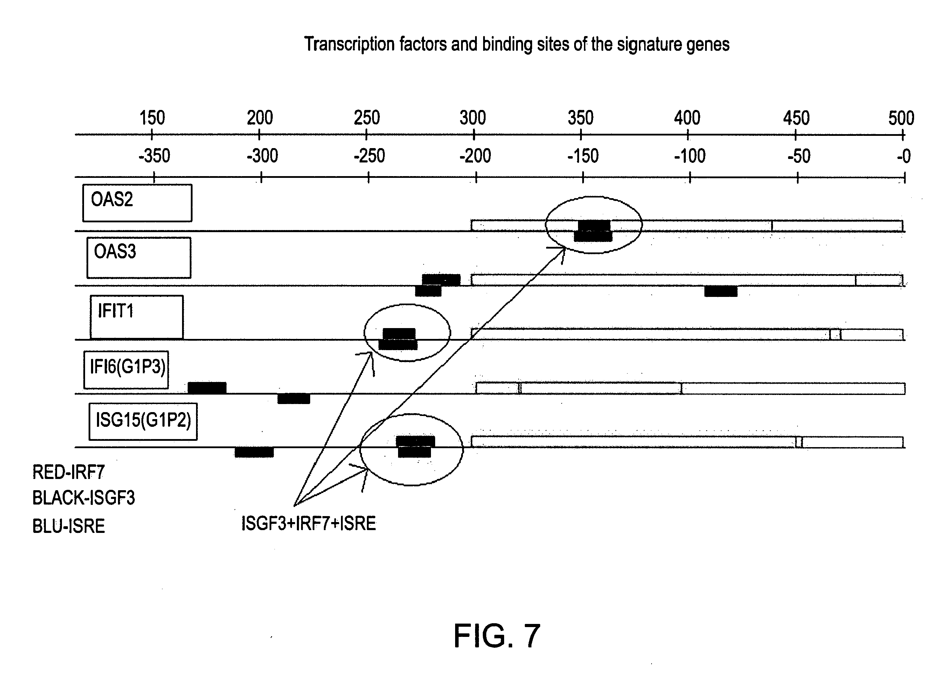

FIG. 7 is a schematic presentation of the genomic sequences of the signature genes OAS2, OAS3, IFIT1, IFI6 (G1P3) and ISG15 (G1P2) depicting analysis of the transcription factors and binding sites of the signature genes. Red - IRF7; Black-ISGf3; Blue - ISRE. Note that the regulatory sequences of all analyzed genes include the ISRE promoter where ISGF3 complex and IRF7 are the controlling elements.

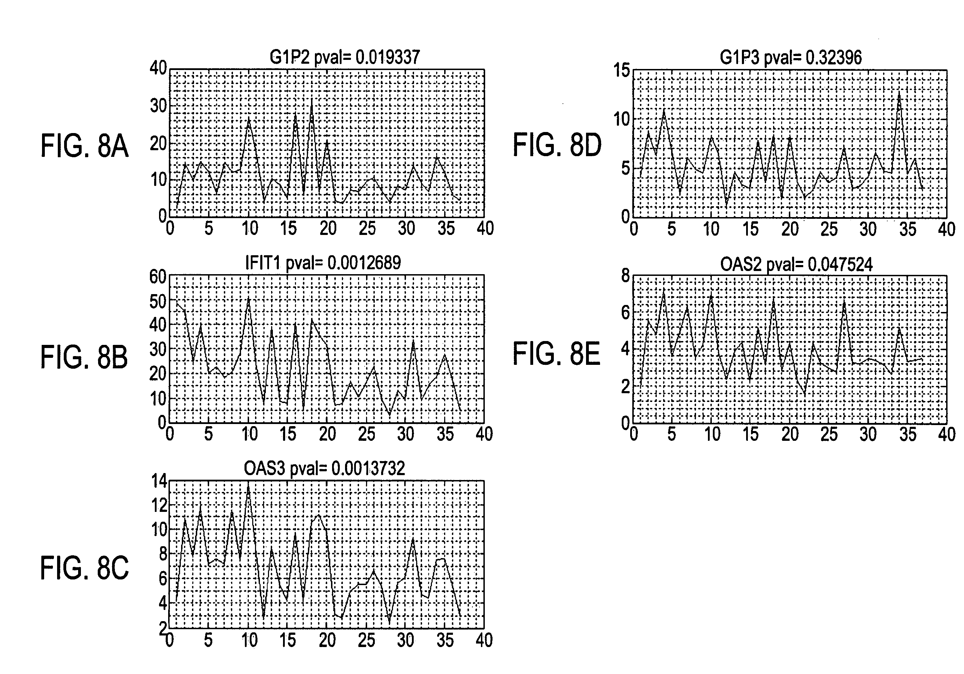

FIGs. 8A-E depict the fold change gene expression levels of G1P2 (Figure 8A), IFIT1 (Figure 8B), OAS3 (Figure 8C), G1P3 (Figure 8D) and OAS2 (Figure 8E) in PBMC of type 1 HCV patients detected 24 hours after interferon treatment compared to the level detected prior to interferon treatment. Numbers on the "X" axis refer to subjects as follows: Subjects 1-20 - responders to interferon; subjects 21-37 - non-responders to interferon. Numbers on the "y" axis refer to fold change in gene expression level. The t-test p-values were calculated on the average and standard variation between the 2 groups. RMA normalized data down loaded from GSE7123.

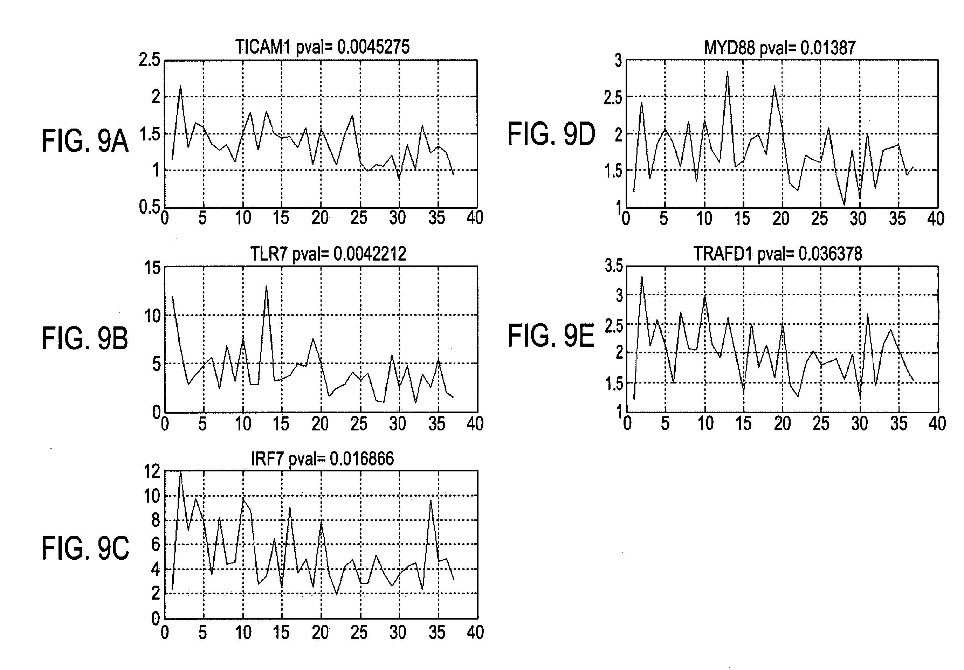

FIGs. 9A-E depict the fold change in gene expression level of key genes from the TLR-Mediated Type I IFN induction pathways in PBMC of type 1 HCV patients detected 24 hours after the interferon injection treatment as compared to the level detected before interferon treatment. Figure 9A - TICAM1; Figure 9B - TLR7; Figure 9C - IRF7; Figure 9D - MYD88; Figure 9E - TRAFD1. Sequences of the probes and genes are provided in Table 7 in the Example section which follows. RMA normalized data down loaded from GSE7123. Numbers in the "x" axis refer to subjects as follows: Subjects 1-20 - responders to interferon; subjects 21-37 - non-responders to interferon. "Y" axis = numbers refer to fold change gene expression levels [fold change =2^ log2 level at 24 - log2 level at 0 on RMA data]. The t-test p-values were calculated on the average and standard variation between the 2 groups. Note that 24 hours following interferon treatment the key genes of the Tlr 9 mediated pathway show significant up-regulation as compared to before treatment in PBMC of responders, but not in non-responder.

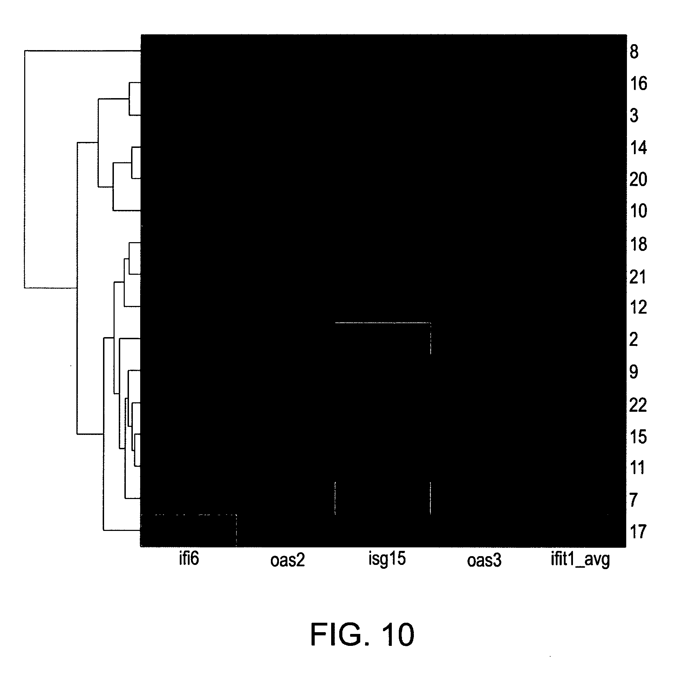

FIG. 10 is a clustergram of the 5 signature genes IFI6, OAS2, ISG15, OAS3, IFIT1_avg, which were found to be switch response genes (for interferon response) in HCV (WO2007039906), using the expression level in a multiple sclerosis (MS) microarray data as downloaded from the Gene Expression Omnibus Accession No. GSE10655 [publicly available from Hypertext Transfer Protocol://World Wide Web (dot) ncbi (dot) nlm (dot) nih (dot) gov/projects/geo/; van Baarsen LGM, Vosslamber S, Tijssen M, Baggen JMC, van der Voort LF, et al. (2008) Pharmacogenomics of Interferon-ß Therapy in Multiple Sclerosis: Baseline IFN Signature Determines Pharmacological Differences between Patients. PLoS ONE 3(4): e1927. doi:10.1371/journal.pone.0001927]. The expression level of IFI6, OAS2, ISG15, OAS3 and IFIT1 was determined in blood before interferon treatment in all MS patients (subjects 8, 16, 3, 14, 20, 10, 18, 21, 12, 2, 9, 22, 15, 11, 7 and 17). The color index of expression level is as follows: Green - low expression; black - middle expression; red - high expression. Note that MS patients numbers 7, 17 (non-responders to interferon as indicated by an increase in relapse rate from 3 relapses per year to 5 relapses per year) and MS patients 10 and 18 (responders to interferon as indicated by the switch from 3 relapses per year to 0 relapses per year) exhibit the same signature genes expression pattern as their parallel non responders and responders to interferon among HCV patients. For example, a very high expression of isg15 and ifi6 followed by a high expression of oas2 oas3 and ifit1 in non-responders (subjects 7 and 17) and the opposite (i.e., low expression) of these genes in the responders (subjects 10 and18).

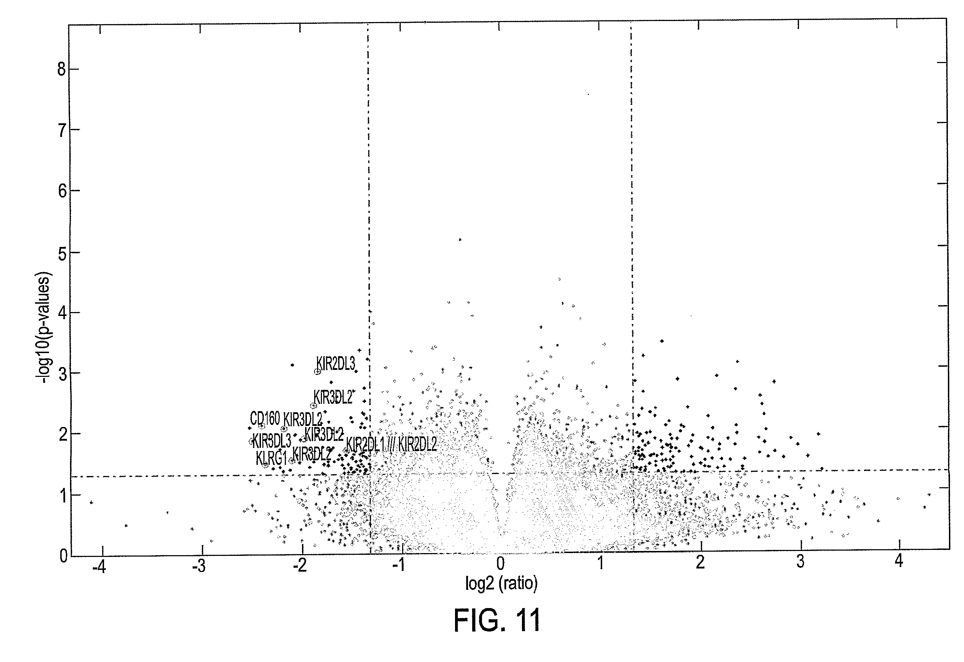

FIG. 11 is a volcano plot of the expression level of selected genes which compares the level of expression measured in PBMC of interferon responders versus non-responders HCV type 1 patients prior to interferon treatment (time 0). Data was up-loaded from the Gene Expression Omnibus Accession No. gse11190. The vertical red lines on the right and left represent fold change of 3.5, meaning that points marked on the left of the left vertical line are up-regulated in non responders and points marked on the right of the right vertical red line are up-regulated more than 3.5 folds in the responders; The horizontal red line corresponds to a p value of 0.05. Points appearing above the red horizontal line corresponds to p values lower than 0.05 (i.e., more significant). Note that the KIR2DL1, KIR2DL2, KIR2DL3, CD160, KLRG1, KIR3DL1, KIR3DL2, KIR3DL3, and KIR3DS1 are significantly down-regulated in interferon responders than in non-responders.

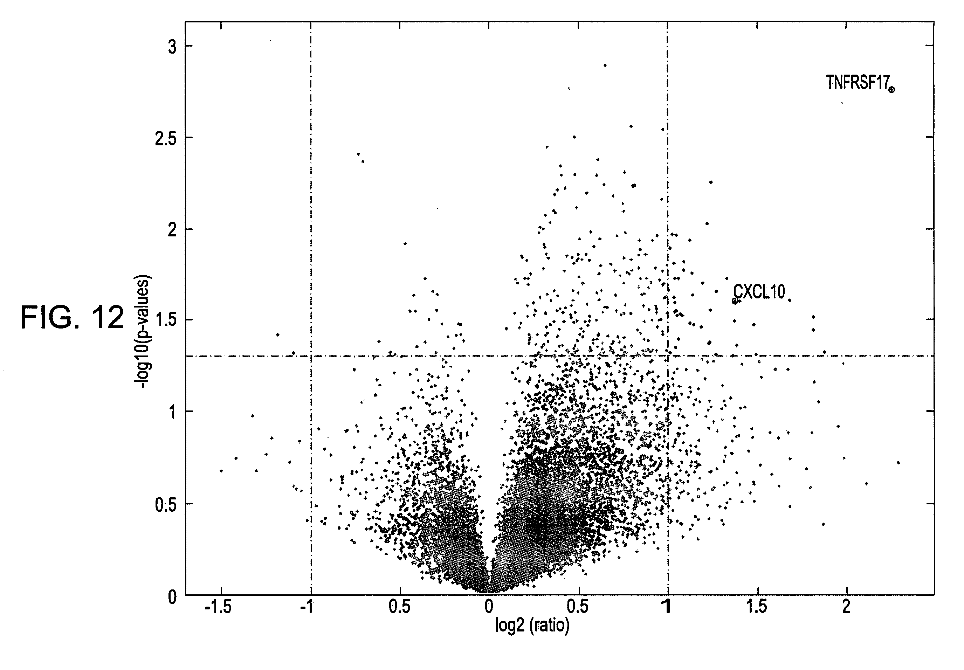

FIG. 12 is a volcano plot which compares the changes in gene expression in PBMC of type 1 HCV patients following interferon treatment between responders and non-responders to interferon. The changes in gene expression are calculated by the ratio between the expression level of a gene measured 4 hours after interferon injection and the expression level of the gene measured prior to interferon injection. "X" and "Y" axes and vertical and horizontal lines are as described with respect to Figure 4. Data was up-loaded from the Gene Expression Omnibus Accession No. gse11190. Note that following interferon injection, the TNFRSF17, CXCL10 and CE24 are significantly up-regulated in interferon responders as compared to interferon non-responders.

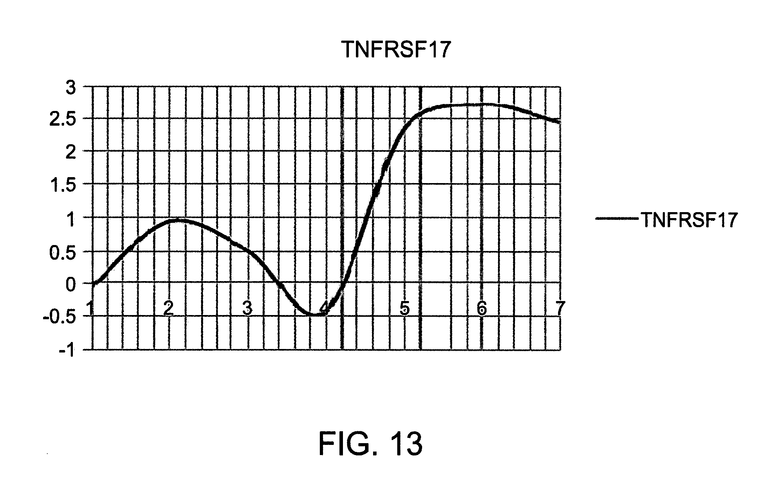

FIG. 13 depicts the changes in the expression level of TNFRSF17 in blood of patients having type 1 HCV 4 hours after interferon treatment as compared to before interferon treatment. The changes in the expression level (Y axis) are presented in log2 values of the expression level measured 4 hours after interferon treatment as compared to the expression level measured prior to interferon treatment. Numbers in the "x" axis refer to subjects as follows: Subjects 1-4 - non-responders to interferon; subjects 5-7 - responders to interferon. Note that the change in TNFRSF17 is highly persistent and significance.

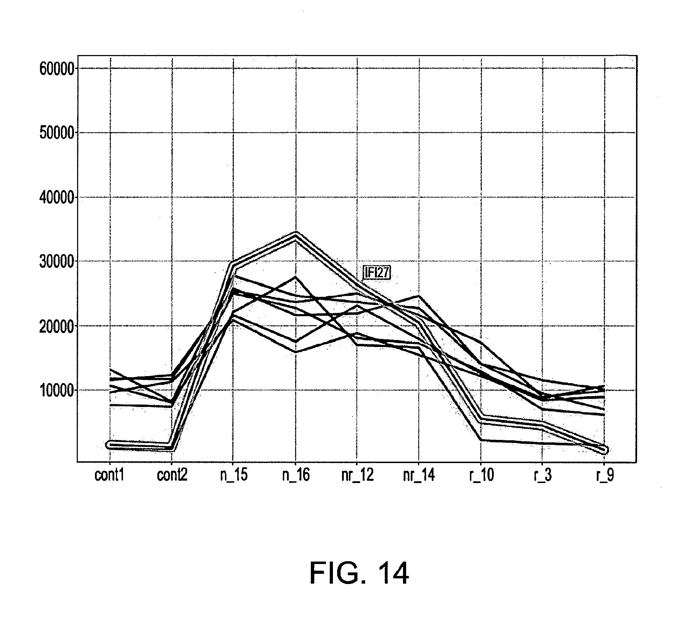

FIG. 14 is a graph depicting the expression level of IFI27, HLA-A, HLA-B and HLA-C in HCV type 1 liver tissues before interferon treatment. Subjects include healthy individuals (Cont1 and Cont2), non-responders to interferon treatment (n_15, n_16, nr_12 and nr_14) and responders to interferon treatment (r_10, r_3 and r_9). Data was up-loaded from the Gene Expression Omnibus Accession No. gse11190 (Affymetrix hu133 plus 2). Note that the IFI27, HLA-A, HLA-B and HLA-C exhibit the most similar (most correlated) expression pattern as the ISG15 gene; e.g., upregulated in non-responders and downregulated in interferon responders.

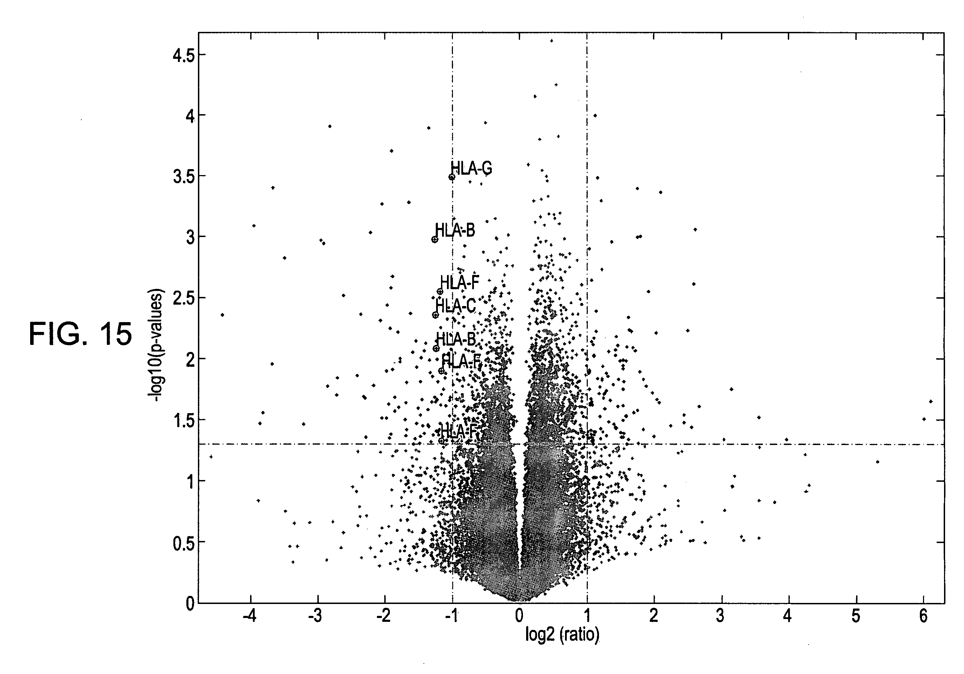

FIG. 15 is a volcano plot depicting the significance of changes in expression levels of various genes in liver of HCV type 1 measured in responders as compared to non responders prior to injection. "X" and "Y" axes and vertical and horizontal lines are as described with respect to Figure 4. Data set was downloaded from the Gene Expression Omnibus Accession No. gse11190. Note that in tissues of HCV type 1 responder patients the HLA-B, HLA-F, HLA-C and HLA-G are significantly down-regulated in more than 2 fold change and with a p value lower than 0.05 as compared to responders, thus demonstrating a significant up-regulation of these genes in liver tissues of HCV type 1 non-responders.

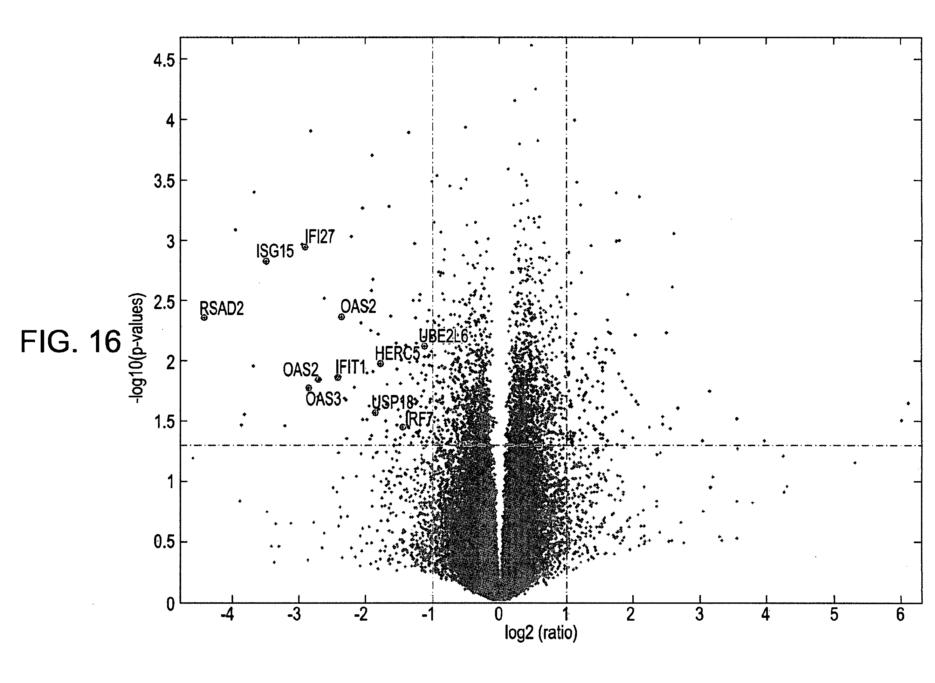

FIG. 16 is a volcano plot depicting the significance of changes in expression levels of various genes in liver of HCV type 1 measured at time 0 in responders versus non responders. The vertical red lines on the right and left represent fold change of 4.6, meaning that points marked on the left of the left vertical line are up-regulated in non responders and points marked on the right of the right vertical red line are up-regulated more than 4.6 folds in the responders; The horizontal red line corresponds to a p value of 0.05. Points appearing above the red horizontal line corresponds to p values lower than 0.05 (i.e., more significant). Note that in parallel to the HLA genes the up-regulated switch genes (e.g., ISG15, IFIT1, USP18, OAS2, OAS3, and HERC6) from the Chen L. et al., 2005 set [Hepatic Gene Expression Discriminates Responders and Nonresponders in Treatment of Chronic Hepatitis C Viral Infection, Gastroenterology, Volume 128, Issue 5, Pages 1437-1444; Hypertext Transfer Protocol://142.150.56.35/∼LiverArrayProject/home (dot) html] are also up regulated in the gse11190 set with a higher fold change *(i.e.,* at least 4.6 folds) as compared to the HLA genes (which is in the range of 2 folds, Figure 15).

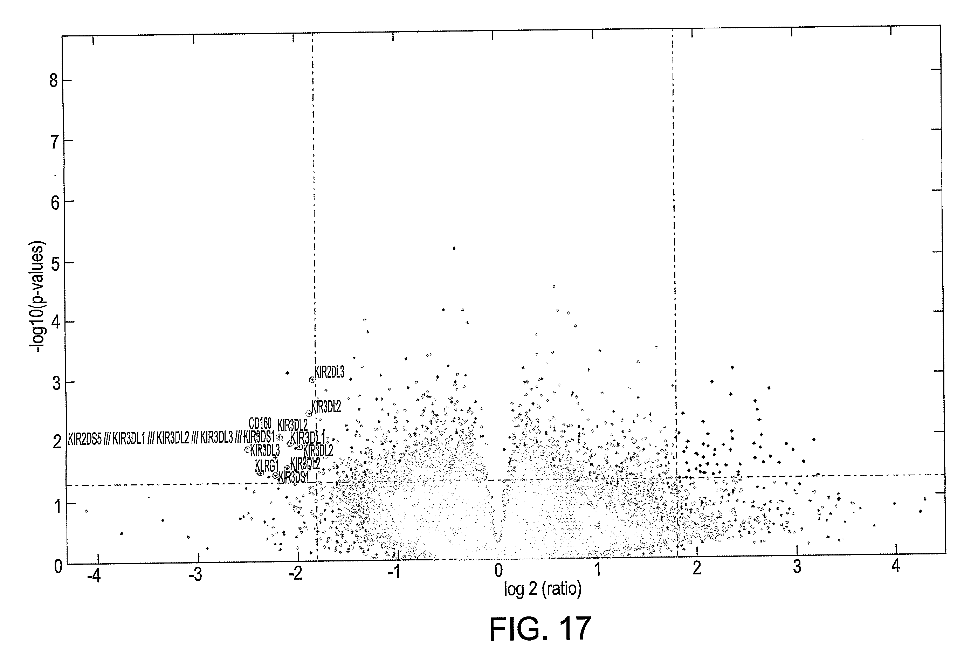

FIG. 17 is a volcano plot depicting the significance of changes in expression levels of various genes in PBMC of HCV type 1 patients as measured at time 0 between responders versus non-responders prior to interferon treatment. Data set was downloaded from the Gene Expression Omnibus Accession No. gse11190. The vertical red lines on the right and left represent fold change of 3.5, meaning that points marked on the left of the left vertical line are up-regulated in non responders and points marked on the right of the right vertical red line are up-regulated more than 3.5 folds in the responders; The horizontal red line corresponds to a p value of 0.05. Points appearing above the red horizontal line corresponds to p values lower than 0.05 (i.e., more significant). Note that in PBMC of type 1 HCV at time 0 (being naive to interferon treatment, *i.e.,* never received interferon) the KIR2D and KIR3D inhibitory natural killer (NK) receptors are up-regulated (at least 3.5 folds) in non responders compared to responders prior to Interferon injection.

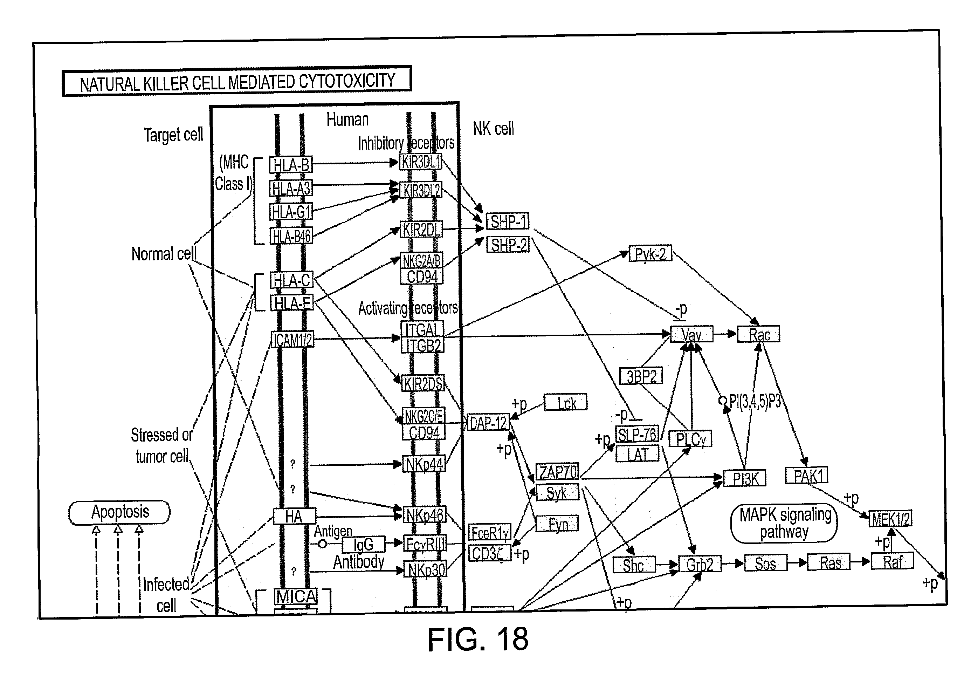

FIG. 18 is a schematic presentation of the natural killer cell mediated cytotoxicity pathway in which genes which are significantly upregulated in liver tissues of non responders type 1 at time 0 are highlighted in yellow. The analysis was performed using the ontoexpress software (Intelligent Systems and Bioinformatics Laboratory, Computer Science Department, Wayne State University).

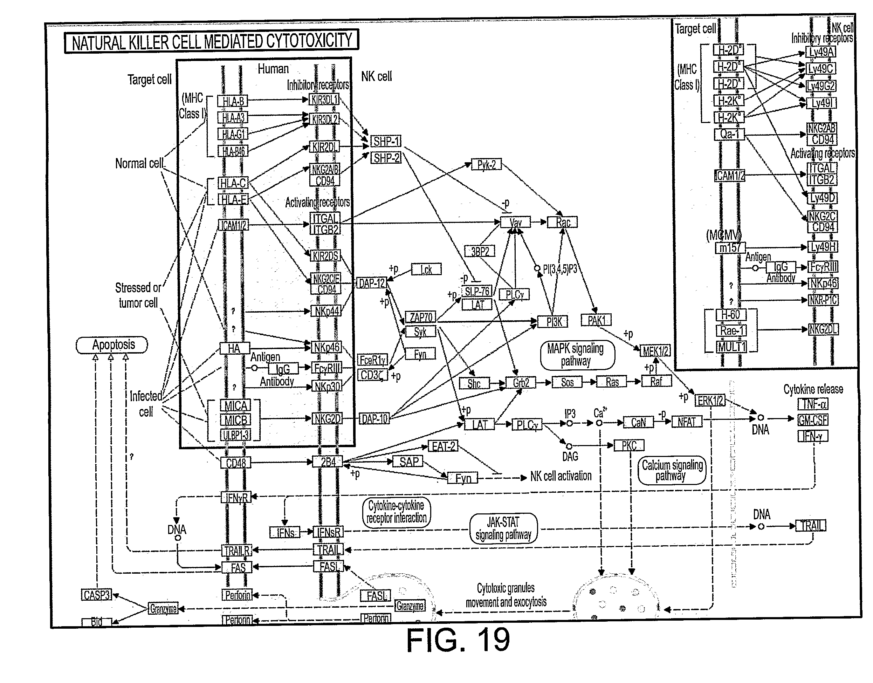

FIG. 19 is a schematic presentation of the natural killer cell mediated cytotoxicity pathway in which genes which are significantly upregulated in PBMC of non responders type 1 at time 0 are highlighted in yellow. The analysis was performed using the ontoexpress software (Intelligent Systems and Bioinformatics Laboratory, Computer Science Department, Wayne State University). Note that in the blood of non-responders the significant upregulation of the kir inhibitor NK receptors (e.g., KIR3D and KIR2D) matches the upregulation of the HLA genes in the liver as shown in Figure 18.

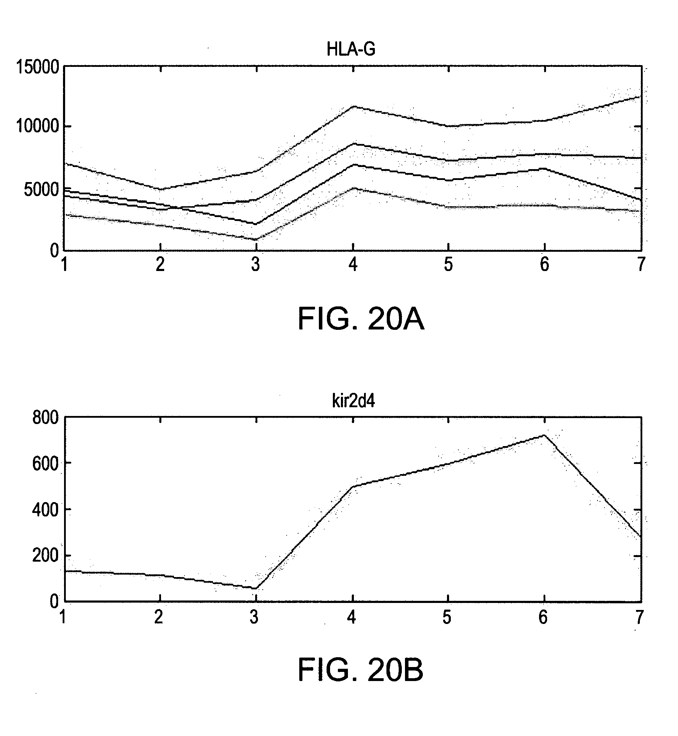

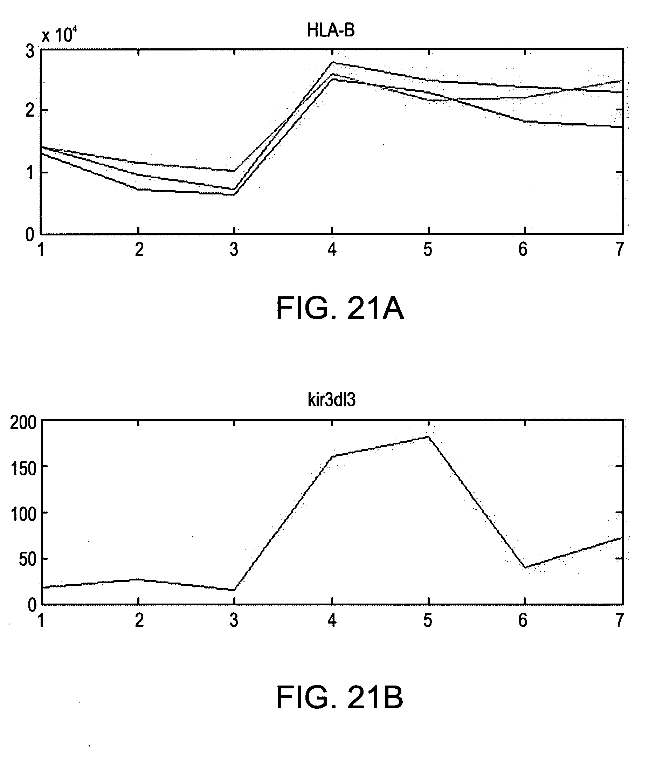

FIGs. 20A-B are graphs depicting the expression level of HLA-G in a liver tissue and of his matched KIR2d4 in the blood of HCV type 1 subjects before interferon treatment. Subjects 1-3 (interferon responders); subjects 4-7 (interferon non-responders). The various colors in Figure 20A indicate expression level using several HLA-G probes. Figure 21A - HLA-G (in liver tissue); Figure 21B - KIR2D4 (in PBMC).
FIGs. 21A-B are graphs depicting the expression level of HLA-B in a liver tissue and of his matched KIR3DL3 in the blood of HCV type 1 subjects before interferon treatment. Subjects 1-3 (interferon responders); subjects 4-7 (interferon non-responders). The various colors in Figure 21A indicate expression level using several HLA-B probes. Figure 21A - HLA-B (in liver tissue); Figure 21B - KIR3DL3 (in PBMC).

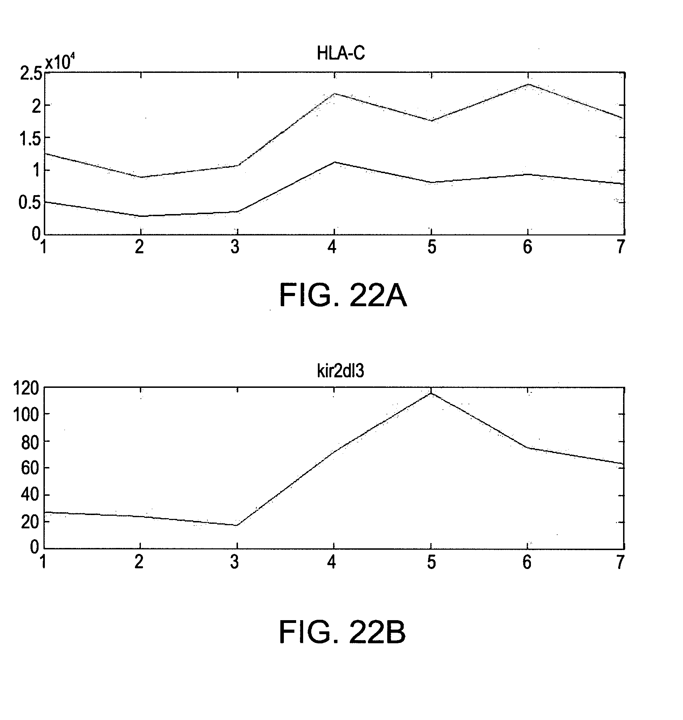

FIGs. 22A-B are graphs depicting the expression level of HLA-C in a liver tissue and of his matched KIR3DL3 in the blood of HCV type 1 subjects before interferon treatment. Subjects 1-3 (interferon responders); subjects 4-7 (interferon non-responders). The various colors in Figure 22A indicate expression level using several HLA-C probes. Figure 22A - HLA-C (in liver tissue); Figure 22B - KIR2DL3 (in PBMC).

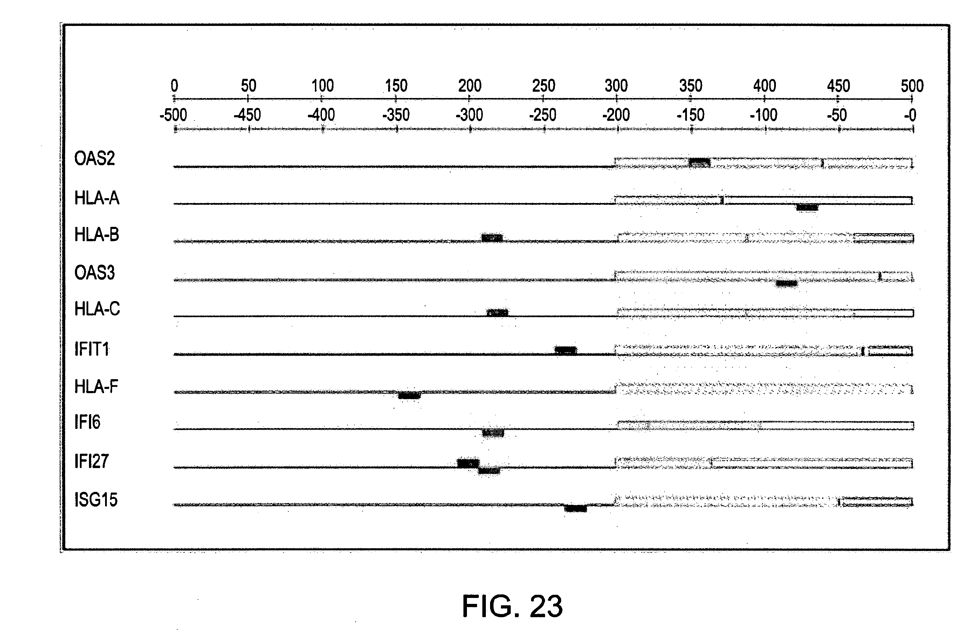

FIG. 23 is a schematic presentation of the OAS2, HLA-A, HLA-B, OAS3, HLA-C, IFIT1, HLA-F, IFI6, IFI27 and ISG15 genes along with their regulatory sequences. Note that the ISRE (light blue solid bars) is a common promoter to all of these genes and is positioned within the 300 bp upstream sequence. Green empty bars = gene coding sequences; Blue empty bars = first exon.

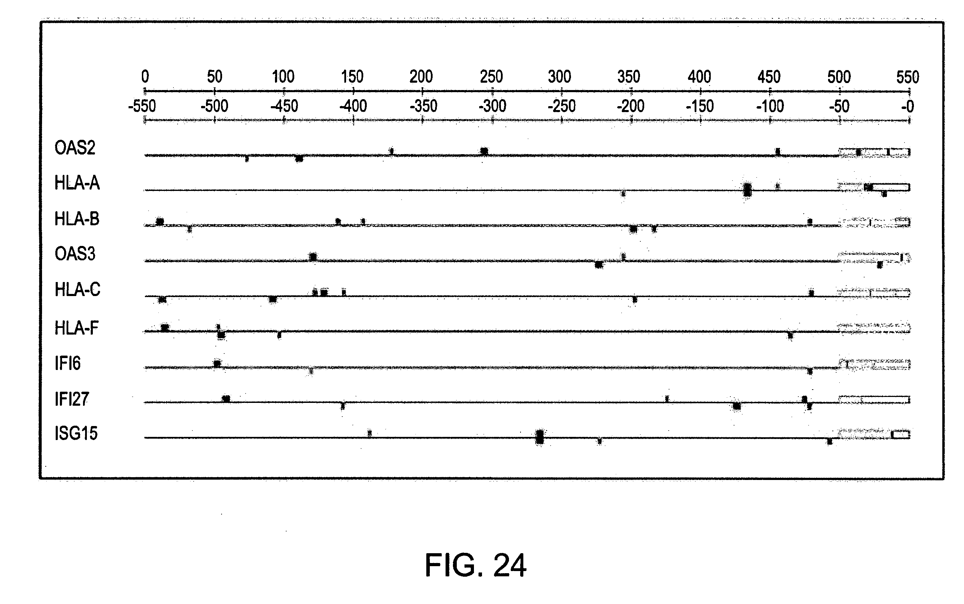

FIG. 24 is a schematic presentation of the OAS2, HLA-A, HLA-B, OAS3, HLA-C, HLA-F, IFI6, IFI27 and ISG15 genes along with their upstream regulatory sequences. Note the 3 promoters ISRE (green squares), OCT1 _04 (pink solid squares) and FOXD3_01 (light blue solid squares) are positioned in the 2000 bp upstream region. The ISRE sequence is closer than the two other promoters and appears already in the 300 bp upstream region.

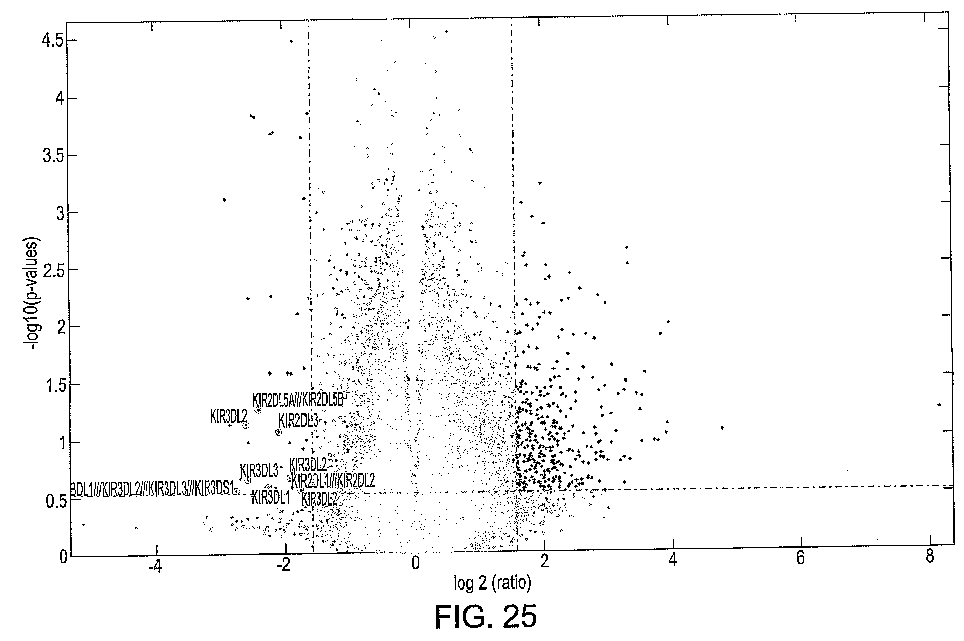

FIG. 25 is a volcano plot depicting the significance of changes between responders and non-responders in expression levels of various genes in PBMC of HCV type 2-4 at time 0 (being naive to interferon treatment). Data was downloaded from the Gene Expression Omnibus Accession No. GSE 11190. "X" and "Y" axes and vertical and horizontal lines are as described with respect to Figure 4. Note the significant downregulation in expression level of the inhibitory KIR genes (e.g., KIR2DL5A, KIR2DL5B, KIR2DL3, KIR3DL1, KIR2DL1, KIR2DL2, KIR3DL3) in PBMC of responders HCV type 2-4 patients as compared to non-responders, similar to the expression pattern of these genes in subjects infected with HCV type 1.

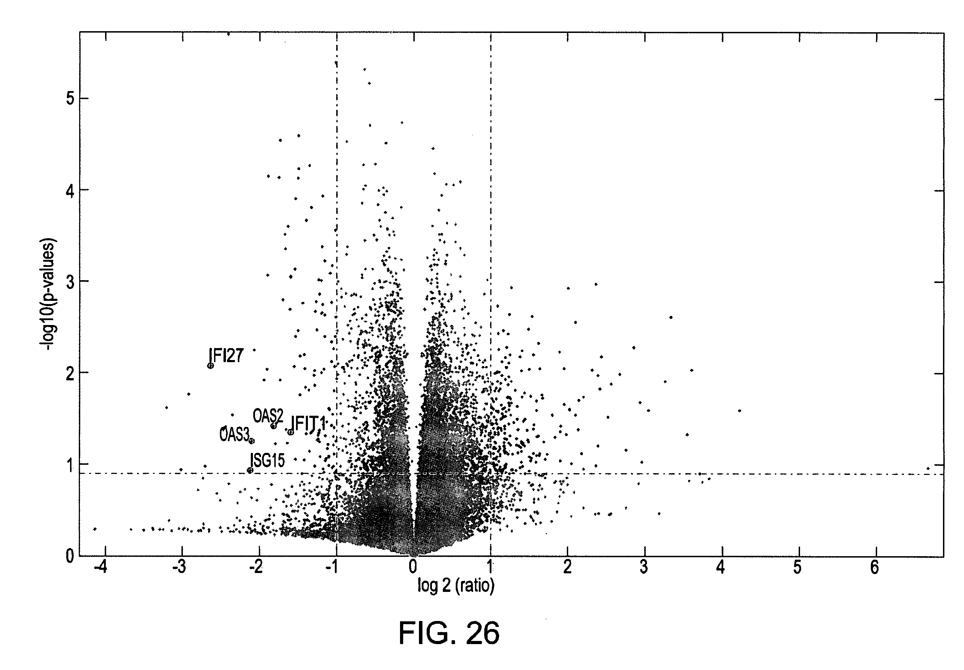

FIG. 26 is a volcano plot depicting the significance of changes between responders and non-responders in expression levels of various genes in liver tissue of HCV type 2-4 at time 0 (being naive to interferon treatment). Data was downloaded from the Gene Expression Omnibus Accession No. gse11190. "X" and "Y" axes and vertical and horizontal lines are as described with respect to Figure 4. Note the significant downregulation in expression level of the HCV type 1 five switch genes *i.e.,* IFI27, ISG15, IFIT1, OAS3 and OAS2 in responder HCV type 2-4 patients as compared to non-responders.

### DESCRIPTION OF SPECIFIC EMBODIMENTS OF THE INVENTION

The present invention, in some embodiments thereof, relates to methods of predicting responsiveness to interferon treatment and methods of treating hepatitis C infection.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

The present inventor has identified signature genes which can be used to predict responsiveness of a subject to interferon treatment.

Thus, as shown in the Examples section which follows, the present inventor used a statistical scoring tool to identify genes which affect interferon response in a subject and found that the expression level of the 5-signature genes ISG15, IFIT1, IFI6, OAS2 and OAS3 in the liver can be used to predict response to interferon in all types of HCV virus infections, *i.e.,* types 1-4, wherein upregulation of the level of expression before interferon treatment indicates that the subject will not response to interferon treatment (Example 4, Figures 4 and 5); that the ratio between the expression level of ISG15, IFI6, IFIT1, OAS2 and OAS3 as determined in liver biopsies following interferon treatment is significantly higher among interferon responders as compared to interferon non-responders of HCV type 1 subjects (Example 3, Figures 2A-E, Table 4); that upregulation of the HERC5, ISG15, USP18 and UBE2L6 genes of the ISGylation process in the liver following interferon treatment predicts responsiveness to interferon treatment (Example 5, Figures 6A-D, Table 6); that the signature genes IFI6, OAS2, ISG15, OAS3 and IFIT1 can predict the response to interferon treatment in subjects diagnosed with multiple sclerosis (Example 8 and Figure 10); that the 5-siganture genes (G1P2, G1P3, IFIT1, OAS2 and OSA3) and the TLR7-meidated pathway genes (TICAM1, MYD88, TLR7, TRAFD1, IRF7) are upregulated in blood of responders following the first interferon injection (Example 7, Figures 8A-E and 9A-E); and that the natural killer (NK) receptor genes KIR2DL1, KIR2DL2, KIR2DL3, KIR3DL1, KIR3DL2, KIR3DL3, KLRG1, KIR3DS1 and CD160 are upregulated in PBMC of HCV type 1 patients who are non-responders to interferon as compared to responders, thus, expression of these genes indicates poor prognosis of a subject infected with the HCV virus (Example 9, Figure 11 and Tables 8 and 9). In addition, as shown in Example 11 (Tables 10-12, Figures 14, 15, 16) the expression pattern of the HLA family of genes (e.g., HLA-A, HLA-B, HLA-C, HLA-F, HLA-G) and of IFI27 was found to be similar to that of ISG15 in liver tissues of HCV type 1 patients prior to treatment. Moreover, the present inventor found that prior to treatment (in subjects naive to interferon treatment) there is a coordinated upregulation of the HLA genes in the liver and the kir genes in the blood of HCV type 1 non-responders, as well as upregulation of the kir genes in blood samples of non-responders HCV types 2-4 patients prior to treatment (Example 12, Figures 18, 19, 20, 21, 22, 25, 26). Furthermore, the present inventor found that upregulation of the expression level of TNFRSF17, and optionally also of CXCL10 and CD24, following interferon treatment predicts the success of interferon treatment (Example 10, Figures 12 and 13). In addition, the present inventor uncovered that ISRE promoter is common to all of the signature genes (e.g., ISG15, IFI6, IFIT1, OAS2, OAS3, HLA-A, HLA-B, HLA-C and HLA-F) involved in determining the fate of interferon treatment (Example 6, Figure 7; Example 13, Figure 23, Tables 14 and 15).

Thus, according to an aspect of some embodiments of the invention there is provided a method of predicting responsiveness of a subject to interferon treatment. The method is effected by comparing a level of expression in a cell of the subject of at least one gene selected from the group consisting KIR3DL3, KIR3DL2, KIR3DL1, KIR2DL1, KIR2DL2, KIR2DL3, KLRG1, KIR3DS1, CD160, HLA-A, HLA-B, HLA-C, HLA-F, HLA-G and IFI27 to a reference expression data of the at least one gene obtained from at least one interferon responder subject and/or at least one interferon non-responder subject, thereby predicting the responsiveness of the subject to interferon treatment.

As used herein the term "interferon" or "IFN" which is interchangeably used herein, refers to a synthetic, recombinant or purified interferon, and encompasses interferon type I [in human include IFN-α (GenBank Accession No. NM_024013 and NP_076918; SEQ ID NOs:165 and 169 respectively), interferon alpha 2a (GenBank Accession No. NM_000605 and NP_000596; SEQ ID NO:173 and 174, respectively), IFN-β (GenBank Accession No. NM_002176 and NP_002167; SEQ ID NOs:166 and 170 respectively), interferon beta 1a [AVONEX (Biogen Idec); REBIF (EMD Serono)] or interferon beta 1b (BETASERON) and IFN-ω (GenBank Accession No. NM_002177 and NP_002168; SEQ ID NOs:167 and 171 respectively)], which bind to the cell surface receptor complex IFN-a receptor (IFNAR) consisting of IFNAR1 and IFNAR2 chains; interferon type II [in human is IFN-γ (GenBank Accession No. NM_000619 and NP_000610; SEQ ID NOs:168 and 172 respectively)], which binds to the IFNGR receptor; and interferon type III, which bind to a receptor complex consisting of IL10R2 (also called CRF2-4) and IFNLR1 (also called CRF2-12).

As used herein the phrase "interferon treatment" refers to administration of interferon into a subject in need thereof. It should be noted that administration of interferon may comprise a single or multiple dosages, as well as a continuous administration, depending on the pathology to be treated and the subject receiving the treatment.

Interferon is used in the treatment of various pathologies such as autoimmune disorders (e.g., multiple sclerosis using e.g., interferon beta-1a and/or interferon beta-1b), various cancers (e.g., hematological malignancy, leukemia and lymphomas including hairy cell leukemia, chronic myeloid leukemia, nodular lymphoma, cutaneous T-cell lymphoma, recurrent melanomas, using e.g., recombinant IFN-α2b), and viral infections (e.g., hepatitis C virus infection, hepatitis B virus infection, viral respiratory diseases such as cold and flu).

Various modes of interferon administration are known in the art. These include, but are not limited to, injection (e.g., using a subcutaneous, intramuscular, intravenous, or intradermal injection), intranasal administration and oral administration.

According to some embodiments of the invention, interferon treatment is provided to the subject in doses matching his weight, at a frequency of once a week, for a period of up to 48 weeks.

According to some embodiments of the invention, the interferon treatment comprises type I interferon such as interferon alpha (SEQ ID NO:169), interferon alpha 2a (SEQ ID NO:174), interferon beta 1a or interferon beta 1b.

According to some embodiments of the invention, the interferon treatment comprises PEGylated interferon [*i.e.,* conjugated to a polyethylene glycol (PEG) polymer].

According to some embodiments of the invention, the interferon treatment comprises interferon and ribavirin.

The term "subject" as used herein refers to a mammal, preferably a human being (male or female) at any age.

According to some embodiments of the invention, the subject is diagnosed with a pathology (disease, disorder or condition) which requires interferon treatment such as an autoimmune disease, a viral infection or cancer as described above.

According to some embodiments of the invention, the subject is diagnosed with hepatitis C virus (HCV) infection.

As used herein the term "HCV" refers to hepatitis C virus having genotype 1 (also known as HCV Type 1), genotype 2 (also known as HCV Type 2), genotype 3 (also known as HCV Type 3), genotype 4 (also known as HCV Type 4), genotype 5 (also known as HCV Type 5) or genotype 6 (also known as HCV Type 6).

The phrase "HCV infection" encompasses acute (refers to the first 6 months after infection) and chronic (refers to infection with hepatitis C virus which persists more than 6 month) infection with the hepatitis C virus.

According to some embodiments of the invention, the subject is diagnosed with chronic HCV infection.

According to some embodiments of the invention, the subject is infected with HCV type 1.

According to some embodiments of the invention, the subject is infected with HCV type 2, 3 or 4

According to some embodiments of the invention, the subject is diagnosed with multiple sclerosis.

As used herein the phrase "multiple sclerosis" refers to a pathology characterized by presence of at least two neurological attacks affecting the central nervous system (CNS) and accompanied by demyelinating lesions on brain magnetic resonance imaging (MRI). The disease course of patients diagnosed with multiple sclerosis can be a relapsing-remitting multiple sclerosis (RRMS) (occurring in 85 % of the patients) or a progressive multiple sclerosis (occurring in 15 % of the patients).

According to some embodiments of the invention, the subject is diagnosed with RRMS.

According to some embodiments of the invention, the subject is a healthy subject (e.g., not diagnosed with any disease which require interferon treatment). It should be noted that determining the responsiveness of a healthy subject to interferon treatment can be performed as part of a genetic testing of the healthy subject, which can be recorded in the subject's medical file for future use (e.g., in case the subject will be diagnosed with a disease requiring interferon treatment).

As used herein the phrase "predicting responsiveness of a subject to interferon treatment" refers to determining the likelihood that the subject will respond to interferon treatment, e.g., the success or failure of interferon treatment.

The term "response" to interferon treatment refers to an improvement in at least one relevant clinical parameter as compared to an untreated subject diagnosed with the same pathology (e.g., the same type, stage, degree and/or classification of the pathology), or as compared to the clinical parameters of the same subject prior to interferon treatment.

Typically only 50 % of HCV type I and MS patients respond to interferon treatment. Therefore a "low probability of response to interferon" in connection with these diseases is a probability significantly lower than about 50 %, e.g., a probability lower than about 40 %, e.g., a probability lower than about 30%, e.g., a probability lower than about 20 %, e.g., a probability lower than about 10 % or 5 %, and a "high probability of response to interferon" is a probability significantly higher than about 50 %, e.g., a probability higher than about 60 %, e.g., a probability higher than about 70 %, e.g., a probability higher than about 80 %, e.g., a probability higher than about 85 %, e.g., a probability higher than about 90 %, e.g., a probability higher than about 95 %, e.g., a probability higher than about 99%.

In connection with HCV types 2, 3, 4 the typical rate of success of interferon treatment is about 80 %, so a low probability of success is a probability lower than about 80 %, e.g., a probability lower than about 70 %, e.g., a probability lower than 60 about %, e.g., a probability lower than 50 about %, e.g., a probability lower than 40 about %, e.g., a probability lower than 30 about %, e.g., lower than 20 about %, e.g., a probability lower than about 10 %, and a high probability is a probability higher than about 80 %, e.g., a probability higher than about 90%, e.g., a probability higher than about 95 %, e.g., a probability higher than about 99%.

For example, a successful interferon treatment in HCV patients can result in clearance of the virus from the subject's body (e.g., from the blood), decreased probability of liver damage and/or cirrhosis, and decreased probability of hepatocellular carcinoma. In addition, if HCV infection is diagnosed immediately after infection, interferon treatment can results in clearance of the virus from the body and prevention of chronic hepatitis C.

For example, a successful interferon treatment in multiple sclerosis patients can result in slowing disease progression and activity in relapsing-remitting multiple sclerosis and reducing attacks in secondary progressive multiple sclerosis.

In HCV infected subjects, the responsiveness to interferon can be evaluated by measuring virus load in blood, and presence and/or level of HCV RNA in liver cells or blood cells of the subject. Such tests can be done prior to interferon treatment, during interferon treatment and at a predetermined period after completion of the treatment course with interferon.

In multiple sclerosis subjects, the responsiveness to interferon can be evaluated using the Kurtzke Expanded Disability Status Scale (EDSS) of the subject which quantifies disability in MS by scoring eight Functional Systems (FS) (pyramidal, cerebellar, brainstem, sensory, bowel and bladder, visual, cerebral, and other), the number or relapses per year and/or the severity thereof.

According to some embodiments of the invention an HCV infected subject is considered an interferon responder when exhibiting a complete clearance of the hepatitis C virus from the subject's body (e.g., tissues, cells, body fluid) as determined within one about month after beginning of interferon treatment, e.g., within about 2 months, e.g., within about 3 months, e.g., within about 4 months, e.g., within about 5 months, e.g., within about 6 months, e.g., within about 7 months, e.g., within about 8 months, e.g., within about 9 months, e.g., within about 10 months, e.g., within about 11 months, e.g., within about 12 months, e.g., within about 3 months, e.g., within about 11 months, e.g., within about 12 months, e.g., within about 13 months, e.g., within about 14 months, e.g., within about 15 months, e.g., within about 16 months, e.g., within about 17 months, e.g., within about 18 months, e.g., within about 19 months, e.g., within about 20 months, e.g., within about 21 months, e.g., within about 22 months, e.g., within about 23 months, e.g., within about 24 after beginning of interferon treatment.

According to some embodiments of the invention an HCV infected subject is considered an interferon non-responder when exhibiting persistent levels [e.g., levels which are similar (± about 10-20 %) or not significantly reduced as compared to before interferon treatment] of the hepatitis C virus in the subject's body (e.g., tissues, cells, body fluid) as determined at least about one month after beginning of interferon treatment, e.g., at least about 2 months, e.g., at least about 3 months, e.g., at least about 4 months, e.g., at least about 5 months, e.g., at least about 6 months, e.g., at least about 7 months, e.g., at least about 8 months, e.g., at least about 9 months, e.g., at least about 10 months, e.g., at least about 11 months, e.g., at least about 12 months, e.g., at least about 13 months, e.g., at least about 14 months, e.g., at least about 15 months, e.g., at least about 16 months, e.g., at least about 17 months, e.g., at least about 18 months, e.g., at least about 19 months, e.g., at least about 20 months, e.g., at least about 21 months, e.g., at least about 22 months, e.g., at least about 23 months, e.g., at least about 24 months, or more after beginning of interferon treatment.

As mentioned, the method according to this aspect of the invention is effected by comparing a level of expression in a cell of the subject of at least one gene selected from the group consisting KIR3DL3, KIR3DL2, KIR3DL1, KIR2DL1, KIR2DL2, KIR2DL3, KLRG1, KIR3DS1, CD160, HLA-A, HLA-B, HLA-C, HLA-F, HLA-G and IFI27 to a reference expression data of the at least one gene obtained from at least one interferon responder subject and/or at least one interferon non-responder subject, thereby predicting the responsiveness of the subject to interferon treatment.

As used herein, the phrase "level of expression" refers to the degree of gene expression and/or gene product activity in a specific cell. For example, up-regulation or down-regulation of various genes can affect the level of the gene product *(i.e.,* RNA and/or protein) in a specific cell.

Sequence information regarding gene products (*i.e.,* RNA transcripts and polypeptide sequences) of KIR2DL1, KIR2DL2, KIR2DL3, KIR3DL1, KIR3DL2, KIR3DL3, KLRG1, KIR3DS1, CD160, HLA-A, HLA-B, HLA-C, HLA-F, HLA-G and IFI27, can be found in Table 16 in the Examples section which follows. In addition, probes which can be used to detect transcripts of these genes are provided in Table 16 (Examples section).

It should be noted that the level of expression can be determined in arbitrary absolute units, or in normalized units (relative to known expression levels of a control reference). For example, when using DNA chips, the expression levels are normalized according to the chips' internal controls or by using quantile normalization such as RMA.

As used herein the phrase "a cell of the subject" refers to any cell (e.g., an isolated cell), cell culture, cell content and/or cell secreted content which contains RNA and/or proteins of the subject. Examples include a blood cell, a cell obtained from any tissue biopsy [e.g., liver biopsy, cerebrospinal fluid, (CSF), brain biopsy], a bone marrow cell, body fluids such as plasma, serum, saliva, spinal fluid, lymph fluid, the external sections of the skin, respiratory, intestinal, and genitourinary tracts, tears, saliva, sputum and milk. According to an embodiment of the invention, the cell is a blood cell (e.g., white blood cells, macrophages, B- and T-lymphocytes, monocytes, neutrophiles, eosinophiles, and basophiles) which can be obtained using a syringe needle from a vein of the subject. It should be noted that the cell may be isolated from the subject (e.g., *for in vitro* detection) or may optionally comprise a cell that has not been physically removed from the subject (e.g., *in vivo* detection).

According to some embodiments of the invention, the white blood cell comprises peripheral blood mononuclear cells (PBMC). The phrase, "peripheral blood mononuclear cells (PBMCs)" as used herein, refers to a mixture of monocytes and lymphocytes. Several methods for isolating white blood cells are known in the art. For example, PBMCs can be isolated from whole blood samples using density gradient centrifugation procedures. Typically, anticoagulated whole blood is layered over the separating medium. At the end of the centrifugation step, the following layers are visually observed from top to bottom: plasma/platelets, PBMCs, separating medium and erythrocytes/granulocytes. The PBMC layer is then removed and washed to remove contaminants (e.g., red blood cells) prior to determining the expression level of the polynucleotide(s) therein.

According to some embodiments of the invention, the cell is a liver cell.

It should be noted that liver cells (hepatic cell) can be obtained by a liver biopsy (e.g., using a surgical tool or a needle).

According to some embodiments of the invention, the level of expression of the gene(s) of the invention is determined using an RNA or a protein detection method.

According to some embodiments of the invention, the RNA or protein molecules are extracted from the cell of the subject.

Methods of extracting RNA or protein molecules from cells of a subject are well known in the art. Once obtained, the RNA or protein molecules can be characterized for the expression and/or activity level of various RNA and/or protein molecules using methods known in the arts.

Non-limiting examples of methods of detecting RNA molecules in a cell sample include Northern blot analysis, RT-PCR, RNA *in situ* hybridization (using e.g., DNA or RNA probes to hybridize RNA molecules present in the cells or tissue sections), *in situ* RT-PCR (e.g., as described in Nuovo GJ, et al. Am J Surg Pathol. 1993, 17: 683-90; Komminoth P, et al. Pathol Res Pract. 1994, 190: 1017-25), and oligonucleotide microarray (e.g., by hybridization of polynucleotide sequences derived from a sample to oligonucleotides attached to a solid surface [e.g., a glass wafer) with addressable location, such as Affymetrix microarray (Affymetrix®, Santa Clara, CA)].

Non-limiting examples of methods of detecting the level and/or activity of specific protein molecules in a cell sample include Enzyme linked immunosorbent assay (ELISA), Western blot analysis, radio-immunoassay (RIA), Fluorescence activated cell sorting (FACS), immunohistochemical *analysis, in situ* activity assay (using e.g., a chromogenic substrate applied on the cells containing an active enzyme), in vitro activity assays (in which the activity of a particular enzyme is measured in a protein mixture extracted from the cells). For example, in case the detection of the expression level of a secreted protein is desired, ELISA assay may be performed on a sample of fluid obtained from the subject (e.g., serum), which contains cell-secreted content.

As used herein the phrase "reference expression data" refers to the expression level of the gene in a cell of a subject whose responsiveness to interferon is already known (e.g., a reference responder or non-responder subject). Such as an expression level can be known from the literature, from the database [e.g., using the Gene Expression Omnibus at Hypertext Transfer Protocol://World Wide Web (dot) ncbi (dot) nlm (dot) nih (dot) gov/projects/geo/], or from biological samples comprising RNA or protein molecules obtained from a reference responder or non-responder subject.

According to some embodiments of the invention, the reference expression data is obtained from at least one interferon responder subject (e.g., from one interferon responder subject), e.g., from at least 2, from at least 3, from at least 4, from at least 5, from at least 6, from at least 7, from at least 8, from at least 9, from at least 10, from at least 20, from at least 30, from at least 40, from at least 50, from at least 100 or more interferon responder subjects.

According to some embodiments of the invention, the reference expression data is obtained from at least one interferon non-responder subject (e.g., from one interferon non-responder subject), e.g., from at least 2, from at least 3, from at least 4, from at least 5, from at least 6, from at least 7, from at least 8, from at least 9, from at least 10, from at least 20, from at least 30, from at least 40, from at least 50, from at least 100 or more interferon non-responder subjects.

It should be noted that when more than one reference subjects (i.e., interferon responders or non-responders) is used, the reference expression data may comprise an average of the expression level of several or all subjects, and those of skills in the art are capable of averaging expression levels from 2 or more subject, using e.g., normalized expression values.

According to some embodiments of the invention, a decrease above a predetermined threshold in the level of expression of the at least one gene in the cell of the subject relative to the reference expression data of the at least one gene obtained from the at least one interferon non-responder subject predicts responsiveness of the subject to interferon treatment, e.g., classifies the subject as responsive to interferon treatment (e.g., indicates that the subject is an interferon responder).

As used herein the phrase "a decrease above a predetermined threshold" refers to a decrease in the level of expression in the cell of the subject relative to the reference expression data obtained from the at least one interferon non-responder subject which is higher than a predetermined threshold such as a about 10 %, e.g., higher than about 20 %, e.g., higher than about 30 %, e.g., higher than about 40 %, e.g., higher than about 50 %, e.g., higher than about 60 %, higher than about 70 %, higher than about 80 %, higher than about 90 %, higher than about 2 times, higher than about three times, higher than about four time, higher than about five times, higher than about six times, higher than about seven times, higher than about eight times, higher than about nine times, higher than about 20 times, higher than about 50 times, higher than about 100 times, higher than about 200 times, higher than about 350, higher than about 500 times, higher than about 1000 times, or more relative to the reference expression data obtained from the at least one interferon non-responder subject.

According to some embodiments of the invention, an increase above a predetermined threshold in the level of expression of the at least one gene in the cell of the subject relative to the reference expression data of the at least one gene obtained from the at least one interferon responder subject predicts lack of responsiveness of the subject to interferon treatment, e.g., classifies the subject as non-responsive to interferon treatment (e.g., indicates that the subject is an interferon non-responder).

As used herein the phrase "an increase above a predetermined threshold" refers to an increase in the level of expression in the cell of the subject relative to the reference expression data obtained from the at least one interferon responder subject which is higher than a predetermined threshold such as a about 10 %, e.g., higher than about 20 %, e.g., higher than about 30 %, e.g., higher than about 40 %, e.g., higher than about 50 %, e.g., higher than about 60 %, higher than about 70 %, higher than about 80 %, higher than about 90 %, higher than about 2 times, higher than about three times, higher than about four time, higher than about five times, higher than about six times, higher than about seven times, higher than about eight times, higher than about nine times, higher than about 20 times, higher than about 50 times, higher than about 100 times, higher than about 200 times, higher than about 350, higher than about 500 times, higher than about 1000 times, or more relative to the reference expression data obtained from the at least one interferon responder subject.

According to some embodiments of the invention, when a level of expression of the at least one gene in the cell of the subject is identical or changed below a predetermined threshold as compared to the reference expression data of the at least one gene obtained from the at least one interferon responder subject, then the subject is classified as responsive to interferon (e.g., indicates that the subject is an interferon responder).

As used herein the phrase "changed below a predetermined threshold" refers to an increase or a decrease in the level of expression in the cell of the subject relative to the reference expression data obtained from the at least one interferon responder subject which is lower than a predetermined threshold, such as lower than about 10 times, e.g., lower than about 9 times, e.g., lower than about 8 times, e.g., lower than about 7 times, e.g., lower than about 6 times, e.g., lower than about 5 times, e.g., lower than about 4 times, e.g., lower than about 3 times, e.g., lower than about 2 times, e.g., lower than about 90%, e.g., lower than about 80%, e.g., lower than about 70%, e.g., lower than about 60%, e.g., lower than about 50%, e.g., lower than about 40%, e.g., lower than about 30%, e.g., lower than about 20%, e.g., lower than about 10%, e.g., lower than about 9%, e.g., lower than about 8%, e.g., lower than about 7%, e.g., lower than about 6%, e.g., lower than about 5%, e.g., lower than about 4%, e.g., lower than about 3%, e.g., lower than about 2%, e.g., lower than about 1% relative to the reference expression data obtained from the at least one interferon responder subject.

According to some embodiments of the invention, when a level of expression of the at least one gene in the cell of the subject is identical or changed below a predetermined threshold as compared to the reference expression data of the at least one gene obtained from the at least one interferon non-responder subject, then the subject is classified as a non-responsive to interferon.

As used herein the phrase "changed below a predetermined threshold" refers to an increase or a decrease in the level of expression in the cell of the subject relative to the reference expression data obtained from the at least one interferon non-responder subject which is lower than a predetermined threshold, such as lower than about 10 times, e.g., lower than about 9 times, e.g., lower than about 8 times, e.g., lower than about 7 times, e.g., lower than about 6 times, e.g., lower than about 5 times, e.g., lower than about 4 times, e.g., lower than about 3 times, e.g., lower than about 2 times, e.g., lower than about 90%, e.g., lower than about 80%, e.g., lower than about 70%, e.g., lower than about 60%, e.g., lower than about 50%, e.g., lower than about 40%, e.g., lower than about 30%, e.g., lower than about 20%, e.g., lower than about 10%, e.g., lower than about 9%, e.g., lower than about 8%, e.g., lower than about 7%, e.g., lower than about 6%, e.g., lower than about 5%, e.g., lower than about 4%, e.g., lower than about 3%, e.g., lower than about 2%, e.g., lower than about 1% relative to the reference expression data obtained from the at least one interferon non-responder subject.

For example, as is shown in Table 8 (Example 7 of the Examples section), while the level of expression of the KIR3DL2 gene among interferon responders was on average of 31 arbitrary units, the level of expression of this gene among interferon non-responders was on average of 134 arbitrary units, which demonstrates an increase of more than about 4 times in cells of non-responders as compared to cells of responders. Similar findings with respect to additional genes are presented in Table 9 (Example 7 of the Examples section).

According to some embodiments of the invention the level of expression is determined prior to interferon treatment.

As used herein the phrase "prior to interferon treatment" refers to any time before the first administration of interferon to the subject. Thus, prior to interferon treatment the subject is considered naive to interferon treatment.

According to some embodiments of the invention the at least one gene comprises at least two genes, wherein a first gene is selected from the group consisting of KIR3DL3, KIR3DL2, KIR3DL1, KIR2DL1, KIR2DL2, KIR2DL3, KLRG1, and CD160, and wherein a second gene is selected from the group consisting of HLA-A, HLA-B, HLA-C, HLA-F, HLA-G and IFI27.

According to some embodiments of the invention the level of expression of the first gene is determined in a blood cell.

According to some embodiments of the invention the level of expression of the second gene is determined in a liver cell.

According to some embodiments of the invention the level of expression of the at least one gene comprises at least two, at least 3, at least 4, at least 5, at least 6, at least 7, at least 8, at least 9, at least 10, at least 11, at least 12, at least 13, at least 14, at least 15 genes from the group of KIR3DL3, KIR3DL2, KIR3DL1, KIR2DL1, KIR2DL2, KIR2DL3, KLRG1, KIR3DS1, CD160, HLA-A, HLA-B, HLA-C, HLA-F, HLA-G and IFI27 genes.

According to an aspect of some embodiments of the invention there is provided a method of predicting responsiveness to interferon treatment of a subject diagnosed with multiple sclerosis or infected with HCV type 2, 3 or 4. The method is effected by comparing a level of expression in a cell of the subject of IFI6, OAS2, ISG15, OAS3 and IFIT1 genes to a reference expression data of the genes obtained from at least one interferon responder subject and/or at least one interferon non-responder subject, thereby predicting the responsiveness of the subject to interferon treatment.

According to some embodiments of the invention the cell is a blood cell.

According to some embodiments of the invention when the subject is diagnosed with multiple sclerosis the cell of the subject is a blood cell.

According to some embodiments of the invention when the subject is infected with HCV type 2, 3 or 4 the cell of the subject is a liver cell.

As mentioned above and further described in the Examples section which follows, the present inventor has uncovered that in addition to testing the expression level of genes prior to interferon treatment, there is a significant value, with a high predictive power, to test the expression of certain genes in a cell of a subject prior to interferon treatment and at a predetermined time period following interferon treatment, since the switch in gene expression immediately after interferon treatment is significant among interferon responders as compared to interferon non-responders.

According to an aspect of some embodiments of the invention there is provided a method of predicting responsiveness of a subject to interferon treatment. The method is effected by comparing a ratio determined between an expression level of TNFRSF17 gene in a cell of the subject following interferon treatment and an expression level of the gene in the cell of the subject prior to interferon treatment, or visa versa, namely, comparing a ratio determined between an expression level of TNFRSF17 gene in a cell of the subject prior to interferon treatment and an expression level of the gene in the cell of the subject following interferon treatment, to a reference ratio determined in a cell of at least one interferon responder subject and/or at least one interferon non-responder subject, the reference ratio is determined between an expression level of the gene following interferon treatment and an expression level of the gene prior to interferon treatment, or visa versa, namely, the reference ratio is determined between an expression level of the gene prior to interferon treatment and an expression level of the gene following interferon treatment, thereby predicting the responsiveness to interferon treatment of a subject.

According to some embodiments of the invention, the method further comprising comparing a ratio determined between an expression level of at least one gene selected from the group consisting of CXCL10 and CD24 in a cell of the subject following interferon treatment and an expression level of the gene in the cell of the subject prior to interferon treatment, or visa versa, namely, comparing a ratio determined between an expression level of the at least one gene selected from the group consisting of CXCL10 and CD24 in a cell of the subject prior to interferon treatment and an expression level of the gene in the cell of the subject following interferon treatment, to a reference ratio determined in a cell of at least one interferon responder subject and/or at least one interferon non-responder subject, the reference ratio is determined between an expression level of the at least one gene following interferon treatment and an expression level of the at least one gene prior to interferon treatment, or visa versa, namely, the reference ratio is determined between an expression level of the at least one gene prior to interferon treatment and an expression level of the at least one gene following interferon treatment, thereby predicting the responsiveness to interferon treatment of a subject.

Sequence information regarding the gene products *(i.e.,* RNA transcripts and polypeptide sequences) of TNFRSF17, CXCL10 and CD24 which can be detected according to the method of some embodiments of the invention is provided in Table 16 in the Examples section which follows. In addition, sequence information of probes which can be used to detect the TNFRSF17, CXCL10 and CD24 RNA transcripts is provided in Table 16 in the Examples section which follows.

The phrase "following interferon treatment" refers to any time ranging from at least about 1 hour after interferon administration to about 1 week after interferon administration. For example, from about 1 hour to about 24-72 hours after administration, e.g., from about 4 hours to about 24 hours after interferon administration.

According to some embodiments of the invention, following interferon treatment is effected about 4 hours following interferon treatment.

According to some embodiments of the invention, following interferon treatment is effected about 24 hours following interferon treatment.

According to some embodiments of the invention, prior to interferon treatment is effected any time before the first interferon administration, such as a few minutes before interferon administration, a few hours before interferon administration or a few days, weeks, months or years before interferon administration.

According to some embodiments of the invention administration of interferon is carried *out in vivo (i.e.,* to the subject in need of therapy).

According to some embodiments of the invention administration of interferon is carried out *in vitro* (e.g., in a cell culture).

According to some embodiments of the invention an increase above a predetermined threshold in the ratio of the subject relative to the reference ratio of the at least one interferon non-responder subject predicts responsiveness of the subject to interferon treatment of the subject.

According to some embodiments of the invention, the increase in the ratio of the subject is of at least about 2 %, e.g., at least about 4 %, at least about 6 %, e.g., at least about 10 %, at least about 20 %, e.g., at least about 30 %, at least about 40 %, e.g., at least about 50 %, at least about 60 %, e.g., at least about 70 %, at least about 80 %, e.g., at least about 90 %, e.g., at least about 2 times, e.g., at least about 3 times, e.g., at least about 4 times, e.g., at least about 5 times, e.g., at least about 6 times, e.g., at least about 7 times, e.g., at least about 8 times, e.g., at least about 9 times, e.g., at least about 10 times, e.g., at least about 20 times, e.g., at least about 30 times, e.g., at least about 40 times, e.g., at least about 50 times, e.g., at least about 60 times, or more relative to the reference ratio determined in the at least one interferon non-responder subject.

According to some embodiments of the invention a decrease above a predetermined threshold in the ratio of the subject relative to the reference ratio of the at least one interferon responder subject predicts lack of responsiveness of the subject to interferon treatment of the subject.

According to some embodiments of the invention, the decrease in the ratio of the subject is of at least about 2 %, e.g., at least about 4 %, at least about 6 %, e.g., at least about 10 %, at least about 20 %, e.g., at least about 30 %, at least about 40 %, e.g., at least about 50 %, at least about 60 %, e.g., at least about 70 %, at least about 80 %, e.g., at least about 90 %, e.g., at least about 2 times, e.g., at least about 3 times, e.g., at least about 4 times, e.g., at least about 5 times, e.g., at least about 6 times, e.g., at least about 7 times, e.g., at least about 8 times, e.g., at least about 9 times, e.g., at least about 10 times, e.g., at least about 20 times, e.g., at least about 30 times, e.g., at least about 40 times, e.g., at least about 50 times, e.g., at least about 60 times, or more relative to the reference ratio determined in the at least one interferon responder subject.

According to some embodiments of the invention, when the ratio of the subject is identical or changed below a predetermined threshold as compared to the reference ratio of the at least one interferon responder subject, then the subject is classified as responsive to interferon.

According to some embodiments of the invention, the change (increase or decrease) between the ratio of the subject and the reference ratio obtained from at least one interferon responder subject is below a predetermined threshold such as below about 10 times, e.g., below about 9 times, e.g., below about 8 times, e.g., below about 7 times, e.g., below about 6 times, e.g., below about 5 times, e.g., below about 4 times, e.g., below about 3 times, e.g., below about 2 times, e.g., below about 90%, e.g., below about 80%, e.g., below about 70%, e.g., below about 60%, e.g., below about 50%, e.g., below about 40%, e.g., below about 30%, e.g., below about 20%, e.g., below about 10%, e.g., below about 9%, e.g., below about 8%, e.g., below about 7%, e.g., below about 6%, e.g., below about 5%, e.g., below about 4%, e.g., below about 3%, e.g., below about 2%, e.g., below about 1% relative to the reference ratio of the at least one interferon responder subject.

According to some embodiments of the invention, when the ratio of the subject is identical or changed below a predetermined threshold as compared to the reference ratio of the at least one interferon non-responder subject, then the subject is classified as non-responsive to interferon.

According to some embodiments of the invention, the change (increase or decrease) between the ratio of the subject and the reference ratio obtained from at least one interferon non-responder subject is below a predetermined threshold such as below about 10 times, e.g., below about 9 times, e.g., below about 8 times, e.g., below about 7 times, e.g., below about 6 times, e.g., below about 5 times, e.g., below about 4 times, e.g., below about 3 times, e.g., below about 2 times, e.g., below about 90%, e.g., below about 80%, e.g., below about 70%, e.g., below about 60%, e.g., below about 50%, e.g., below about 40%, e.g., below about 30%, e.g., below about 20%, e.g., below about 10%, e.g., below about 9%, e.g., below about 8%, e.g., below about 7%, e.g., below about 6%, e.g., below about 5%, e.g., below about 4%, e.g., below about 3%, e.g., below about 2%, e.g., below about 1% relative to the reference expression data obtained from the at least one interferon non-responder subject.

According to an aspect of some embodiments of the invention, there is provided a method of predicting responsiveness of a subject to interferon treatment, comprising comparing a ratio determined between an expression level of ISG15, IFI6, IFIT1, OAS2 and OAS3 genes in a cell of the subject following interferon treatment and an expression level of the genes in the cell of the subject prior to interferon treatment, or visa versa, namely, comparing a ratio determined between an expression level of ISG15, IFI6, IFIT1, OAS2 and OAS3 genes in a cell of the subject prior to interferon treatment and an expression level of the genes in the cell of the subject following interferon treatment, to a reference ratio determined in a cell of at least one interferon responder subject and/or at least one interferon non-responder subject, the reference ratio is determined between an expression level of the genes following interferon treatment and an expression level of the genes prior to interferon treatment, or visa versa, namely, the reference ratio is determined between an expression level of the genes prior to interferon treatment and an expression level of the genes following interferon treatment, thereby predicting the responsiveness to interferon treatment of a subject.

Sequence information regarding the gene products *(i.e.,* RNA transcripts and polypeptide sequences) of ISG15, IFI6, IFIT1, OAS2 and OAS3 which can be detected according to the method of some embodiments of the invention is provided in Table 2 in the Examples section which follows. In addition, sequence information of probes which can be used to detect the ISG15, IFI6, IFIT1, OAS2 and OAS3 RNA transcripts is provided in Table 2 in the Examples section which follows.

According to an aspect of some embodiments of the invention, there is provided a method of predicting responsiveness of a subject to interferon treatment, comprising comparing a ratio determined between an expression level of at least one gene selected from the group consisting of: TICAM1, MYD88, TLR7, TRAFD1 and IRF7 in a cell of the subject following interferon treatment and an expression level of the at least one gene in the cell of the subject prior to interferon treatment, or visa versa, namely, comparing a ratio determined between an expression level of at least one gene selected from the group consisting of: TICAM1, MYD88, TLR7, TRAFD1 and IRF7 in a cell of the subject prior to interferon treatment and an expression level of the at least one gene in the cell of the subject following interferon treatment, to a reference ratio determined in a cell of at least one interferon responder subject and/or at least one interferon non-responder subject, the reference ratio is determined between an expression level of the gene following interferon treatment and an expression level of the gene prior to interferon treatment, or visa versa, namely, the reference ratio is determined between an expression level of the gene prior to interferon treatment and an expression level of the gene following interferon treatment, thereby predicting the responsiveness to interferon treatment of a subject.

According to some embodiments of the invention the at least one gene comprises one gene, at least two genes, at least three genes, at least four genes or at least 5 genes from the group of TICAM1, MYD88, TLR7, TRAFD1 and IRF7 genes.

Sequence information regarding the gene products *(i.e.,* RNA transcripts and polypeptide sequences) of TICAM1, MYD88, TLR7, TRAFD1 and IRF7 which can be detected according to the method of some embodiments of the invention is provided in Table 7 in the Examples section which follows. In addition, sequence information of probes which can be used to detect the TICAM1, MYD88, TLR7, TRAFD1 and IRF7 RNA transcripts is provided in Table 7 in the Examples section which follows.

According to an aspect of some embodiments of the invention, there is provided a method of predicting responsiveness of a subject to interferon treatment, comprising comparing a ratio determined between an expression level of at least one gene selected from the group consisting of HERC5 and UBE2L6 in a liver cell of the subject following interferon treatment and an expression level of the at least one gene in the liver cell of the subject prior to interferon treatment, or visa versa, namely, comparing a ratio determined between an expression level of at least one gene selected from the group consisting of HERC5 and UBE2L6 in a liver cell of the subject prior to interferon treatment and an expression level of the at least one gene in the liver cell of the subject following interferon treatment, to a reference ratio determined in a cell of at least one interferon responder subject and/or at least one interferon non-responder subject, the reference ratio is determined between an expression level of the at least one gene following interferon treatment and an expression level of the at least one gene prior to interferon treatment, or visa versa, namely, the reference ratio is determined between an expression level of the at least one gene prior to interferon treatment and an expression level of the at least one gene following interferon treatment, thereby predicting the responsiveness to interferon treatment of a subject.

For example, as shown in Table 6 (Examples section), while the average ratio between the expression level of HERC5 following interferon injection as compared to prior interferon injection was about 5 in interferon responders, the average ratio between the expression level of HERC5 following interferon injection as compared to prior interferon injection in interferon non-responders was about 1.18. Thus, there is an increase of about 5 times between the ratio in responders to the ratio in non-responder.

According to some embodiments of the invention the method further comprising comparing a ratio determined between an expression level of at least one gene selected from the group consisting of ISG15 and USP18 in a liver cell of the subject following interferon treatment and an expression level of the at least one gene in the liver cell of the subject prior to interferon treatment, or visa versa, namely, comparing a ratio determined between an expression level of at least one gene selected from the group consisting of ISG15 and USP18 in a liver cell of the subject prior to interferon treatment and an expression level of the at least one gene in the liver cell of the subject following interferon treatment, to a reference ratio determined in a cell of at least one interferon responder subject and/or at least one interferon non-responder subject, the reference ratio is determined between an expression level of the at least one gene following interferon treatment and an expression level of the at least one gene prior to interferon treatment, or visa versa, namely, the reference ratio is determined between an expression level of the at least one gene prior to interferon treatment and an expression level of the at least one gene following interferon treatment, thereby predicting the responsiveness to interferon treatment of a subject.

According to some embodiments of the invention the level of expression of the at least one gene comprises at least two, at least 3, at least 4 genes from the group of HERC5, UBE2L6, ISG15 and USP18 genes.

Sequence information regarding the gene products (*i.e.,* RNA transcripts and polypeptide sequences) of HERC5, UBE2L6, ISG15 and USP18 which can be detected according to the method of some embodiments of the invention is provided in Table 5 in the Examples section which follows. In addition, sequence information of probes which can be used to detect the HERC5, UBE2L6, ISG15 and USP18 RNA transcripts is provided in Table 5 in the Examples section which follows.

According to an aspect of some embodiments of the invention, there is provided a method of predicting responsiveness of a subject to interferon treatment, comprising comparing a ratio determined between an expression level of at least one gene selected from the group consisting ISG15, IFI6, IFIT1, OAS2 and OAS3 in a liver cell of the subject following interferon treatment and an expression level of the at least one gene in the liver cell of the subject prior to interferon treatment, or visa versa, namely, comparing a ratio determined between an expression level of at least one gene selected from the group consisting ISG15, IFI6, IFIT1, OAS2 and OAS3 in a liver cell of the subject prior to interferon treatment and an expression level of the at least one gene in the liver cell of the subject following interferon treatment, to a reference ratio determined in a liver cell of at least one interferon responder subject and/or at least one interferon non-responder subject, the reference ratio is determined between an expression level of the at least one gene following interferon treatment and an expression level of the at least one gene prior to interferon treatment, or visa versa, namely, the reference ratio is determined between an expression level of the at least one gene prior to interferon treatment and an expression level of the at least one gene following interferon treatment, thereby predicting the responsiveness to interferon treatment of a subject.

According to some embodiments of the invention the at least one gene comprises one gene, at least two genes, at least three genes, at least four genes or at least 5 genes from the group of ISG15, IFI6, IFIT1, OAS2 and OAS3 genes.

It should be noted that for predication of responsiveness to interferon treatment several of the above methods may be used in combination for example combination of static (e.g., determination prior to interferon treatment), dynamic (e.g., comparing the level of expression before and after interferon treatment) and in vitro methods, combination of samples from blood or liver biopsy, combination of checking various sets of genes.

According to some embodiments of the invention, the method further comprising selecting the subject as an HCV infected subject (e.g., chronic HCV).

According to some embodiments of the invention, the method further comprising selecting the subject as a multiple sclerosis diagnosed subject.

The method of predicting the responsiveness of a subject to interferon treatment according to some embodiments of the invention enables the classification of a subject as an interferon responder or an interferon non-responder.

Since as mentioned above the responsiveness to interferon treatment may affect disease outcome, the teachings of the invention can be used to determine the prognosis of a subject in need of interferon treatment.

According to some embodiments of the invention, the method further comprising informing the subject of the predicted responsiveness to interferon treatment (e.g., responder or non-responder) and/or the predicted prognosis of the subject.

As used herein the phrase "informing the subject" refers to advising the subject that based on the predicted responsiveness to interferon treatment the subject should seek a suitable treatment regimen. For example, if the subject is predicted to respond to interferon treatment and is diagnosed or suffers from a pathology requiring interferon treatment that such a treatment is advisable.

Once the responsiveness to interferon treatment is determined, the results can be recorded in the subject's medical file, which may assist in selecting a treatment regimen and/or determining prognosis of the subject.

According to some embodiments of the invention, the method further comprising recording the responsiveness of the subject to interferon treatment in the subject's medical file.

As mentioned, the prediction of the responsiveness of a subject to interferon treatment can be used to select the treatment regimen of a subject and thereby treat the subject in need thereof.

Thus, according to an aspect of some embodiments of the invention, there is provided a method of treating of a subject in need of interferon treatment, the method comprising: (a) predicting the responsiveness of the subject to interferon treatment according to the method of the invention, and (b) selecting a treatment regimen based on the responsiveness; thereby treating the subject in need of interferon treatment.

As used herein the phrase "a subject in need of interferon treatment" refers to any subject who is diagnosed with or suffers from a pathology (e.g., condition, disease or disorder) requiring interferon treatment. Non-limiting examples of such pathologies include as autoimmune disorder (e.g., multiple sclerosis), cancer (e.g., hematological malignancy, leukemia and lymphomas including hairy cell leukemia, chronic myeloid leukemia, nodular lymphoma, cutaneous T-cell lymphoma, recurrent melanomas), and viral infection (e.g., hepatitis C virus infection, hepatitis B virus infection, viral respiratory diseases such as cold and flu).

According to some embodiments of the invention, a subject in need of interferon treatment is diagnosed with multiple sclerosis.

According to some embodiments of the invention, a subject in need of interferon treatment is infected with HCV (any type of HCV as described above).

The term "treating" refers to inhibiting, preventing or arresting the development of a pathology (disease, disorder or condition) and/or causing the reduction, remission, or regression of a pathology. Those of skill in the art will understand that various methodologies and assays can be used to assess the development of a pathology, and similarly, various methodologies and assays may be used to assess the reduction, remission or regression of a pathology.

As used herein the phrase "treatment regimen" refers to a treatment plan that specifies the type of treatment, dosage, schedule and/or duration of a treatment provided to a subject in need thereof (e.g., a subject diagnosed with multiple sclerosis or infected with HCV). The selected treatment regimen can be an aggressive one which is expected to result in the best clinical outcome (e.g., complete cure of the pathology), yet may be associated with some discomfort to the subject or adverse side effects (e.g., a damage to healthy cells or tissue); or a more moderate one which may relief symptoms of the pathology yet may results in incomplete cure of the pathology. The type of treatment, dosage, schedule and duration of treatment can vary, depending on the severity of pathology and the predicted responsiveness of the subject to the treatment, and those of skills in the art are capable of adjusting the type of treatment with the dosage, schedule and duration of treatment.

According to some embodiments of the invention, when the subject is classified as a responder to interferon treatment the treatment regimen comprises administration of interferon.

As mentioned above and described in the Examples section which follows, the present inventor has uncovered that in interferon responders there is a coordinated increase in the level of the KIR inhibitory receptor genes in the blood and of their matched HLA genes in the liver. Thus, the inventor uncovered that downregulation of the interaction between the HLA and the KIR inhibitory receptors would increase the responsiveness of a subject to interferon.

According to an aspect of some embodiments of the invention, there is provided a method of treating a subject in need of interferon therapy, comprising co-administering to the subject interferon and an agent capable of downregulating an expression level and/or activity of an HLA gene product or of a KIR inhibitory receptor gene product, thereby treating the subject in need of interferon therapy.

According to some embodiments of the invention, co-administering is effected so as to enable a pharmacokinetic overlap between the interferon and the agent which is capable of downregulating HLA or KIR inhibitory receptor gene product(s).

As used herein the phrase "pharmacokinetic overlap" refers to a substantial overlap between the efficacy window of the agent capable of downregulating HLA or KIR inhibitory receptor gene products and the efficacy window of interferon.

As used herein, the phrase "efficacy window" describes a time frame during which an active agent exhibits a desired pharmacological effect, herein prevention of the interaction between HLA and the KIR inhibitory receptor by the agent capable of downregulating HLA or KIR inhibitory receptor gene product(s); and clearance of HCV from the body by interferon. In other words, this phrase describes the time period at which the plasma concentration of an active agent is equal to or higher than a minimal pharmacologically effective concentration thereof.

According to some embodiments of the invention, the co-administering is effected concomitantly. In some embodiments, concomitant co-administration is effected such that both agents [*i.e.,* interferon and agent which is capable of downregulating HLA or KIR inhibitory receptor gene product(s)] are administered at the same time, or such that the agents are co-formulated in a unit dosage form, as is detailed hereinafter.

According to some embodiments of the invention, the method further comprising administering ribavirin to the subject.

According to some embodiments of the invention, the subject is a non-responder to interferon treatment.

According to some embodiments of the invention, the agent which is capable of downregulating HLA or KIR inhibitory receptor gene product(s) can be an antibody, an RNA silencing molecule, a ribozyme or a DNAzyme.

According to some embodiments of the invention, the HLA gene product which expression level is downregulated by the agent is HLA-A, HLA-B, HLA-C, HLA-F and/or HLA-G.

According to some embodiments of the invention, the KIR inhibitory receptor gene product which expression level is downregulated by the agent is KIR3DL3, KIR3DL2, KIR3DL1, KIR2DL1, KIR2DL2, KIR2DL3, CD160 and/or KLRG1.

According to some embodiments of the invention, the antibody is an anti-KIR inhibitory receptor antibody.

According to some embodiments of the invention the anti-KIR inhibitory receptor antibody is the human monoclonal antibody 1-7F9 (Shah N., et al., 2009, Blood 114:2567-2568) which targets KIR2DL1, KIR2DL2 and KIR2DL3 on natural killer (NK) cells. This antibody activates NK cells by blocking the interaction between inhibitory KIRs and target cell HLA class I molecules.

According to some embodiments of the invention the anti-KIR inhibitory receptor antibody is the ECM41 monoclonal antibody (Vitale M., et al., Int Immunol. 2004 Oct;16(10):1459-66) which is specific to KIR2DL3.

The term "antibody" as used in this invention includes intact molecules as well as functional fragments thereof, such as Fab, F(ab')2, and Fv that are capable of binding to macrophages. These functional antibody fragments are defined as follows: (1) Fab, the fragment which contains a monovalent antigen-binding fragment of an antibody molecule, can be produced by digestion of whole antibody with the enzyme papain to yield an intact light chain and a portion of one heavy chain; (2) Fab', the fragment of an antibody molecule that can be obtained by treating whole antibody with pepsin, followed by reduction, to yield an intact light chain and a portion of the heavy chain; two Fab' fragments are obtained per antibody molecule; (3) (Fab')2, the fragment of the antibody that can be obtained by treating whole antibody with the enzyme pepsin without subsequent reduction; F(ab')2 is a dimer of two Fab' fragments held together by two disulfide bonds; (4) Fv, defined as a genetically engineered fragment containing the variable region of the light chain and the variable region of the heavy chain expressed as two chains; and (5) Single chain antibody ("SCA"), a genetically engineered molecule containing the variable region of the light chain and the variable region of the heavy chain, linked by a suitable polypeptide linker as a genetically fused single chain molecule.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988, incorporated herein by reference).

Antibody fragments according to the present invention can be prepared by proteolytic hydrolysis of the antibody or by expression in E. coli or mammalian cells (e.g. Chinese hamster ovary cell culture or other protein expression systems) of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, by Goldenberg, U.S. Pat. Nos. 4,036,945 and 4,331,647, and references contained therein, which patents are hereby incorporated by reference in their entirety. See also Porter, R. R. [Biochem. J. 73: 119-126 (1959)]. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of VH and VL chains. This association may be noncovalent, as described in Inbar et al. [Proc. Nat'l Acad. Sci. USA 69:2659-62 (19720]. Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (sFv) are prepared by constructing a structural gene comprising DNA sequences encoding the VH and VL domains connected by an oligonucleotide. The structural gene is inserted into an expression vector, which is subsequently introduced into a host cell such as E. coli. The recombinant host cells synthesize a single polypeptide chain with a linker peptide bridging the two V domains. Methods for producing sFvs are described, for example, by [Whitlow and Filpula, Methods 2: 97-105 (1991); Bird et al., Science 242:423-426 (1988); Pack et al., Bio/Technology 11:1271-77 (1993); and U.S. Pat. No. 4,946,778, which is hereby incorporated by reference in its entirety.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry [Methods, 2: 106-10 (1991)].

Humanized forms of non-human (e.g., murine) antibodies are chimeric molecules of immunoglobulins, immunoglobulin chains or fragments thereof (such as Fv, Fab, Fab', F(ab').sub2 or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers [Jones et al., Nature, 321:522-525 (1986); Riechmann et al., Nature 332:323-327 (1988); Verhoeyen et al., Science, 239:1534-1536 (1988)], by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introduction of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995).

As used herein, the phrase "RNA silencing" refers to a group of regulatory mechanisms [e.g. RNA interference (RNAi), transcriptional gene silencing (TGS), post-transcriptional gene silencing (PTGS), quelling, co-suppression, and translational repression] mediated by RNA molecules which result in the inhibition or "silencing" of the expression of a corresponding protein-coding gene. RNA silencing has been observed in many types of organisms, including plants, animals, and fungi.

In certain embodiments, the RNA silencing agent is capable of preventing complete processing (e.g., the full translation and/or expression) of an mRNA molecule through a post-transcriptional silencing mechanism. RNA silencing agents include noncoding RNA molecules, for example RNA duplexes comprising paired strands, as well as precursor RNAs from which such small non-coding RNAs can be generated. Exemplary RNA silencing agents include dsRNAs such as siRNAs, miRNAs and shRNAs. In one embodiment, the RNA silencing agent is capable of inducing RNA interference. In another embodiment, the RNA silencing agent is capable of mediating translational repression.

According to one embodiment, the dsRNA is greater than 30 bp. The use of long dsRNAs (i.e. dsRNA greater than 30 bp) has been very limited owing to the belief that these longer regions of double stranded RNA will result in the induction of the interferon and PKR response. However, the use of long dsRNAs can provide numerous advantages in that the cell can select the optimal silencing sequence alleviating the need to test numerous siRNAs; long dsRNAs will allow for silencing libraries to have less complexity than would be necessary for siRNAs; and, perhaps most importantly, long dsRNA could prevent viral escape mutations when used as therapeutics.

The term "siRNA" refers to small inhibitory RNA duplexes (generally between 18-30 basepairs) that induce the RNA interference (RNAi) pathway. Typically, siRNAs are chemically synthesized as 21 mers with a central 19 bp duplex region and symmetric 2-base 3'-overhangs on the termini, although it has been recently described that chemically synthesized RNA duplexes of 25-30 base length can have as much as a 100-fold increase in potency compared with 21mers at the same location. The observed increased potency obtained using longer RNAs in triggering RNAi is theorized to result from providing Dicer with a substrate (27mer) instead of a product (21mer) and that this improves the rate or efficiency of entry of the siRNA duplex into RISC.

It has been found that position of the 3'-overhang influences potency of an siRNA and asymmetric duplexes having a 3'-overhang on the antisense strand are generally more potent than those with the 3'-overhang on the sense strand (Rose et al., 2005). This can be attributed to asymmetrical strand loading into RISC, as the opposite efficacy patterns are observed when targeting the antisense transcript.

The strands of a double-stranded interfering RNA (e.g., an siRNA) may be connected to form a hairpin or stem-loop structure (e.g., an shRNA). Thus, as mentioned the RNA silencing agent of the present invention may also be a short hairpin RNA (shRNA).

The term "shRNA", as used herein, refers to an RNA agent having a stem-loop structure, comprising a first and second region of complementary sequence, the degree of complementarity and orientation of the regions being sufficient such that base pairing occurs between the regions, the first and second regions being joined by a loop region, the loop resulting from a lack of base pairing between nucleotides (or nucleotide analogs) within the loop region. The number of nucleotides in the loop is a number between and including 3 to 23, or 5 to 15, or 7 to 13, or 4 to 9, or 9 to 11. Some of the nucleotides in the loop can be involved in base-pair interactions with other nucleotides in the loop. Examples of oligonucleotide sequences that can be used to form the loop include 5'-UUCAAGAGA-3' (Brummelkamp, T. R. et al. (2002) Science 296: 550) and 5'-UUUGUGUAG-3' (Castanotto, D. et al. (2002) RNA 8:1454). It will be recognized by one of skill in the art that the resulting single chain oligonucleotide forms a stem-loop or hairpin structure comprising a double-stranded region capable of interacting with the RNAi machinery.

According to another embodiment the RNA silencing agent may be a miRNA. miRNAs are small RNAs made from genes encoding primary transcripts of various sizes. They have been identified in both animals and plants. The primary transcript (termed the "pri-miRNA") is processed through various nucleolytic steps to a shorter precursor miRNA, or "pre-miRNA." The pre-miRNA is present in a folded form so that the final (mature) miRNA is present in a duplex, the two strands being referred to as the miRNA (the strand that will eventually basepair with the target) The pre-miRNA is a substrate for a form of dicer that removes the miRNA duplex from the precursor, after which, similarly to siRNAs, the duplex can be taken into the RISC complex. It has been demonstrated that miRNAs can be transgenically expressed and be effective through expression of a precursor form, rather than the entire primary form (Parizotto et al. (2004) Genes & Development 18:2237-2242 and Guo et al. (2005) Plant Cell 17:1376-1386).

Synthesis of RNA silencing agents suitable for use with the present invention can be effected as follows. First, the target mRNA sequence (e.g, HLA or KIR inhibitory receptors as described above) is scanned downstream of the AUG start codon for AA dinucleotide sequences. Occurrence of each AA and the 3' adjacent 19 nucleotides is recorded as potential siRNA target sites. Preferably, siRNA target sites are selected from the open reading frame, as untranslated regions (UTRs) are richer in regulatory protein binding sites. UTR-binding proteins and/or translation initiation complexes may interfere with binding of the siRNA endonuclease complex [Tuschl ChemBiochem. 2:239-245]. It will be appreciated though, that siRNAs directed at untranslated regions may also be effective, as demonstrated for GAPDH wherein siRNA directed at the 5' UTR mediated about 90 % decrease in cellular GAPDH mRNA and completely abolished protein level (www.ambion.com/techlib/tn/91/912.html).

Second, potential target sites are compared to an appropriate genomic database (e.g., human, mouse, rat etc.) using any sequence alignment software, such as the BLAST software available from the NCBI server (www.ncbi.nlm.nih.gov/BLAST/). Putative target sites which exhibit significant homology to other coding sequences are filtered out.

Qualifying target sequences are selected as template for siRNA synthesis. Preferred sequences are those including low G/C content as these have proven to be more effective in mediating gene silencing as compared to those with G/C content higher than 55 %. Several target sites are preferably selected along the length of the target gene for evaluation. For better evaluation of the selected siRNAs, a negative control is preferably used in conjunction. Negative control siRNA preferably include the same nucleotide composition as the siRNAs but lack significant homology to the genome. Thus, a scrambled nucleotide sequence of the siRNA is preferably used, provided it does not display any significant homology to any other gene.

According to some embodiments of the invention, the RNA silencing molecule is an siRNA directed against a KIR inhibitory receptor or a HLA.

Non-limiting examples of siRNA molecules which can be used according to the method of some embodiments of the invention include the sequences set forth by SEQ ID NOs:175-178 (directed against HLA-B); SEQ ID NOs:179-197 (directed against HLA-F); SEQ ID NOs:198-210 (directed against HLA-C); SEQ ID NOs:244-286 (directed against HLA-G); SEQ ID NOs:325-354 (directed against KIR3DL1); SEQ ID NOs:419-468 (directed against KIRG1); SEQ ID NOs:211-243 (directed against HLA-A); SEQ ID NOs:287-301 (directed against KIR3DL2); SEQ ID NOs:302-324 (directed against KIR3DL3); SEQ ID NOs:355-368 (directed against KIR2DL3); SEQ ID NOs:369-418 (directed against CD160); and/or the siRNA described in Sergio Gonzalez S, et al., 2005 (Molecular Therapy Vol. 11: 811-8. Amplification of RNAi-Targeting HLA mRNAs), which is incorporated herein by reference in its entirety.

It will be appreciated that the RNA silencing agent of the present invention need not be limited to those molecules containing only RNA, but further encompasses chemically-modified nucleotides and non-nucleotides.

In some embodiments, the RNA silencing agent provided herein can be functionally associated with a cell-penetrating peptide." As used herein, a "cell-penetrating peptide" is a peptide that comprises a short (about 12-30 residues) amino acid sequence or functional motif that confers the energy-independent (i.e., non-endocytotic) translocation properties associated with transport of the membrane-permeable complex across the plasma and/or nuclear membranes of a cell. The cell-penetrating peptide used in the membrane-permeable complex of the present invention preferably comprises at least one non-functional cysteine residue, which is either free or derivatized to form a disulfide link with a double-stranded ribonucleic acid that has been modified for such linkage. Representative amino acid motifs conferring such properties are listed in U.S. Pat. No. 6,348,185, the contents of which are expressly incorporated herein by reference. The cell-penetrating peptides of the present invention preferably include, but are not limited to, penetratin, transportan, pIsl, TAT(48-60), pVEC, MTS, and MAP.

Another agent capable of downregulating HLA or KIR inhibitory receptor is a DNAzyme molecule capable of specifically cleaving an mRNA transcript or DNA sequence of the HLA or KIR inhibitory receptor. DNAzymes are single-stranded polynucleotides which are capable of cleaving both single and double stranded target sequences (Breaker, R.R. and Joyce, G. Chemistry and Biology 1995;2:655; Santoro, S.W. & Joyce, G.F. Proc. Natl, Acad. Sci. USA 1997;943:4262) A general model (the "10-23" model) for the DNAzyme has been proposed. "10-23" DNAzymes have a catalytic domain of 15 deoxyribonucleotides, flanked by two substrate-recognition domains of seven to nine deoxyribonucleotides each. This type of DNAzyme can effectively cleave its substrate RNA at purine:pyrimidine junctions (Santoro, S.W. & Joyce, G.F. Proc. Natl, Acad. Sci. USA 199; for rev of DNAzymes see Khachigian, LM [Curr Opin Mol Ther 4:119-21 (2002)].

Examples of construction and amplification of synthetic, engineered DNAzymes recognizing single and double-stranded target cleavage sites have been disclosed in U.S. Pat. No. 6,326,174 to Joyce et al. DNAzymes of similar design directed against the human Urokinase receptor were recently observed to inhibit Urokinase receptor expression, and successfully inhibit colon cancer cell metastasis in vivo (Itoh et al , 20002, Abstract 409, Ann Meeting Am Soc Gen Ther www.asgt.org). In another application, DNAzymes complementary to bcr-ab1 oncogenes were successful in inhibiting the oncogenes expression in leukemia cells, and lessening relapse rates in autologous bone marrow transplant in cases of CML and ALL.

Downregulation of the HLA or KIR inhibitory receptor can also be effected using an antisense polynucleotide capable of specifically hybridizing with an mRNA transcript encoding the HLA or KIR inhibitory receptor.

Design of antisense molecules which can be used to efficiently downregulate a HLA or KIR inhibitory receptor must be effected while considering two aspects important to the antisense approach. The first aspect is delivery of the oligonucleotide into the cytoplasm of the appropriate cells, while the second aspect is design of an oligonucleotide which specifically binds the designated mRNA within cells in a way which inhibits translation thereof.

The prior art teaches of a number of delivery strategies which can be used to efficiently deliver oligonucleotides into a wide variety of cell types [see, for example, Luft J Mol Med 76: 75-6 (1998); Kronenwett et al. Blood 91: 852-62 (1998); Rajur et al. Bioconjug Chem 8: 935-40 (1997); Lavigne et al. Biochem Biophys Res Commun 237: 566-71 (1997) and Aoki et al. (1997) Biochem Biophys Res Commun 231: 540-5 (1997)].

In addition, algorithms for identifying those sequences with the highest predicted binding affinity for their target mRNA based on a thermodynamic cycle that accounts for the energetics of structural alterations in both the target mRNA and the oligonucleotide are also available [see, for example, Walton et al. Biotechnol Bioeng 65: 1-9 (1999)]. In addition, several approaches for designing and predicting efficiency of specific oligonucleotides using an in vitro system were also published (Matveeva et al., Nature Biotechnology 16: 1374 - 1375 (1998)].

Another agent capable of downregulating the HLA or KIR inhibitory receptor is a ribozyme molecule capable of specifically cleaving an mRNA transcript encoding HLA or KIR inhibitory receptor. Ribozymes are being increasingly used for the sequence-specific inhibition of gene expression by the cleavage of mRNAs encoding proteins of interest [Welch et al., Curr Opin Biotechnol. 9:486-96 (1998)]. The possibility of designing ribozymes to cleave any specific target RNA has rendered them valuable tools in both basic research and therapeutic applications. In the therapeutics area, ribozymes have been exploited to target viral RNAs in infectious diseases, dominant oncogenes in cancers and specific somatic mutations in genetic disorders [Welch et al., Clin Diagn Virol. 10:163-71 (1998)]. Most notably, several ribozyme gene therapy protocols for HIV patients are already in Phase 1 trials. More recently, ribozymes have been used for transgenic animal research, gene target validation and pathway elucidation. Several ribozymes are in various stages of clinical trials. ANGIOZYME was the first chemically synthesized ribozyme to be studied in human clinical trials. ANGIOZYME specifically inhibits formation of the VEGF-r (Vascular Endothelial Growth Factor receptor), a key component in the angiogenesis pathway. Ribozyme Pharmaceuticals, Inc., as well as other firms have demonstrated the importance of anti-angiogenesis therapeutics in animal models. HEPTAZYME, a ribozyme designed to selectively destroy Hepatitis C Virus (HCV) RNA, was found effective in decreasing Hepatitis C viral RNA in cell culture assays (Ribozyme Pharmaceuticals, Incorporated - WEB home page).

The agents described hereinabove (e.g., interferon and/or an agent which is capable of downregulating the expression level and/or activity of the HLA or KIR inhibitory receptor as described above) of the present invention can be administered to an organism per se, or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the interferon and/or an agent which is capable of downregulating the expression level and/or activity of the HLA or KIR inhibitory receptor as described above accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition, which is incorporated herein by reference.

Suitable routes of administration may, for example, include oral, rectal, transmucosal, especially transnasal, intestinal or parenteral delivery, including intramuscular, subcutaneous and intramedullary injections as well as intrathecal, direct intraventricular, intracardiac, e.g., into the right or left ventricular cavity, into the common coronary artery, intravenous, inrtaperitoneal, intranasal, or intraocular injections.

Conventional approaches for drug delivery to the central nervous system (CNS) include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide). However, each of these strategies has limitations, such as the inherent risks associated with an invasive surgical procedure, a size limitation imposed by a limitation inherent in the endogenous transport systems, potentially undesirable biological side effects associated with the systemic administration of a chimeric molecule comprised of a carrier motif that could be active outside of the CNS, and the possible risk of brain damage within regions of the brain where the BBB is disrupted, which renders it a suboptimal delivery method.

Alternately, one may administer the pharmaceutical composition in a local rather than systemic manner, for example, via injection of the pharmaceutical composition directly into a tissue region of a patient.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuos infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of the present invention may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose. More specifically, a therapeutically effective amount means an amount of active ingredients effective to prevent, alleviate or ameliorate symptoms of a disorder (e.g., multiple sclerosis or HCV infection) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide tissue and/or blood levels of the active ingredient which are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

The agents described hereinabove (e.g., the oligonucleotides, probes, antibodies) for predicting responsiveness of a subject to interferon treatment may be included in a diagnostic kit/article of manufacture preferably along with appropriate instructions for use and labels indicating FDA approval for use in predicting responsiveness of a subject to interferon treatment and/or treating the subject.

Thus, according to an aspect of some embodiments of the invention there is provided a diagnostic kit. The kit comprises at least one oligonucleotide or antibody for specifically determining an expression level of at least one gene of the genes which are differentially regulated between interferon responders and interferon non-responders as described hereinabove and in the Examples section which follows.

The term "oligonucleotide" refers to a single stranded or double stranded oligomer or polymer of ribonucleic acid (RNA) or deoxyribonucleic acid (DNA) or mimetics thereof. This term includes oligonucleotides composed of naturally-occurring bases, sugars and covalent internucleoside linkages (e.g., backbone) as well as oligonucleotides having non-naturally-occurring portions which function similarly to respective naturally-occurring portions.

According to some embodiments of the invention, the at least one oligonucleotide does not exceed 1000 oligonucleotides, e.g., does not exceed 500 oligonucleotides, e.g., does not exceed 400 oligonucleotides, e.g., does not exceed 300 oligonucleotides, e.g., does not exceed 200 oligonucleotides, e.g., does not exceed 50 oligonucleotides, e.g., does not exceed 40 oligonucleotides, e.g., does not exceed 30 oligonucleotides.

According to some embodiments of the invention, the at least one oligonucleotide or antibody is capable of determining the expression level of at least one gene selected from the group consisting of KIR3DL3, KIR3DL2, KIR3DL1, KIR2DL1, KIR2DL2, KIR2DL3, KLRG1, KIR3DS1, CD160, HLA-A, HLA-B, HLA-C, HLA-F, HLA-G and IFI27.

According to some embodiments of the invention, the at least one oligonucleotide or antibody is capable of determining the expression level of TNFRSF17 gene.

According to some embodiments of the invention, the at least one oligonucleotide or antibody is capable of determining the expression level of at least one gene selected from the group consisting of TNFRSF17, CXCL10 and CD24.

According to some embodiments of the invention, the at least one oligonucleotide or antibody is capable of determining the expression level of at least one gene selected from the group consisting of IFI6, OAS2, ISG15, OAS3 and IFIT1 genes.

According to some embodiments of the invention, the at least one oligonucleotide or antibody is capable of determining the expression level of at least one gene selected from the group consisting of HERC5 and UBE2L6.

According to some embodiments of the invention, the at least one oligonucleotide or antibody is capable of determining the expression level of at least one gene selected from the group consisting of HERC5, UBE2L6, ISG15 and USP18.

According to some embodiments of the invention, the at least one oligonucleotide or antibody is capable of determining the expression level of at least one gene selected from the group consisting of KIR3DL3, KIR3DL2, KIR3DL1, KIR2DL1, KIR2DL2, KIR2DL3, KLRG1, KIR3DS1, CD160, HLA-A, HLA-B, HLA-C, HLA-F, HLA-G, IFI27, TNFRSF17, CXCL10, CD24, IFI6, OAS2, ISG15, OAS3, IFIT1, HERC5, UBE2L6, ISG15 and USP18.

As used herein the term "about" refers to ± 10 %.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### EXAMPLE 1

### IDENTIFICATION OF GENES WHICH ARE DIFFERENTIALLY EXPRESSED BETWEEN RESPONDERS AND NON-RESPONDERS TO INTERFERON TREATMENT - A STATIC METHOD

In order to get a high resolution of the relative importance of the dominate genes which determine the fate of interferon I treatment, the present inventor has developed a statistical scoring tool that is based on the quantitative and qualitative ranking of all the genes in all possible permutations. The assumption behind this process is that the most important genes determining the outcome of treatment are the ones most persistent in all possible combinations of patient comparison selection. In the case of the Chen et al., 2005 dataset [L. Chen, I. Borozan, J. Feld, J. Sun, L. Tannis, C. Coltescu, J. Heathcote, A. Edwards, I. Mcgilvray. "Hepatic Gene Expression Discriminates Responders and Nonresponders in Treatment of Chronic Hepatitis C Viral Infection"; Gastroenterology, 128:1437-1444; Hypertext Transfer Protocol://142.150.56.35/∼LiverArrayProject/home (dot) html] the present inventor has selected all possible 11 combinations out of 15 cases in each group of interferon responders and non responders (13651 possibilities) which yielded 1365² or 18563225 t-test competitions between the genes as they are expressed in the two groups. In each race, the names of the top 10 winning genes were accumulated along with their p value scores, since the place of the winning genes in the race and the relative distance to the other genes in the race (how far away each gene is from the one before him and from the one after him) are important. Based on the tabulation of the results (Table 1, below) the prediction of the critical genes for the success of pegylated interferon (IFNIα) treatment for HCV were found. These are the same five genes identified by the present inventor in WO2007039906. However using the current methods the present inventor was able to pinpoint at the two most important genes required for the prediction, and to indicate the exact nature of the difference between respondents and non-respondents

**Table 1**

| ***Top 10 gene scores*** | | | | | |
|---|---|---|---|---|---|
| | ***Gene*** | ***Score*** | ***Ratio of best score*** | ***Divided by*** | ***g1p2 score divider*** |
| 1 | ISG15 (g1p2) | 9.49E+09 | 1 | 1 | glp2/1 |
| 2 | IFI6 (g1p3) | 4.47E+09 | 0.471521 | 2.120794 | g1p2/2.12 |
| 3 | ifit1 | 3.79E+08 | 0.03999 | 25.00643 | g1p2/25 |
| 4 | oas2 | 2.97E+08 | 0.031293 | 31.95613 | g1p2/31 |
| 5 | oas3 | 17968405 | 0.001894 | 528.0912 | g1p2/528 |
| 6 | | 2829517 | 0.000298 | 3353.56 | g1p2/3353 |
| 7 | | 2450741 | 0.000258 | 3871.872 | |
| 8 | | 896905 | 9.45E-05 | 10579.67 | |
| 9 | | 852981 | 8.99E-05 | 11124.46 | |
| 10 | | 427878.8 | 4.51E-05 | 22176.74 | |

Table 1: Scoring results top 5 critical genes.

In the tabulation process the statistical score of the top up-regulated genes in non-responders compared to responders was much higher than the scores obtained by the top down-regulated genes (which are therefore not included in the Table) in the opposite direction. Thus, an additional significant improvement was added to teachings of WO2007039906, in which during the scoring process, instead of using the median p-value of each of the 10 top places, each gene was scored by his own p-values obtained in the Table of the top 10 places.

These results demonstrate that the level of expression of g1p2 (ISG15) can be used as a predictor for response to interferon treatment before treatment has began.

### EXAMPLE 2

### VALIDATION OF THE PREDICTIVE POWER OF THE SIGNATURE GENES TO

### INTERFERON RESPONSE IN INDEPENDENT DATA SETS

### Experimental procedures

The expression levels of the genes-of-interest were obtained from publicly available data bases [Hypertext Transfer Protocol://World Wide Web (dot) ncbi (dot) nlm (dot) nih (dot) gov/projects/geo/] using the Gene Expression Omnibus Accession No. GSE11190. Analysis of data was performed by custom programs written in MATLAB.

Validation of results was performed by analysing RNA extracted from paraffin embedded liver biopsies which were obtained from of HCV type 1 patients before the first injection of interferon (*i.e.,* in time 0, naive patients).

### Results

***The OAS3, IF16, ISG15, OAS2 and IFIT1 gene signature is differentially expressed between interferon responder and non-responders -*** As shown in Figure 1 and Table 3 below, the OAS3, IF16, ISG15, OAS2 and IFIT1 are up regulated in non-responders to interferon treatment as compared to responders or healthy controls. Sequence information of genes which were found to be differentially regulated in between responders and non-responders to interferon treatment is provided in Table 2, below.

**Table 2**

| ***Sequence information*** | | | | | | |
|---|---|---|---|---|---|---|
| ***Probe Set ID*** | ***Gene Symbol*** | ***Gene Title*** | ***Representati ve Public ID*** | ***RefSeq Protein ID (SEQ ID NO:)*** | ***RefSeq Transcript ID (SEQ ID NO:)*** | ***UniGene ID*** |
| 204415_at (SEQ ID NO:1); | IFI6 (G1P3) | interferon, alpha-inducible protein 6 | NM_022873 | NP_002029 (SEQ ID NO:6); NP_075010 (SEQ ID NO:7); NP_075011 (SEQ ID NO:8); | NM_002038 (SEQ ID NO:15); NM_022872 (SEQ ID NO:16); NM_022873 (SEQ ID NO:17); | Hs.523847 |
| 218400_at (SEQ ID NO:2); | OAS3 | 2'-5'-oligoadenylate synthetase 3, 100kDa | NM_006187 | NP_006178 (SEQ ID NO:9); | NM_006187 (SEQ ID NO:18); | Hs.528634 |
| 205483_s_ at (SEQ ID NO:3); | ISG15 (G1P2) | ISG15 ubiquitin-like modifier | NM_005101 | NP_005092 (SEQ ID NO:10); | NM_005101 (SEQ ID NO:19); | Hs.458485 |
| 204972_at (SEQ ID NO:4); | OAS2 | 2'-5'-oligoadenylate synthetase 2, 69/71kDa | NM_016817 | NP_0010279 03 (SEQ ID NO:11); NP_002526 (SEQ ID NO:12); NP_058197 (SEQ ID NO:13); | NM_0010327 31 (SEQ ID NO:20); NM_002535 (SEQ ID NO:21); NM_016817 (SEQ ID NO:22); | Hs.414332 |
| 203153_at (SEQ ID NO:5); | IFIT1 | interferon-induced protein with tetratricopepti de repeats 1 | NM_001548 | NP_001539 (SEQ ID NO:14); | NM_001548 (SEQ ID NO:23); | Hs.20315 |

Table 2: Probe sets ID refer to the GeneChip Array "Human Genome U133 Plus 2.0 Array" from Affimetrix (Affymetrix hu1333_plus2). Sequences from NCBI refer to Genome version March 2006 (NCBI Build 36.1).

**Table 3**

| ***Expression analysis of signature genes in liver tissues in HCV type 1 patients*** | | | | | | | | | | |
|---|---|---|---|---|---|---|---|---|---|---|
| ***probe*** | ***gene*** | ***c1*** | ***c2*** | ***nr*_*1*** | ***nr_2*** | ***nr_4*** | ***nr_5*** | ***r_1*** | ***r_2*** | ***r_3*** |
| 218400_at | OAS3 | 126 | 193 | 2912 | 2601 | 1377 | 1275 | 236 | 413 | 196 |
| 205483_s_at | ISG15 | 842 | 775 | 22192 | 27471 | 17024 | 16703 | 2257 | 1806 | 1514 |
| 204415_at | IFI6 | 1105 | 1478 | 4118 | 2902 | 14001 | 13068 | 322 | 161 | 583 |
| 204972_at | OAS2 | 157 | 230 | 2385 | 2255 | 1469 | 964 | 362 | 382 | 64 |
| 203153_at | IFIT1 | 1475 | 1659 | 10685 | 17920 | 8919 | 8977 | 2374 | 2261 | 1889 |

Table 3. Provided are the raw data of the expression levels of various genes among 2 control subjects (c1, c2), 4 non-responders (nr_1, nr_2, nr_4 and nr_5) and 3 responders (r_1, r_2 and r_3) as measured in liver tissues of HCV type 1 (Results are presented in Figures 1A-E).

***Determination of expression level of the S-siganture genes OAS3, IF16*, *ISG15, OAS2 and IFIT1 in naïve HCV type 1 patients -*** Archive liver tissues from 21 HCV type 1 patients were used to determine the level of the 5-signature genes before interferon treatment (*i.e.,* at time 0, naive patients) and the ratio between the base line expression level of the signature genes in interferon non-responders and the expression level in each of the interferon responders was determined (Figure 3). The results show a high ratio between the expression level of non-responders base line and the expression level in responders, demonstrating that prior to interferon treatment the level of the 5-signature genes is low in patients which later on appear to be responders to interferon. These results confirm the previous results and demonstrate that the signature gene expression can be used to predict response to interferon.

### EXAMPLE 3

### IDENTIFICATION OFA GENETIC SWITCH IMMEDIATELY AFTER INTERFERON TREATMENT AS A PREDICTOR FOR RESPONSE TO INTERFERON TREATMENT -A DYNAMIC METHOD

The expression levels of the genes-of-interest were obtained from publicly available data bases [Hypertext Transfer Protocol://World Wide Web (dot) ncbi (dot) nlm (dot) nih (dot) gov/projects/geo/] using the Gene Expression Omnibus Accession No. GSE11190. Analysis of data was performed by custom programs written in MATLAB.

### Results

***The expression levels** of **the OAS3, IF16, ISG15, OAS2 and IFIT1 gene signature is significantly upregulated among interferon responders following the first interferon treatment while being unchanged among non-responders -*** Based on the results presented in Figures 1A-E, Table 2 and Figure 3, the present inventor has hypothesized that the gene signature reflects an on/off situation; thus injection of interferon to "on" genes at 0 time (*i.e.,* before the first injection of interferon, naive subjects with respect to interferon) can not upregulate them, while injection to "off' genes will upregulate them. To test this assumption the inventor used the data set described in Sarasin-Filipowicz M, et al., 2008, who analyzed a total of 78 samples of blood or liver biopsy (taken before and after interferon alpha treatment) using Affymetrix Human U133 Plus 2.0 Array. The original data set was divided to two groups of patient: HCV type 1 and HCV types 2, 3 and 4 (types 2-4 hereinafter). The hypothesis was tested in HCV type 1 patients using liver tissue biopsies taken 4 hours after injection of interferon and prior to injection with interferon. As shown in Figures 2A-E and Table 4 below, while in non -responders to interferon treatment (subjects 1-4 in Figures 2A-E) there is nearly no change in the expression of the signature genes following interferon injection, in interferon responders (subjects 5-7 in Figures 2A-E), the expression level of the signature genes is at least log2 2.5 folds higher following interferon treatment. Thus, the ratio (*in vivo* log2) between the expression level determined 4 hours after injection and the expression level determined before injection was significantly higher among interferon responders as compared to interferon non-responders. These results demonstrate a powerful validation and a new prediction power for response to treatment provided by this gene signature by testing the level of the signature genes before and after interferon injection in liver tissues of HCV type 1 subjects.

**Table 4**

| ***Expression analysis of signature genes in liver of HCV type 1 patients*** | | | | | | | | |
|---|---|---|---|---|---|---|---|---|
| ***Probe*** | ***Gene*** | ***Nr_1*** | ***Nr_2*** | ***Nr_4*** | ***Nr_5*** | ***R_1*** | ***R_2*** | ***R_3*** |
| 205483_s_at | ISG15 | 0.161 | -0.081 | 0.019 | 0.243 | 3.078 | 1.609 | 3.557 |
| 203153_at | IFIT1 | 0.540 | -0.174 | 0.189 | 0.950 | 3.299 | 2.078 | 3.763 |
| 204972_at | OAS2 | 0.115 | -0.049 | 0.097 | 0.556 | 3.035 | 1.545 | 4.604 |
| 218400_at | OAS3 | 0.036 | 0.099 | 0.535 | 0.409 | 3.673 | 1.161 | 3.268 |
| 204415_at | IFI6 | 0.222 | 0.308 | -0.135 | -0.133 | 1.511 | 1.276 | 3.008 |

Table 4. Provided are the logarithmic ratios between the expression levels of various genes as measured in liver tissues of HCV type 1 patients 4 hours after interferon treatment and the level measured prior to interferon treatment among non-responders (Nr_1, Nr_2, Nr_4, and Nr_5) and responders (R_1, R_2, and R_3) to interferon treatment (Results are presented in Figures 2A-E).

These results demonstrate that in addition to testing the expression level of the 5 signature genes prior to interferon treatment, there is a significant value, with a high predictive power, to test the expression of the 5 signature genes in liver tissue (biopsies) taken prior to interferon treatment and 4 hours after interferon treatment, since the switch in gene expression immediately after interferon treatment is significant among interferon responders. On the other hand, in non-responders there is no change in the level of expression of these genes.

### EXAMPLE 4

### THE SIGNATURE GENES CAN PREDICT RESPONSE TO INTERFERON TREATMENT IN TYPES 1-4 HCV PATIENTS

The expression levels of the genes-of-interest were obtained from publicly available data bases [Hypertext Transfer Protocol://World Wide Web (dot) ncbi (dot) nlm (dot) nih (dot) gov/projects/geo/] using the Gene Expression Omnibus Accession No. GSE11190. Analysis of data was performed by custom programs written in MATLAB.

### Results

***The 5 signature genes are upregulated in interferon non-responders as compared to responders in liver samples of HCV types 1***-***4 patients -*** To further substantiate the above results and in order to determine if the 5-signature genes can predict responsiveness to interferon also in other types of HCV infections, the present inventor has analyzed data from GSE11190 and compared the level of expression between responders and non-responders to interferon treatment. The results show that in liver tissues of HCV types 1-4 interferon non-responders the 5 genes *i.e.,* ISG15, IFIT1, IFI6, OAS2, OAS3 are significantly up-regulated as compared to the interferon responders (Figure 4).

Further analysis of the same data set showed that in HCV type 1 patients the ISG15, IFIT1, IFI6, OAS2 and OAS3 genes are significantly up-regulated in non-responders as compared to responders (Figure 5).

These results demonstrate that the 5-signature genes (ISG15, IFIT1, IFI6, OAS2 and OAS3) can be used to predict response to interferon in all types of HCV virus infections, i.e., types 1-4, wherein upregulation of the level of expression before interferon treatment indicates that the patient will not response to a subsequent interferon treatment.

### EXAMPLE 5

### INVOLVEMENT OF THE ISGYLATED PROCESS IN RESPONSE TO INTERFERON -A DYNAMIC METHOD

The expression levels of the genes-of-interest were obtained from publicly available data bases [Hypertext Transfer Protocol://World Wide Web (dot) ncbi (dot) nlm (dot) nih (dot) gov/projects/geo/] using the Gene Expression Omnibus Accession No. GSE11190. Analysis of data was performed by custom programs written in MATLAB.

As shown in Example 1 hereinabove, the master gene of the predictive signature set is the ISG15 [interferon (IFN)-stimulated protein of 15 kDa]. G1P2/ISG15 is a ubiquitin-like protein that becomes conjugated to many cellular proteins upon activation by interferon-alpha (IFNA; MIM 147660) and interferon-beta (IFNB; MIM 147640) (Zhao et al., 2005 [PubMed16009940]). The ISGylated process is described in Anthony J. Sadler and Bryan R. G. Williams, 2008 [Nat Rev Immunol. 8(7):559-68. Review].

Table 5 below, provides sequence information of the probes/genes of the ISGylated process which were analyzed by the present inventor.

**Table 5**

| ***Sequence information*** | | | | | | |
|---|---|---|---|---|---|---|
| ***Probe Set ID*** | ***Gene Symbol*** | ***Gene Title*** | ***Representati ve Public ID*** | ***RefSeq Protein ID (SEQ ID NO:)*** | ***RefSeq Transcript ID (SEQ ID NO:)*** | ***UniGene ID*** |
| 205483_s_ at (SEQ ID NO:3) | ISG15 | ISG15 ubiquitin-like modifier | NM_005101 (SEQ ID NO:19) | NP_005092 (SEQ ID NO:10) | NM_005101 (SEQ ID NO:19) | Hs.458485 |
| 219211_at (SEQ ID NO:24) | USP18 | ubiquitin specific peptidase 18 | NM_017414 (SEQ ID NO:27) | NP_059110 (SEQ ID NO:30) | NM_017414 (SEQ ID NO:27) | Hs.38260 |
| 219863_at (SEQ ID NO:25) | HERC5 | hect domain and RLD 5 | NM_016323 (SEQ ID NO:28) | NP_057407 (SEQ ID NO:31) | NM_016323 (SEQ ID NO:28) | Hs.26663 |
| 201649_at (SEQ ID NO:26) | UBE2L6 | ubiquitin-conjugating enzyme E2L 6 | NM_004223 (SEQ ID NO:29) | NP_004214 (SEQ ID NO:32); NP_937826 (SEQ ID NO:33) | NM_004223 (SEQ ID NO:29); NM_198183 (SEQ ID NO:34) | Hs.425777 |

Table 5: Probe sets ID refer to the GeneChip Array "Human Genome U133 Plus 2.0 Array" from Affimetrix (Affymetrix hu1333_plus2). Sequences from NCBI refer to Genome version March 2006 (NCBI Build 36.1).

### Results

In order to determine the relevance of the genes involved in ISGylation in the response to interferon, the present inventor has analyzed the GSE11190 dataset for the expression level of the ISGylated genes. As shown in Figures 6A-D and Table 6 below, following interferon injection the genes belonging to the ISGylated process, ISG15, ISG15, HERC5, UBE2L6 are up-regulated in the responders (e.g., subjects 5, 6, 7 in Figures 6A-D) but not in the non-responders.

**Table 6**

| ***Raw data analysis of expression analysis of genes involved in the ISGylation process*** | | | | | | | |
|---|---|---|---|---|---|---|---|
| | ***nr1*** | ***nr2*** | ***nr3*** | ***nr4*** | ***r1*** | ***r2*** | ***r3*** |
| HERC5 | 0.001 | 0.20 | 0.26 | 0.46 | 2.83 | 1.31 | 2.44 |
| USP18 | 0.62 | -0.07 | 0.22 | 0.74 | 3.18 | 1.38 | 3.40 |
| ISG15 | 0.16 | -0.08 | 0.02 | 0.24 | 3.08 | 1.61 | 3.56 |
| UBE2L6 | 0.17 | -0.17 | -0.06 | 0.19 | 1.10 | 0.48 | 1.09 |

Table 6: Provided are the changes in expression level between 4 hours after interferon treatment as compared to prior to interferon treatment on a log2 scale. "Nr" = non-responder to interferon treatment; "r" = responder to interferon treatment.

These results indicate that the ISGylation process is essential to the success of the interferon treatment and in cases where the ISGylation is not "switched on" (i.e., upregulated following the first injection as compared to the level before the first injection) the treatment of interferon is likely to fail.

***The mechanism of action of ISG15 disabling in non responders -*** The expression of interferon (IFN)-stimulated protein of 15 kDa (ISG15), the E1-activating enzyme UBE1L or UBE2L6 shown here (E1-like ubiquitin-activating enzyme) and multiple E2-conjugating enzymes (shown here as an example is UBCH8) and E3-ligase enzymes [such as HERC5 (homologous to the E6-associated protein C terminus domain and RCC1-like domain containing protein 5)] is coordinately induced by type I IFNs through IFN-stimulated response elements (ISREs) in their respective gene promoter regions. E1, E2 and E3 proteins sequentially catalyse the conjugation of ISG15 to numerous protein substrates to modulate pleiotropic cellular responses to inhibit virus production. This process (known as ISGylation) is reversibly regulated by proteases [such as ubiquitin-specific protease 18 (USP18)]), which are also induced by IFNs Interferon-inducible antiviral effectors.

### Analysis and Discussion

Altogether, these results demonstrate that the top 5 genes ISG15, IFIT1, IFI6, OAS2 and OAS3 are relevant genes for deciding the fate of the treatment among HCV types 1-4 patients. The scores of place 6 and (glp2 score divide by 3353) and further down (as shown in Table 1, Example 1) are of no statistical importance. In addition, the 2 neighboring genes g1p2 (isg15) and g1p3 (ifi6) stand out as most important genes in deciding the fate of the treatment. If their expression level is up-regulated, a person would most probably not respond to the treatment. The next 3 genes are in concert with the top 2 but not as significant as the top 2 genes. IFIT1 and OAS2 add to the certainty of prediction, and with yet less significance, so does OAS3.

These results show that these genes act as a "fate switch". If the switch is "ON" (genes up-regulated), then the interferon treatment can not yield any success as its defense scheme operates by up-regulating these genes. In contrast, if the switch is "OFF" (genes down-regulated), then the interferon treatment can up-regulate these key genes, and start the defense mechanism.

### EXAMPLE 6

### IDENTIFICATION OF COMMON REGULATORY SEQUENCES TO THE INTERFERON RESPONSE SWITCH GENES

The immune cells in the liver include hepatocytes, which are lined with biliary endothelial cells (EC) on the portal facet, and stellate cells (SC) in the space of Disse (SD). EC separate the SD from the blood flow. Dendritic cells of plasmacytoid (PDC) and myeloid (MDC) origin, monocytes (Mo), macrophages (Mf), and Kupffer cells (KC) are located in close proximity to EC.

***The ISRE promoter is common to the 5*-*signature genes -*** The present inventor used Toucan 2 [workbench for regulatory sequence analysis; Hypertext Transfer Protocol://homes (dot) esat (dot) kuleuven (dot) be/∼saerts/software/toucan (dot) php] to search for common regulatory sequences of the signature genes. Thus, the 300 bp upstream sequence (relative to from exon 1) of each of the 5 signature genes was analyzed using a motif scanner with the TRANFAC Public 7.0 Verterbrates PWM Database and for Background using Human DBTSS Promoters(0) Database. As shown in Figure 7, all gene expression controls are localized to the ISRE promoter where ISGF3 complex and IRF7 are the controlling elements.

### EXAMPLE 7

### ACTIVATION OF THE TLR9 PATHWAY GENES IN BLOOD OF RESPONDERS HCV PATIENTS FOLLOWING INTERFERON TREATMENT

The TLR (Toll-like receptor)-mediated type I IFN pathway, in particular the MyD88 signaling pathway for IRF7 activation in pDCs, are in charge of robust type I gene induction.

Upon TLR7 or TLR9 (expressed in endosomes) stimulation, IRF7 interacting with MyD88 is activated by the IRAK4-IRAK1-IKK kinase cascade. Secreted type I IFNs enhance the expression of IRF7 gene, leading to further enhancement of type I IFN gene induction (Kenya Honda, Akinori Takaoka, and Tadatsugu Taniguchi. Type I Interferon Gene Induction Review by the Interferon Regulatory Factor Family of Transcription Factors. Immunity 25, 349-360, September 2006). TLR receptors from the TLR9 subfamily (TLR7 and TLR9) which are expressed in the endosomes transmit downstream signals via the recruitment of TIR-containing adaptor protein, such as MyD88 and TICAM1. IRF7 also interacts with TRAF6, another adaptor molecule functioning downstream of MYD88.

The present inventor has tested the changes in expression level of genes in the TLR mediated type I IFN pathway following treatment with interferon in blood samples of HCV type 1patients. For this purpose, the data set (Gene Expression Omnibus Accession No. Gse7123) published by Taylor MW, et al., 2007 (Virol. 81:3391-401. Changes in gene expression during pegylated interferon and ribavirin therapy of chronic hepatitis C virus distinguish responders from nonresponders to antiviral therapy) was analyzed. This data set includes gene expression data from RNA that was extracted from PBMC of type 1 HCV patients (responders and non-responders to interferon treatment) obtained prior to injection, 24 hours following the treatment, and on other time points along the end of the treatment. After RMA (Robust Multichip Average) normalization of all microarrays, the data was arranged to provide the fold change in a log2 scale in the expression level determined 24 hours after interferon treatment as compared to prior to interferon treatment in both responders and non responders.

### Results

***The 5*-*siganture genes* (*G1P2*,** ***G1P3, IFIT1, OAS2 and OSA3) and the TLR7-meidated pathway** genes* **(*TICAM1*, *MYD88, TLR7, TRAFD1*, *IRF7) are upregulated in responders following the first interferon injection -*** The results of the analyses show that both the switch signature genes (Figures 8A-E) and the TLR7-mediated type I IFN pathway genes (Figures 9A-E) are up-regulated 24 hours following interferon treatment in responders but not in non-responders (p-value of less than 0.05). Thus, these results show that in responders, but not in non-responders to interferon treatment, there is a functionally "on" switch *(i.e.,* upregulation of genes in response to interferon treatment) of the TLR signature genes.

Table 7 hereinbelow, provides sequence information of the TLR signature genes which were found to be upregulated in responders following treatment with interferon.

**Table 7**

| ***Sequence information of the TLR7 pathway genes*** | | | | | | |
|---|---|---|---|---|---|---|
| ***Probe Set ID*** | ***Gene Symbol*** | ***Gene Title*** | ***Transcript ID*** | ***RefSeq Protein ID (SEQ ID NO:)*** | ***RefSeq Transcript ID (SEQ ID NO:)*** | ***UniGene ID*** |
| 209124_at (SEQ ID NO:35) | MYD88 | myeloid differential ion primary response gene (88) | AK097983; AK124685; BC013589; BX537602; ENST00000 396334; ENST00000 415158; ENST00000 416282; ENST00000 417037; ENST00000 421516; ENST00000 421571; ENST00000 443433; NM_002468; U70451; uc003chw.1; uc003chx.1 | NP_002459 (SEQ ID NO:40) | NM_002468 (SEQ ID NO:48) | Hs.82116 |
| 213191_at (SEQ ID NO:36) | TICAM1 | toll-like receptor adaptor molecule 1 | AB086380; ENST00000 248244; NM_182919; uc002mbh.1; uc002mbi.1 | NP_891549 (SEQ ID NO:41) | NM_182919 (SEQ ID NO:49) | Hs.29344 |
| 218400_at (SEQ ID NO:2 | OAS3 | 2'-5'-oligoadeny late synthetase 3, 100kDa | AB044545; AF063613; ENST00000 228928; NM_006187; uc001tug.1 | NP_006178 (SEQ ID NO:9) | NM_006187 (SEQ ID NO:18) | Hs.528634 |
| 220146_at (SEQ ID NO:37 | TLR7 | toll-like receptor 7 | AF240467; ENST00000 380659; NM_016562; uc004cvc.1 | NP_057646 (SEQ ID NO:42) | NM_016562 (SEQ ID NO:50) | Hs.659215 |
| 35254_at (SEQ ID NO:38) | TRAFD1 | TRAF-type zinc finger domain containing 1 | AB007447; BC003553; ENST00000 257604; ENST00000 412615; ENST00000 432758; NM_001143 906; NM_006700; uc001tto.1; uc001ttp.1 | NP_00113737 8 (SEQ ID NO:43); NP_006691 (SEQ ID NO:44) | NM_0011439 06 (SEQ ID NO:51); NM_006700 (SEQ ID NO:52) | Hs.5148 |
| 208436_s_ at (SEQ ID NO:39) | IRF7 | interferon regulatory factor 7 | AF076494; AK303752; BC136555; ENST00000 330243; ENST00000 348655; ENST00000 397562; ENST00000 397566; ENST00000 397570; ENST00000 397574; GENSCAN0 0000065383; NM_001572; NM_004029; NM_004031; U53830; U53831; U53832; U73036; uc001lqf.1; uc001lqg.1; uc001lqh.1; uc001lqi.1 | NP_001563 (SEQ ID NO:45); NP_004020 (SEQ ID NO:46); NP_004022 (SEQ ID NO:47) | NM_001572 (SEQ ID NO:53); NM_004029 (SEQ ID NO:54); NM_004031 (SEQ ID NO:55) | Hs.166120 |

Table 7: Probe sets ID refer to the GeneChip Array "HG-U133A Array" from Affimetrix. Sequences from NCBI refer to Genome version March 2006 (NCBI Build 36.1). The genes were identified using the Gene Expression Omnibus Accession No. GSE7123 data set.

Thus, from the dynamics of the response in PBMC it can be seen that in positive responses the TLR pathway genes get activated (extra up regulation compared to non responders) resulting in the extra up regulation of the signature genes. From the static test (liver tissue of HCV patients before treatment) it can be seen that in non responders the 5 IFN signature genes are already up regulated prior to the treatment, meaning that the switch was pre-triggered to the "on" state, the genes reached their saturation level and can not increase their expression level as needed by the immune response to the interferon injection.

### EXAMPLE 8

### THE SIGNATURE GENES CAN PREDICT RESPONSE TO INTERFERON IN MULTIPLE SCLEROSIS PATIENTS

The differential expression of the 5 signature genes was validated in a data set of subjects receiving interferon I-β treatment for multiple sclerosis (MS). The microarray data set (Gene Expression Omnibus Accession No. GSE10655) was published by Baarsen et al 2008 [van Baarsen LGM, Vosslamber S, Tijssen M, Baggen JMC, van der Voort LF, et al. (2008) Pharmacogenomics of Interferon-ß Therapy in Multiple Sclerosis: Baseline IFN Signature Determines Pharmacological Differences between Patients. PLoS ONE 3(4): e1927]. Interestingly only a third of the patients respond favorably to the treatment (e.g., by decreases of symptoms such as number of relapses per year), so there is a need to predict response to treatment in the MS group of patients as well. In this case the RNA was extracted from PBMC before treatment and at some points after treatment among interferon responders and non responders. Baarsen et al., 2008, found a switch behavior for a set of genes up regulated before treatment as indication for non responders.

### Results

***The 5-signature switch genes (G1P2, G1P3, IFIT1, OAS2 and OSA3) can also predict response to interferon treatment in multiple sclerosis (MS) patients -*** The present inventor has performed a clustergram analysis on the expression of the 5 signature genes using the MS microarray data (GSE10655). As shown in Figure 10, in MS patients which do not respond to interferon (e.g., as determiend by an increase in the number of relapses per year such as patients Nos. 7 and 17 in Figure 10) the expression level of the signature genes is parallel to that of interferon non-responders among HCV patients. On the other hand, in MS patients which respond to interferon treatment (e.g., MS patients numbers 10 and 18 in Figure 10, which switched from 3 relapses per year to 0 relapses per year), the expression level of the signature genes is in parallel to that of interferon responders among the HCV patients. Thus, these results demonstrate that the same expression pattern is observed in MS patients and HCV infected patients with respect to response to interferon.

These results suggest that the same genes act as a switch for the response to interferon among patients of two unrelated diseases, i.e., HCV (type 1, 2, 3 and 4) and multiple sclerosis.

Thus, it seems that the 5 signature genes (ISG15, IFI6, IF1T1, OAS2 and OAS3) act as a general static signature switch for any interferon I treatment, with the up state (genes up regulated) prior to treatment as an indication for a probable non response to the treatment.

As described, determination of the expression level of the 5 signature genes of type 1 HCV patients prior to injection can predict responders vs. non responders. In addition, the results of the dynamic approach (i.e., determining the level of expression in a liver biopsy before and after the first interferon injection) can be used to increase the predictability power of the method.

### EXAMPLE 9

To test the involvement of natural killer inhibitory receptors in the response to interferon treatment the present inventor used the data set designated by Gene Expression Omnibus Accession No. gse11190.

Analysis of the expression pattern of genes in PBMC of HCV type 1 patients before interferon treatment (*i.e.,* naive to the treatment) revealed that the KIR2DL3, KIR3DL2, CD160, KLRG1, KIR3DL1, KIR3DL3, and KIR3DS1 are significantly down-regulated in interferon responders than in non-responders (Figure 11).

Table 8 below provides the expression levels (in arbitrary expression units) of certain probes from the KIR3DL genes (KIR3DL1, KIR3DL2, and KIR3DL3) in PBMC of type 1 HCV patients prior to interferon treatment (time 0).

**Table 8**

| Probe | Gene | NR1_15 | NR1_14 | NR1_16 | NR1_12 | RR1_9 | RR1_10 | RR1_3 |
|---|---|---|---|---|---|---|---|---|
| 211687_ x_at | KIR3DL1 | 54.34 | 99.89 | 154.84 | 45.13 | 29.58 | 15.70 | 17.87 |
| 207313_ x_at | KIR3DL2 | 141.52 | 133.38 | 162.42 | 76.66 | 26.96 | 49.95 | 21.08 |
| 207314_ x_at | KIR3DL2 | 123.76 | 178.36 | 421.33 | 97.30 | 65.26 | 54.63 | 23.61 |
| 211688_ x_at | KIR3DL2 | 84.45 | 130.23 | 173.63 | 48.05 | 21.58 | 30.67 | 19.83 |
| 216907_ x_at | KIR3DL2 | 91.87 | 135.88 | 148.08 | 81.13 | 29.06 | 41.32 | 22.43 |
| 216676_ x_at | KIR3DL3 | 159.90 | 72.64 | 180.55 | 39.88 | 26.70 | 18.44 | 14.92 |

Table 8. Provided are the expression levels (arbitrary units) of the indicated KIR3DL genes in PBMC of type 1 HCV patients prior to interferon treatment. NR1_15, NR1_14, NR1_16 and NT1_12 are non-responders to interferon; RR1_9, RR1_10 and RR1_3 are responders to interferon.

Table 9 shows the fold change between the level of expression of the KIR inhibitory genes in non-responders as compared to responders in PBMC of HCV type 1 before interferon treatment (at time 0).

**Table 9**

| ***Gene name*** | ***Ratio non***-***responders* /*responders*** | ***p***-***value*** |
|---|---|---|
| LOC730432 | 5.75848 | 0.006407 |
| KIR3DL3* | 5.65701 | 0.015599 |
| CD160* | 5.30257 | 0.004108 |
| KLRG1 | 5.17789 | 0.042744 |
| RAB8B | 5.16744 | 0.004787 |
| PLXDC1 | 5.02109 | 0.015832 |
| CLIC3 | 4.66021 | 0.001968 |
| KIR3DS1 | 4.65987 | 0.037145 |
| KIR3DL2* | 4.54073 | 0.009583 |
| KIR3DL2* | 4.28976 | 0.030791 |
| ETS1 | 4.25805 | 0.001047 |
| KIR3DL1* | 4.20611 | 0.014854 |
| KIR2DL1*/// KIR2DL2*; KIR2DL3*; KIR2DL5B*; KIR2DS1*; KIR2DS2*; KIR2DS3*; KIR2DS4*; KIR2DS5*; KIR3DL1*; KIR3DL2*; KIR3DL3*; KIR3DS1* | 4.19161 | 0.005581 |
| APH1A | 4.14032 | 0.025299 |
| BAG2 | 4.07818 | 0.013842 |
| KIR3DL2* | 3.93383 | 0.004767 |
| KLRC3 | 3.93159 | 0.04951 |
| FLJ14213 | 3.81649 | 0.019087 |
| EDG8 | 3.79551 | 0.023646 |
| SLC16A3 | 3.79164 | 0.047258 |
| KIR3DL2* | 3.69283 | 0.002271 |
| SH2D1A | 3.68506 | 0.033533 |
| SENP7 | 3.67986 | 0.012762 |
| EDG8 | 3.67212 | 0.011557 |
| CCL4 | 3.63626 | 0.01551 |
| B3GNT2 | 3.58503 | 0.006146 |
| KIR2DL3* | 3.58336 | 0.001072 |
| ZNF146 | 3.53781 | 0.020323 |
| C1orf174 | 3.51223 | 0.044396 |
| SRR | 3.50893 | 0.014816 |

Table 9. Provided are the fold changes in expression levels of the indicated genes in non-responders versus responders to interferon treatment at time 0 (i.e., before the first interferon treatment). The data used for analysis is Gene Expression Omnibus Accession No. GSE11190. Genes marked with (*) are kir inhibitors.

The results presented in Table 9 above show that in type 1 HCV PBMC there are of 5 NK inhibitory receptors KIR3DL1, KIR3DL2, KIR3DL3, KLRG1 and CD160 which are upregulated in non-responders to interferon, thus indicating a poor prognosis to the subject infected with the HCV virus. Of them, CD160 exhibits a weak homology to KIR2DL4 and shows specific association with MHC class I molecule, and KLRG1 which belongs to the Natural Killer Receptor Family (KLR).

Altogether, these results show that in PBMC of non-responders the inhibitor KIR genes are significantly up regulated in comparison to the responders. Thus, upregulation of the KIR genes in HCV patients indicates probable failure of treatment by interferon. These results can explain the inability of the non responders to benefit from the support provided by the interferon injection.

### EXAMPLE 10

### TNFRSF17 AND CXCL10 CAN BE USED IN A DYNAMIC METHOD OF PREDICTING RESPONSE TO INTERFERON

In order to provide a further understanding and enhanced prediction power, the present inventor has analyzed the Gene Expression Omnibus Accession No. gse11190 data set by splitting into 2-groups (*i.e.,* responders and non-responders), calculating log2 ratios between the expression level in PBMC obtained 4 hours following interferon treatment and the expression level in PBMC before interferon treatment.

### Results

***Prediction power of the dynamic method using TNFRSF17 and CXCL10 -*** As shown in Figures 12 and 13, using a Volcano graph analysis it is clear that the expression level of TNFRSF17, a receptor for the B cell growth factor BLyS-BAFF, by itself can predict the success (greater than 4 fold up-regulation) or failure (less than 2 fold up-regulation) of the IFN treatment.

In addition, as is further shown in Figure 12, to further enhance and increase confidence of the prediction one can determine the expression level of CXCL10 or IP-10 (Interferon -inducible cytokine IP-10), which upregulation thereof 4 hours after interferon treatment as compared to prior to interferon treatment can predict success of interferon treatment.

Thus, the dynamic signature of both TNFRSF17 as a major indicator and IP-10 as a minor indicator, added to the previous KIR genes static expression prior to treatment and can provide a close to complete certainty prediction for type 1 HCV patients, taken from their PBMC samples.

### EXAMPLE 11

### IDENTIFICATION OF GENES WHICH EXHIBIT THE MOST CORRELATED

### EXPRESSION PATTERN TO ISG15

The microarray used in the analyses described in Example 1 above, containing 14000 probes (Chen et al., 2005), revealed the exceptional consistency of ISG15 up-regulation in non responders compared to responders in type 1 HCV patients receiving pegylated IFN treatment. To identify additional genes which exhibit the most correlated expression pattern to ISG15 in liver tissues of HCV type 1 patients the present inventor analyzed the Gene Expression Omnibus Accession No. gse11190 data set (Affymetrix arrays u133 PLUS 2) which includes 47000 probe.

### Results

As shown in Tables 10-12 below, the correlation between the expression pattern of various genes in non-responders and responders was compared to that of ISG15. It was found that the expression pattern of IFI27 is highly correlated with that of ISG15.

**Table 10**

| ***Analysis of genes exhibiting a similar differential expression as ISG15 and IFI27 between responders and non-responders to interferon*** | | | | | | | | | | |
|---|---|---|---|---|---|---|---|---|---|---|
| ***Probe name*** | ***Gen name*** | ***cont1*** | ***cont2*** | ***n_15*** | ***n_16*** | ***nr_12*** | ***nr_14*** | ***r_10*** | ***r_3*** | ***r*_*9*** |
| 202411 _at | IFI27 | 1468 | 1089 | 29259 | 33957 | 26342 | 20426 | 5577 | 4610 | 789 |
| 205483 s_at | ISG15 | 842 | 775 | 22192 | 27471 | 17024 | 16703 | 2257 | 1806 | 1514 |
| 211911 x_at | HLA-B | 10661 | 8206 | 27722 | 24707 | 23653 | 22816 | 14110 | 9484 | 7138 |
| 208729 x_at | HLA-B | 7812 | 7493 | 25039 | 22816 | 18026 | 17346 | 13035 | 7078 | 6264 |
| 215313 x_at | HLA-A | 11617 | 12395 | 25427 | 23653 | 25037 | 21670 | 17399 | 9009 | 9960 |
| 209140 x_at | HLA-B | 11882 | 11742 | 25831 | 21593 | 21928 | 24707 | 14074 | 11568 | 10117 |
| 214459 x_at | HLA-C | 13247 | 8304 | 21673 | 17499 | 23184 | 17873 | 12568 | 8840 | 10641 |
| 213932 x_at | HLA-A | 9655 | 11361 | 20901 | 15883 | 18819 | 15491 | 12174 | 8508 | 9090 |
| 214478 _at | SPP2 | 8855 | 5969 | 18821 | 13627 | 16660 | 19769 | 11668 | 10096 | 7360 |
| 208812 _x_at | HLA-C; LOC73 2037 | 16610 | 9282 | 19466 | 17824 | 23357 | 18076 | 11696 | 9655 | 11775 |
| 206293 _at | SULT2 A1 | 4136 | 8292 | 17026 | 18132 | 11312 | 15666 | 12454 | 13345 | 6519 |
| 217757 _at | A2M | 11914 | 13627 | 26660 | 25424 | 21359 | 19896 | 24807 | 7744 | 13175 |
| 208980 s_at | UBC | 9938 | 10703 | 16512 | 21359 | 13549 | 13695 | 13627 | 11964 | 11173 |
| 216526 x_at | HLA-C | 10434 | 10773 | 20486 | 13344 | 15624 | 15113 | 11052 | 9534 | 9219 |
| 206292 s_at | SULT2 A1 | 5997 | 10618 | 18190 | 17973 | 11076 | 15193 | 13848 | 11775 | 9672 |
| 217933 s_at | LAP3 | 6364 | 7871 | 15842 | 16221 | 12075 | 8304 | 8746 | 7107 | 7255 |
| 211296 x_at | UBC | 14816 | 13035 | 18999 | 21438 | 16737 | 20761 | 16879 | 13280 | 12568 |
| 203153 _at | IFIT1 | 1475 | 1659 | 10685 | 17920 | 8919 | 8977 | 2374 | 2261 | 1889 |
| 214328 s_at | HSP90 AA1 | 11266 | 10272 | 14365 | 16275 | 15356 | 13549 | 12542 | 12846 | 8883 |
| 204533 _at | CXCL1 0 | 261 | 697 | 14322 | 14364 | 10272 | 7097 | 9884 | 3145 | 1833 |
| 213738 s_at | ATP5A 1 | 11853 | 14110 | 16416 | 20622 | 16082 | 19341 | 14542 | 13737 | 14699 |
| 224187 x_at | HSPA8 | 15024 | 11991 | 21595 | 19961 | 18248 | 16737 | 17697 | 18658 | 12454 |
| 209937 _at | TM4SF 4 | 7449 | 7549 | 12815 | 12937 | 13590 | 12568 | 8718 | 7838 | 10191 |
| 221891 x_at | HSPA8 | 10839 | 8780 | 18772 | 19278 | 14979 | 9906 | 14735 | 18998 | 12019 |
| 208687 x_at | HSPA8 | 12075 | 8152 | 15800 | 14320 | 14699 | 13590 | 12484 | 12783 | 11026 |
| 203382 s_at | APOE | 15883 | 12049 | 23359 | 17346 | 18366 | 14699 | 13411 | 18305 | 12938 |
| 217732 s_at | ITM2B | 13411 | 13445 | 15194 | 14857 | 18132 | 13411 | 11567 | 9549 | 10839 |
| 201553 s_at | LAMP1 | 7282 | 8584 | 13412 | 16703 | 10434 | 11076 | 11076 | 12543 | 9451 |
| 204532 x_at | UGT1A 4 | 10389 | 12484 | 17401 | 16782 | 10250 | 16660 | 9250 | 14940 | 11441 |
| 205480 _s_at | UGP2 | 13961 | 14901 | 18420 | 22443 | 17873 | 20691 | 16511 | 15955 | 16925 |

Table 10. Provided are the expression levels of the indicated genes among control subjects (contl, cont2), non-responders (n_15, n_16, nr_12, nr_14) and responders (r_10, r_3, r_9).

**Table 11**

| ***Continued analysis of genes exhibiting a similar differential expression as ISG15 and IFI27 between responders and non-responders to interferon*** | | | | | | | |
|---|---|---|---|---|---|---|---|
| ***Probe*** | ***Similarity to isg15 euc*** | ***Similarity to isg15 euc (rank)*** | ***Similarity to 1sg15 corr*** | ***Similarity to 1sg15 corr (rank)*** | ***avg_nr*** | ***avg_r*** | ***log2_nr_ r*** |
| 202411 at | 14580.22 | 4.00 | 0.99 | 3.00 | 27495.63 | 3658.46 | 2.91 |
| 205483_s at | 0.00 | 3.00 | 1.00 | 1.00 | 20847.43 | 1858.92 | 3.49 |
| 211911_x at | 22410.78 | 11.00 | 0.94 | 43.00 | 24724.51 | 10243.84 | 1.27 |
| 208729_x at | 17071.10 | 5.00 | 0.95 | 39.00 | 20806.84 | 8792.21 | 1.24 |
| 215313_x at | 26786.77 | 42.00 | 0.90 | 131.00 | 23946.58 | 12122.75 | 0.98 |
| 209140_x at | 26210.76 | 35.00 | 0.91 | 97.00 | 23514.59 | 11919.48 | 0.98 |
| 214459_x at | 24262.36 | 20.00 | 0.83 | 401.00 | 20057.34 | 10682.67 | 0.91 |
| 213932_x at | 23043.64 | 12.00 | 0.86 | 290.00 | 17773.48 | 9923.83 | 0.84 |
| 214478 at | 22253.19 | 10.00 | 0.79 | 663.00 | 17219.24 | 9708.23 | 0.83 |
| 208812_x at | 26822.54 | 44.00 | 0.76 | 934.00 | 19680.85 | 11042.10 | 0.83 |
| 206293_at | 21843.55 | 9.00 | 0.79 | 626.00 | 15533.96 | 10772.65 | 0.53 |
| 217757 at | 31947.92 | 317.00 | 0.75 | 999.00 | 23334.58 | 15242.09 | 0.61 |
| 208980_s at | 24449.96 | 22.00 | 0.88 | 185.00 | 16278.71 | 12254.79 | 0.41 |
| 216526_x at | 24400.16 | 21.00 | 0.79 | 659.00 | 16141.86 | 9935.13 | 0.70 |
| 206292_s at | 23825.51 | 17.00 | 0.79 | 670.00 | 15607.89 | 11765.17 | 0.41 |
| 217933_s at | 21116.89 | 8.00 | 0.90 | 129.00 | 13110.50 | 7702.71 | 0.77 |
| 211296_x at | 29603.65 | 126.00 | 0.88 | 206.00 | 19483.84 | 14242.53 | 0.45 |
| 203153 at | 18722.86 | 6.00 | 0.98 | 15.00 | 11625.39 | 2174.41 | 2.42 |
| 214328_s at | 26079.73 | 34.00 | 0.85 | 301.00 | 14886.25 | 11423.97 | 0.38 |
| 204533 at | 20785.14 | 7.00 | 0.86 | 278.00 | 11513.50 | 4954.22 | 1.22 |
| 213738_s at | 29228.83 | 105.00 | 0.86 | 266.00 | 18115.11 | 14326.35 | 0.34 |
| 224187_x at | 32050.92 | 334.00 | 0.71 | 1336.00 | 19135.46 | 16269.75 | 0.23 |
| 209937 at | 23895.80 | 18.00 | 0.90 | 121.00 | 12977.32 | 8915.67 | 0.54 |
| 221891_x at | 29237.70 | 106.00 | 0.50 | 5362.00 | 15733.82 | 15250.82 | 0.04 |
| 208687_x at | 26927.02 | 46.00 | 0.77 | 835.00 | 14602.18 | 12097.82 | 0.27 |
| 203382_s at | 31466.61 | 272.00 | 0.61 | 2842.00 | 18442.67 | 14884.66 | 0.31 |
| 217732_s at | 27789.18 | 63.00 | 0.67 | 1849.00 | 15398.42 | 10651.75 | 0.53 |
| 201553_s at | 25032.81 | 24.00 | 0.76 | 945.00 | 12906.13 | 11023.31 | 0.23 |

| ***Probe*** | ***Similarity to isg15 euc*** | ***Similarity to isg15 euc (rank)*** | ***Similarity to 1sg15 corr*** | ***Similarity to 1sg15 corr (rank)*** | ***avg_nr*** | ***avg_r*** | ***log2_nr_ r*** |
|---|---|---|---|---|---|---|---|
| 204532_x at | 27042.20 | 52.00 | 0.66 | 2049.00 | 15273.05 | 11876.76 | 0.36 |
| 205480_s _at | 32690.47 | 418.00 | 0.88 | 203.00 | 19856.97 | 16463.68 | 0.27 |

Table 11. Provided is continued analysis of similarity of the expression of the indicated probes/genes to ISG15. isg15 euc (using Euclidean distance measurement); Similarity to isg15 euc (euc distance ranking distance rank); Similarity to 1sg15 corr (correlation measurement to isg15); Similarity to 1sg15 corr (ranking correlation rank); avg_nr (average value of non responders); avg_r (average value of responders); log2_nr_r (log2 ratio responders to non responders);

**Table 12**

| ***Continued analysis of genes exhibiting a similar differential expression as ISG15 and IFI27 between responders and non-responders to interferon*** | | | | | | | | |
|---|---|---|---|---|---|---|---|---|
| Probe | Similarit y to Average (Edited) square | Similarit y to Average (Edited) square (rank) | Similarit y to Average (Edited) square* 2 | Similarit y to Average (Edited) square* 2 (rank) | Similarity to 211799_ x_at | Similarity to 211799_x_ at(rank) | Similarit y to IFI27 | Similarit y to IFI27(ra nk) |
| 202411_ at | 36952.5 8 | 54435.0 0 | 23456.3 6 | 159.00 | 38727.1 8 | 54514.00 | 0.00 | 1.00 |
| 205483_ s_at | 23549.6 9 | 54257.0 0 | 11358.1 3 | 3.00 | 26451.9 7 | 54403.00 | 14580.2 2 | 2.00 |
| 211911_ x_at | 36915.0 8 | 54433.0 0 | 25553.2 8 | 485.00 | 33978.0 2 | 54475.00 | 19310.9 9 | 3.00 |
| 208729_ x_at | 29190.1 8 | 54353.0 0 | 19314.8 1 | 19.00 | 26082.7 2 | 54398.00 | 19836.9 8 | 4.00 |
| 215313_ x_at | 38869.6 2 | 54460.0 0 | 28776.7 1 | 1355.00 | 34800.9 1 | 54483.00 | 24457.2 7 | 5.00 |
| 209140_ x_at | 37552.3 7 | 54440.0 0 | 27522.5 2 | 942.00 | 33601.9 1 | 54469.00 | 25139.5 1 | 6.00 |
| 214459_ x_at | 31306.5 7 | 54375.0 0 | 23040.7 5 | 133.00 | 26371.2 8 | 54402.00 | 26448.2 4 | 7.00 |
| 213932_ x_at | 27339.6 5 | 54322.0 0 | 20758.7 1 | 38.00 | 21923.7 9 | 54353.00 | 27885.5 5 | 8.00 |
| 214478_ at | 24523.0 9 | 54274.0 0 | 18138.9 8 | 16.00 | 20159.2 7 | 8643.00 | 28375.5 4 | 9.00 |
| 208812_ x_at | 32862.7 9 | 54394.0 0 | 25436.2 7 | 455.00 | 27342.7 4 | 54410.00 | 29164.6 2 | 10.00 |
| 206293_ at | 23941.7 2 | 54266.0 0 | 19687.3 7 | 24.00 | 20066.2 5 | 8218.00 | 29394.3 8 | 11.00 |
| 217757_ at | 43143.7 6 | 54494.0 0 | 34465.5 0 | 54435.0 0 | 38186.9 4 | 54506.00 | 30067.7 5 | 12.00 |
| 208980_ s_at | 28608.6 8 | 54340.0 0 | 23894.1 3 | 201.00 | 22966.7 2 | 54361.00 | 30348.4 4 | 13.00 |
| 216526_ x_at | 25736.1 8 | 54290.0 0 | 20952.5 1 | 42.00 | 19466.0 5 | 6208.00 | 30730.9 6 | 14.00 |
| 206292_ s_at | 26243.8 0 | 54303.0 0 | 22074.8 4 | 84.00 | 21094.4 2 | 28256.00 | 30796.3 0 | 15.00 |
| 217933_ s_at | 18241.2 9 | 4296.00 | 17221.7 8 | 12.00 | 12543.3 3 | 888.00 | 31194.4 3 | 16.00 |
| 211296_ x_at | 36412.4 5 | 54430.0 0 | 29699.1 3 | 1819.00 | 30625.4 6 | 54440.00 | 31888.6 4 | 17.00 |
| 203153_ at | 8799.94 | 7.00 | 13110.9 0 | 4.00 | 12069.0 3 | 786.00 | 32466.4 4 | 18.00 |
| 214328_ s_at | 26252.9 7 | 54304.0 0 | 22979.6 9 | 129.00 | 19760.3 8 | 7096.00 | 32628.4 6 | 19.00 |
| 204533_ at | 11969.0 5 | 34.00 | 15047.2 1 | 5.00 | 11585.2 2 | 693.00 | 32654.1 0 | 20.00 |
| 213738_ s_at | 34336.5 0 | 54411.0 0 | 28540.2 1 | 1255.00 | 28708.8 1 | 54423.00 | 32920.8 7 | 21.00 |
| 224187_ x_at | 38361.1 5 | 54450.0 0 | 32152.7 0 | 4241.00 | 32084.1 7 | 54454.00 | 33416.6 1 | 22.00 |
| 209937_ at | 18879.1 2 | 6303.00 | 18046.0 3 | 14.00 | 12778.1 3 | 944.00 | 33507.8 4 | 23.00 |
| 221891_ x_at | 32407.0 7 | 54389.0 0 | 28549.6 3 | 1258.00 | 26184.3 9 | 54399.00 | 33603.2 4 | 24.00 |
| 208687_ x_at | 26359.6 8 | 54307.0 0 | 23394.3 9 | 154.00 | 19425.6 9 | 6104.00 | 33626.3 0 | 25.00 |
| 203382_ s_at | 36695.1 3 | 54431.0 0 | 30907.5 3 | 2670.00 | 30108.0 3 | 54436.00 | 33638.5 4 | 26.00 |
| 217732_ s_at | 28051.8 7 | 54331.0 0 | 24413.9 1 | 284.00 | 21050.0 7 | 20970.00 | 33701.8 4 | 27.00 |
| 201553_ s_at | 22667.6 6 | 54239.0 0 | 21696.9 7 | 64.00 | 16688.7 7 | 2525.00 | 33874.3 6 | 28.00 |
| 204532_ x_at | 28327.0 5 | 54334.0 0 | 24835.4 0 | 359.00 | 22453.4 4 | 54356.00 | 33938.1 5 | 29.00 |
| 205480_ s_at | 39619.0 5 | 54466.0 0 | 33024.6 1 | 6547.00 | 33908.8 7 | 54472.00 | 34312.2 5 | 30.00 |

Table 12. Continued analysis of similarity of the indicated probes/genes to ISG15. Similarity to Average (edited signal 250 250 1000 1000 1000 1000 500 500 500 euclidean distance) square; Similarity to Average (Edited) square (ranking of previous measured distance ); Similarity to Average (Edited) square*² (edited signal 700 700 17000 17000 17000 17000 1500 1500 1500); Similarity to Average (Edited) square*² (ranking of the previous data); Similarity to 211799_x_at (Euclidean distance tO HLA-C); Similarity to 211799_x_at (ranking of the former); Similarity to IFI27 = (oclidean distance to the IFI27 profile); Similarity to IFI27(rank) (ranking of the previous);

As shown in Tables 10-12 above and Figure 14, the expression pattern of the HLA family of genes is similar to that of ISG15 in liver tissues of HCV type 1 patients. In addition, as shown in Figure 15, in the liver of HCV type 1 patients the level of HLA-B, HLA-F, HLA-C and HLA-G before any injection of interferon is significantly upregulated (at least 2 fold change) in non-responders as compared to interferon responders. In parallel and even with a stronger up-regulation, the former described switch genes (Example 1) show similar behavior in non responders using the gse11190 data set. Thus, as shown in Figure 16 the level of ISG15, IFIT1, USP18, OAS2, OAS3, and HERC6 in PBMC before any injection of interferon is significantly upregulated (at least 4.6 fold change) in non-responders HCV type 1 patients as compared to responders.

As described in Example 9 above, in PBMC of non responders type 1 HCV patients there is a significant up-regulation of the inhibitor KIR genes. Thus, one of the major actions of the HCV virus is deciphered here: In the liver tissue the virus succeeds in activating the HLA genes (HLA-A, HLA-B, HLA-C) which results in the appearance of their complementary inhibitory KIR (e.g., KIR3D, KIR2D) in the PBMC of these patients.

### EXAMPLE 12

### MATCHING EXPRESSION PATTERN OF HLA GENES IN LIVER AND KIR GENES IN BLOOD OF NON-RESPONDER HCV TYPES 1-4 PATIENTS

Further analysis of the upregulated genes in various biological pathways (using the ontoexpress software (Intelligent Systems and Bioinformatics Laboratory, Computer Science Department, Wayne State University) revealed that the most statistically significant pathway for both expression in liver and blood of non-responders versus responder is the natural killer cell mediated cytotoxicity pathway as shown in Figures 18 and 19. These analyses show that while the MHC class 1 genes are up-regulated in the target cells (liver) of non-responders, there is a matching upregulation of the KIR inhibitory genes in the PBMC of non-responders, resulting in inhibition of NK cells and preventing their action against the liver cells hosting the HCV virus.

***Coordinated upregulation of HLA genes in liver and kir genes in blood of non-responders HCV type 1 patients at time 0 (naïve to interferon) -*** Figures 20A-B, 21A-B and 22A-B demonstrate the expression of various HLA genes in liver tissues and of their matching kir genes in the blood of HCV type 1 patients. Hence in non responders to the IFN treatment the liver tissue shows up regulation of the HLA (MHC CLASS 1) genes while at the same time their corresponding inhibitory KIR genes are up regulated compared to the responders.

***The kir genes are upregulated in blood samples of non-responders HCV types 2-4 patients at time 0 (naïve to interferon) -*** As shown in Figure 25, in HCV types 2-4 patients the same kir genes (e.g., KIR2DL5A, KIR2DL5B, KIR2DL3, KIR3DL1, KIR2DL1, KIR2DL2, KIR3DL3) are upregulated in PBMC of non-responders as compared to responders, similar to the profile in blood of HCV type 1 non-responders. In addition, in liver tissues of non-responders HCV types 2-4, the same ISG15 pathway is activated in non-responders as compared to responders (Figure 26), similar to the liver profile of HCV type 1 non-responders.

### EXAMPLE 13

### IDENTIFICATION OF A COMMON PROMOTER TO THE GENES WHICH ARE UP-REGULATED IN NON-RESPONDERS TO INTERFERON TREATMENT

The present inventor has surprisingly uncovered that the genes which are upregulated in non-responders to interferon (e.g., HLA-A, HLA-B, HLA-C, HLA-F, ISG15, IFI27, IFIT1, IFI6, OAS2, and OAS3) have the same promoter ISRE in the near 300 bp upstream region of the gene (Figure 23). Tables 13 and 14 depict the frequency score [determined toucan workbench for regulatory sequence analysis; Hypertext Transfer Protocol://homes (dot) esat (dot) kuleuven (dot) be/∼saerts/software/toucan (dot) php] of promoters in the 300 bp (Table 14) and 2000 bp (Table 15) upstream region of the analyzed genes which are upregulated in liver of HCV type 1 non-responders. It should be noted that higher scores indicate higher probabilities of the promoter being active for these genes.

**Table 14**

| | |
|---|---|
| ***Promoters in the 300 bp upstream region*** | |

| ***Promoter name*** | ***Score*** |
|---|---|
| M00302-V$NFAT_Q6 | 0.001097804 |
| M00258-V$ISRE_01 | 0.001097804 |
| M00062-V$IRF1_01 | 9.98E-04 |
| M00453-V$IRF7_01 | 9.98E-04 |
| M00063-V$IRF2_01 | 7.98E-04 |
| M00138-V$OCT1_04 | 6.99E-04 |
| M00148-V$SRY_01 | 6.99E-04 |
| M00380-V$PAX4_04 | 6.99E-04 |
| M00083-V$MZF1_01 | 5.99E-04 |
| M00141-V$LYF1_01 | 5.99E-04 |
| M00208-V$NFKB_C | 5.99E-04 |
| M00456-V$FAC1_01 | 5.99E-04 |
| M00194-V$NFKB Q6 | 4.99E-04 |
| M00471-V$TBP_01 | 4.99E-04 |
| M00486-V$PAX2_02 | 4.99E-04 |
| M00145-V$BRN2_01 | 4.99E-04 |
| M00054-V$NFKAPPAB_01 | 4.99E-04 |
| M00051-V$NFKAPPAB50_01 | 4.99E-04 |
| M00130-V$FOXD3_01 | 4.99E-04 |

Table 14: Provided are the promoters found in the 300 bp upstream sequence of the analyzed genes (upregulated in liver tissue of HCV type 1 non-responders) along with the frequency score of each promoter.

**Table 15**

| ***Promoters in the 2000 bp upstream region*** | |
|---|---|
| ***Promoter name*** | ***Score*** |
| M00138-V$OCT1_04 | 4.54E-04 |
| M00130-V$FOXD3_01 | 4.32E-04 |
| M00258-V$ISRE_01 | 3.86E-04 |
| M00096-V$PBX1_01 | 3.86E-04 |
| M00453-V$IRF7 01 | 3.86E-04 |
| M00380-V$PAX4_04 | 3.86E-04 |
| M00081-V$EVI1_04 | 3.63E-04 |
| M00456-V$FAC1_01 | 3.63E-04 |
| M00131-V$HNF3B_01 | 3.41E-04 |
| M00160-V$SRY_02 | 3.41E-04 |
| M00116-V$CEBPA_01 | 3.41E-04 |
| M00471-V$TBP_01 | 3.18E-04 |
| M00268-V$XFD2_01 | 3.18E-04 |
| FivePrimeUTR | 2.95E-04 |
| M00216-V$TATA_C | 2.95E-04 |
| M00302-V$NFAT_Q6 | 2.95E-04 |
| M00377-V$PAX4_02 | 2.95E-04 |
| exon | 2.95E-04 |
| M00472-V$FOXO4_01 | 2.95E-04 |
| M00473-V$FOXO1_01 | 2.73E-04 |
| M00289-V$HFH3_01 | 2.73E-04 |

Table 15: Provided are the promoters found in the 2000 bp upstream sequence of the analyzed genes (upregulated in liver tissue of HCV type 1 non-responders) along with the frequency score of each promoter.

Table 16 provides sequence information of identified genes according to some embodiments of the invention.

**Table 16**

| ***Probe Set ID*** | ***Gene Symbol*** | ***Gene Title*** | ***RefSeq Protein ID*** | ***RefSeq Transcript ID*** |
|---|---|---|---|---|
| 202411_at (SEQ ID NO:56) | IFI27 | interferon alpha-inducible protein 27 | NP_001123552 (SEQ_ID NO:91); NP_005523 (SEQ ID NO:164) | NM_001130080 (SEQ ID NO:123); NM_005532 (SEQ ID NO:124) |
| 204533_at (SEQ ID NO:57) | CXCL10 | chemokine (C-X-C motif) ligand 10 | NP_001556 (SEQ ID NO:92) | NM_001565 (SEQ ID NO:125) |
| 206641_at (SEQ ID NO:58) | TNFRSF17 | tumor necrosis factor receptor superfamily, member 17 | NP_001183 (SEQ ID NO:93) | NM_001192 (SEQ ID NO:126) |
| 207313_x _at (SEQ ID NO:59) | KIR3DL2; LOC72778 7 | killer cell immunoglobulin-like receptor, three domains, long cytoplasmic tail, 2; | NP_006728 (SEQ ID NO:94); XP_001718677 (SEQ ID NO:95) | NM_006737 (SEQ ID NO:127); XM_001718625 (SEQ ID NO:128) |
| 207314_x _at (SEQ ID NO:60) | KIR3DL2; LOC72778 7 | killer cell immunoglobulin-like receptor, three domains, long cytoplasmic tail, 2; | NP_006728 (SEQ ID NO:94); XP_001718677 (SEQ ID NO:95) | NM_006737 (SEQ ID NO:127); XM_001718625 (SEQ ID NO:128) |
| 211688_x _at (SEQ ID NO:61) | KIR3DL2; LOC72778 7 | killer cell immunoglobulin-like receptor, three domains, long cytoplasmic tail, 2; | NP_006728 (SEQ ID NO:94); XP_001718677 (SEQ ID NO:95) | NM_006737 (SEQ ID NO:127); XM_001718625 (SEQ ID NO:128) |
| 216907_x _at (SEQ ID NO:62) | KIR3DL2; LOC72778 7 | killer cell immunoglobulin-like receptor, three domains, long cytoplasmic tail, 2; | NP_006728 (SEQ ID NO:94); XP_001718677 (SEQ ID NO:95) | NM_006737 (SEQ ID NO:127); XM_001718625 (SEQ ID NO:128) |
| 211397_x _at (SEQ ID NO:63) | KIR2DL2 | killer cell immunoglobulin-like receptor, two domains, long cytoplasmic tail, 2 | NP_055034 (SEQ ID NO:96) | NM_014219 (SEQ ID NO:129) |
| 207840_at (SEQ ID NO:64) | CD160 | CD160 molecule | NP_008984 (SEQ ID NO:97) | NM_007053 (SEQ ID NO:130) |
| 208179_x _at (SEQ ID NO:65) | KIR2DL3 | killer cell immunoglobulin-like receptor, two domains, long cytoplasmic tail, 3 | NP_055326 (SEQ ID NO:98); NP_056952 (SEQ ID NO:99) | NM_014511 (SEQ ID NO:131); NM_015868 (SEQ ID NO:132) |
| 208426_x _at (SEQ ID NO:66) | KIR2DL3; KIR2DL4; KIR2DL5A | killer cell immunoglobulin-like receptor, two domains, long cytoplasmic tail, 3 (KIR2DL3); killer cell immunoglobulin-like receptor, two domains, long cytoplasmic tail, 4 (KIR2DL4); killer cell immunoglobulin-like receptor, two domains, long cytoplasmic tail, 5A (KIR2DL5A) | NP_001074239 (SEQ ID NO:100); NP_001074241 (SEQ ID NO:101); NP_002246 (SEQ ID NO:102); NP_055326 (SEQ ID NO:98); NP_056952 (SEQ ID NO:99); NP_065396 (SEQ ID NO:105) | NM_001080770 (SEQ ID NO:133); NM_001080772 (SEQ ID NO:134); NM_002255 (SEQ ID NO:135); NM_014511 (SEQ ID NO:131); NM_015868 (SEQ ID NO:132); NM_020535 (SEQ ID NO:136) |
| 208650_s_ at (SEQ ID NO:67) | CD24 | CD24 molecule | NP_037362 (SEQ ID NO:106) | NM_013230 (SEQ ID NO:137) |
| 208651_x _at (SEQ ID NO:68) | CD24 | CD24 molecule | NP_037362 (SEQ ID NO:106) | NM_013230 (SEQ ID NO:137) |
| 216379_x _at (SEQ ID NO:69) | CD24 | CD24 molecule | NP_037362 (SEQ ID NO:106) | NM_013230 (SEQ ID NO:137) |
| 209771_x_at (SEQ ID NO:70) | CD24 | CD24 molecule | NP_037362 (SEQ ID NO:106) | NM_013230 (SEQ ID NO:137) |
| 209772_s_ at (SEQ ID NO:71) | CD24 | CD24 molecule | NP_037362 (SEQ ID NO:106) | NM_013230 (SEQ ID NO:137) |
| 266_s_at (SEQ ID NO:72) | CD24 | CD24 molecule | NP_037362 (SEQ ID NO:106) | NM_013230 (SEQ ID NO:137) |
| 208729_x _at (SEQ ID NO:73) | HLA-B | major histocompatibility complex, class I, B | NP_005505 (SEQ ID NO:107) | NM_005514 (SEQ ID NO:138) |
| 209140_x _at (SEQ ID NO:74) | HLA-B | major histocompatibility complex, class I, B | NP_005505 (SEQ ID NO:107) | NM_005514 (SEQ ID NO:138) |
| 211911_x _at (SEQ ID NO:75) | HLA-B | major histocompatibility complex, class I, B | NP_005505 (SEQ ID NO:107) | NM_005514 (SEQ ID NO:138) |
| 208812_x _at (SEQ ID NO:76) | HLA-C | major histocompatibility complex, class I, C | NP_002108 (SEQ ID NO:108) | NM_002117 (SEQ ID NO:139) |
| 211146_at (SEQ ID NO:77) | HLA-C | Major histocompatibility complex, class I, C | NP_002108 (SEQ ID NO:108) | NM_002117 (SEQ ID NO:139) |
| 211799_x _at (SEQ ID NO:78) | HLA-C | major histocompatibility complex, class I, C | NP_002108 (SEQ ID NO:108) | NM_002117 (SEQ ID NO:139) |
| 214459_x _at (SEQ ID NO:79) | HLA-C | major histocompatibility complex, class I, C | NP_002108 (SEQ ID NO: 108) | NM_002117 (SEQ ID NO:139) |
| 216526_x _at (SEQ ID NO:80) | HLA-C | major histocompatibility complex, class I, C | NP_002108 (SEQ ID NO: 108) | NM_002117 (SEQ ID NO:139 same as above) |
| 210288_at (SEQ ID NO:81) | KLRG1 | killer cell lectin-like receptor subfamily G, member 1 | NP_005801 (SEQ ID NO:109) | NM_005810 (SEQ ID NO:140) |
| 210514_x _at (SEQ ID NO:82) | HLA-G | major histocompatibility complex, class I, G | NP_002118 (SEQ ID NO:110) | NM_002127 (SEQ ID NO:141) |
| 211528_x _at (SEQ ID NO:83) | HLA-G | major histocompatibility complex, class I, G | NP_002118 (SEQ ID NO: 110) | NM_002127 (SEQ ID NO:141) |
| 211529_x _at (SEQ ID NO:84) | HLA-G | major histocompatibility complex, class I, G | NP_002118 (SEQ ID NO: 110) | NM_002127 (SEQ ID NO:141) |
| _at (SEQ ID NO:85) | HLA-G | major histocompatibility complex, class I, G | NP_002118 (SEQ ID NO: 110) | NM_002127 (SEQ ID NO:141) |
| 211389_x _at (SEQ ID NO:86) | KIR3DS1 | killer cell immunoglobulin-like receptor, three domains, short cytoplasmic tail, 1 | NP_001077008 (SEQ ID NO:111) | NM_001083539 (SEQ ID NO:142) |
| 211687_x _at (SEQ ID NO:87) | KIR3DL1 | killer cell immunoglobulin-like receptor, three domains, long cytoplasmic tail, 1 | NP_037421 (SEQ ID NO:145) | NM_013289 (SEQ ID NO:143) |
| 213932_x _at (SEQ ID NO:88) | HLA-A | major histocompatibility complex, class I, A | NP_002107 (SEQ ID NO:112) | NM_002116 (SEQ ID NO:144) |
| 215313_x _at (SEQ ID NO:89) | HLA-A | major histocompatibility complex, class I, A | NP_002107 (SEQ ID NO:112) | NM_002116 (SEQ ID NO:144) |
| 216676_x _at (SEQ ID NO:90) | KIR3DL3 | killer cell immunoglobulin-like receptor, three domains, long cytoplasmic tail, 3 | NP_703144 (SEQ ID NO:113) | NM_153443 (SEQ ID NO:146) |
| 217318_x _at (SEQ ID NO:160) | KIR2DL1; KIR2DL2; KIR2DL3; KIR2DL5A ; KIR2DL5B KIR2DS1; KIR2DS2; KIR2DS3; KIR2DS4; KIR2DS5; KIR3DL2; KIR3DL3; KIR3DP1; LOC72778 7 | killer cell immunoglobulin-like receptor, two domains, long cytoplasmic tail, 1 (KIR2DL1); killer cell immunoglobulin-like receptor, two domains, long cytoplasmic tail, 2 (KIR2DL2); killer cell immunoglobulin-like receptor, two domains, long cytoplasmic tail, 3 (KIR2DL3); killer cell immunoglobulin-like receptor, two domains, long cytoplasmic tail, 5A (KIR2DL5A); killer cell immunoglobulin-like receptor, two domains, long cytoplasmic tail, 5B (KIR2DL5B); killer cell immunoglobulin-like receptor, two domains, short cytoplasmic tail, 1 (KIR2DS1); killer cell immunoglobulin-like receptor, two domains, short cytoplasmic tail, 2 (KIR2DS2); killer cell immunoglobulin-like receptor, two domains, short cytoplasmic tail, 3 (KIR2DS3); killer cell immunoglobulin-like receptor, two domains, short cytoplasmic tail, 4 (KIR2DS4); killer cell immunoglobulin-like receptor, two domains, short cytoplasmic tail, 5 (KIR2DS5); killer cell immunoglobulin-like receptor, three domains, long cytoplasmic tail, 2 (KIR3DL2); killer cell immunoglobulin-like receptor, three domains, | NP_001015070 (SEQ ID NO:114); NP_001018091 (SEQ ID NO:148); NP_006728 (SEQ ID NO:94); NP_036444 (SEQ ID NO:115); NP_036445 (SEQ ID NO:116); NP_036446 (SEQ ID NO:117); NP_055033 (SEQ ID NO:118); NP_055034 (SEQ ID NO:96); NP_055326 (SEQ ID NO:98); NP_055327 (SEQ ID NO:119); NP_055328 (SEQ ID NO:103); NP_056952 (SEQ ID NO:99); NP_065396 (SEQ ID NO:105); NP_703144 (SEQ ID NO:113); XP_001718677 (SEQ ID NO:95); XP_002346955 (SEQ ID NO:104) | NM_001015070 (SEQ ID NO:147); NM_001018081 (SEQ ID NO:149); NM_006737 (SEQ ID NO:127); NM_012312 (SEQ ID NO:150); NM_012313 (SEQ ID NO:151); NM_012314 (SEQ ID NO:152); NM_014218 (SEQ ID NO:153); NM_014219 (SEQ ID NO:129); NM_014511 (SEQ ID NO:131); NM_014512 (SEQ ID NO:154); NM_014513 (SEQ ID NO:155); NM_015868 (SEQ ID NO:132); NM_020535 (SEQ ID NO:136); NM_153443 (SEQ ID NO:146); XM_001718625 (SEQ ID NO:128); XM_002346914 (SEQ ID NO:156) |
| | | long cytoplasmic tail, 3 (KIR3DL3); killer-cell Ig-like receptor; similar to killer cell immunoglobulin-like receptor 3DL2 precursor (MHC class I NK cell receptor) (KIR3DP1) (Natural killer-associated transcript 4) (NKAT-4) (p70 natural killer cell receptor clone CL-5) (CD158k antigen) | | |
| 221875_x _at (SEQ ID NO:161) | HLA-F | major histocompatibility complex, class I, F | NP_001091948 (SEQ ID NO:120); NP_001091949 (SEQ ID NO:121); NP_061823 (SEQ ID NO:122) | NM_001098478 (SEQ ID NO:157); NM_001098479 (SEQ ID NO:158); NM_018950 (SEQ ID NO:159) |
| 221978_at (SEQ ID NO:162) | HLA-F | major histocompatibility complex, class I, F | NP_001091948 (SEQ ID NO:120 same as above); NP_001091949 (SEQ ID NO:121 same as above); NP_061823 (SEQ ID NO:122 same as above) | NM_001098478 (SEQ ID NO:157); NM_001098479 (SEQ ID NO:158); NM_018950 (SEQ ID NO:159) |
| 204806_x _at (SEQ ID NO:163) | HLA-F | major histocompatibility complex, class I, F | NP_001091948 (SEQ ID NO:120); NP_001091949 (SEQ ID NO:121); NP_061823 (SEQ ID NO:122) | NM_001098478 (SEQ ID NO:157); NM_001098479 (SEQ ID NO:158); NM_018950 (SEQ ID NO:159) |

Table 16.

### EXAMPLE 14

### ANTIBODIES AGAINST KIR RECEPTORS ON NATURAL KILLER CELLS FOR CONVERSION OF NON RESPONDERS TO RESPONDERS OF INTERFERON

The present inventor has uncovered that a potential solution for interferon non responders can be using a monoclonal antibody directed against KIR. For example, the 1-7F9, a human monoclonal antibody targeting KIRs on NK cells (Romagne F., et al., 2009, Blood 114:2667-2677, "Preclinical characterization of 1-7F9, a novel human anti-KIR receptor therapeutic antibody that augments natural killer-mediated killing of tumor cells"). This antibody activates NK cells by blocking the interaction between inhibitory KIRs and target cell HLA class I molecules.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

### REFERENCES

### (Additional references are cited in text)

Ahmad A and Alvarez F. 2004 (J. of Leukocyte Biology, 76:743-759)
Chen Limin, Borozan Ivan, Feld Jordan, Sun Jing, Tannis Laura-Lee, Coltescu Catalina, Heathcote Jenny, Edwards Aled M, And Mcgilvray Ian D. Hepatic Gene Expression Discriminates Responders and Nonresponders in Treatment of Chronic Hepatitis C Viral Infection. Gastroenterology 2005, 128:1437-1444.
Giannini C, et al., 2008, Blood. 112:4353-4. "Can BAFF promoter polymorphism be a predisposing condition for HCV-related mixed cryoglobulinemia?"
Gonzalez S, Castanotto D, Li H, Olivares S, Jensen MC, Forman SJ, Rossi JJ, Cooper LJ. 2005, Molecular Therapy Vol. 11: 811-8. Amplification of RNAi-Targeting HLA mRNAs.
Khakoo SI., Thio CL., et al., 2004. Science 305:872-874. HLA and NK cell inhibitory receptor genes in resolving hepatitis C virus infection.
Kenya Honda, Akinori Takaoka, and Tadatsugu Taniguchi. Type I Inteferon Gene Induction by the Interferon Regulatory Factor ,Family of Transcription Factors. Immunity 25, 349-360, 2006, Review.
Lopez- Vazquez et al., 2005 (JID 192: 162-165)
Magdalena Sarasin-Filipowicz*, Edward J. Oakeley†, Francois H. T. Duong*, Verena Christen*, Luigi Terracciano‡, Witold Filipowicz†, and Markus H. Heim. Chronic hepatitis C. PNAS _ May 13, 2008 _ vol. 105 _ no. 19:7034-7039.
Paladino N., et al., 2007 (Tissue Antigens 69 Suppl 1:109-111)
Parham P, 2004 (Science 305:786-787)
Parham P, 2005 (Nature Reviews, Immunology 5:201-214)
Querec TD, Akondy RS, Lee EK, Cao W, Nakaya HI, Teuwen D, Pirani A, Gernert K, Deng J, Marzolf B, Kennedy K, Wu H, Bennouna S, Oluoch H, Miller J, Vencio RZ, Mulligan M, Aderem A, Ahmed R, Pulendran B. Nat Immunol. 2009, 10:116-25
Rajagopalan S, and Long EO. 2005. JEM 201:1025-1029. Understanding how combination of HLA and KIR genes influence disease.
Rauch A., et al. 2007 (Tissue Antigens 69 Suppl 1:237-40)
Sarasin-Filipowicz Magdalena, Oakeley Edward J., Duong Francois H. T., Christen Verena, et al. Interferon signaling and treatment outcome in chronic hepatitis C. PNAS May 13, 2008, vol. 105 no. 19, Pages 7034-7039.
Shah N., et al., 2009, Blood 114:2567-2568
Stein Aerts, Peter Van Loo, Gert Thijs, Herbert Mayer, Rainer de Martin, Yves Moreau and Bart De Moor (2005) "TOUCAN 2: the all-inclusive open source workbench for regulatory sequence analysis". Nucl Acids Res, vol. 33 (Web Server issue), W393-6.
Tarantino G. et al., 2008, SERUM BLYS/BAFF LEVELS IN ACUTE HEPATITIS C PREDICT CLINICAL OUTCOME. Digestive and Liver Disease 40:A1-A40)
Taylor, Milton W., Takuma Tsukahara, Leonid Brodsky, Joel Schaley, Corneliu Sanda, Matthew J. Stephens, Jeanette N. McClintick, Howard J. Edenberg, Lang Li, John E. Tavis, Charles Howell, and Steven H. Belle. Changes in Gene Expression during Pegylated Interferon and Ribavirin Therapy of Chronic Hepatitis C Virus Distinguish Responders from Nonresponders to Antiviral Therapy. Journal Of Virology, Apr. 2007, p. 3391-3401.
van Baarsen LGM, Vosslamber S, Tijssen M, Baggen JMC, van der Voort LF, et al. (2008) Pharmacogenomics of Interferon-ß Therapy in Multiple Sclerosis:Baseline IFN Signature Determines Pharmacological Differences between Patients. PLoS ONE 3(4): e1927. doi:10.1371/journal.pone.0001927
Vidal-Casrineira JR., et al., 2009, JVI online 21 October 2009. Effect of killer immunoglobulin-like receptors (KIR) in the response to combined treatment in patients with chronic hepatitis C.
Vitale M., et al., Int Immunol. 2004 Oct;16(10):1459-66.
Zeremski M, et al., 2007 (J Acquir Immune Defic Syndr. 2007 Jul 1;45(3):262-8)
Zuniga J., et al., 2009 (Molecular Immunology 46:2723-2727)

### FURTHER NUMBERED ASPECTS OF THE INVENTION

The following represent further numbered aspects of the invention, but do not at present form claims.
(1) A method of predicting responsiveness of a subject to interferon treatment, comprising comparing a level of expression in a cell of the subject of at least one gene selected from the group consisting KIR3DL3, KIR3DL2, KIR3DL1, KIR2DL1, KIR2DL2, KIR2DL3, KLRG1, KIR3DS1, CD160, HLA-A, HLA-B, HLA-C, HLA-F, HLA-G and IFI27 to a reference expression data of said at least one gene obtained from at least one interferon responder subject and/or at least one interferon non-responder subject, thereby predicting the responsiveness of the subject to interferon treatment.
(2) A method of predicting responsiveness of a subject to interferon treatment, comprising comparing a ratio determined between an expression level of TNFRSF17 gene in a cell of the subject following interferon treatment and an expression level of said gene in said cell of the subject prior to interferon treatment, or visa versa, to a reference ratio determined in a cell of at least one interferon responder subject and/or at least one interferon non-responder subject, said reference ratio is determined between an expression level of said gene following interferon treatment and an expression level of said gene prior to interferon treatment, or visa versa, thereby predicting the responsiveness to interferon treatment of a subject.
(3) A method of predicting responsiveness to interferon treatment of a subject diagnosed with multiple sclerosis or infected with HCV type 2, 3 or 4, comprising comparing a level of expression in a cell of the subject of IFI6, OAS2, ISG15, OAS3 and IFIT1 genes to a reference expression data of said genes obtained from at least one interferon responder subject and/or at least one interferon non-responder subject, thereby predicting the responsiveness of the subject to interferon treatment.
(4) A method of predicting responsiveness of a subject to interferon treatment, comprising comparing a ratio determined between an expression level of ISG15, IFI6, IFIT1, OAS2 and OAS3 genes in a cell of the subject following interferon treatment and an expression level of said genes in said cell of the subject prior to interferon treatment, or visa versa, to a reference ratio determined in a cell of at least one interferon responder subject and/or at least one interferon non-responder subject, said reference ratio is determined between an expression level of said genes following interferon treatment and an expression level of said genes prior to interferon treatment, or visa versa, thereby predicting the responsiveness to interferon treatment of a subject.
(5) A method of predicting responsiveness of a subject to interferon treatment, comprising comparing a ratio determined between an expression level of at least one gene selected from the group consisting of: TICAM1, MYD88, TLR7, TRAFD1 and IRF7 in a cell of the subject following interferon treatment and an expression level of said at least one gene in said cell of the subject prior to interferon treatment, or visa versa, to a reference ratio determined in a cell of at least one interferon responder subject and/or at least one interferon non-responder subject, said reference ratio is determined between an expression level of said gene following interferon treatment and an expression level of said gene prior to interferon treatment, or visa versa, thereby predicting the responsiveness to interferon treatment of a subject.
(6) A method of predicting responsiveness of a subject to interferon treatment, comprising comparing a ratio determined between an expression level of at least one gene selected from the group consisting of HERC5 and UBE2L6 in a liver cell of the subject following interferon treatment and an expression level of said at least one gene in said liver cell of the subject prior to interferon treatment, or visa versa, to a reference ratio determined in a cell of at least one interferon responder subject and/or at least one interferon non-responder subject, said reference ratio is determined between an expression level of said at least one gene following interferon treatment and an expression level of said at least one gene prior to interferon treatment, or visa versa, thereby predicting the responsiveness to interferon treatment of a subject.
(7) A method of predicting responsiveness of a subject to interferon treatment, comprising comparing a ratio determined between an expression level of at least one gene selected from the group consisting ISG15, IFI6, IFIT1, OAS2 and OAS3 in a liver cell of the subject following interferon treatment and an expression level of said at least one gene in said liver cell of the subject prior to interferon treatment, or visa versa, to a reference ratio determined in a liver cell of at least one interferon responder subject and/or at least one interferon non-responder subject, said reference ratio is determined between an expression level of said at least one gene following interferon treatment and an expression level of said at least one gene prior to interferon treatment, or visa versa, thereby predicting the responsiveness to interferon treatment of a subject.
(8) A method of treating of a subject in need of interferon treatment, the method comprising:
   (a) predicting the responsiveness of the subject to interferon treatment according to the method of any of (1)-(7), and
   (b) selecting a treatment regimen based on said responsiveness;
   thereby treating the subject in need of interferon treatment.
(9) A method of treating a subject in need of interferon therapy, comprising co-administering to the subject interferon and an agent capable of downregulating HLA or KIR inhibitory receptor, thereby treating the subject in need of interferon therapy.
(10) The method of (1) or (3), wherein a decrease above a predetermined threshold in said level of expression of said at least one gene in said cell of the subject relative to said reference expression data of said at least one gene obtained from said at least one interferon non-responder subject predicts responsiveness of the subject to interferon treatment of the subject.
(11) The method of (1) or (3), wherein an increase above a predetermined threshold in said level of expression of said at least one gene in said cell of the subject relative to said reference expression data of said at least one gene obtained from said at least one interferon responder subject predicts lack of responsiveness of the subject to interferon treatment of the subject.
(12) The method of (1) or (3), wherein when a level of expression of said at least one gene in said cell of the subject is identical or changed below a predetermined threshold as compared to said reference expression data of said at least one gene obtained from said at least one interferon responder subject, then the subject is classified as responsive to interferon.
(13) The method of (1) or (3), wherein when a level of expression of said at least one gene in said cell of the subject is identical or changed below a predetermined threshold as compared to said reference expression data of said at least one gene obtained from said at least one interferon non-responder subject, then the subject is classified as a non-responsive to interferon.
(14) The method of (2), (4), (5), (6) or (7), wherein an increase above a predetermined threshold in said ratio of the subject relative to said reference ratio of said at least one interferon non-responder subject predicts responsiveness of the subject to interferon treatment of the subject.
(15) The method of (2), (4), (5), (6) or (7),, wherein a decrease above a predetermined threshold in said ratio of the subject relative to said reference ratio of said at least one interferon responder subject predicts lack of responsiveness of the subject to interferon treatment of the subject.
(16) The method of (2), (4), (5), (6) or (7),, wherein when said ratio of the subject is identical or changed below a predetermined threshold as compared to said reference ratio of said at least one interferon responder subject, then the subject is classified as responsive to interferon.
(17) The method of (2), (4), (5), (6) or (7), wherein when said ratio of the subject is identical or changed below a predetermined threshold as compared to said reference ratio of said at least one interferon non-responder subject, then the subject is classified as non-responsive to interferon.
(18) The method of (1) or (3), wherein said level of expression is determined prior to interferon treatment.
(19) The method of any of (1), (2), (3), (4), (5) and (8), wherein said cell is a blood cell.
(20) The method of any of (1), (2), (3), (4), (5) and (8), wherein said cell is a liver cell.
(21) The method of any of (2), (4), (5), (6), (7) and (8), wherein said following interferon treatment is effected about 4 hours after interferon treatment.
(22) The method of any of (2), (4), (5), (6), (7) and (8), wherein said following interferon treatment is effected about 24 hours after interferon treatment.
(23) The method of (1), (2), (4), (5), (6), (7), (8) or (9), wherein said subject is diagnosed with HCV infection type 1, 2, 3 or 4.
(24) The method of (2), further comprising comparing a ratio determined between an expression level of at least one gene selected from the group consisting of CXCL10 and CD24 in a cell of the subject following interferon treatment and an expression level of said gene in said cell of the subject prior to interferon treatment, or visa versa, to a reference ratio determined in a cell of at least one interferon responder subject and/or at least one interferon non-responder subject, said reference ratio is determined between an expression level of said at least one gene following interferon treatment and an expression level of said at least one gene prior to interferon treatment, or visa versa, thereby predicting the responsiveness to interferon treatment of a subject.
(25) The method of (3), wherein when said subject is diagnosed with multiple sclerosis said cell of the subject is a blood cell.
(26) The method of (3), wherein when said subject is infected with HCV type 2, 3 or 4 said cell of the subject is a liver cell.
(27) The method of (4), wherein said cell is a blood cell.
(28) The method of (6), further comprising comparing a ratio determined between an expression level of at least one gene selected from the group consisting of ISG15 and USP18 in a liver cell of the subject following interferon treatment and an expression level of said at least one gene in said liver cell of the subject prior to interferon treatment, or visa versa, to a reference ratio determined in a cell of at least one interferon responder subject and/or at least one interferon non-responder subject, said reference ratio is determined between an expression level of said at least one gene following interferon treatment and an expression level of said at least one gene prior to interferon treatment, or visa versa, thereby predicting the responsiveness to interferon treatment of a subject.
(29) The method of (9), wherein said co-administering is effected so as to enable a pharmacokinetic overlap between said interferon and said agent.
(30) A pharmaceutical composition comprising interferon, an agent capable of downregulating HLA or KIR inhibitory receptor, and a pharmaceutically acceptable carrier.
(31) The method of (1), (2), (4), (5), (6), (7), (8) or (9), wherein the subject is infected with HCV type 1.
(32) The method of (1), (2), (4), (5), (6), (7), (8) or (9), wherein the subject is infected with HCV type 2, 3 or 4.
(33) The method of (9), wherein the subject is diagnosed with multiple sclerosis.
(34) The method of any of (1)-(8), wherein said level of expression is determined using an RNA detection method.
(35) The method of any of (1)-(8), wherein said level of expression is determined using a protein detection method.
(36) The method of (9), wherein said agent is selected from the group consisting of an antibody, an RNA silencing molecule, a ribozyme and a DNAzyme.
(37) The method of (36), wherein said antibody is an anti-KIR inhibitory receptor antibody.
(38) The method of (36), wherein said RNA silencing molecule is an siRNA directed against a KIR inhibitory receptor or a HLA.
(39) The method of (9), wherein the subject is a non-responder to interferon treatment.

## Claims

1. A method of predicting responsiveness to interferon treatment of a subject diagnosed with multiple sclerosis or infected with HCV type 2, 3 or 4, comprising comparing a level of expression in a cell of the subject of IFI6, OAS2, ISG15, OAS3 and IFIT1 genes to a reference expression data of said genes obtained from at least one interferon responder subject and/or at least one interferon non-responder subject, thereby predicting the responsiveness of the subject to interferon treatment.

2. The method according to claim 1, wherein:
an identical or decreased level of expression of said at least one gene in said cell of the subject relative to a predetermined reference threshold of interferon responder subjects, is an indication for responsiveness of the subject to interferon treatment; or
wherein an identical or increased level of expression of said at least one gene in said cell of the subject relative to a predetermined reference threshold of interferon non-responder subjects, is an indication for lack of responsiveness of the subject to interferon treatment; thereby predicting the responsiveness of the subject to interferon treatment.

3. The method according to claim 1 or 2, wherein said level of expression is determined prior to interferon treatment.

4. A method of predicting responsiveness of a subject to interferon treatment, comprising comparing a ratio determined between an expression level of ISG15, IFI6, IFIT1, OAS2 and OAS3 genes in a cell of the subject following interferon treatment and an expression level of said genes in said cell of the subject prior to interferon treatment, or visa versa, to a reference ratio determined in a cell of at least one interferon responder subject and/or at least one interferon non-responder subject, said reference ratio is determined between an expression level of said genes following interferon treatment and an expression level of said genes prior to interferon treatment, or visa versa, thereby predicting the responsiveness to interferon treatment of a subject.

5. The method according to claim 4, wherein:
a) an increase ratio of the subject relative to said reference ratio of said at least one interferon non-responder subject, or an identical or increased ratio of the subject relative to said reference ratio of said at least one interferon responder subject, predicts responsiveness of the subject to interferon treatment; or
b) a decreased ratio of the subject relative to said reference ratio of said at least one interferon responder subject, or an identical or decreased ratio of the subject relative to said reference ratio of said at least one interferon non-responder subject, predicts lack of responsiveness of the subject to interferon treatment.

6. The method according to claims 4 and 5, wherein said following interferon treatment is effected about 4 hours after interferon treatment

7. The method according to claims 4 and 5, wherein said following interferon treatment is effected about 24 hours after interferon treatment.

8. A method of selecting a treatment regimen for a subject in need of interferon treatment, the method comprising:
(a) predicting the responsiveness of the subject to interferon treatment according to the method of any of claims 1-5, and
(b) selecting a treatment regimen based on said responsiveness;
thereby treating the subject in need of interferon treatment.

9. The method according to any of claims 1 to 8, wherein said cell is a blood cell.

10. The method according to any of claims 1 to 8, wherein said cell is a liver cell.

11. The method according to any one of claims 1 to 10, wherein said subject is diagnosed with HCV infection type 1, 2, 3 or 4.

12. The method according to claim 1, wherein when said subject is diagnosed with multiple sclerosis said cell of the subject is a blood cell.

13. The method according to claim 1, wherein when said subject is infected with HCV type 2, 3 or 4 said cell of the subject is a liver cell.

14. The method according to any of claims 1-8, wherein said level of expression is determined using an RNA detection method or a protein detection method.

15. A method of predicting responsiveness of a subject to interferon treatment, comprising comparing a ratio determined between an expression level of at least one gene selected from the group consisting of HERC5 and UBE2L6 in a liver cell of the subject following interferon treatment and an expression level of said at least one gene in said liver cell of the subject prior to interferon treatment, or visa versa, to a reference ratio determined in a cell of at least one interferon responder subject and/or at least one interferon non-responder subject, said reference ratio is determined between an expression level of said at least one gene following interferon treatment and an expression level of said at least one gene prior to interferon treatment, or visa versa, preferably, said method further comprising comparing a ratio determined between an expression level of at least one gene selected from the group consisting of ISG15 and USP18 in a liver cell of the subject following interferon treatment and an expression level of said at least one gene in said liver cell of the subject prior to interferon treatment, or visa versa, to a reference ratio determined in a cell of at least one interferon responder subject and/or at least one interferon non-responder subject, said reference ratio is determined between an expression level of said at least one gene following interferon treatment and an expression level of said at least one gene prior to interferon treatment, or visa versa, thereby predicting the responsiveness to interferon treatment.


SEQUENCE LISTING

<110>  Yissum Research Development Company of the Hebrew
       University of Jerusalem Ltd.
       Smith, Yoav

<120>  METHODS OF PREDICTING RESPONSIVENESS TO INTERFERON TREATMENT AND
       TREATING HEPATITIS C INFECTION

<130>  51632

<150>  US 61/141,048
<151>  2008-12-29

<150>  US 61/146,150
<151>  2009-01-21

<150>  US 61/184,910
<151>  2009-06-08

<150>  US 61/253,703
<151>  2009-10-21

<160>  468

<170>  PatentIn version 3.5

<210>  1
<211>  528
<212>  DNA
<213>  Homo sapiens

<400>  1
tgaccttcat ggccgtcgga ggaggactcg cagtcgccgg gctgcccgcg ctgggcttca      60

ccggcgccgg catcgcggcc aactcggtgg ctgcctcgct gatgagctgg tctgcgatcc     120

tgaatggggg cggcgtgccc gccggggggc tagtggccac gctgcagagc ctcggggctg     180

gtggcagcag cgtcgtcata ggtaatattg gtgccctgat gggctacgcc acccacaagt     240

atctcgatag tgaggaggat gaggagtagc cagcagctcc cagaacctct tcttccttct     300

tggcctaact cttccagtta ggatctagaa ctttgccttt tttttttttt tttttttttt     360

ttgagatggg ttctcactat attgtccagg ctagagtgca gtggctattc acagatgcga     420

acatagtaca ctgcagcctc caactcctag cctcaagtga tcctcctgtc tcaacctccc     480

aagtaggatt acaagcatgc gccgacgatg cccagaatcc agaacttt                  528


<210>  2
<211>  465
<212>  DNA
<213>  Homo sapiens

<400>  2
ttggcatatt ggccccactg taacttttgg gggcttcccg gtctagccac accctcggat      60

ggaaagactt gactgcataa agatgtcagt tctccctgag ttgattgata ggcttaatgg     120

tcaccctaaa aacacccaca tatgcttttc gatggaacca gataagttga cgctaaagtt     180

cttatggaaa aatacacacg caatagctag gaaaacacag ggaaagaaga gttctgagca 240

gggcctagtc ttagccaata ttaaaacata ctatgaagcc tctgatactt aaacagcatg 300

gcgctggtac gtaaatagac caatgcagtt aggtggctct ttccaagact ctggggaaaa 360

aagtagtaaa aagctaaatg caatcaatca gcaattgaaa gctaagtgag agagccagag 420

ggcctccttg gtggtaaaag agggttgcat ttcttgcagc cagaa 465

<210> 3
<211> 432
<212> DNA
<213> Homo sapiens

<400> 3
tggcgggcaa cgaattccag gtgtccctga gcagctccat gtcggtgtca gagctgaagg 60

cgcagatcac ccagaagatt ggcgtgcacg ccttccagca gcgtctggct gtccacccga 120

gcggtgtggc gctgcaggac agggtccccc ttgccagcca gggcctgggc cctggcagca 180

cggtcctgct ggtggtggac aaatgcgacg aacctctgag catcctggtg aggaataaca 240

agggccgcag cagcacctac gaggtccggc tgacgcagac cgtggcccac ctgaagcagc 300

aagtgagcgg gctggagggt gtgcaggacg acctgttctg gctgaccttc gaggggaagc 360

ccctggagga ccagctcccg ctgggggagt acggcctcaa gcccctgagc accgtgttca 420

tgaatctgcg cc 432

<210> 4
<211> 555
<212> DNA
<213> Homo sapiens

<400> 4
agactgcaaa ccctttcata aagctgcctt gctgaactcc tctctgcagg agccctgctt 60

aaaatagttg atgtcatcac tttatgtgca tcttatttct gtcaacttgt attttttttt 120

cttgtatttt tccaattagc tcctcctttt tccttccagt ctaaaaaagg aatcctctgt 180

gtcttcaaag caaagctctt tactttcccc ttggttctca taactctgtg atcttgctct 240

cggtgcttcc aactcatcca cgtcctgtct gtttcctctg tatacaaaac cctttctgcc 300

cctgctgaca cagacatcct ctatgccagc agccaggcca accctttcat tagaacttca 360

agctctccaa aggctcagat tataactgtt gtcatattta tatgaggctg ttgtcttttc 420

cttctgagcc tgcctttatc cccccaccca ggagtatcct cttgccaaag caaaagactt 480

tttccttggc tttagcctta aagatacttg aaggtctagg tgctttaacc tcacataccc 540

tcacttaaac tttta 555

<210> 5

<211>  509
<212>  DNA
<213>  Homo sapiens

<400>  5
aaaaagccca catttgaggt ggctcatcta gacctggcaa gaatgtatat agaagcaggc      60

aatcacagaa aagctgaaga gaattttcaa aaattgttat gcatgaaacc agtggtagaa     120

gaaacaatgc aagacataca tttctactat ggtcggtttc aggaatttca aaagaaatct     180

gacgtcaatg caattatcca ttatttaaaa gctataaaaa tagaacaggc atcattaaca     240

agggataaaa gtatcaattc tttgaagaaa ttggttttaa ggaaacttcg gagaaaggca     300

ttagatctgg aaagcttgag cctccttggg ttcgtctata aattggaagg aaatatgaat     360

gaagccctgg agtactatga gcgggccctg agactggctg ctgactttga gaactctgtg     420

agacaaggtc cttaggcacc cagatatcag ccactttcac atttcatttc attttatgct     480

aacatttact aatcatcttt tctgcttac                                       509


<210>  6
<211>  130
<212>  PRT
<213>  Homo sapiens

<400>  6

Met Arg Gln Lys Ala Val Ser Leu Phe Leu Cys Tyr Leu Leu Leu Phe
1               5                   10                  15


Thr Cys Ser Gly Val Glu Ala Gly Lys Lys Lys Cys Ser Glu Ser Ser
            20                  25                  30


Asp Ser Gly Ser Gly Phe Trp Lys Ala Leu Thr Phe Met Ala Val Gly
        35                  40                  45


Gly Gly Leu Ala Val Ala Gly Leu Pro Ala Leu Gly Phe Thr Gly Ala
    50                  55                  60


Gly Ile Ala Ala Asn Ser Val Ala Ala Ser Leu Met Ser Trp Ser Ala
65                  70                  75                  80


Ile Leu Asn Gly Gly Gly Val Pro Ala Gly Gly Leu Val Ala Thr Leu
                85                  90                  95


Gln Ser Leu Gly Ala Gly Gly Ser Ser Val Val Ile Gly Asn Ile Gly
            100                 105                 110


Ala Leu Met Gly Tyr Ala Thr His Lys Tyr Leu Asp Ser Glu Glu Asp
        115                 120                 125

Glu Glu
    130

<210>  7
<211>  134
<212>  PRT
<213>  Homo sapiens

<400>  7

Met Arg Gln Lys Ala Val Ser Leu Phe Leu Cys Tyr Leu Leu Leu Phe
1               5                   10                  15

Thr Cys Ser Gly Val Glu Ala Gly Glu Asn Ala Gly Lys Lys Lys Cys
            20                  25                  30

Ser Glu Ser Ser Asp Ser Gly Ser Gly Phe Trp Lys Ala Leu Thr Phe
        35                  40                  45

Met Ala Val Gly Gly Gly Leu Ala Val Ala Gly Leu Pro Ala Leu Gly
    50                  55                  60

Phe Thr Gly Ala Gly Ile Ala Ala Asn Ser Val Ala Ala Ser Leu Met
65                  70                  75                  80

Ser Trp Ser Ala Ile Leu Asn Gly Gly Gly Val Pro Ala Gly Gly Leu
                85                  90                  95

Val Ala Thr Leu Gln Ser Leu Gly Ala Gly Gly Ser Ser Val Val Ile
            100                 105                 110

Gly Asn Ile Gly Ala Leu Met Gly Tyr Ala Thr His Lys Tyr Leu Asp
        115                 120                 125

Ser Glu Glu Asp Glu Glu
    130

<210>  8
<211>  138
<212>  PRT
<213>  Homo sapiens

<400>  8

Met Arg Gln Lys Ala Val Ser Leu Phe Leu Cys Tyr Leu Leu Leu Phe
1               5                   10                  15

Thr Cys Ser Gly Val Glu Ala Gly Glu Asn Ala Gly Lys Asp Ala Gly
            20                  25                  30

Lys Lys Lys Cys Ser Glu Ser Ser Asp Ser Gly Ser Gly Phe Trp Lys

35                  40                  45

Ala Leu Thr Phe Met Ala Val Gly Gly Gly Leu Ala Val Ala Gly Leu
    50                  55                  60

Pro Ala Leu Gly Phe Thr Gly Ala Gly Ile Ala Ala Asn Ser Val Ala
65                  70                  75                  80

Ala Ser Leu Met Ser Trp Ser Ala Ile Leu Asn Gly Gly Gly Val Pro
                85                  90                  95

Ala Gly Gly Leu Val Ala Thr Leu Gln Ser Leu Gly Ala Gly Gly Ser
            100                 105                 110

Ser Val Val Ile Gly Asn Ile Gly Ala Leu Met Gly Tyr Ala Thr His
        115                 120                 125

Lys Tyr Leu Asp Ser Glu Glu Asp Glu Glu
    130                 135

<210>  9
<211>  1087
<212>  PRT
<213>  Homo sapiens

<400>  9

Met Asp Leu Tyr Ser Thr Pro Ala Ala Ala Leu Asp Arg Phe Val Ala
1               5                   10                  15

Arg Arg Leu Gln Pro Arg Lys Glu Phe Val Glu Lys Ala Arg Arg Ala
            20                  25                  30

Leu Gly Ala Leu Ala Ala Ala Leu Arg Glu Arg Gly Gly Arg Leu Gly
        35                  40                  45

Ala Ala Ala Pro Arg Val Leu Lys Thr Val Lys Gly Gly Ser Ser Gly
    50                  55                  60

Arg Gly Thr Ala Leu Lys Gly Gly Cys Asp Ser Glu Leu Val Ile Phe
65                  70                  75                  80

Leu Asp Cys Phe Lys Ser Tyr Val Asp Gln Arg Ala Arg Arg Ala Glu
                85                  90                  95

Ile Leu Ser Glu Met Arg Ala Ser Leu Glu Ser Trp Trp Gln Asn Pro
            100                 105                 110

Val Pro Gly Leu Arg Leu Thr Phe Pro Glu Gln Ser Val Pro Gly Ala

115                 120                 125

Leu Gln Phe Arg Leu Thr Ser Val Asp Leu Glu Asp Trp Met Asp Val
    130                 135                 140

Ser Leu Val Pro Ala Phe Asn Val Leu Gly Gln Ala Gly Ser Gly Val
145                 150                 155                 160

Lys Pro Lys Pro Gln Val Tyr Ser Thr Leu Leu Asn Ser Gly Cys Gln
                165                 170                 175

Gly Gly Glu His Ala Ala Cys Phe Thr Glu Leu Arg Arg Asn Phe Val
            180                 185                 190

Asn Ile Arg Pro Ala Lys Leu Lys Asn Leu Ile Leu Leu Val Lys His
        195                 200                 205

Trp Tyr His Gln Val Cys Leu Gln Gly Leu Trp Lys Glu Thr Leu Pro
    210                 215                 220

Pro Val Tyr Ala Leu Glu Leu Leu Thr Ile Phe Ala Trp Glu Gln Gly
225                 230                 235                 240

Cys Lys Lys Asp Ala Phe Ser Leu Ala Glu Gly Leu Arg Thr Val Leu
                245                 250                 255

Gly Leu Ile Gln Gln His Gln His Leu Cys Val Phe Trp Thr Val Asn
            260                 265                 270

Tyr Gly Phe Glu Asp Pro Ala Val Gly Gln Phe Leu Gln Arg Gln Leu
        275                 280                 285

Lys Arg Pro Arg Pro Val Ile Leu Asp Pro Ala Asp Pro Thr Trp Asp
    290                 295                 300

Leu Gly Asn Gly Ala Ala Trp His Trp Asp Leu Leu Ala Gln Glu Ala
305                 310                 315                 320

Ala Ser Cys Tyr Asp His Pro Cys Phe Leu Arg Gly Met Gly Asp Pro
                325                 330                 335

Val Gln Ser Trp Lys Gly Pro Gly Leu Pro Arg Ala Gly Cys Ser Gly
            340                 345                 350

Leu Gly His Pro Ile Gln Leu Asp Pro Asn Gln Lys Thr Pro Glu Asn
        355                 360                 365

Ser Lys Ser Leu Asn Ala Val Tyr Pro Arg Ala Gly Ser Lys Pro Pro
    370                 375                 380

Ser Cys Pro Ala Pro Gly Pro Thr Gly Ala Ala Ser Ile Val Pro Ser
385                 390                 395                 400

Val Pro Gly Met Ala Leu Asp Leu Ser Gln Ile Pro Thr Lys Glu Leu
                405                 410                 415

Asp Arg Phe Ile Gln Asp His Leu Lys Pro Ser Pro Gln Phe Gln Glu
            420                 425                 430

Gln Val Lys Lys Ala Ile Asp Ile Ile Leu Arg Cys Leu His Glu Asn
        435                 440                 445

Cys Val His Lys Ala Ser Arg Val Ser Lys Gly Gly Ser Phe Gly Arg
    450                 455                 460

Gly Thr Asp Leu Arg Asp Gly Cys Asp Val Glu Leu Ile Ile Phe Leu
465                 470                 475                 480

Asn Cys Phe Thr Asp Tyr Lys Asp Gln Gly Pro Arg Arg Ala Glu Ile
                485                 490                 495

Leu Asp Glu Met Arg Ala Gln Leu Glu Ser Trp Trp Gln Asp Gln Val
            500                 505                 510

Pro Ser Leu Ser Leu Gln Phe Pro Glu Gln Asn Val Pro Glu Ala Leu
        515                 520                 525

Gln Phe Gln Leu Val Ser Thr Ala Leu Lys Ser Trp Thr Asp Val Ser
    530                 535                 540

Leu Leu Pro Ala Phe Asp Ala Val Gly Gln Leu Ser Ser Gly Thr Lys
545                 550                 555                 560

Pro Asn Pro Gln Val Tyr Ser Arg Leu Leu Thr Ser Gly Cys Gln Glu
                565                 570                 575

Gly Glu His Lys Ala Cys Phe Ala Glu Leu Arg Arg Asn Phe Met Asn
            580                 585                 590

Ile Arg Pro Val Lys Leu Lys Asn Leu Ile Leu Leu Val Lys His Trp
        595                 600                 605

Tyr Arg Gln Val Ala Ala Gln Asn Lys Gly Lys Gly Pro Ala Pro Ala
    610                 615                 620

Ser Leu Pro Pro Ala Tyr Ala Leu Glu Leu Leu Thr Ile Phe Ala Trp
625                 630                 635                 640

Glu Gln Gly Cys Arg Gln Asp Cys Phe Asn Met Ala Gln Gly Phe Arg
                645                 650                 655

Thr Val Leu Gly Leu Val Gln Gln His Gln Gln Leu Cys Val Tyr Trp
            660                 665                 670

Thr Val Asn Tyr Ser Thr Glu Asp Pro Ala Met Arg Met His Leu Leu
        675                 680                 685

Gly Gln Leu Arg Lys Pro Arg Pro Leu Val Leu Asp Pro Ala Asp Pro
    690                 695                 700

Thr Trp Asn Val Gly His Gly Ser Trp Glu Leu Leu Ala Gln Glu Ala
705                 710                 715                 720

Ala Ala Leu Gly Met Gln Ala Cys Phe Leu Ser Arg Asp Gly Thr Ser
                725                 730                 735

Val Gln Pro Trp Asp Val Met Pro Ala Leu Leu Tyr Gln Thr Pro Ala
            740                 745                 750

Gly Asp Leu Asp Lys Phe Ile Ser Glu Phe Leu Gln Pro Asn Arg Gln
        755                 760                 765

Phe Leu Ala Gln Val Asn Lys Ala Val Asp Thr Ile Cys Ser Phe Leu
    770                 775                 780

Lys Glu Asn Cys Phe Arg Asn Ser Pro Ile Lys Val Ile Lys Val Val
785                 790                 795                 800

Lys Gly Gly Ser Ser Ala Lys Gly Thr Ala Leu Arg Gly Arg Ser Asp
                805                 810                 815

Ala Asp Leu Val Val Phe Leu Ser Cys Phe Ser Gln Phe Thr Glu Gln
            820                 825                 830

Gly Asn Lys Arg Ala Glu Ile Ile Ser Glu Ile Arg Ala Gln Leu Glu
        835                 840                 845

Ala Cys Gln Gln Glu Arg Gln Phe Glu Val Lys Phe Glu Val Ser Lys
    850                 855                 860

Trp Glu Asn Pro Arg Val Leu Ser Phe Ser Leu Thr Ser Gln Thr Met
865                 870                 875                 880

Leu Asp Gln Ser Val Asp Phe Asp Val Leu Pro Ala Phe Asp Ala Leu
                885                 890                 895

Gly Gln Leu Val Ser Gly Ser Arg Pro Ser Ser Gln Val Tyr Val Asp
            900                 905                 910

Leu Ile His Ser Tyr Ser Asn Ala Gly Glu Tyr Ser Thr Cys Phe Thr
        915                 920                 925

Glu Leu Gln Arg Asp Phe Ile Ile Ser Arg Pro Thr Lys Leu Lys Ser
    930                 935                 940

Leu Ile Arg Leu Val Lys His Trp Tyr Gln Gln Cys Thr Lys Ile Ser
945                 950                 955                 960

Lys Gly Arg Gly Ser Leu Pro Pro Gln His Gly Leu Glu Leu Leu Thr
                965                 970                 975

Val Tyr Ala Trp Glu Gln Gly Gly Lys Asp Ser Gln Phe Asn Met Ala
            980                 985                 990

Glu Gly Phe Arg Thr Val Leu Glu  Leu Val Thr Gln Tyr  Arg Gln Leu
        995                 1000                1005

Cys Ile  Tyr Trp Thr Ile Asn  Tyr Asn Ala Lys Asp  Lys Thr Val
    1010                1015                1020

Gly Asp  Phe Leu Lys Gln Gln  Leu Gln Lys Pro Arg  Pro Ile Ile
    1025                1030                1035

Leu Asp  Pro Ala Asp Pro Thr  Gly Asn Leu Gly His  Asn Ala Arg
    1040                1045                1050

Trp Asp  Leu Leu Ala Lys Glu  Ala Ala Ala Cys Thr  Ser Ala Leu
    1055                1060                1065

Cys Cys  Met Gly Arg Asn Gly  Ile Pro Ile Gln Pro  Trp Pro Val
    1070                1075                1080

Lys Ala  Ala Val
    1085

<210>  10
<211>  165
<212>  PRT
<213>  Homo sapiens

<400>  10

Met Gly Trp Asp Leu Thr Val Lys Met Leu Ala Gly Asn Glu Phe Gln
1               5                   10                  15

Val Ser Leu Ser Ser Ser Met Ser Val Ser Glu Leu Lys Ala Gln Ile
            20                  25                  30

Thr Gln Lys Ile Gly Val His Ala Phe Gln Gln Arg Leu Ala Val His
        35                  40                  45

Pro Ser Gly Val Ala Leu Gln Asp Arg Val Pro Leu Ala Ser Gln Gly
    50                  55                  60

Leu Gly Pro Gly Ser Thr Val Leu Leu Val Val Asp Lys Cys Asp Glu
65                  70                  75                  80

Pro Leu Ser Ile Leu Val Arg Asn Asn Lys Gly Arg Ser Ser Thr Tyr
                85                  90                  95

Glu Val Arg Leu Thr Gln Thr Val Ala His Leu Lys Gln Gln Val Ser
            100                 105                 110

Gly Leu Glu Gly Val Gln Asp Asp Leu Phe Trp Leu Thr Phe Glu Gly
        115                 120                 125

Lys Pro Leu Glu Asp Gln Leu Pro Leu Gly Glu Tyr Gly Leu Lys Pro
    130                 135                 140

Leu Ser Thr Val Phe Met Asn Leu Arg Leu Arg Gly Gly Gly Thr Glu
145                 150                 155                 160

Pro Gly Gly Arg Ser
                165


<210>  11
<211>  172
<212>  PRT
<213>  Homo sapiens

<400>  11

Met Gly Asn Gly Glu Ser Gln Leu Ser Ser Val Pro Ala Gln Lys Leu
1               5                   10                  15

Gly Trp Phe Ile Gln Glu Tyr Leu Lys Pro Tyr Glu Glu Cys Gln Thr
            20                  25                  30

Leu Ile Asp Glu Met Val Asn Thr Ile Cys Asp Val Leu Gln Glu Pro
        35                  40                  45

Glu Gln Phe Pro Leu Val Gln Gly Val Ala Ile Gly Gly Ser Tyr Gly
    50                  55                  60

Arg Lys Thr Val Leu Arg Gly Asn Ser Asp Gly Thr Leu Val Leu Phe
65                  70                  75                  80

Phe Ser Asp Leu Lys Gln Phe Gln Asp Gln Lys Arg Ser Gln Arg Asp
                85                  90                  95

Ile Leu Asp Lys Thr Gly Asp Lys Leu Lys Phe Cys Leu Phe Thr Lys
            100                 105                 110

Trp Leu Lys Asn Asn Phe Glu Ile Gln Lys Ser Leu Asp Gly Phe Thr
        115                 120                 125

Ile Gln Val Phe Thr Lys Asn Gln Arg Ile Ser Phe Glu Val Leu Ala
    130                 135                 140

Ala Phe Asn Ala Leu Ser Lys His Cys Trp Val Ser Gly Glu Lys Ser
145                 150                 155                 160

Gln Arg Ser Gly Cys Gln Thr Ala Leu Cys Asn Leu
                165                 170

<210>  12
<211>  687
<212>  PRT
<213>  Homo sapiens

<400>  12

Met Gly Asn Gly Glu Ser Gln Leu Ser Ser Val Pro Ala Gln Lys Leu
1               5                   10                  15

Gly Trp Phe Ile Gln Glu Tyr Leu Lys Pro Tyr Glu Glu Cys Gln Thr
            20                  25                  30

Leu Ile Asp Glu Met Val Asn Thr Ile Cys Asp Val Leu Gln Glu Pro
        35                  40                  45

Glu Gln Phe Pro Leu Val Gln Gly Val Ala Ile Gly Gly Ser Tyr Gly
    50                  55                  60

Arg Lys Thr Val Leu Arg Gly Asn Ser Asp Gly Thr Leu Val Leu Phe
65                  70                  75                  80

Phe Ser Asp Leu Lys Gln Phe Gln Asp Gln Lys Arg Ser Gln Arg Asp
                85                  90                  95

Ile Leu Asp Lys Thr Gly Asp Lys Leu Lys Phe Cys Leu Phe Thr Lys
            100                 105                 110

Trp Leu Lys Asn Asn Phe Glu Ile Gln Lys Ser Leu Asp Gly Phe Thr
        115                 120                 125

Ile Gln Val Phe Thr Lys Asn Gln Arg Ile Ser Phe Glu Val Leu Ala
    130                 135                 140

Ala Phe Asn Ala Leu Ser Leu Asn Asp Asn Pro Ser Pro Trp Ile Tyr
145                 150                 155                 160

Arg Glu Leu Lys Arg Ser Leu Asp Lys Thr Asn Ala Ser Pro Gly Glu
                165                 170                 175

Phe Ala Val Cys Phe Thr Glu Leu Gln Gln Lys Phe Phe Asp Asn Arg
            180                 185                 190

Pro Gly Lys Leu Lys Asp Leu Ile Leu Leu Ile Lys His Trp His Gln
        195                 200                 205

Gln Cys Gln Lys Lys Ile Lys Asp Leu Pro Ser Leu Ser Pro Tyr Ala
    210                 215                 220

Leu Glu Leu Leu Thr Val Tyr Ala Trp Glu Gln Gly Cys Arg Lys Asp
225                 230                 235                 240

Asn Phe Asp Ile Ala Glu Gly Val Arg Thr Val Leu Glu Leu Ile Lys
                245                 250                 255

Cys Gln Glu Lys Leu Cys Ile Tyr Trp Met Val Asn Tyr Asn Phe Glu
            260                 265                 270

Asp Glu Thr Ile Arg Asn Ile Leu Leu His Gln Leu Gln Ser Ala Arg
        275                 280                 285

Pro Val Ile Leu Asp Pro Val Asp Pro Thr Asn Asn Val Ser Gly Asp
    290                 295                 300

Lys Ile Cys Trp Gln Trp Leu Lys Lys Glu Ala Gln Thr Trp Leu Thr
305                 310                 315                 320

Ser Pro Asn Leu Asp Asn Glu Leu Pro Ala Pro Ser Trp Asn Val Leu
                325                 330                 335

Pro Ala Pro Leu Phe Thr Thr Pro Gly His Leu Leu Asp Lys Phe Ile
            340                 345                 350

Lys Glu Phe Leu Gln Pro Asn Lys Cys Phe Leu Glu Gln Ile Asp Ser
        355                 360                 365

Ala Val Asn Ile Ile Arg Thr Phe Leu Lys Glu Asn Cys Phe Arg Gln
    370                 375                 380

Ser Thr Ala Lys Ile Gln Ile Val Arg Gly Gly Ser Thr Ala Lys Gly
385                 390                 395                 400

Thr Ala Leu Lys Thr Gly Ser Asp Ala Asp Leu Val Val Phe His Asn
                405                 410                 415

Ser Leu Lys Ser Tyr Thr Ser Gln Lys Asn Glu Arg His Lys Ile Val
            420                 425                 430

Lys Glu Ile His Glu Gln Leu Lys Ala Phe Trp Arg Glu Lys Glu Glu
        435                 440                 445

Glu Leu Glu Val Ser Phe Glu Pro Pro Lys Trp Lys Ala Pro Arg Val
    450                 455                 460

Leu Ser Phe Ser Leu Lys Ser Lys Val Leu Asn Glu Ser Val Ser Phe
465                 470                 475                 480

Asp Val Leu Pro Ala Phe Asn Ala Leu Gly Gln Leu Ser Ser Gly Ser
                485                 490                 495

Thr Pro Ser Pro Glu Val Tyr Ala Gly Leu Ile Asp Leu Tyr Lys Ser
            500                 505                 510

Ser Asp Leu Pro Gly Gly Glu Phe Ser Thr Cys Phe Thr Val Leu Gln
        515                 520                 525

Arg Asn Phe Ile Arg Ser Arg Pro Thr Lys Leu Lys Asp Leu Ile Arg
    530                 535                 540

Leu Val Lys His Trp Tyr Lys Glu Cys Glu Arg Lys Leu Lys Pro Lys
545                 550                 555                 560

Gly Ser Leu Pro Pro Lys Tyr Ala Leu Glu Leu Leu Thr Ile Tyr Ala
                565                 570                 575

Trp Glu Gln Gly Ser Gly Val Pro Asp Phe Asp Thr Ala Glu Gly Phe
            580                 585                 590

Arg Thr Val Leu Glu Leu Val Thr Gln Tyr Gln Gln Leu Cys Ile Phe
        595                 600                 605

Trp Lys Val Asn Tyr Asn Phe Glu Asp Glu Thr Val Arg Lys Phe Leu
    610                 615                 620

Leu Ser Gln Leu Gln Lys Thr Arg Pro Val Ile Leu Asp Pro Ala Glu
625                 630                 635                 640

Pro Thr Gly Asp Val Gly Gly Gly Asp Arg Trp Cys Trp His Leu Leu
                645                 650                 655

Ala Lys Glu Ala Lys Glu Trp Leu Ser Ser Pro Cys Phe Lys Asp Gly
            660                 665                 670

Thr Gly Asn Pro Ile Pro Pro Trp Lys Val Pro Val Lys Val Ile
        675                 680                 685

<210>  13
<211>  719
<212>  PRT
<213>  Homo sapiens

<400>  13

Met Gly Asn Gly Glu Ser Gln Leu Ser Ser Val Pro Ala Gln Lys Leu
1               5                   10                  15

Gly Trp Phe Ile Gln Glu Tyr Leu Lys Pro Tyr Glu Glu Cys Gln Thr
            20                  25                  30

Leu Ile Asp Glu Met Val Asn Thr Ile Cys Asp Val Leu Gln Glu Pro
        35                  40                  45

Glu Gln Phe Pro Leu Val Gln Gly Val Ala Ile Gly Gly Ser Tyr Gly
    50                  55                  60

Arg Lys Thr Val Leu Arg Gly Asn Ser Asp Gly Thr Leu Val Leu Phe
65                  70                  75                  80

Phe Ser Asp Leu Lys Gln Phe Gln Asp Gln Lys Arg Ser Gln Arg Asp
                85                  90                  95

Ile Leu Asp Lys Thr Gly Asp Lys Leu Lys Phe Cys Leu Phe Thr Lys
            100                 105                 110

Trp Leu Lys Asn Asn Phe Glu Ile Gln Lys Ser Leu Asp Gly Phe Thr
        115                 120                 125

Ile Gln Val Phe Thr Lys Asn Gln Arg Ile Ser Phe Glu Val Leu Ala
    130                 135                 140

Ala Phe Asn Ala Leu Ser Leu Asn Asp Asn Pro Ser Pro Trp Ile Tyr
145                 150                 155                 160

Arg Glu Leu Lys Arg Ser Leu Asp Lys Thr Asn Ala Ser Pro Gly Glu
                165                 170                 175

Phe Ala Val Cys Phe Thr Glu Leu Gln Gln Lys Phe Phe Asp Asn Arg
            180                 185                 190

Pro Gly Lys Leu Lys Asp Leu Ile Leu Leu Ile Lys His Trp His Gln
        195                 200                 205

Gln Cys Gln Lys Lys Ile Lys Asp Leu Pro Ser Leu Ser Pro Tyr Ala
    210                 215                 220

Leu Glu Leu Leu Thr Val Tyr Ala Trp Glu Gln Gly Cys Arg Lys Asp
225                 230                 235                 240

Asn Phe Asp Ile Ala Glu Gly Val Arg Thr Val Leu Glu Leu Ile Lys
                245                 250                 255

Cys Gln Glu Lys Leu Cys Ile Tyr Trp Met Val Asn Tyr Asn Phe Glu
            260                 265                 270

Asp Glu Thr Ile Arg Asn Ile Leu Leu His Gln Leu Gln Ser Ala Arg
        275                 280                 285

Pro Val Ile Leu Asp Pro Val Asp Pro Thr Asn Asn Val Ser Gly Asp
    290                 295                 300

Lys Ile Cys Trp Gln Trp Leu Lys Lys Glu Ala Gln Thr Trp Leu Thr
305                 310                 315                 320

Ser Pro Asn Leu Asp Asn Glu Leu Pro Ala Pro Ser Trp Asn Val Leu
                325                 330                 335

Pro Ala Pro Leu Phe Thr Thr Pro Gly His Leu Leu Asp Lys Phe Ile
            340                 345                 350

Lys Glu Phe Leu Gln Pro Asn Lys Cys Phe Leu Glu Gln Ile Asp Ser
        355                 360                 365

Ala Val Asn Ile Ile Arg Thr Phe Leu Lys Glu Asn Cys Phe Arg Gln

370                 375                 380

Ser Thr Ala Lys Ile Gln Ile Val Arg Gly Gly Ser Thr Ala Lys Gly
385                 390                 395                 400

Thr Ala Leu Lys Thr Gly Ser Asp Ala Asp Leu Val Val Phe His Asn
                405                 410                 415

Ser Leu Lys Ser Tyr Thr Ser Gln Lys Asn Glu Arg His Lys Ile Val
            420                 425                 430

Lys Glu Ile His Glu Gln Leu Lys Ala Phe Trp Arg Glu Lys Glu Glu
        435                 440                 445

Glu Leu Glu Val Ser Phe Glu Pro Pro Lys Trp Lys Ala Pro Arg Val
    450                 455                 460

Leu Ser Phe Ser Leu Lys Ser Lys Val Leu Asn Glu Ser Val Ser Phe
465                 470                 475                 480

Asp Val Leu Pro Ala Phe Asn Ala Leu Gly Gln Leu Ser Ser Gly Ser
                485                 490                 495

Thr Pro Ser Pro Glu Val Tyr Ala Gly Leu Ile Asp Leu Tyr Lys Ser
            500                 505                 510

Ser Asp Leu Pro Gly Gly Glu Phe Ser Thr Cys Phe Thr Val Leu Gln
        515                 520                 525

Arg Asn Phe Ile Arg Ser Arg Pro Thr Lys Leu Lys Asp Leu Ile Arg
    530                 535                 540

Leu Val Lys His Trp Tyr Lys Glu Cys Glu Arg Lys Leu Lys Pro Lys
545                 550                 555                 560

Gly Ser Leu Pro Pro Lys Tyr Ala Leu Glu Leu Leu Thr Ile Tyr Ala
                565                 570                 575

Trp Glu Gln Gly Ser Gly Val Pro Asp Phe Asp Thr Ala Glu Gly Phe
            580                 585                 590

Arg Thr Val Leu Glu Leu Val Thr Gln Tyr Gln Gln Leu Cys Ile Phe
        595                 600                 605

Trp Lys Val Asn Tyr Asn Phe Glu Asp Glu Thr Val Arg Lys Phe Leu
    610                 615                 620

Leu Ser Gln Leu Gln Lys Thr Arg Pro Val Ile Leu Asp Pro Ala Glu
625                 630                 635                 640

Pro Thr Gly Asp Val Gly Gly Gly Asp Arg Trp Cys Trp His Leu Leu
                645                 650                 655

Ala Lys Glu Ala Lys Glu Trp Leu Ser Ser Pro Cys Phe Lys Asp Gly
            660                 665                 670

Thr Gly Asn Pro Ile Pro Pro Trp Lys Val Pro Thr Met Gln Thr Pro
        675                 680                 685

Gly Ser Cys Gly Ala Arg Ile His Pro Ile Val Asn Glu Met Phe Ser
    690                 695                 700

Ser Arg Ser His Arg Ile Leu Asn Asn Asn Ser Lys Arg Asn Phe
705                 710                 715

<210>  14
<211>  478
<212>  PRT
<213>  Homo sapiens

<400>  14

Met Ser Thr Asn Gly Asp Asp His Gln Val Lys Asp Ser Leu Glu Gln
1               5                   10                  15

Leu Arg Cys His Phe Thr Trp Glu Leu Ser Ile Asp Asp Asp Glu Met
            20                  25                  30

Pro Asp Leu Glu Asn Arg Val Leu Asp Gln Ile Glu Phe Leu Asp Thr
        35                  40                  45

Lys Tyr Ser Val Gly Ile His Asn Leu Leu Ala Tyr Val Lys His Leu
    50                  55                  60

Lys Gly Gln Asn Glu Glu Ala Leu Lys Ser Leu Lys Glu Ala Glu Asn
65                  70                  75                  80

Leu Met Gln Glu Glu His Asp Asn Gln Ala Asn Val Arg Ser Leu Val
                85                  90                  95

Thr Trp Gly Asn Phe Ala Trp Met Tyr Tyr His Met Gly Arg Leu Ala
            100                 105                 110

Glu Ala Gln Thr Tyr Leu Asp Lys Val Glu Asn Ile Cys Lys Lys Leu
        115                 120                 125

Ser Asn Pro Phe Arg Tyr Arg Met Glu Cys Pro Glu Ile Asp Cys Glu
    130                 135                 140

Glu Gly Trp Ala Leu Leu Lys Cys Gly Gly Lys Asn Tyr Glu Arg Ala
145                 150                 155                 160

Lys Ala Cys Phe Glu Lys Val Leu Glu Val Asp Pro Glu Asn Pro Glu
                165                 170                 175

Ser Ser Ala Gly Tyr Ala Ile Ser Ala Tyr Arg Leu Asp Gly Phe Lys
            180                 185                 190

Leu Ala Thr Lys Asn His Lys Pro Phe Ser Leu Leu Pro Leu Arg Gln
        195                 200                 205

Ala Val Arg Leu Asn Pro Asp Asn Gly Tyr Ile Lys Val Leu Leu Ala
    210                 215                 220

Leu Lys Leu Gln Asp Glu Gly Gln Glu Ala Glu Gly Glu Lys Tyr Ile
225                 230                 235                 240

Glu Glu Ala Leu Ala Asn Met Ser Ser Gln Thr Tyr Val Phe Arg Tyr
                245                 250                 255

Ala Ala Lys Phe Tyr Arg Arg Lys Gly Ser Val Asp Lys Ala Leu Glu
            260                 265                 270

Leu Leu Lys Lys Ala Leu Gln Glu Thr Pro Thr Ser Val Leu Leu His
        275                 280                 285

His Gln Ile Gly Leu Cys Tyr Lys Ala Gln Met Ile Gln Ile Lys Glu
    290                 295                 300

Ala Thr Lys Gly Gln Pro Arg Gly Gln Asn Arg Glu Lys Leu Asp Lys
305                 310                 315                 320

Met Ile Arg Ser Ala Ile Phe His Phe Glu Ser Ala Val Glu Lys Lys
                325                 330                 335

Pro Thr Phe Glu Val Ala His Leu Asp Leu Ala Arg Met Tyr Ile Glu
            340                 345                 350

Ala Gly Asn His Arg Lys Ala Glu Glu Asn Phe Gln Lys Leu Leu Cys
        355                 360                 365

Met Lys Pro Val Val Glu Glu Thr Met Gln Asp Ile His Phe His Tyr
    370                 375                 380

Gly Arg Phe Gln Glu Phe Gln Lys Lys Ser Asp Val Asn Ala Ile Ile
385                 390                 395                 400

His Tyr Leu Lys Ala Ile Lys Ile Glu Gln Ala Ser Leu Thr Arg Asp
                405                 410                 415

Lys Ser Ile Asn Ser Leu Lys Lys Leu Val Leu Arg Lys Leu Arg Arg
            420                 425                 430

Lys Ala Leu Asp Leu Glu Ser Leu Ser Leu Leu Gly Phe Val Tyr Lys
        435                 440                 445

Leu Glu Gly Asn Met Asn Glu Ala Leu Glu Tyr Tyr Glu Arg Ala Leu
    450                 455                 460

Arg Leu Ala Ala Asp Phe Glu Asn Ser Val Arg Gln Gly Pro
465                 470                 475

<210>  15
<211>  836
<212>  DNA
<213>  Homo sapiens

<400>  15
ccagccttca gccggagaac cgtttactcg ctgctgtgcc catctatcag caggctccgg      60

gctgaagatt gcttctcttc tctcctccaa ggtctagtga cggagcccgc gcgcggcgcc     120

accatgcggc agaaggcggt atcgcttttc ttgtgctacc tgctgctctt cacttgcagt     180

ggggtggagg caggtaagaa aaagtgctcg gagagctcgg acagcggctc cgggttctgg     240

aaggccctga ccttcatggc cgtcggagga ggactcgcag tcgccgggct gcccgcgctg     300

ggcttcaccg gcgccggcat cgcggccaac tcggtggctg cctcgctgat gagctggtct     360

gcgatcctga atgggggcgg cgtgcccgcc ggggggctag tggccacgct gcagagcctc     420

ggggctggtg gcagcagcgt cgtcataggt aatattggtg ccctgatggg ctacgccacc     480

cacaagtatc tcgatagtga ggaggatgag gagtagccag cagctcccag aacctcttct     540

tccttcttgg cctaactctt ccagttagga tctagaactt tgcctttttt tttttttttt     600

tttttttgag atgggttctc actatattgt ccaggctaga gtgcagtggc tattcacaga     660

tgcgaacata gtacactgca gcctccaact cctagcctca agtgatcctc ctgtctcaac     720

ctcccaagta ggattacaag catgcgccga cgatgcccag aatccagaac tttgtctatc     780

actctcccca acaacctaga tgtgaaaaca gaataaactt cacccagaaa acactt         836

<210>  16
<211>  848
<212>  DNA

<213>  Homo sapiens

<400>  16
ccagccttca gccggagaac cgtttactcg ctgctgtgcc catctatcag caggctccgg      60

gctgaagatt gcttctcttc tctcctccaa ggtctagtga cggagcccgc gcgcggcgcc     120

accatgcggc agaaggcggt atcgcttttc ttgtgctacc tgctgctctt cacttgcagt     180

ggggtggagg caggtgagaa tgcgggtaag aaaaagtgct cggagagctc ggacagcggc     240

tccgggttct ggaaggccct gaccttcatg gccgtcggag gaggactcgc agtcgccggg     300

ctgcccgcgc tgggcttcac cggcgccggc atcgcggcca actcggtggc tgcctcgctg     360

atgagctggt ctgcgatcct gaatgggggc ggcgtgcccg ccggggggct agtggccacg     420

ctgcagagcc tcggggctgg tggcagcagc gtcgtcatag gtaatattgg tgccctgatg     480

ggctacgcca cccacaagta tctcgatagt gaggaggatg aggagtagcc agcagctccc     540

agaacctctt cttccttctt ggcctaactc ttccagttag gatctagaac tttgcctttt     600

tttttttttt tttttttttg agatgggttc tcactatatt gtccaggcta gagtgcagtg     660

gctattcaca gatgcgaaca tagtacactg cagcctccaa ctcctagcct caagtgatcc     720

tcctgtctca acctcccaag taggattaca agcatgcgcc gacgatgccc agaatccaga     780

actttgtcta tcactctccc caacaaccta gatgtgaaaa cagaataaac ttcacccaga     840

aaacactt                                                              848

<210>  17
<211>  860
<212>  DNA
<213>  Homo sapiens

<400>  17
ccagccttca gccggagaac cgtttactcg ctgctgtgcc catctatcag caggctccgg      60

gctgaagatt gcttctcttc tctcctccaa ggtctagtga cggagcccgc gcgcggcgcc     120

accatgcggc agaaggcggt atcgcttttc ttgtgctacc tgctgctctt cacttgcagt     180

ggggtggagg caggtgagaa tgcgggtaag gatgcaggta agaaaaagtg ctcggagagc     240

tcggacagcg gctccgggtt ctggaaggcc ctgaccttca tggccgtcgg aggaggactc     300

gcagtcgccg ggctgcccgc gctgggcttc accggcgccg gcatcgcggc caactcggtg     360

gctgcctcgc tgatgagctg gtctgcgatc ctgaatgggg gcggcgtgcc cgccgggggg     420

ctagtggcca cgctgcagag cctcggggct ggtggcagca gcgtcgtcat aggtaatatt     480

ggtgccctga tgggctacgc cacccacaag tatctcgata gtgaggagga tgaggagtag     540

ccagcagctc ccagaacctc ttcttccttc ttggcctaac tcttccagtt aggatctaga     600

actttgcctt tttttttttt tttttttttt tgagatgggt tctcactata ttgtccaggc     660

tagagtgcag tggctattca cagatgcgaa catagtacac tgcagcctcc aactcctagc     720

ctcaagtgat cctcctgtct caacctccca agtaggatta caagcatgcg ccgacgatgc 780

ccagaatcca gaactttgtc tatcactctc cccaacaacc tagatgtgaa aacagaataa 840

acttcaccca gaaaacactt 860

<210> 18
<211> 6646
<212> DNA
<213> Homo sapiens

<400> 18
gttcggagag ccgggcggga aaacgaaacc agaaatccga aggccgcgcc agagccctgc 60

ttccccttgc acctgcgccg ggcggccatg gacttgtaca gcaccccggc cgctgcgctg 120

gacaggttcg tggccagaag gctgcagccg cggaaggagt tcgtagagaa ggcgcggcgc 180

gctctgggcg ccctggccgc tgccctgagg gagcgcgggg gccgcctcgg tgctgctgcc 240

ccgcgggtgc tgaaaactgt caagggaggc tcctcgggcc ggggcacagc tctcaagggt 300

ggctgtgatt ctgaacttgt catcttcctc gactgcttca agagctatgt ggaccagagg 360

gcccgccgtg cagagatcct cagtgagatg cgggcatcgc tggaatcctg gtggcagaac 420

ccagtccctg gtctgagact cacgtttcct gagcagagcg tgcctggggc cctgcagttc 480

cgcctgacat ccgtagatct tgaggactgg atggatgtta gcctggtgcc tgccttcaat 540

gtcctgggtc aggccggctc cggcgtcaaa cccaagccac aagtctactc taccctcctc 600

aacagtggct gccaaggggg cgagcatgcg gcctgcttca cagagctgcg gaggaacttt 660

gtgaacattc gcccagccaa gttgaagaac ctaatcttgc tggtgaagca ctggtaccac 720

caggtgtgcc tacaggggtt gtggaaggag acgctgcccc cggtctatgc cctggaattg 780

ctgaccatct tcgcctggga gcagggctgt aagaaggatg ctttcagcct agccgaaggc 840

ctccgaactg tcctgggcct gatccaacag catcagcacc tgtgtgtttt ctggactgtc 900

aactatggct tcgaggaccc tgcagttggg cagttcttgc agcggcagct taagagaccc 960

aggcctgtga tcctggaccc agctgacccc acatgggacc tggggaatgg ggcagcctgg 1020

cactgggatt tgctagccca ggaggcagca tcctgctatg accacccatg ctttctgagg 1080

gggatggggg acccagtgca gtcttggaag gggccgggcc ttccacgtgc tggatgctca 1140

ggtttgggcc accccatcca gctagaccct aaccagaaga cccctgaaaa cagcaagagc 1200

ctcaatgctg tgtacccaag agcagggagc aaacctccct catgcccagc tcctggcccc 1260

actggggcag ccagcatcgt cccctctgtg ccgggaatgg ccttggacct gtctcagatc 1320

cccaccaagg agctggaccg cttcatccag gaccacctga agccgagccc ccagttccag 1380

gagcaggtga aaaaggccat cgacatcatc ttgcgctgcc tccatgagaa ctgtgttcac 1440

aaggcctcaa gagtcagtaa agggggctca tttggccggg gcacagacct aagggatggc 1500

tgtgatgttg aactcatcat cttcctcaac tgcttcacgg actacaagga ccaggggccc 1560

cgccgcgcag agatccttga tgagatgcga gcgcagctag aatcctggtg gcaggaccag 1620

gtgcccagcc tgagccttca gtttcctgag cagaatgtgc ctgaggctct gcagttccag 1680

ctggtgtcca cagccctgaa gagctggacg gatgttagcc tgctgcctgc cttcgatgct 1740

gtggggcagc tcagttctgg caccaaacca aatccccagg tctactcgag gctcctcacc 1800

agtggctgcc aggagggcga gcataaggcc tgcttcgcag agctgcggag gaacttcatg 1860

aacattcgcc ctgtcaagct gaagaacctg attctgctgg tgaagcactg gtaccgccag 1920

gttgcggctc agaacaaagg aaaaggacca gcccctgcct ctctgccccc agcctatgcc 1980

ctggagctcc tcaccatctt tgcctgggag cagggctgca ggcaggattg tttcaacatg 2040

gcccaaggct tccggacggt gctggggctc gtgcaacagc atcagcagct ctgtgtctac 2100

tggacggtca actatagcac tgaggaccca gccatgagaa tgcaccttct tggccagctt 2160

cgaaaaccca gacccctggt cctggacccc gctgatccca cctggaacgt gggccacggt 2220

agctgggagc tgttggccca ggaagcagca gcgctgggga tgcaggcctg ctttctgagt 2280

agagacggga catctgtgca gccctgggat gtgatgccag ccctccttta ccaaacccca 2340

gctggggacc ttgacaagtt catcagtgaa tttctccagc ccaaccgcca gttcctggcc 2400

caggtgaaca aggccgttga taccatctgt tcatttttga aggaaaactg cttccggaat 2460

tctcccatca aagtgatcaa ggtggtcaag ggtggctctt cagccaaagg cacagctctg 2520

cgaggccgct cagatgccga cctcgtggtg ttcctcagct gcttcagcca gttcactgag 2580

cagggcaaca agcgggccga gatcatctcc gagatccgag cccagctgga ggcatgtcaa 2640

caggagcggc agttcgaggt caagtttgaa gtctccaaat gggagaatcc ccgcgtgctg 2700

agcttctcac tgacatccca gacgatgctg gaccagagtg tggactttga tgtgctgcca 2760

gcctttgacg ccctaggcca gctggtctct ggctccaggc ccagctctca agtctacgtc 2820

gacctcatcc acagctacag caatgcgggc gagtactcca cctgcttcac agagctacaa 2880

cgggacttca tcatctctcg ccctaccaag ctgaagagcc tgatccggct ggtgaagcac 2940

tggtaccagc agtgtaccaa gatctccaag gggagaggct ccctaccccc acagcacggg 3000

ctggaactcc tgactgtgta tgcctgggag cagggcggga aggactccca gttcaacatg 3060

gctgagggct tccgcacggt cctggagctg gtcacccagt accgccagct ctgtatctac 3120

tggaccatca actacaacgc caaggacaag actgttggag acttcctgaa acagcagctt 3180

cagaagccca ggcctatcat cctggatccg gctgacccga caggcaacct gggccacaat 3240

gcccgctggg acctgctggc caaggaagct gcagcctgca catctgccct gtgctgcatg 3300

ggacggaatg gcatccccat ccagccatgg ccagtgaagg ctgctgtgtg aagttgagaa 3360

aatcagcggt cctactggat gaagagaaga tggacaccag ccctcagcat gaggaaattc 3420

agggtcccct accagatgag agagattgtg tacatgtgtg tgtgagcaca tgtgtgcatg 3480

tgtgtgcaca cgtgtgcatg tgtgtgtttt agtgaatctg ctctcccagc tcacacactc 3540

ccctgcctcc catggcttac acactaggat ccagactcca tggtttgaca ccagcctgcg 3600

tttgcagctt ctctgtcact tccatgactc tatcctcata ccaccactgc tgcttcccac 3660

ccagctgaga atgccccctc ctccctgact cctctctgcc catgcaaatt agctcacatc 3720

tttcctcctg ctgcaatcca tcccttcctc ccattggcct ctccttgcca aatctaaata 3780

gtttatatag ggatggcaga gagttcccat ctcatctgtc agccacagtc atttggtact 3840

ggctacctgg agccttatct tctgaagggt tttaaagaat ggccaattag ctgagaagaa 3900

ttatctaatc aattagtgat gtctgccatg gatgcagtag aggaaagtgg tggtacaagt 3960

gccatgattg attagcaatg tctgcactgg atacggaaaa aagaaggtgc ttgcaggttt 4020

acagtgtata tgtgggctat tgaagagccc tctgagctcg gttgctagca ggagagcatg 4080

cccatattgg cttactttgt ctgccacaga cacagacaga gggagttggg acatgcatgc 4140

tatggggacc ctcttgttgg acacctaatt ggatgcctct tcatgagagg cctccttttc 4200

ttcacctttt atgctgcact cctcccctag tttacacatc ttgatgctgt ggctcagttt 4260

gccttcctga atttttattg ggtccctgtt ttctctccta acatgctgag attctgcatc 4320

cccacagcct aaactgagcc agtggccaaa caaccgtgct cagcctgttt ctctctgccc 4380

tctagagcaa ggcccaccag gtccatccag gaggctctcc tgacctcaag tccaacaaca 4440

gtgtccacac tagtcaaggt tcagcccaga aaacagaaag cactctagga atcttaggca 4500

gaaagggatt ttatctaaat cactggaaag gctggaggag cagaaggcag aggccaccac 4560

tggactattg gtttcaatat tagaccactg tagccgaatc agaggccaga gagcagccac 4620

tgctactgct aatgccacca ctacccctgc catcactgcc ccacatggac aaaactggag 4680

tcgagaccta ggttagattc ctgcaaccac aaacatccat cagggatggc cagctgccag 4740

agctgcggga agacggatcc cacctccctt tcttagcaga atctaaatta cagccagacc 4800

tctggctgca gaggagtctg agacatgtat gattgaatgg gtgccaagtg ccagggggcg 4860

gagtccccag cagatgcatc ctggccatct gttgcgtgga tgagggagtg ggtctatctc 4920

agaggaagga acaggaaaca aagaaaggaa gccactgaac atcccttctc tgctccacag 4980

gagtgcctta gacagcctga ctctccacaa accactgtta aaacttacct gctaggaatg 5040

ctagattgaa tgggatggga agagccttcc ctcattattg tcattcttgg agagaggtga 5100

gcaaccaagg gaagctcctc tgattcacct agaacctgtt ctctgccgtc tttggctcag 5160

cctacagaga ctagagtagg tgaaggacag gaggacaggg cttctaatac ctgtgccata 5220

ttgacagcct ccatccctgt cccccatctt ggtgctgaac caacgctaag ggcaccttct 5280

tagactcacc tcatcgatac tgcctggtaa tccaaagcta gaactctcag gaccccaaac 5340

tccacctctt ggattggccc tggctgctgc cacacacata tccaagagct cagggccagt 5400

tctggtgggc agcagagacc tgctctgcca agttgtccag cagcagagtg gccctggcct 5460

gggcatcaca agccagtgat gctcctggga agaccaggtg gcaggtcgca gttgggtacc 5520

ttccattccc accacacaga ctctgggcct ccccgcaaaa tggctccaga attagagtaa 5580

ttatgagatg gtgggaacca gagcaactca ggtgcatgat acaaggagag gttgtcatct 5640

gggtagggca gagaggaggg cttgctcatc tgaacagggg tgtatttcat tccaggccct 5700

cagtctttgg caatggccac cctggtgttg gcatattggc cccactgtaa cttttggggg 5760

cttcccggtc tagccacacc ctcggatgga aagacttgac tgcataaaga tgtcagttct 5820

ccctgagttg attgataggc ttaatggtca ccctaaaaac acccacatat gcttttcgat 5880

ggaaccaggt aagttgacgc taaagttctt atggaaaaat acacacgcaa tagctaggaa 5940

aacacaggga aagaagagtt ctgagcaggg cctagtctta gccaatatta aaacatacta 6000

tgaagcctct gatacttaaa cagcatggcg ctggtacgta aatagaccaa tgcagttagg 6060

tggctctttc caagactctg gggaaaaaag tagtaaaaag ctaaatgcaa tcaatcagca 6120

attgaaagct aagtgagaga gccagagggc ctccttggtg gtaaaagagg gttgcatttc 6180

ttgcagccag aaggcagaga aagtgaagac caagtccaga actgaatcct aagaaatgca 6240

ggactgcaaa gaaattggtg tgtgtgtgtg tgtgtgtgtg tgtgtgtgtg tttaattttt 6300

aaaaagtttt tattgagata caagtcaata ccataaagct ctcacccttc taaagtgtac 6360

aattcagtgg tgtgagtata ttcataagat ttatacttgg tgtctattca taagacttat 6420

atccagcata ttcataacta gagccatatc acagatgcat tcatcataat aattccagac 6480

attttcatca ccctaaaagg aaaccctgaa acccattagc agtcattccc cattcctcca 6540

acccattctc tccctaatcc ctagaaacca ccaatctgct gtgtatttca tctattgcca 6600

acatttcata taaatggcat catacaaaaa aaaaaaaaaa aaaaaa 6646

<210> 19
<211> 685
<212> DNA
<213> Homo sapiens

<400> 19
ataatagggc cggtgctgcc tgccgaagcc ggcggctgag aggcagcgaa ctcatctttg 60

ccagtacagg agcttgtgcc gtggcccaca gcccacagcc cacagccatg ggctgggacc 120

tgacggtgaa gatgctggcg ggcaacgaat tccaggtgtc cctgagcagc tccatgtcgg 180

tgtcagagct gaaggcgcag atcacccaga agatcggcgt gcacgccttc cagcagcgtc 240

tggctgtcca cccgagcggt gtggcgctgc aggacagggt cccccttgcc agccagggcc 300

tgggccccgg cagcacggtc ctgctggtgg tggacaaatg cgacgaacct ctgagcatcc 360

tggtgaggaa taacaagggc cgcagcagca cctacgaggt acggctgacg cagaccgtgg 420

cccacctgaa gcagcaagtg agcgggctgg agggtgtgca ggacgacctg ttctggctga 480

ccttcgaggg gaagcccctg gaggaccagc tcccgctggg ggagtacggc ctcaagcccc 540

tgagcaccgt gttcatgaat ctgcgcctgc ggggaggcgg cacagagcct ggcgggcgga 600

gctaagggcc tccaccagca tccgagcagg atcaagggcc ggaaataaag gctgttgtaa 660

agagaaaaaa aaaaaaaaaa aaaaa 685

<210> 20
<211> 2123
<212> DNA
<213> Homo sapiens

<400> 20
caagagttgg taagctcgct gcagtgggtg gagagaggcc tctagacttc agtttcagtt 60

tcctggctct gggcagcagc aagaattcct ctgcctccca tcctaccatt cactgtcttg 120

ccggcagcca gctgagagca atgggaaatg gggagtccca gctgtcctcg gtgcctgctc 180

agaagctggg ttggtttatc caggaatacc tgaagcccta cgaagaatgt cagacactga 240

tcgacgagat ggtgaacacc atctgtgacg tcctgcagga acccgaacag ttccccctgg 300

tgcagggagt ggccataggt ggctcctatg gacggaaaac agtcttaaga ggcaactccg 360

atggtaccct tgtcctcttc ttcagtgact taaaacaatt ccaggatcag aagagaagcc 420

aacgtgacat cctcgataaa actggggata agctgaagtt ctgtctgttc acgaagtggt 480

tgaaaaacaa tttcgagatc cagaagtccc ttgatgggtt caccatccag gtgttcacaa 540

aaaatcagag aatctctttc gaggtgctgg ccgccttcaa cgctctgagt aagcattgct 600

gggtgtcagg agagaaaagc caaagaagcg ggtgccagac agctctgtgc aacctctagg 660

ccatgagtgg gatagatacc actgctgctt taaaaaatgg gagaccatag accctcagga 720

gagaagaatc ccttctaccc tggactcgct ctcttctctg gaactaactt ctcccccata 780

ccctgattgt ctttggagaa aatgttctgg attctagaat ctaaggcaga gccttttaag 840

ccatactgta cacataaatc acctggaacc ttgttaaaat gcagatcctg actcaggagg 900

tctgagttag agcccaggat ttcatatttc tagccagctc catgatgagc tgctggtccg 960

cagatcatgc ttgcaggttt tgaccagagt cagtgttggt tagagtaaga ggatgaggca 1020

gacatctggg aaaagtccag ctggggcaag catttgaagt ctgccttcct accaggtcaa 1080

aatcaaggca acgaccttcc atagataact atcaaagctt gagggggtgc cttgaaccca 1140

actcctaaat ccctaagacc tgcccacctc ttgtgtctcc tgtctcagca aacattccca 1200

cactcttgca tattgttaaa gtaacctctg cttaccaggc ttctggttta ataaaagatg 1260

gctagagtga ctccatctta aagcaagtag ctaggcactc aaaaggaacc tacaggctta 1320

atacttgggt ctgaaaatag ccacagtcta agctgaccac caattataat tgcagaatat 1380

ttaaggccat acaaaacatc tcccactaag cctacaaaat gtccaggtgt cctaaaagtt 1440

cagcccactt aaaggcagca ttaatgagca ggtttaggtt gaaggattaa tggtcatcaa 1500

taccactgtt aagaagaaaa ttcttggcca aattgaattt aatggagttt aactgagcag 1560

acaattcaca aatctagaag cctcctgagc cagagtaggt tcagagagtc ttgaacacag 1620

ccacgtggtg gaagaagatt tatggacagg aaaaggaaaa tgatgtactg aaaatgaaag 1680

tgaggtacag aaacagccag actggttata gctcagcatt ggccttattt gaacgagatt 1740

tgaacagttg gccacctttg attggccgaa actcagtgat tggcacaaga gtaggttgca 1800

gtctgtttac acatcctttt aggttatagt tcaccatgta cagagaaatt ttaggccaaa 1860

cttaaaatat gtaaggaggc agctttaggc taaacttgat ttaacagcac caataccccc 1920

tacctttagt gagcacatct gcacattcca attttaatga cagctcctta gaatttctta 1980

tcaacgaaga cactaacaaa gaatggcgca ttcctccttc tcctttctga ggatgcccta 2040

ccctgtaaca aagtcgtttc taataaattt gcttctttca ccataaaaaa aaaaaaaaaa 2100

aaaaaaaaaa aaaaaaaaaa aaa 2123

<210> 21
<211> 3647
<212> DNA
<213> Homo sapiens

<400> 21
caagagttgg taagctcgct gcagtgggtg gagagaggcc tctagacttc agtttcagtt 60

tcctggctct gggcagcagc aagaattcct ctgcctccca tcctaccatt cactgtcttg 120

ccggcagcca gctgagagca atgggaaatg gggagtccca gctgtcctcg gtgcctgctc 180

agaagctggg ttggtttatc caggaatacc tgaagcccta cgaagaatgt cagacactga 240

tcgacgagat ggtgaacacc atctgtgacg tcctgcagga acccgaacag ttccccctgg 300

tgcagggagt ggccataggt ggctcctatg gacggaaaac agtcttaaga ggcaactccg 360

atggtaccct tgtcctcttc ttcagtgact taaaacaatt ccaggatcag aagagaagcc 420

aacgtgacat cctcgataaa actggggata agctgaagtt ctgtctgttc acgaagtggt 480

tgaaaaacaa tttcgagatc cagaagtccc ttgatgggtt caccatccag gtgttcacaa 540

aaaatcagag aatctctttc gaggtgctgg ccgccttcaa cgctctgagc ttaaatgata 600

atcccagccc ctggatctat cgagagctca aaagatcctt ggataagaca aatgccagtc 660

ctggtgagtt tgcagtctgc ttcactgaac tccagcagaa gttttttgac aaccgtcctg 720

gaaaactaaa ggatttgatc ctcttgataa agcactggca tcaacagtgc cagaaaaaaa 780

tcaaggattt accctcgctg tctccgtatg ccctggagct gcttacggtg tatgcctggg 840

aacaggggtg cagaaaagac aactttgaca ttgctgaagg cgtcagaacc gtactggagc 900

tgatcaaatg ccaggagaag ctgtgtatct attggatggt caactacaac tttgaagatg 960

agaccatcag gaacatcctg ctgcaccagc tccaatcagc gaggccagta atcttggatc 1020

cagttgaccc aaccaataat gtgagtggag ataaaatatg ctggcaatgg ctgaaaaaag 1080

aagctcaaac ctggttgact tctcccaacc tggataatga gttacctgca ccatcttgga 1140

atgttctgcc tgcaccactc ttcacgaccc caggccacct tctggataag ttcatcaagg 1200

agtttctcca gcccaacaaa tgcttcctag agcagattga cagtgctgtt aacatcatcc 1260

gtacattcct taaagaaaac tgcttccgac aatcaacagc caagatccag attgtccggg 1320

gaggatcaac cgccaaaggc acagctctga agactggctc tgatgccgat ctcgtcgtgt 1380

tccataactc acttaaaagc tacacctccc aaaaaaacga gcggcacaaa atcgtcaagg 1440

aaatccatga acagctgaaa gccttttgga gggagaagga ggaggagctt gaagtcagct 1500

ttgagcctcc caagtggaag gctcccaggg tgctgagctt ctctctgaaa tccaaagtcc 1560

tcaacgaaag tgtcagcttt gatgtgcttc ctgcctttaa tgcactgggt cagctgagtt 1620

ctggctccac acccagcccc gaggtttatg cagggctcat tgatctgtat aaatcctcgg 1680

acctcccggg aggagagttt tctacctgtt tcacagtcct gcagcgaaac ttcattcgct 1740

cccggcccac caaactaaag gatttaattc gcctggtgaa gcactggtac aaagagtgtg 1800

aaaggaaact gaagccaaag gggtctttgc ccccaaagta tgccttggag ctgctcacca 1860

tctatgcctg ggagcagggg agtggagtgc cggattttga cactgcagaa ggtttccgga 1920

cagtcctgga gctggtcaca caatatcagc agctctgcat cttctggaag gtcaattaca 1980

actttgaaga tgagaccgtg aggaagtttc tactgagcca gttgcagaaa accaggcctg 2040

tgatcttgga cccagccgaa cccacaggtg acgtgggtgg aggggaccgt tggtgttggc 2100

atcttctggc aaaagaagca aaggaatggt tatcctctcc ctgcttcaag gatgggactg 2160

gaaacccaat accaccttgg aaagtgccgg taaaagtcat ctaaaggagg cgttgtctgg 2220

aaatagccct gtaacaggct tgaatcaaag aacttctcct actgtagcaa cctgaaatta 2280

actcagacac aaataaagga aacccagctc acaggagctt aaacagctgg tcagccccct 2340

aagcccccac tacaagtgat cctcaggcag gtaaccccag attcatgcac tgtagggtgc 2400

tgcgcagcat ccctagtctc tacccagtag atgccactag ccctcctctc ccagtgacaa 2460

ccaaaagtct tcagacattg tcaaacgttc ccctgggttc acagatcttt ctgcctttgg 2520

cttttggctc caccctcttt agctgttaat ttgagtactt atggccctga aagcggccac 2580

ggtgcctcca gatggcaggt ttgcaatcca agcaggaaga aggaaaagat acccaaaggt 2640

caagaacaca gtgattttat tagaagtttc atccgcaaat tttcttccat ttcattgctc 2700

agaaatgtca tgtggctacc tgtaacttga aggtggctac aaagatgact gtggacgtgg 2760

gttgcactgg ccacccaagg atgtctgcca cacctctcca aagccctccc tacctaccaa 2820

gatatacctg atatattcca ccaggatatc ctccctccag atatacttgg ttctctccac 2880

caggttcttt ctttaaagca ggatttctca actttgatac ttactcacat ttggggctag 2940

acagttcttt gtttggaggc tctcttgtgc attgtaggat gttgagcagc atctctggcc 3000

tgtacccagt agatgccacc cagttgtgac aattaaaagt gtcttgagac tttatcatgt 3060

gtcttctgcc ctaggtgaga acccttgcac tagaggaacc ctacacccca accctggggg 3120

gaatgtaggg aagaggtggc caagccaacc gtggggttag ctctaattat taagatatgc 3180

attataaata aataccaaaa aattgtctct ggcaatagtt accttcccag atacaggtcc 3240

cccctttttt cccctaactc ttttaagcaa tgattgtaac tattaggaga cattgctctc 3300

ccacgtatgt tttctttttt agacaatgca gacaccagga agttgtggag ctaggatcca 3360

tcctattgtc aatgagatgt tctcatccag aagccataga atcctgaata ataattctaa 3420

aagaaacttc tagagatcat ctggcaatcg cttttaaaga ctcggctcac cgtgagaaag 3480

agtcactcac atccattctt cccttgatgg tccctattcc tccttccctt gcttcttgga 3540

cttcttgaaa tcaatcaaga ctgcaaaccc tttcataaag tcttgccttg ctgaactccc 3600

tctctgcagg cagcctgcct ttaaaaatag ttgctgtcat ccacttt 3647

<210> 22
<211> 3539
<212> DNA
<213> Homo sapiens

<400> 22
caagagttgg taagctcgct gcagtgggtg gagagaggcc tctagacttc agtttcagtt 60

tcctggctct gggcagcagc aagaattcct ctgcctccca tcctaccatt cactgtcttg 120

ccggcagcca gctgagagca atgggaaatg gggagtccca gctgtcctcg gtgcctgctc 180

agaagctggg ttggtttatc caggaatacc tgaagcccta cgaagaatgt cagacactga 240

tcgacgagat ggtgaacacc atctgtgacg tcctgcagga acccgaacag ttccccctgg 300

tgcagggagt ggccataggt ggctcctatg gacggaaaac agtcttaaga ggcaactccg 360

atggtaccct tgtcctcttc ttcagtgact taaaacaatt ccaggatcag aagagaagcc 420

aacgtgacat cctcgataaa actggggata agctgaagtt ctgtctgttc acgaagtggt 480

tgaaaaacaa tttcgagatc cagaagtccc ttgatgggtt caccatccag gtgttcacaa 540

aaaatcagag aatctctttc gaggtgctgg ccgccttcaa cgctctgagc ttaaatgata 600

atcccagccc ctggatctat cgagagctca aaagatcctt ggataagaca aatgccagtc 660

ctggtgagtt tgcagtctgc ttcactgaac tccagcagaa gttttttgac aaccgtcctg 720

gaaaactaaa ggatttgatc ctcttgataa agcactggca tcaacagtgc cagaaaaaaa 780

tcaaggattt accctcgctg tctccgtatg ccctggagct gcttacggtg tatgcctggg 840

aacaggggtg cagaaaagac aactttgaca ttgctgaagg cgtcagaacc gtactggagc 900

tgatcaaatg ccaggagaag ctgtgtatct attggatggt caactacaac tttgaagatg 960

agaccatcag gaacatcctg ctgcaccagc tccaatcagc gaggccagta atcttggatc 1020

cagttgaccc aaccaataat gtgagtggag ataaaatatg ctggcaatgg ctgaaaaaag 1080

aagctcaaac ctggttgact tctcccaacc tggataatga gttacctgca ccatcttgga 1140

atgttctgcc tgcaccactc ttcacgaccc caggccacct tctggataag ttcatcaagg 1200

agtttctcca gcccaacaaa tgcttcctag agcagattga cagtgctgtt aacatcatcc 1260

gtacattcct taaagaaaac tgcttccgac aatcaacagc caagatccag attgtccggg 1320

gaggatcaac cgccaaaggc acagctctga agactggctc tgatgccgat ctcgtcgtgt 1380

tccataactc acttaaaagc tacacctccc aaaaaaacga gcggcacaaa atcgtcaagg 1440

aaatccatga acagctgaaa gccttttgga gggagaagga ggaggagctt gaagtcagct 1500

ttgagcctcc caagtggaag gctcccaggg tgctgagctt ctctctgaaa tccaaagtcc 1560

tcaacgaaag tgtcagcttt gatgtgcttc ctgcctttaa tgcactgggt cagctgagtt 1620

ctggctccac acccagcccc gaggtttatg cagggctcat tgatctgtat aaatcctcgg 1680

acctcccggg aggagagttt tctacctgtt tcacagtcct gcagcgaaac ttcattcgct 1740

cccggcccac caaactaaag gatttaattc gcctggtgaa gcactggtac aaagagtgtg 1800

aaaggaaact gaagccaaag gggtctttgc ccccaaagta tgccttggag ctgctcacca 1860

tctatgcctg ggagcagggg agtggagtgc cggattttga cactgcagaa ggtttccgga 1920

cagtcctgga gctggtcaca caatatcagc agctctgcat cttctggaag gtcaattaca 1980

actttgaaga tgagaccgtg aggaagtttc tactgagcca gttgcagaaa accaggcctg 2040

tgatcttgga cccagccgaa cccacaggtg acgtgggtgg aggggaccgt tggtgttggc 2100

atcttctggc aaaagaagca aaggaatggt tatcctctcc ctgcttcaag gatgggactg 2160

gaaacccaat accaccttgg aaagtgccga caatgcagac accaggaagt tgtggagcta 2220

ggatccatcc tattgtcaat gagatgttct catccagaag ccatagaatc ctgaataata 2280

attctaaaag aaacttctag agatcatctg gcaatcgctt ttaaagactc ggctcaccgt 2340

gagaaagagt cactcacatc cattcttccc ttgatggtcc ctattcctcc ttcccttgct 2400

tcttggactt cttgaaatca atcaagactg caaacccttt cataaagtct tgccttgctg 2460

aactccctct ctgcaggcag cctgccttta aaaatagttg ctgtcatcca ctttatgtgc 2520

atcttatttc tgtcaacttg tatttttttt cttgtatttt tccaattagc tcctcctttt 2580

tccttccagt ctaaaaaagg aatcctctgt gtcttcaaag caaagctctt tactttcccc 2640

ttggttctca taactctgtg atcttgctct cggtgcttcc aactcatcca cgtcctgtct 2700

gtttcctctg tatacaaaac cctttctgcc cctgctgaca cagacatcct ctatgccagc 2760

agccagccaa ccctttcatt agaacttcaa gctctccaaa ggctcagatt ataactgttg 2820

tcatatttat atgaggctgt tgtcttttcc ttctgagcct gcctttctcc cccccaccca 2880

ggagtatcct cttgccaaat caaaagactt tttccttggg ctttagcctt aaagatactt 2940

gaaggtctag gtgctttaac ctcacatacc ctcacttaaa cttttatcac tgttgcatat 3000

accagttgtg atacaataaa gaatgtatct ggattttgtg cctagttcct agcacacagc 3060

ttcaaaaatt ctagagtttc ctgataggag tgtcttttgt attcataaca agcccttttc 3120

acccatgcct gggtttatgc taacaaggtt acccatggtg ggcccttagt ttcaaggaag 3180

gagttggcca agccagaaag accaagcatg tggttaaagc attggaattt tcagccccat 3240

cccaccccca atctccaagg aggtgatggg gctggaaatt gagttcaatt ttaacatggc 3300

cagtgattta agcaatgctg cctatgtaaa gaaaccccaa taaaaactct ggacagtgag 3360

gcttggggag cttcctgatt ggcagacatt ccaatgtact aggaaggtag cgcatcttga 3420

ttccacaggg acaaaggctc ctgagctctg ggcccttcca gtgcttgcca ccctacatac 3480

tctttgtctg gctcttcatt tgtattcttt ataataaaat ggtgattgta agtagagca 3539

<210> 23
<211> 1876
<212> DNA
<213> Homo sapiens

<400> 23
ttacaccatt ggctgctgtt tagctccctt atataacact gtcttggggt ttaaacgtaa 60

ctgaaaatcc acaagacaga atagccagat ctcagaggag cctggctaag caaaaccctg 120

cagaacggct gcctaattta cagcaaccat gagtacaaat ggtgatgatc atcaggtcaa 180

ggatagtctg gagcaattga gatgtcactt tacatgggag ttatccattg atgacgatga 240

aatgcctgat ttagaaaaca gagtcttgga tcagattgaa ttcctagaca ccaaatacag 300

tgtgggaata cacaacctac tagcctatgt gaaacacctg aaaggccaga atgaggaagc 360

cctgaagagc ttaaaagaag ctgaaaactt aatgcaggaa gaacatgaca accaagcaaa 420

tgtgaggagt ctggtgacct ggggcaactt tgcctggatg tattaccaca tgggcagact 480

ggcagaagcc cagacttacc tggacaaggt ggagaacatt tgcaagaagc tttcaaatcc 540

cttccgctat agaatggagt gtccagaaat agactgtgag gaaggatggg ccttgctgaa 600

gtgtggagga aagaattatg aacgggccaa ggcctgcttt gaaaaggtgc ttgaagtgga 660

ccctgaaaac cctgaatcca gcgctgggta tgcgatctct gcctatcgcc tggatggctt 720

taaattagcc acaaaaaatc acaagccatt ttctttgctt cccctaaggc aggctgtccg 780

cttaaatcca gacaatggat atattaaggt tctccttgcc ctgaagcttc aggatgaagg 840

acaggaagct gaaggagaaa agtacattga agaagctcta gccaacatgt cctcacagac 900

ctatgtcttt cgatatgcag ccaagtttta ccgaagaaaa ggctctgtgg ataaagctct 960

tgagttatta aaaaaggcct tgcaggaaac acccacttct gtcttactgc atcaccagat 1020

agggctttgc tacaaggcac aaatgatcca aatcaaggag gctacaaaag ggcagcctag 1080

agggcagaac agagaaaagc tagacaaaat gataagatca gccatatttc attttgaatc 1140

tgcagtggaa aaaaagccca catttgaggt ggctcatcta gacctggcaa gaatgtatat 1200

agaagcaggc aatcacagaa aagctgaaga gaattttcaa aaattgttat gcatgaaacc 1260

agtggtagaa gaaacaatgc aagacataca tttccactat ggtcggtttc aggaatttca 1320

aaagaaatct gacgtcaatg caattatcca ttatttaaaa gctataaaaa tagaacaggc 1380

atcattaaca agggataaaa gtatcaattc tttgaagaaa ttggttttaa ggaaacttcg 1440

gagaaaggca ttagatctgg aaagcttgag cctccttggg ttcgtctaca aattggaagg 1500

aaatatgaat gaagccctgg agtactatga gcgggccctg agactggctg ctgactttga 1560

gaactctgtg agacaaggtc cttaggcacc cagatatcag ccactttcac atttcatttc 1620

attttatgct aacatttact aatcatcttt tctgcttact gttttcagaa acattataat 1680

tcactgtaat gatgtaattc ttgaataata aatctgacaa aatattagtt gtgttcaaca 1740

attagtgaaa cagaatgtgt gtatgcatgt aagaaagaga aatcatttgt atgagtgcta 1800

tgtagtagag aaaaaatgtt agttaacttt gtaggaaata aaacattgga cttacactaa 1860

aaaaaaaaaa aaaaaa 1876

<210> 24
<211> 532
<212> DNA
<213> Homo sapiens

<400> 24
gaaatgccca aaaccttcag agattgacac gctgtcattt tccatttccg ttcctggatc 60

tacggagtct tctaagagat tttgcaatga ggagaagcat tgttttcaaa ctatataact 120

gaccttattt ataattaggg atattatcaa aatatgtaac catgaggccc ctcaggtcct 180

gatcagtcag aatggatgct ttcaccagca gacccggcca tgtggctgct cggtcctggg 240

tgctcgctgc tgtgcaagac attagccctt tagttatgag cctgtgggaa cttcaggggt 300

tcccagtggg gagagcagtg gcagtgggag gcatctgggg gccaaaggtc agtggcaggg 360

ggtatttcag tattatacaa ctgctgtgac cagacttgta tactggctga atatcagtgc 420

tgtttgtaat tttcactttt gagaaccaac attaattcca tatgaatcaa gtgttttgta 480

actgctattc atttattcag caaatattta ttgagcatct cttctccata ag 532

<210> 25
<211> 532
<212> DNA
<213> Homo sapiens

<400> 25
acagttcaca tcccaccata gtgatgtttt ggaaggcttt ccacaaattg actctggaag 60

aaaagaaaaa attccttgta tttcttacag gaactgacag actacaaatg aaagatttaa 120

ataatatgaa aataacattt tgctgtcctg aaagttggaa tgaaagagac cctataagag 180

cactgacatg tttcagtgtc ctcttcctcc ctaaatattc tacaatggaa acagttgaag 240

aagcgcttca agaagccatc aacaacaaca gaggatttgg ctgaccagct tgcttgtcca 300

acagccttat tttgttgttg ttatcgttgt tgttgttgtt gttgttgttg tttctctact 360

ttgttttgtt ttaggctttt agcagcctga agccatggtt tttcatttct gtctctagtg 420

ataagcagga aagagggatg aagaagaggg tttactggcc ggttagaacc cgtgactgta 480

ttctctccct tggatacccc tatgcctaca tcatattcct tacctctttt gg 532

<210> 26
<211> 511
<212> DNA
<213> Homo sapiens

<400> 26
gtgtgttgtg tatggactca ctcccaggtt cacctggcca caggtgcacc cttcccacac 60

cctttacatt ccccagagcc aagggagttt aagtttgcag ttacaggcca gttctccagc 120

tctccatctt agagagacag gtcaccttgc aggcctgctt gcaggaaatg aatccagcag 180

ccaactcgaa tccccctagg gctcaggcac tgagggcctg gggacagtgg agcatatggg 240

tgggagacag atggagggta ccctatttac aactgagtca gccaagccac tgatgggaat 300

atacagattt aggtgctaaa ccgtttattt tccacggatg agtcacaatc tgaagaatca 360

aacttccatc ctgaaaatct atatgtttca aaaccacttg ccatcctgtt agattgccag 420

ttcctgggac caggcctcag actgtgaagt atatatcctc cagcattcag tccaggggga 480

gccacggaaa ccatgttctt gcttaagcca t 511

<210> 27
<211> 2037
<212> DNA
<213> Homo sapiens

<400> 27
gggaagctcg ggccggcagg gtttccccgc acgctggcgc ccagctcccg gcgcggaggc 60

cgctgtaagt ttcgctttcc attcagtgga aaacgaaagc tgggcggggt gccacgagcg 120

cggggccaga ccaaggcggg cccggagcgg aacttcggtc ccagctcggt ccccggctca 180

gtcccgacgt ggaactcagc agcggaggct ggacgcttgc atggcgcttg agagattcca 240

tcgtgcctgg ctcacataag cgcttcctgg aagtgaagtc gtgctgtcct gaacgcgggc 300

caggcagctg cggcctgggg gttttggagt gatcacgaat gagcaaggcg tttgggctcc 360

tgaggcaaat ctgtcagtcc atcctggctg agtcctcgca gtccccggca gatcttgaag 420

aaaagaagga agaagacagc aacatgaaga gagagcagcc cagagagcgt cccagggcct 480

gggactaccc tcatggcctg gttggtttac acaacattgg acagacctgc tgccttaact 540

ccttgattca ggtgttcgta atgaatgtgg acttcaccag gatattgaag aggatcacgg 600

tgcccagggg agctgacgag cagaggagaa gcgtcccttt ccagatgctt ctgctgctgg 660

agaagatgca ggacagccgg cagaaagcag tgcggcccct ggagctggcc tactgcctgc 720

agaagtgcaa cgtgcccttg tttgtccaac atgatgctgc ccaactgtac ctcaaactct 780

ggaacctgat taaggaccag atcactgatg tgcacttggt ggagagactg caggccctgt 840

atacgatccg ggtgaaggac tccttgattt gcgttgactg tgccatggag agtagcagaa 900

acagcagcat gctcaccctc ccactttctc tttttgatgt ggactcaaag cccctgaaga 960

cactggagga cgccctgcac tgcttcttcc agcccaggga gttatcaagc aaaagcaagt 1020

gcttctgtga gaactgtggg aagaagaccc gtgggaaaca ggtcttgaag ctgacccatt 1080

tgccccagac cctgacaatc cacctcatgc gattctccat caggaattca cagacgagaa 1140

agatctgcca ctccctgtac ttcccccaga gcttggattt cagccagatc cttccaatga 1200

agcgagagtc ttgtgatgct gaggagcagt ctggagggca gtatgagctt tttgctgtga 1260

ttgcgcacgt gggaatggca gactccggtc attactgtgt ctacatccgg aatgctgtgg 1320

atggaaaatg gttctgcttc aatgactcca atatttgctt ggtgtcctgg gaagacatcc 1380

agtgtaccta cggaaatcct aactaccact ggcaggaaac tgcatatctt ctggtttaca 1440

tgaagatgga gtgctaatgg aaatgcccaa aaccttcaga gattgacacg ctgtcatttt 1500

ccatttccgt tcctggatct acggagtctt ctaagagatt ttgcaatgag gagaagcatt 1560

gttttcaaac tatataactg agccttattt ataattaggg atattatcaa aatatgtaac 1620

catgaggccc ctcaggtcct gatcagtcag aatggatgct ttcaccagca gacccggcca 1680

tgtggctgct cggtcctggg tgctcgctgc tgtgcaagac attagccctt tagttatgag 1740

cctgtgggaa cttcaggggt tcccagtggg gagagcagtg gcagtgggag gcatctgggg 1800

gccaaaggtc agtggcaggg ggtatttcag tattatacaa ctgctgtgac cagacttgta 1860

tactggctga atatcagtgc tgtttgtaat tttcactttt gagaaccaac attaattcca 1920

tatgaatcaa gtgttttgta actgctattc atttattcag caaatattta ttgatcatct 1980

cttctccata agatagtgtg ataaacacag tcatgaataa agttattttc cacaaaa 2037

<210> 28
<211> 3525
<212> DNA
<213> Homo sapiens

<400> 28
tcagtagctg aggctgcggt tccccgacgc cacgcagctg cgcgcagctg gttcccgctc 60

tgcagcgcaa cgcctgaggc agtgggcgcg ctcagtcccg ggaccaggcg ttctcctcct 120

tcgcctctgg gcctgggacc ccgcaaagcg gcgatggagc ggaggtcgcg gaggaagtcg 180

cggcgcaacg ggcgctcgac cgcgggcaag gccgccgcga cccagcccgc gaagtctccg 240

ggcgcacagc tctggctctt tcccagcgcc gcgggcctcc accgcgcgct gctccggagg 300

gtggaggtga cgcgccaact ctgctgctcg ccggggcgcc tcgcggtctt ggaacgcggc 360

ggggcgggcg tccaggttca ccagctgctc gccgggagcg gcggcgcccg gacgccgaaa 420

tgcattaaat taggaaaaaa catgaagata cattccgtgg accaaggagc agagcacatg 480

ctgattctct catcagatgg aaaaccattt gagtatgaca actatagcat gaaacatcta 540

aggtttgaaa gcattttaca agaaaaaaaa ataattcaga tcacatgtgg agattaccat 600

tctcttgcac tctcaaaagg tggtgagctt tttgcctggg gacagaacct gcatgggcag 660

cttggagttg gaaggaaatt tccctcaacc accacaccac agattgtgga gcacctcgca 720

ggagtaccct tggctcagat ttctgccgga gaagcccaca gcatggcctt atccatgtct 780

ggcaacattt attcatgggg aaaaaatgaa tgtggacaac taggcctggg ccacactgag 840

agtaaagatg atccatccct tattgaagga ctagacaatc agaaagttga atttgtcgct 900

tgtggtggct ctcacagtgc cctactcaca caggatgggc tgctgtttac tttcggtgct 960

ggaaaacatg ggcaacttgg tcataattca acacagaatg agctaagacc ctgtttggtg 1020

gctgagcttg ttgggtatag agtgactcag atagcatgtg gaaggtggca cacacttgcc 1080

tatgtttctg atttgggaaa ggtcttttcc tttggttctg gaaaagatgg acaactggga 1140

aatggtggaa cacgtgacca gctgatgccg cttccagtga aagtatcatc aagtgaagaa 1200

ctcaaacttg aaagccatac ctcagaaaag gagttaataa tgattgctgg agggaatcaa 1260

agcattttgc tctggataaa gaaagagaat tcatatgtta atctgaagag gacaattcct 1320

actctgaatg aagggactgt aaagagatgg attgctgatg tggagactaa acggtggcag 1380

agcacaaaaa gggaaatcca agagatattt tcatctcctg cttgtctaac tggaagtttt 1440

ttaaggaaaa gaagaactac agaaatgatg cctgtttatt tggacttaaa taaagcaaga 1500

aacatcttca aggagttaac ccaaaaggac tggattacta acatgataac cacctgcctc 1560

aaagataatc tgctcaaaag acttccattt cattctccac cccaagaagc tttagaaatt 1620

ttcttccttc tcccagaatg tcctatgatg catatttcca acaactggga gagccttgtg 1680

gttccatttg caaaggttgt ttgtaaaatg agtgaccagt cttcactggt tctggaagag 1740

tattgggcaa ctctgcaaga atccactttc agcaaactgg tccagatgtt taaaacagcc 1800

gtcatatgcc agttggatta ctgggatgaa agtgctgagg agaatggtaa tgttcaagct 1860

ctcctagaaa tgttgaagaa gctgcacagg gtaaaccagg tgaaatgtca actacctgaa 1920

agtattttcc aagtagacga actcttgcac cgtctcaatt tttttgtaga agtatgcaga 1980

aggtacttgt ggaaaatgac tgtggacgct tcagaaaatg tacaatgctg cgtcatattc 2040

agtcactttc catttatctt taataatctg tcgaaaatta aactactaca tacagacaca 2100

cttttaaaaa tagagagtaa aaaacataaa gcttatctta ggtcggcagc aattgaggaa 2160

gaaagagagt ctgaattcgc tttgaggccc acgtttgatc taacagtcag aaggaatcac 2220

ttgattgagg atgttttgaa tcagctaagt caatttgaga atgaagacct gaggaaagag 2280

ttatgggttt catttagtgg agaaattggg tatgacctcg gaggagtcaa gaaagagttc 2340

ttctactgtc tgtttgcaga gatgatccag ccggaatatg ggatgttcat gtatcctgaa 2400

ggggcttcct gcatgtggtt tcctgtcaag cctaaatttg agaagaaaag atacttcttt 2460

tttggggttc tatgtggact ttccctgttc aattgcaatg ttgccaacct tcctttccca 2520

ctggcactgt ttaagaaact tttggaccaa atgccatcat tggaagactt gaaagaactc 2580

agtcctgatt tgggaaagaa tttgcaaaca cttctggatg atgaaggtga taactttgag 2640

gaagtatttt acatccattt taatgtgcac tgggacagaa acgacacaaa cttaattcct 2700

aatggaagta gcataactgt caaccagact aacaagagag actatgtttc taagtatatc 2760

aattacattt tcaacgactc tgtaaaggcg gtttatgaag aatttcggag aggattttat 2820

aaaatgtgcg acgaagacat tatcaaatta ttccaccccg aagaactgaa ggatgtgatt 2880

gttggaaata cagattatga ttggaaaaca tttgaaaaga atgcacgtta tgaaccagga 2940

tataacagtt cacatcccac catagtgatg ttttggaagg ctttccacaa attgactctg 3000

gaagaaaaga aaaaattcct tgtatttctt acaggaactg acagactaca aatgaaagat 3060

ttaaataata tgaaaataac attttgctgt cctgaaagtt ggaatgaaag agaccctata 3120

agagcactga catgtttcag tgtcctcttc ctccctaaat attctacaat ggaaacagtt 3180

gaagaagcgc ttcaagaagc catcaacaac aacagaggat ttggctgacc agcttgcttg 3240

tccaacagcc ttattttgtt gttgttatcg ttgttgttgt tgttgttgtt gttgtttctc 3300

tactttgttt tgttttaggc ttttagcagc ctgaagccat ggtttttcat ttctgtctct 3360

agtgataagc aggaaagagg gatgaagaag agggtttact ggccggttag aacccgtgac 3420

tgtattctct cccttggata cccctatgcc tacatcatat tccttacctc ttttgggaaa 3480

tatttttcaa aaataaaata accgaaaaat taaaaaaaaa aaaaa 3525

<210>  29
<211>  1260
<212>  DNA
<213>  Homo sapiens

<400>  29
gggggtgggg tccccggggc ggggcggggc gcgctgtgtc gcgggtcgga gctcggtcct      60

gctggaggcc acgggtgcca cacactcggt cccgacatga tggcgagcat gcgagtggtg     120

aaggagctgg aggatcttca gaagaagcct cccccatacc tgcggaacct gtccagcgat     180

gatgccaatg tcctggtgtg gcacgctctc ctcctacccg accaacctcc ctaccacctg     240

aaagccttca acctgcgcat cagcttcccg ccggagtatc cgttcaagcc tcccatgatc     300

aaattcacaa ccaagatcta ccaccccaac gtggacgaga acggacagat ttgcctgccc     360

atcatcagca gtgagaactg gaagccttgc accaagactt gccaagtcct ggaggccctc     420

aatgtgctgg tgaatagacc gaatatcagg gagcccctgc ggatggacct cgctgacctg     480

ctgacacaga atccggagct gttcagaaag aatgccgaag agttcaccct ccgattcgga     540

gtggaccggc cctcctaact catgttctga ccctctgtgc actggatcct cggcatagcg     600

gacggacaca cctcatggac tgaggccaga gccccctgtg gcccattccc cattcatttt     660

tcccttctta ggttgttagt cattagtttg tgtgtgtgtg tggtggaggg aagggagcta     720

tgagtgtgtg tgttgtgtat ggactcactc ccaggttcac ctggccacag gtgcaccctt     780

cccacaccct ttacattccc cagagccaag ggagtttaag tttgcagtta caggccagtt     840

ctccagctct ccatcttaga gagacaggtc accttgcagg cctgcttgca ggaaatgaat     900

ccagcagcca actcgaatcc ccctagggct caggcactga gggcctgggg acagtggagc     960

atatgggtgg gagacagatg gagggtaccc tatttacaac tgagtcagcc aagccactga    1020

tgggaatata cagatttagg tgctaaaccg tttattttcc acggatgagt cacaatctga    1080

agaatcaaac ttccatcctg aaaatctata tgtttcaaaa ccacttgcca tcctgttaga    1140

ttgccagttc ctgggaccag gcctcagact gtgaagtata tatcctccag cattcagtcc    1200

agggggagcc acggaaacca tgttcttgct taagccatta aagtcagaga tgaattctgg    1260


<210>  30
<211>  372
<212>  PRT
<213>  Homo sapiens

<400>  30

Met Ser Lys Ala Phe Gly Leu Leu Arg Gln Ile Cys Gln Ser Ile Leu
1               5                   10                  15


Ala Glu Ser Ser Gln Ser Pro Ala Asp Leu Glu Glu Lys Lys Glu Glu

20                  25                  30

Asp Ser Asn Met Lys Arg Glu Gln Pro Arg Glu Arg Pro Arg Ala Trp
        35                  40                  45

Asp Tyr Pro His Gly Leu Val Gly Leu His Asn Ile Gly Gln Thr Cys
    50                  55                  60

Cys Leu Asn Ser Leu Ile Gln Val Phe Val Met Asn Val Asp Phe Thr
65                  70                  75                  80

Arg Ile Leu Lys Arg Ile Thr Val Pro Arg Gly Ala Asp Glu Gln Arg
                85                  90                  95

Arg Ser Val Pro Phe Gln Met Leu Leu Leu Leu Glu Lys Met Gln Asp
            100                 105                 110

Ser Arg Gln Lys Ala Val Arg Pro Leu Glu Leu Ala Tyr Cys Leu Gln
        115                 120                 125

Lys Cys Asn Val Pro Leu Phe Val Gln His Asp Ala Ala Gln Leu Tyr
    130                 135                 140

Leu Lys Leu Trp Asn Leu Ile Lys Asp Gln Ile Thr Asp Val His Leu
145                 150                 155                 160

Val Glu Arg Leu Gln Ala Leu Tyr Thr Ile Arg Val Lys Asp Ser Leu
                165                 170                 175

Ile Cys Val Asp Cys Ala Met Glu Ser Ser Arg Asn Ser Ser Met Leu
            180                 185                 190

Thr Leu Pro Leu Ser Leu Phe Asp Val Asp Ser Lys Pro Leu Lys Thr
        195                 200                 205

Leu Glu Asp Ala Leu His Cys Phe Phe Gln Pro Arg Glu Leu Ser Ser
    210                 215                 220

Lys Ser Lys Cys Phe Cys Glu Asn Cys Gly Lys Lys Thr Arg Gly Lys
225                 230                 235                 240

Gln Val Leu Lys Leu Thr His Leu Pro Gln Thr Leu Thr Ile His Leu
                245                 250                 255

Met Arg Phe Ser Ile Arg Asn Ser Gln Thr Arg Lys Ile Cys His Ser
            260                 265                 270

Leu Tyr Phe Pro Gln Ser Leu Asp Phe Ser Gln Ile Leu Pro Met Lys
        275                 280                 285

Arg Glu Ser Cys Asp Ala Glu Glu Gln Ser Gly Gly Gln Tyr Glu Leu
    290                 295                 300

Phe Ala Val Ile Ala His Val Gly Met Ala Asp Ser Gly His Tyr Cys
305                 310                 315                 320

Val Tyr Ile Arg Asn Ala Val Asp Gly Lys Trp Phe Cys Phe Asn Asp
                325                 330                 335

Ser Asn Ile Cys Leu Val Ser Trp Glu Asp Ile Gln Cys Thr Tyr Gly
            340                 345                 350

Asn Pro Asn Tyr His Trp Gln Glu Thr Ala Tyr Leu Leu Val Tyr Met
        355                 360                 365

Lys Met Glu Cys
    370

<210>  31
<211>  1024
<212>  PRT
<213>  Homo sapiens

<400>  31

Met Glu Arg Arg Ser Arg Arg Lys Ser Arg Arg Asn Gly Arg Ser Thr
1               5                   10                  15

Ala Gly Lys Ala Ala Ala Thr Gln Pro Ala Lys Ser Pro Gly Ala Gln
            20                  25                  30

Leu Trp Leu Phe Pro Ser Ala Ala Gly Leu His Arg Ala Leu Leu Arg
        35                  40                  45

Arg Val Glu Val Thr Arg Gln Leu Cys Cys Ser Pro Gly Arg Leu Ala
    50                  55                  60

Val Leu Glu Arg Gly Gly Ala Gly Val Gln Val His Gln Leu Leu Ala
65                  70                  75                  80

Gly Ser Gly Gly Ala Arg Thr Pro Lys Cys Ile Lys Leu Gly Lys Asn
                85                  90                  95

Met Lys Ile His Ser Val Asp Gln Gly Ala Glu His Met Leu Ile Leu
            100                 105                 110

Ser Ser Asp Gly Lys Pro Phe Glu Tyr Asp Asn Tyr Ser Met Lys His
        115                 120                 125

Leu Arg Phe Glu Ser Ile Leu Gln Glu Lys Lys Ile Ile Gln Ile Thr
    130                 135                 140

Cys Gly Asp Tyr His Ser Leu Ala Leu Ser Lys Gly Gly Glu Leu Phe
145                 150                 155                 160

Ala Trp Gly Gln Asn Leu His Gly Gln Leu Gly Val Gly Arg Lys Phe
                165                 170                 175

Pro Ser Thr Thr Thr Pro Gln Ile Val Glu His Leu Ala Gly Val Pro
            180                 185                 190

Leu Ala Gln Ile Ser Ala Gly Glu Ala His Ser Met Ala Leu Ser Met
        195                 200                 205

Ser Gly Asn Ile Tyr Ser Trp Gly Lys Asn Glu Cys Gly Gln Leu Gly
    210                 215                 220

Leu Gly His Thr Glu Ser Lys Asp Asp Pro Ser Leu Ile Glu Gly Leu
225                 230                 235                 240

Asp Asn Gln Lys Val Glu Phe Val Ala Cys Gly Gly Ser His Ser Ala
                245                 250                 255

Leu Leu Thr Gln Asp Gly Leu Leu Phe Thr Phe Gly Ala Gly Lys His
            260                 265                 270

Gly Gln Leu Gly His Asn Ser Thr Gln Asn Glu Leu Arg Pro Cys Leu
        275                 280                 285

Val Ala Glu Leu Val Gly Tyr Arg Val Thr Gln Ile Ala Cys Gly Arg
    290                 295                 300

Trp His Thr Leu Ala Tyr Val Ser Asp Leu Gly Lys Val Phe Ser Phe
305                 310                 315                 320

Gly Ser Gly Lys Asp Gly Gln Leu Gly Asn Gly Gly Thr Arg Asp Gln
                325                 330                 335

Leu Met Pro Leu Pro Val Lys Val Ser Ser Ser Glu Glu Leu Lys Leu
            340                 345                 350

Glu Ser His Thr Ser Glu Lys Glu Leu Ile Met Ile Ala Gly Gly Asn
        355                 360                 365

Gln Ser Ile Leu Leu Trp Ile Lys Lys Glu Asn Ser Tyr Val Asn Leu
    370                 375                 380

Lys Arg Thr Ile Pro Thr Leu Asn Glu Gly Thr Val Lys Arg Trp Ile
385                 390                 395                 400

Ala Asp Val Glu Thr Lys Arg Trp Gln Ser Thr Lys Arg Glu Ile Gln
                405                 410                 415

Glu Ile Phe Ser Ser Pro Ala Cys Leu Thr Gly Ser Phe Leu Arg Lys
            420                 425                 430

Arg Arg Thr Thr Glu Met Met Pro Val Tyr Leu Asp Leu Asn Lys Ala
        435                 440                 445

Arg Asn Ile Phe Lys Glu Leu Thr Gln Lys Asp Trp Ile Thr Asn Met
    450                 455                 460

Ile Thr Thr Cys Leu Lys Asp Asn Leu Leu Lys Arg Leu Pro Phe His
465                 470                 475                 480

Ser Pro Pro Gln Glu Ala Leu Glu Ile Phe Phe Leu Leu Pro Glu Cys
                485                 490                 495

Pro Met Met His Ile Ser Asn Asn Trp Glu Ser Leu Val Val Pro Phe
            500                 505                 510

Ala Lys Val Val Cys Lys Met Ser Asp Gln Ser Ser Leu Val Leu Glu
        515                 520                 525

Glu Tyr Trp Ala Thr Leu Gln Glu Ser Thr Phe Ser Lys Leu Val Gln
    530                 535                 540

Met Phe Lys Thr Ala Val Ile Cys Gln Leu Asp Tyr Trp Asp Glu Ser
545                 550                 555                 560

Ala Glu Glu Asn Gly Asn Val Gln Ala Leu Leu Glu Met Leu Lys Lys
                565                 570                 575

Leu His Arg Val Asn Gln Val Lys Cys Gln Leu Pro Glu Ser Ile Phe
            580                 585                 590

Gln Val Asp Glu Leu Leu His Arg Leu Asn Phe Phe Val Glu Val Cys
        595                 600                 605

Arg Arg Tyr Leu Trp Lys Met Thr Val Asp Ala Ser Glu Asn Val Gln
    610                 615                 620

Cys Cys Val Ile Phe Ser His Phe Pro Phe Ile Phe Asn Asn Leu Ser
625                 630                 635                 640

Lys Ile Lys Leu Leu His Thr Asp Thr Leu Leu Lys Ile Glu Ser Lys
                645                 650                 655

Lys His Lys Ala Tyr Leu Arg Ser Ala Ala Ile Glu Glu Glu Arg Glu
            660                 665                 670

Ser Glu Phe Ala Leu Arg Pro Thr Phe Asp Leu Thr Val Arg Arg Asn
        675                 680                 685

His Leu Ile Glu Asp Val Leu Asn Gln Leu Ser Gln Phe Glu Asn Glu
    690                 695                 700

Asp Leu Arg Lys Glu Leu Trp Val Ser Phe Ser Gly Glu Ile Gly Tyr
705                 710                 715                 720

Asp Leu Gly Gly Val Lys Lys Glu Phe Phe Tyr Cys Leu Phe Ala Glu
                725                 730                 735

Met Ile Gln Pro Glu Tyr Gly Met Phe Met Tyr Pro Glu Gly Ala Ser
            740                 745                 750

Cys Met Trp Phe Pro Val Lys Pro Lys Phe Glu Lys Lys Arg Tyr Phe
        755                 760                 765

Phe Phe Gly Val Leu Cys Gly Leu Ser Leu Phe Asn Cys Asn Val Ala
    770                 775                 780

Asn Leu Pro Phe Pro Leu Ala Leu Phe Lys Lys Leu Leu Asp Gln Met
785                 790                 795                 800

Pro Ser Leu Glu Asp Leu Lys Glu Leu Ser Pro Asp Leu Gly Lys Asn
                805                 810                 815

Leu Gln Thr Leu Leu Asp Asp Glu Gly Asp Asn Phe Glu Glu Val Phe
            820                 825                 830

Tyr Ile His Phe Asn Val His Trp Asp Arg Asn Asp Thr Asn Leu Ile
        835                 840                 845

Pro Asn Gly Ser Ser Ile Thr Val Asn Gln Thr Asn Lys Arg Asp Tyr
    850                 855                 860

Val Ser Lys Tyr Ile Asn Tyr Ile Phe Asn Asp Ser Val Lys Ala Val

865                 870                 875                 880

Tyr Glu Glu Phe Arg Arg Gly Phe Tyr Lys Met Cys Asp Glu Asp Ile
                885                 890                 895

Ile Lys Leu Phe His Pro Glu Glu Leu Lys Asp Val Ile Val Gly Asn
            900                 905                 910

Thr Asp Tyr Asp Trp Lys Thr Phe Glu Lys Asn Ala Arg Tyr Glu Pro
        915                 920                 925

Gly Tyr Asn Ser Ser His Pro Thr Ile Val Met Phe Trp Lys Ala Phe
    930                 935                 940

His Lys Leu Thr Leu Glu Glu Lys Lys Lys Phe Leu Val Phe Leu Thr
945                 950                 955                 960

Gly Thr Asp Arg Leu Gln Met Lys Asp Leu Asn Asn Met Lys Ile Thr
                965                 970                 975

Phe Cys Cys Pro Glu Ser Trp Asn Glu Arg Asp Pro Ile Arg Ala Leu
            980                 985                 990

Thr Cys Phe Ser Val Leu Phe Leu  Pro Lys Tyr Ser Thr  Met Glu Thr
        995                 1000                1005

Val Glu  Glu Ala Leu Gln Glu  Ala Ile Asn Asn Asn  Arg Gly Phe
    1010                1015                1020

Gly

<210>  32
<211>  153
<212>  PRT
<213>  Homo sapiens

<400>  32

Met Met Ala Ser Met Arg Val Val Lys Glu Leu Glu Asp Leu Gln Lys
1               5                   10                  15

Lys Pro Pro Pro Tyr Leu Arg Asn Leu Ser Ser Asp Asp Ala Asn Val
            20                  25                  30

Leu Val Trp His Ala Leu Leu Leu Pro Asp Gln Pro Pro Tyr His Leu
        35                  40                  45

Lys Ala Phe Asn Leu Arg Ile Ser Phe Pro Pro Glu Tyr Pro Phe Lys

50                  55                  60

Pro Pro Met Ile Lys Phe Thr Thr Lys Ile Tyr His Pro Asn Val Asp
65                  70                  75                  80

Glu Asn Gly Gln Ile Cys Leu Pro Ile Ile Ser Ser Glu Asn Trp Lys
                85                  90                  95

Pro Cys Thr Lys Thr Cys Gln Val Leu Glu Ala Leu Asn Val Leu Val
            100                 105                 110

Asn Arg Pro Asn Ile Arg Glu Pro Leu Arg Met Asp Leu Ala Asp Leu
        115                 120                 125

Leu Thr Gln Asn Pro Glu Leu Phe Arg Lys Asn Ala Glu Glu Phe Thr
    130                 135                 140

Leu Arg Phe Gly Val Asp Arg Pro Ser
145                 150

<210>  33
<211>  87
<212>  PRT
<213>  Homo sapiens

<400>  33

Met Ile Lys Phe Thr Thr Lys Ile Tyr His Pro Asn Val Asp Glu Asn
1               5                   10                  15

Gly Gln Ile Cys Leu Pro Ile Ile Ser Ser Glu Asn Trp Lys Pro Cys
            20                  25                  30

Thr Lys Thr Cys Gln Val Leu Glu Ala Leu Asn Val Leu Val Asn Arg
        35                  40                  45

Pro Asn Ile Arg Glu Pro Leu Arg Met Asp Leu Ala Asp Leu Leu Thr
    50                  55                  60

Gln Asn Pro Glu Leu Phe Arg Lys Asn Ala Glu Glu Phe Thr Leu Arg
65                  70                  75                  80

Phe Gly Val Asp Arg Pro Ser
                85

<210>  34
<211>  1269
<212>  DNA
<213>  Homo sapiens

<400>  34
aagactggaa cccgtatgag cgccccccag cgcccctgag cgctcgccgc cggtgcacgg      60

cgcaccccgc gggaggcagg gatcagcaaa gccgtgcgcc ccgaggcccg cccccgtctc     120

cgcacaaaga ccgagctgga ggatcttcag aagaagcctc ccccatacct gcggaacctg     180

tccagcgatg atgccaatgt cctggtgtgg cacgctctcc tcctacccga ccaacctccc     240

taccacctga aagccttcaa cctgcgcatc agcttcccgc cggagtatcc gttcaagcct     300

cccatgatca aattcacaac caagatctac caccccaacg tggacgagaa cggacagatt     360

tgcctgccca tcatcagcag tgagaactgg aagccttgca ccaagacttg ccaagtcctg     420

gaggccctca atgtgctggt gaatagaccg aatatcaggg agcccctgcg gatggacctc     480

gctgacctgc tgacacagaa tccggagctg ttcagaaaga atgccgaaga gttcaccctc     540

cgattcggag tggaccggcc ctcctaactc atgttctgac cctctgtgca ctggatcctc     600

ggcatagcgg acggacacac ctcatggact gaggccagag ccccctgtgg cccattcccc     660

attcattttt cccttcttag gttgttagtc attagtttgt gtgtgtgtgt ggtggaggga     720

agggagctat gagtgtgtgt gttgtgtatg gactcactcc caggttcacc tggccacagg     780

tgcacccttc ccacaccctt tacattcccc agagccaagg gagtttaagt ttgcagttac     840

aggccagttc tccagctctc catcttagag agacaggtca ccttgcaggc ctgcttgcag     900

gaaatgaatc cagcagccaa ctcgaatccc cctagggctc aggcactgag ggcctgggga     960

cagtggagca tatgggtggg agacagatgg agggtaccct atttacaact gagtcagcca    1020

agccactgat gggaatatac agatttaggt gctaaaccgt ttattttcca cggatgagtc    1080

acaatctgaa gaatcaaact tccatcctga aaatctatat gtttcaaaac cacttgccat    1140

cctgttagat tgccagttcc tgggaccagg cctcagactg tgaagtatat atcctccagc    1200

attcagtcca gggggagcca cggaaaccat gttcttgctt aagccattaa agtcagagat    1260

gaattctgg                                                            1269

<210>  35
<211>  507
<212>  DNA
<213>  Homo sapiens

<400>  35
ttgtgtccct actcattgaa accaaactct ggaaaggacc caatgtacca gtatttatac      60

ctctaatgaa gcacagagag aggaagagag ctgcttaaac tcacacaaca atgaactgca     120

gacacagctg ttctctccct ctctccttcc cagagcaatt tatactttac cctcaggctg     180

tcctctgggg agaaggtgcc atggtcttag gtgtctgtgc cccaggacag accctaggac     240

cctaaatcca atagaaaatg catatctttg ctccactttc agccaggctg gagcaaggta     300

ccttttctta ggatcttggg agggaatgga tgcccctctc tgcatgatct tgttgaggca     360

tttagctgcc atgcacctgt ccccctttaa tactgggcat tttaaagcca tctcaagagg 420

catcttctac atgttttgta cgcattaaaa taatttcaaa gatatctgag aaaagccgat 480

atttgccatt cttcctatat cctggaa 507

<210> 36
<211> 491
<212> DNA
<213> Homo sapiens

<220>
<221> misc_feature
<222> (35)..(35)
<223> n is a, c, g, or t

<220>
<221> misc_feature
<222> (96)..(96)
<223> n is a, c, g, or t

<220>
<221> misc_feature
<222> (254)..(254)
<223> n is a, c, g, or t

<400> 36
cctgcacgca tggcaggctg gcaccccccc accgncctcc ccacagccag cagcctttcc 60

acagtcactg cccttcccgc agtccccagc cttccntacg gcctcacccg caccccctca 120

gagcccaggg ctgcaacccc tcattatcca ccacgcacag atggtacagc tggggctgaa 180

caaccacatg tggaaccaga gagggtccca ggcgcccgag gacaagacgc aggaggcaga 240

atgaccgcgt gtcnttgcct gaccacctgg ggaacacccc tggacccagg catcggccag 300

gaccccatag agcaccccgg tctgccctgt gccctgtgga cagtggaaga tgaggtcatc 360

tgccactttc aggacattgt ccgggagccc ttcatttagg acaaaacggg cgcgatgatg 420

ccctggcttt cagggtggtc agaactggat acggtgttta caattccaat ctctctattt 480

ctgggtgaag g 491

<210> 37
<211> 522
<212> DNA
<213> Homo sapiens

<400> 37
atctgcacac ttgatacagc aacgttagat ggttttgatg gtaaacccta aaggaggact 60

ccaagagtgt gtatttattt atagttttat cagagatgac aattatttga atgccaatta 120

tatggattcc tttcattttt tgctggagga tgggagaaga aaccaaagtt tatagacctt 180

cacattgaga aagcttcagt tttgaacttc agctatcaga ttcaaaaaca acagaaagaa 240

ccaagacatt cttaagatgc ctgtactttc agctgggtat aaattcatga gttcaaagat 300

tgaaacctga ccaatttgct ttatttcatg gaagaagtga tctacaaagg tgtttgtgcc 360

atttggaaaa cagcgtgcat gtgttcaagc cttagattgg cgatgtcgta ttttcctcac 420

gtgtggcaat gccaaaggct ttactttacc tgtgagtaca cactatatga attatttcca 480

acgtacattt aatcaataag ggtcacaaat tcccaaatca at 522

<210> 38
<211> 445
<212> DNA
<213> Homo sapiens

<220>
<221> misc_feature
<222> (164)..(164)
<223> n is a, c, g, or t

<220>
<221> misc_feature
<222> (168)..(168)
<223> n is a, c, g, or t

<220>
<221> misc_feature
<222> (174)..(174)
<223> n is a, c, g, or t

<220>
<221> misc_feature
<222> (188)..(188)
<223> n is a, c, g, or t

<220>
<221> misc_feature
<222> (191)..(211)
<223> n is a, c, g, or t

<220>
<221> misc_feature
<222> (214)..(214)
<223> n is a, c, g, or t

<220>
<221> misc_feature
<222> (391)..(391)
<223> n is a, c, g, or t

<220>
<221> misc_feature
<222> (393)..(394)
<223> n is a, c, g, or t

<400> 38
cccttggtgc tgtgtggtcc cagtggaagg aggggaagat tttggaaacc tggtagccac 60

cagtaaggtg attctctgcc ctgttggggc ctaaatttgg gggcttttgg gcaacctctc 120

cgtgtactgc gtctgtccac actcgattgg gccccaggtg tgtnatgnag gcgnctctgg 180

taaggtgnct nnnnnnnnnn nnnnnnnnnn ntcnagtaac gaggcttttg atgtgttgag 240

ctggaggtga gtggaccggg ggctgtgttt taagctgctt ccttggcatt tggcatcact 300

gccttctgtt cccgggggag catggatctt ttgtcctcac tgctttctaa tggggagggc 360

tgagggctcc ctgtccccac agcaggtatg ntnngctctg ccccagcccc acacttgctc 420

tgaaaaccaa gtgtcagagc ccctt 445

<210> 39
<211> 477
<212> DNA
<213> Homo sapiens

<400> 39
cagctgcgct acacggagga actgctgcgg cacgtggccc ctgggttgca cctggagctt 60

cgggggccac agctgtgggc ccggcgcatg ggcaagtgca aggtgtactg ggaggtgggc 120

ggacccccag gctccgccag cccctccacc ccagcctgcc tgctgcctcg gaactgtgac 180

acccccatct tcgacttcag agtcttcttc caagagctgg tggaattccg ggcacggcag 240

cgccgtggct ccccacgcta taccatctac ctgggcttcg ggcaggacct gtcagctggg 300

aggcccaagg agaagagcct ggtcctggtg aagctggaac cctggctgtg ccgagtgcac 360

ctagagggca cgcagcgtga gggtgtgtct tccctggata gcagcagcct cagcctctgc 420

ctgtccagcg ccaacagcct ctatgacgac atcgagtgct tccttatgga gctggag 477

<210> 40
<211> 309
<212> PRT
<213> Homo sapiens

<400> 40

Met Arg Pro Asp Arg Ala Glu Ala Pro Gly Pro Pro Ala Met Ala Ala
1               5                   10                  15

Gly Gly Pro Gly Ala Gly Ser Ala Ala Pro Val Ser Ser Thr Ser Ser
            20                  25                  30

Leu Pro Leu Ala Ala Leu Asn Met Arg Val Arg Arg Arg Leu Ser Leu
        35                  40                  45

Phe Leu Asn Val Arg Thr Gln Val Ala Ala Asp Trp Thr Ala Leu Ala
    50                  55                  60

Glu Glu Met Asp Phe Glu Tyr Leu Glu Ile Arg Gln Leu Glu Thr Gln
65                  70                  75                  80

Ala Asp Pro Thr Gly Arg Leu Leu Asp Ala Trp Gln Gly Arg Pro Gly

85                  90                  95

Ala Ser Val Gly Arg Leu Leu Glu Leu Leu Thr Lys Leu Gly Arg Asp
            100                 105                 110

Asp Val Leu Leu Glu Leu Gly Pro Ser Ile Glu Glu Asp Cys Gln Lys
        115                 120                 125

Tyr Ile Leu Lys Gln Gln Gln Glu Glu Ala Glu Lys Pro Leu Gln Val
    130                 135                 140

Ala Ala Val Asp Ser Ser Val Pro Arg Thr Ala Glu Leu Ala Gly Ile
145                 150                 155                 160

Thr Thr Leu Asp Asp Pro Leu Gly His Met Pro Glu Arg Phe Asp Ala
                165                 170                 175

Phe Ile Cys Tyr Cys Pro Ser Asp Ile Gln Phe Val Gln Glu Met Ile
            180                 185                 190

Arg Gln Leu Glu Gln Thr Asn Tyr Arg Leu Lys Leu Cys Val Ser Asp
        195                 200                 205

Arg Asp Val Leu Pro Gly Thr Cys Val Trp Ser Ile Ala Ser Glu Leu
    210                 215                 220

Ile Glu Lys Arg Cys Arg Arg Met Val Val Val Val Ser Asp Asp Tyr
225                 230                 235                 240

Leu Gln Ser Lys Glu Cys Asp Phe Gln Thr Lys Phe Ala Leu Ser Leu
                245                 250                 255

Ser Pro Gly Ala His Gln Lys Arg Leu Ile Pro Ile Lys Tyr Lys Ala
            260                 265                 270

Met Lys Lys Glu Phe Pro Ser Ile Leu Arg Phe Ile Thr Val Cys Asp
        275                 280                 285

Tyr Thr Asn Pro Cys Thr Lys Ser Trp Phe Trp Thr Arg Leu Ala Lys
    290                 295                 300

Ala Leu Ser Leu Pro
305

<210>  41
<211>  712
<212>  PRT
<213>  Homo sapiens

<400>  41

Met Ala Cys Thr Gly Pro Ser Leu Pro Ser Ala Phe Asp Ile Leu Gly
1               5                   10                  15

Ala Ala Gly Gln Asp Lys Leu Leu Tyr Leu Lys His Lys Leu Lys Thr
            20                  25                  30

Pro Arg Pro Gly Cys Gln Gly Gln Asp Leu Leu His Ala Met Val Leu
        35                  40                  45

Leu Lys Leu Gly Gln Glu Thr Glu Ala Arg Ile Ser Leu Glu Ala Leu
    50                  55                  60

Lys Ala Asp Ala Val Ala Arg Leu Val Ala Arg Gln Trp Ala Gly Val
65                  70                  75                  80

Asp Ser Thr Glu Asp Pro Glu Glu Pro Pro Asp Val Ser Trp Ala Val
                85                  90                  95

Ala Arg Leu Tyr His Leu Leu Ala Glu Glu Lys Leu Cys Pro Ala Ser
            100                 105                 110

Leu Arg Asp Val Ala Tyr Gln Glu Ala Val Arg Thr Leu Ser Ser Arg
        115                 120                 125

Asp Asp His Arg Leu Gly Glu Leu Gln Asp Glu Ala Arg Asn Arg Cys
    130                 135                 140

Gly Trp Asp Ile Ala Gly Asp Pro Gly Ser Ile Arg Thr Leu Gln Ser
145                 150                 155                 160

Asn Leu Gly Cys Leu Pro Pro Ser Ser Ala Leu Pro Ser Gly Thr Arg
                165                 170                 175

Ser Leu Pro Arg Pro Ile Asp Gly Val Ser Asp Trp Ser Gln Gly Cys
            180                 185                 190

Ser Leu Arg Ser Thr Gly Ser Pro Ala Ser Leu Ala Ser Asn Leu Glu
        195                 200                 205

Ile Ser Gln Ser Pro Thr Met Pro Phe Leu Ser Leu His Arg Ser Pro
    210                 215                 220

His Gly Pro Ser Lys Leu Cys Asp Asp Pro Gln Ala Ser Leu Val Pro
225                 230                 235                 240

Glu Pro Val Pro Gly Gly Cys Gln Glu Pro Glu Glu Met Ser Trp Pro
                245                 250                 255

Pro Ser Gly Glu Ile Ala Ser Pro Pro Glu Leu Pro Ser Ser Pro Pro
            260                 265                 270

Pro Gly Leu Pro Glu Val Ala Pro Asp Ala Thr Ser Thr Gly Leu Pro
        275                 280                 285

Asp Thr Pro Ala Ala Pro Glu Thr Ser Thr Asn Tyr Pro Val Glu Cys
    290                 295                 300

Thr Glu Gly Ser Ala Gly Pro Gln Ser Leu Pro Leu Pro Ile Leu Glu
305                 310                 315                 320

Pro Val Lys Asn Pro Cys Ser Val Lys Asp Gln Thr Pro Leu Gln Leu
                325                 330                 335

Ser Val Glu Asp Thr Thr Ser Pro Asn Thr Lys Pro Cys Pro Pro Thr
            340                 345                 350

Pro Thr Thr Pro Glu Thr Ser Pro Pro Pro Pro Pro Pro Pro Pro Ser
        355                 360                 365

Ser Thr Pro Cys Ser Ala His Leu Thr Pro Ser Ser Leu Phe Pro Ser
    370                 375                 380

Ser Leu Glu Ser Ser Ser Glu Gln Lys Phe Tyr Asn Phe Val Ile Leu
385                 390                 395                 400

His Ala Arg Ala Asp Glu His Ile Ala Leu Arg Val Arg Glu Lys Leu
                405                 410                 415

Glu Ala Leu Gly Val Pro Asp Gly Ala Thr Phe Cys Glu Asp Phe Gln
            420                 425                 430

Val Pro Gly Arg Gly Glu Leu Ser Cys Leu Gln Asp Ala Ile Asp His
        435                 440                 445

Ser Ala Phe Ile Ile Leu Leu Leu Thr Ser Asn Phe Asp Cys Arg Leu
    450                 455                 460

Ser Leu His Gln Val Asn Gln Ala Met Met Ser Asn Leu Thr Arg Gln
465                 470                 475                 480

Gly Ser Pro Asp Cys Val Ile Pro Phe Leu Pro Leu Glu Ser Ser Pro
                485                 490                 495

Ala Gln Leu Ser Ser Asp Thr Ala Ser Leu Leu Ser Gly Leu Val Arg
            500                 505                 510

Leu Asp Glu His Ser Gln Ile Phe Ala Arg Lys Val Ala Asn Thr Phe
        515                 520                 525

Lys Pro His Arg Leu Gln Ala Arg Lys Ala Met Trp Arg Lys Glu Gln
    530                 535                 540

Asp Thr Arg Ala Leu Arg Glu Gln Ser Gln His Leu Asp Gly Glu Arg
545                 550                 555                 560

Met Gln Ala Ala Ala Leu Asn Ala Ala Tyr Ser Ala Tyr Leu Gln Ser
                565                 570                 575

Tyr Leu Ser Tyr Gln Ala Gln Met Glu Gln Leu Gln Val Ala Phe Gly
            580                 585                 590

Ser His Met Ser Phe Gly Thr Gly Ala Pro Tyr Gly Ala Arg Met Pro
        595                 600                 605

Phe Gly Gly Gln Val Pro Leu Gly Ala Pro Pro Pro Phe Pro Thr Trp
    610                 615                 620

Pro Gly Cys Pro Gln Pro Pro Pro Leu His Ala Trp Gln Ala Gly Thr
625                 630                 635                 640

Pro Pro Pro Pro Ser Pro Gln Pro Ala Ala Phe Pro Gln Ser Leu Pro
                645                 650                 655

Phe Pro Gln Ser Pro Ala Phe Pro Thr Ala Ser Pro Ala Pro Pro Gln
            660                 665                 670

Ser Pro Gly Leu Gln Pro Leu Ile Ile His His Ala Gln Met Val Gln
        675                 680                 685

Leu Gly Leu Asn Asn His Met Trp Asn Gln Arg Gly Ser Gln Ala Pro
    690                 695                 700

Glu Asp Lys Thr Gln Glu Ala Glu
705                 710

<210>  42
<211>  1049
<212>  PRT
<213>  Homo sapiens

<400>  42

Met Val Phe Pro Met Trp Thr Leu Lys Arg Gln Ile Leu Ile Leu Phe
1               5                   10                  15

Asn Ile Ile Leu Ile Ser Lys Leu Leu Gly Ala Arg Trp Phe Pro Lys
            20                  25                  30

Thr Leu Pro Cys Asp Val Thr Leu Asp Val Pro Lys Asn His Val Ile
        35                  40                  45

Val Asp Cys Thr Asp Lys His Leu Thr Glu Ile Pro Gly Gly Ile Pro
    50                  55                  60

Thr Asn Thr Thr Asn Leu Thr Leu Thr Ile Asn His Ile Pro Asp Ile
65                  70                  75                  80

Ser Pro Ala Ser Phe His Arg Leu Asp His Leu Val Glu Ile Asp Phe
                85                  90                  95

Arg Cys Asn Cys Val Pro Ile Pro Leu Gly Ser Lys Asn Asn Met Cys
            100                 105                 110

Ile Lys Arg Leu Gln Ile Lys Pro Arg Ser Phe Ser Gly Leu Thr Tyr
        115                 120                 125

Leu Lys Ser Leu Tyr Leu Asp Gly Asn Gln Leu Leu Glu Ile Pro Gln
    130                 135                 140

Gly Leu Pro Pro Ser Leu Gln Leu Leu Ser Leu Glu Ala Asn Asn Ile
145                 150                 155                 160

Phe Ser Ile Arg Lys Glu Asn Leu Thr Glu Leu Ala Asn Ile Glu Ile
                165                 170                 175

Leu Tyr Leu Gly Gln Asn Cys Tyr Tyr Arg Asn Pro Cys Tyr Val Ser
            180                 185                 190

Tyr Ser Ile Glu Lys Asp Ala Phe Leu Asn Leu Thr Lys Leu Lys Val
        195                 200                 205

Leu Ser Leu Lys Asp Asn Asn Val Thr Ala Val Pro Thr Val Leu Pro
    210                 215                 220

Ser Thr Leu Thr Glu Leu Tyr Leu Tyr Asn Asn Met Ile Ala Lys Ile
225                 230                 235                 240

Gln Glu Asp Asp Phe Asn Asn Leu Asn Gln Leu Gln Ile Leu Asp Leu
                245                 250                 255

Ser Gly Asn Cys Pro Arg Cys Tyr Asn Ala Pro Phe Pro Cys Ala Pro
            260                 265                 270

Cys Lys Asn Asn Ser Pro Leu Gln Ile Pro Val Asn Ala Phe Asp Ala
        275                 280                 285

Leu Thr Glu Leu Lys Val Leu Arg Leu His Ser Asn Ser Leu Gln His
    290                 295                 300

Val Pro Pro Arg Trp Phe Lys Asn Ile Asn Lys Leu Gln Glu Leu Asp
305                 310                 315                 320

Leu Ser Gln Asn Phe Leu Ala Lys Glu Ile Gly Asp Ala Lys Phe Leu
                325                 330                 335

His Phe Leu Pro Ser Leu Ile Gln Leu Asp Leu Ser Phe Asn Phe Glu
            340                 345                 350

Leu Gln Val Tyr Arg Ala Ser Met Asn Leu Ser Gln Ala Phe Ser Ser
        355                 360                 365

Leu Lys Ser Leu Lys Ile Leu Arg Ile Arg Gly Tyr Val Phe Lys Glu
    370                 375                 380

Leu Lys Ser Phe Asn Leu Ser Pro Leu His Asn Leu Gln Asn Leu Glu
385                 390                 395                 400

Val Leu Asp Leu Gly Thr Asn Phe Ile Lys Ile Ala Asn Leu Ser Met
                405                 410                 415

Phe Lys Gln Phe Lys Arg Leu Lys Val Ile Asp Leu Ser Val Asn Lys
            420                 425                 430

Ile Ser Pro Ser Gly Asp Ser Ser Glu Val Gly Phe Cys Ser Asn Ala
        435                 440                 445

Arg Thr Ser Val Glu Ser Tyr Glu Pro Gln Val Leu Glu Gln Leu His
    450                 455                 460

Tyr Phe Arg Tyr Asp Lys Tyr Ala Arg Ser Cys Arg Phe Lys Asn Lys
465                 470                 475                 480

Glu Ala Ser Phe Met Ser Val Asn Glu Ser Cys Tyr Lys Tyr Gly Gln
                485                 490                 495

Thr Leu Asp Leu Ser Lys Asn Ser Ile Phe Phe Val Lys Ser Ser Asp
        500                 505                 510

Phe Gln His Leu Ser Phe Leu Lys Cys Leu Asn Leu Ser Gly Asn Leu
    515                 520                 525

Ile Ser Gln Thr Leu Asn Gly Ser Glu Phe Gln Pro Leu Ala Glu Leu
    530                 535                 540

Arg Tyr Leu Asp Phe Ser Asn Asn Arg Leu Asp Leu Leu His Ser Thr
545                 550                 555                 560

Ala Phe Glu Glu Leu His Lys Leu Glu Val Leu Asp Ile Ser Ser Asn
                565                 570                 575

Ser His Tyr Phe Gln Ser Glu Gly Ile Thr His Met Leu Asn Phe Thr
            580                 585                 590

Lys Asn Leu Lys Val Leu Gln Lys Leu Met Met Asn Asp Asn Asp Ile
        595                 600                 605

Ser Ser Ser Thr Ser Arg Thr Met Glu Ser Glu Ser Leu Arg Thr Leu
    610                 615                 620

Glu Phe Arg Gly Asn His Leu Asp Val Leu Trp Arg Glu Gly Asp Asn
625                 630                 635                 640

Arg Tyr Leu Gln Leu Phe Lys Asn Leu Leu Lys Leu Glu Glu Leu Asp
                645                 650                 655

Ile Ser Lys Asn Ser Leu Ser Phe Leu Pro Ser Gly Val Phe Asp Gly
            660                 665                 670

Met Pro Pro Asn Leu Lys Asn Leu Ser Leu Ala Lys Asn Gly Leu Lys
        675                 680                 685

Ser Phe Ser Trp Lys Lys Leu Gln Cys Leu Lys Asn Leu Glu Thr Leu
    690                 695                 700

Asp Leu Ser His Asn Gln Leu Thr Thr Val Pro Glu Arg Leu Ser Asn
705                 710                 715                 720

Cys Ser Arg Ser Leu Lys Asn Leu Ile Leu Lys Asn Asn Gln Ile Arg
                725                 730                 735

Ser Leu Thr Lys Tyr Phe Leu Gln Asp Ala Phe Gln Leu Arg Tyr Leu
            740                 745                 750

Asp Leu Ser Ser Asn Lys Ile Gln Met Ile Gln Lys Thr Ser Phe Pro
        755                 760                 765

Glu Asn Val Leu Asn Asn Leu Lys Met Leu Leu Leu His His Asn Arg
    770                 775                 780

Phe Leu Cys Thr Cys Asp Ala Val Trp Phe Val Trp Trp Val Asn His
785                 790                 795                 800

Thr Glu Val Thr Ile Pro Tyr Leu Ala Thr Asp Val Thr Cys Val Gly
                805                 810                 815

Pro Gly Ala His Lys Gly Gln Ser Val Ile Ser Leu Asp Leu Tyr Thr
            820                 825                 830

Cys Glu Leu Asp Leu Thr Asn Leu Ile Leu Phe Ser Leu Ser Ile Ser
        835                 840                 845

Val Ser Leu Phe Leu Met Val Met Met Thr Ala Ser His Leu Tyr Phe
    850                 855                 860

Trp Asp Val Trp Tyr Ile Tyr His Phe Cys Lys Ala Lys Ile Lys Gly
865                 870                 875                 880

Tyr Gln Arg Leu Ile Ser Pro Asp Cys Cys Tyr Asp Ala Phe Ile Val
                885                 890                 895

Tyr Asp Thr Lys Asp Pro Ala Val Thr Glu Trp Val Leu Ala Glu Leu
            900                 905                 910

Val Ala Lys Leu Glu Asp Pro Arg Glu Lys His Phe Asn Leu Cys Leu
        915                 920                 925

Glu Glu Arg Asp Trp Leu Pro Gly Gln Pro Val Leu Glu Asn Leu Ser
    930                 935                 940

Gln Ser Ile Gln Leu Ser Lys Lys Thr Val Phe Val Met Thr Asp Lys
945                 950                 955                 960

Tyr Ala Lys Thr Glu Asn Phe Lys Ile Ala Phe Tyr Leu Ser His Gln
                965                 970                 975

Arg Leu Met Asp Glu Lys Val Asp Val Ile Ile Leu Ile Phe Leu Glu
            980                 985                 990

Lys Pro Phe Gln Lys Ser Lys Phe  Leu Gln Leu Arg Lys  Arg Leu Cys

995                    1000                    1005

Gly Ser  Ser Val Leu Glu Trp  Pro Thr Asn Pro Gln  Ala His Pro
    1010                 1015                 1020

Tyr Phe  Trp Gln Cys Leu Lys  Asn Ala Leu Ala Thr  Asp Asn His
    1025                 1030                 1035

Val Ala  Tyr Ser Gln Val Phe  Lys Glu Thr Val
    1040                 1045

<210>  43
<211>  582
<212>  PRT
<213>  Homo sapiens

<400>  43

Met Ala Glu Phe Leu Asp Asp Gln Glu Thr Arg Leu Cys Asp Asn Cys
1               5                   10                  15

Lys Lys Glu Ile Pro Val Phe Asn Phe Thr Ile His Glu Ile His Cys
            20                  25                  30

Gln Arg Asn Ile Gly Met Cys Pro Thr Cys Lys Glu Pro Phe Pro Lys
        35                  40                  45

Ser Asp Met Glu Thr His Met Ala Ala Glu His Cys Gln Val Thr Cys
    50                  55                  60

Lys Cys Asn Lys Lys Leu Glu Lys Arg Leu Leu Lys Lys His Glu Glu
65                  70                  75                  80

Thr Glu Cys Pro Leu Arg Leu Ala Val Cys Gln His Cys Asp Leu Glu
                85                  90                  95

Leu Ser Ile Leu Lys Leu Lys Glu His Glu Asp Tyr Cys Gly Ala Arg
            100                 105                 110

Thr Glu Leu Cys Gly Asn Cys Gly Arg Asn Val Leu Val Lys Asp Leu
        115                 120                 125

Lys Thr His Pro Glu Val Cys Gly Arg Glu Gly Glu Glu Lys Arg Asn
    130                 135                 140

Glu Val Ala Ile Pro Pro Asn Ala Tyr Asp Glu Ser Trp Gly Gln Asp
145                 150                 155                 160

Gly Ile Trp Ile Ala Ser Gln Leu Leu Arg Gln Ile Glu Ala Leu Asp

                165                 170                 175

Pro Pro Met Arg Leu Pro Arg Arg Pro Leu Arg Ala Phe Glu Ser Asp
            180                 185                 190

Val Phe His Asn Arg Thr Thr Asn Gln Arg Asn Ile Thr Ala Gln Val
        195                 200                 205

Ser Ile Gln Asn Asn Leu Phe Glu Glu Gln Glu Arg Gln Glu Arg Asn
    210                 215                 220

Arg Gly Gln Gln Pro Pro Lys Glu Gly Gly Glu Glu Ser Ala Asn Leu
225                 230                 235                 240

Asp Phe Met Leu Ala Leu Ser Leu Gln Asn Glu Gly Gln Ala Ser Ser
                245                 250                 255

Val Ala Glu Gln Asp Phe Trp Arg Ala Val Cys Glu Ala Asp Gln Ser
            260                 265                 270

His Gly Gly Pro Arg Ser Leu Ser Asp Ile Lys Gly Ala Ala Asp Glu
        275                 280                 285

Ile Met Leu Pro Cys Glu Phe Cys Glu Glu Leu Tyr Pro Glu Glu Leu
    290                 295                 300

Leu Ile Asp His Gln Thr Ser Cys Asn Pro Ser Arg Ala Leu Pro Ser
305                 310                 315                 320

Leu Asn Thr Gly Ser Ser Ser Pro Arg Gly Val Glu Glu Pro Asp Val
                325                 330                 335

Ile Phe Gln Asn Phe Leu Gln Gln Ala Ala Ser Asn Gln Leu Asp Ser
            340                 345                 350

Leu Met Gly Leu Ser Asn Ser His Pro Val Glu Glu Ser Ile Ile Ile
        355                 360                 365

Pro Cys Glu Phe Cys Gly Val Gln Leu Glu Glu Glu Val Leu Phe His
    370                 375                 380

His Gln Asp Gln Cys Asp Gln Arg Pro Ala Thr Ala Thr Asn His Val
385                 390                 395                 400

Thr Glu Gly Ile Pro Arg Leu Asp Ser Gln Pro Gln Glu Thr Ser Pro
                405                 410                 415

Glu Leu Pro Arg Arg Arg Val Arg His Gln Gly Asp Leu Ser Ser Gly
            420                 425                 430

Tyr Leu Asp Asp Thr Lys Gln Glu Thr Ala Asn Gly Pro Thr Ser Cys
        435                 440                 445

Leu Pro Pro Ser Arg Pro Ile Asn Asn Met Thr Ala Thr Tyr Asn Gln
    450                 455                 460

Leu Ser Arg Ser Thr Ser Gly Pro Arg Pro Gly Cys Gln Pro Ser Ser
465                 470                 475                 480

Pro Cys Val Pro Lys Leu Ser Asn Ser Asp Ser Gln Asp Ile Gln Gly
                485                 490                 495

Arg Asn Arg Asp Ser Gln Asn Gly Ala Ile Ala Pro Gly His Val Ser
            500                 505                 510

Val Ile Arg Pro Pro Gln Asn Leu Tyr Pro Glu Asn Ile Val Pro Ser
        515                 520                 525

Phe Ser Pro Gly Pro Ser Gly Arg Tyr Gly Ala Ser Gly Arg Ser Glu
    530                 535                 540

Gly Gly Arg Asn Ser Arg Val Thr Pro Ala Ala Ala Asn Tyr Arg Ser
545                 550                 555                 560

Arg Thr Ala Lys Ala Lys Pro Ser Lys Gln Gln Gly Ala Gly Asp Ala
                565                 570                 575

Glu Glu Glu Glu Glu Glu
            580

<210>  44
<211>  582
<212>  PRT
<213>  Homo sapiens

<400>  44

Met Ala Glu Phe Leu Asp Asp Gln Glu Thr Arg Leu Cys Asp Asn Cys
1               5                   10                  15

Lys Lys Glu Ile Pro Val Phe Asn Phe Thr Ile His Glu Ile His Cys
            20                  25                  30

Gln Arg Asn Ile Gly Met Cys Pro Thr Cys Lys Glu Pro Phe Pro Lys
        35                  40                  45

Ser Asp Met Glu Thr His Met Ala Ala Glu His Cys Gln Val Thr Cys
    50                  55                  60

Lys Cys Asn Lys Lys Leu Glu Lys Arg Leu Leu Lys Lys His Glu Glu
65                  70                  75                  80

Thr Glu Cys Pro Leu Arg Leu Ala Val Cys Gln His Cys Asp Leu Glu
                85                  90                  95

Leu Ser Ile Leu Lys Leu Lys Glu His Glu Asp Tyr Cys Gly Ala Arg
            100                 105                 110

Thr Glu Leu Cys Gly Asn Cys Gly Arg Asn Val Leu Val Lys Asp Leu
        115                 120                 125

Lys Thr His Pro Glu Val Cys Gly Arg Glu Gly Glu Glu Lys Arg Asn
    130                 135                 140

Glu Val Ala Ile Pro Pro Asn Ala Tyr Asp Glu Ser Trp Gly Gln Asp
145                 150                 155                 160

Gly Ile Trp Ile Ala Ser Gln Leu Leu Arg Gln Ile Glu Ala Leu Asp
                165                 170                 175

Pro Pro Met Arg Leu Pro Arg Arg Pro Leu Arg Ala Phe Glu Ser Asp
            180                 185                 190

Val Phe His Asn Arg Thr Thr Asn Gln Arg Asn Ile Thr Ala Gln Val
        195                 200                 205

Ser Ile Gln Asn Asn Leu Phe Glu Glu Gln Glu Arg Gln Glu Arg Asn
    210                 215                 220

Arg Gly Gln Gln Pro Pro Lys Glu Gly Gly Glu Glu Ser Ala Asn Leu
225                 230                 235                 240

Asp Phe Met Leu Ala Leu Ser Leu Gln Asn Glu Gly Gln Ala Ser Ser
                245                 250                 255

Val Ala Glu Gln Asp Phe Trp Arg Ala Val Cys Glu Ala Asp Gln Ser
            260                 265                 270

His Gly Gly Pro Arg Ser Leu Ser Asp Ile Lys Gly Ala Ala Asp Glu
        275                 280                 285

Ile Met Leu Pro Cys Glu Phe Cys Glu Glu Leu Tyr Pro Glu Glu Leu

290                 295                 300

Leu Ile Asp His Gln Thr Ser Cys Asn Pro Ser Arg Ala Leu Pro Ser
305                 310                 315                 320

Leu Asn Thr Gly Ser Ser Ser Pro Arg Gly Val Glu Glu Pro Asp Val
                325                 330                 335

Ile Phe Gln Asn Phe Leu Gln Gln Ala Ala Ser Asn Gln Leu Asp Ser
            340                 345                 350

Leu Met Gly Leu Ser Asn Ser His Pro Val Glu Glu Ser Ile Ile Ile
        355                 360                 365

Pro Cys Glu Phe Cys Gly Val Gln Leu Glu Glu Glu Val Leu Phe His
    370                 375                 380

His Gln Asp Gln Cys Asp Gln Arg Pro Ala Thr Ala Thr Asn His Val
385                 390                 395                 400

Thr Glu Gly Ile Pro Arg Leu Asp Ser Gln Pro Gln Glu Thr Ser Pro
                405                 410                 415

Glu Leu Pro Arg Arg Arg Val Arg His Gln Gly Asp Leu Ser Ser Gly
            420                 425                 430

Tyr Leu Asp Asp Thr Lys Gln Glu Thr Ala Asn Gly Pro Thr Ser Cys
        435                 440                 445

Leu Pro Pro Ser Arg Pro Ile Asn Asn Met Thr Ala Thr Tyr Asn Gln
    450                 455                 460

Leu Ser Arg Ser Thr Ser Gly Pro Arg Pro Gly Cys Gln Pro Ser Ser
465                 470                 475                 480

Pro Cys Val Pro Lys Leu Ser Asn Ser Asp Ser Gln Asp Ile Gln Gly
                485                 490                 495

Arg Asn Arg Asp Ser Gln Asn Gly Ala Ile Ala Pro Gly His Val Ser
            500                 505                 510

Val Ile Arg Pro Pro Gln Asn Leu Tyr Pro Glu Asn Ile Val Pro Ser
        515                 520                 525

Phe Ser Pro Gly Pro Ser Gly Arg Tyr Gly Ala Ser Gly Arg Ser Glu
    530                 535                 540

Gly Gly Arg Asn Ser Arg Val Thr Pro Ala Ala Ala Asn Tyr Arg Ser
545                 550                 555                 560

Arg Thr Ala Lys Ala Lys Pro Ser Lys Gln Gln Gly Ala Gly Asp Ala
                565                 570                 575

Glu Glu Glu Glu Glu Glu
            580

<210>  45
<211>  503
<212>  PRT
<213>  Homo sapiens

<400>  45

Met Ala Leu Ala Pro Glu Arg Ala Ala Pro Arg Val Leu Phe Gly Glu
1               5                   10                  15

Trp Leu Leu Gly Glu Ile Ser Ser Gly Cys Tyr Glu Gly Leu Gln Trp
            20                  25                  30

Leu Asp Glu Ala Arg Thr Cys Phe Arg Val Pro Trp Lys His Phe Ala
        35                  40                  45

Arg Lys Asp Leu Ser Glu Ala Asp Ala Arg Ile Phe Lys Ala Trp Ala
    50                  55                  60

Val Ala Arg Gly Arg Trp Pro Pro Ser Ser Arg Gly Gly Gly Pro Pro
65                  70                  75                  80

Pro Glu Ala Glu Thr Ala Glu Arg Ala Gly Trp Lys Thr Asn Phe Arg
                85                  90                  95

Cys Ala Leu Arg Ser Thr Arg Arg Phe Val Met Leu Arg Asp Asn Ser
            100                 105                 110

Gly Asp Pro Ala Asp Pro His Lys Val Tyr Ala Leu Ser Arg Glu Leu
        115                 120                 125

Cys Trp Arg Glu Gly Pro Gly Thr Asp Gln Thr Glu Ala Glu Ala Pro
    130                 135                 140

Ala Ala Val Pro Pro Pro Gln Gly Gly Pro Pro Gly Pro Phe Leu Ala
145                 150                 155                 160

His Thr His Ala Gly Leu Gln Ala Pro Gly Pro Leu Pro Ala Pro Ala
                165                 170                 175

Gly Asp Lys Gly Asp Leu Leu Leu Gln Ala Val Gln Gln Ser Cys Leu
        180                 185                 190

Ala Asp His Leu Leu Thr Ala Ser Trp Gly Ala Asp Pro Val Pro Thr
        195                 200                 205

Lys Ala Pro Gly Glu Gly Gln Glu Gly Leu Pro Leu Thr Gly Ala Cys
    210                 215                 220

Ala Gly Gly Pro Gly Leu Pro Ala Gly Glu Leu Tyr Gly Trp Ala Val
225                 230                 235                 240

Glu Thr Thr Pro Ser Pro Gly Pro Gln Pro Ala Ala Leu Thr Thr Gly
                245                 250                 255

Glu Ala Ala Ala Pro Glu Ser Pro His Gln Ala Glu Pro Tyr Leu Ser
            260                 265                 270

Pro Ser Pro Ser Ala Cys Thr Ala Val Gln Glu Pro Ser Pro Gly Ala
        275                 280                 285

Leu Asp Val Thr Ile Met Tyr Lys Gly Arg Thr Val Leu Gln Lys Val
    290                 295                 300

Val Gly His Pro Ser Cys Thr Phe Leu Tyr Gly Pro Pro Asp Pro Ala
305                 310                 315                 320

Val Arg Ala Thr Asp Pro Gln Gln Val Ala Phe Pro Ser Pro Ala Glu
                325                 330                 335

Leu Pro Asp Gln Lys Gln Leu Arg Tyr Thr Glu Glu Leu Leu Arg His
            340                 345                 350

Val Ala Pro Gly Leu His Leu Glu Leu Arg Gly Pro Gln Leu Trp Ala
        355                 360                 365

Arg Arg Met Gly Lys Cys Lys Val Tyr Trp Glu Val Gly Gly Pro Pro
    370                 375                 380

Gly Ser Ala Ser Pro Ser Thr Pro Ala Cys Leu Leu Pro Arg Asn Cys
385                 390                 395                 400

Asp Thr Pro Ile Phe Asp Phe Arg Val Phe Phe Gln Glu Leu Val Glu
                405                 410                 415

Phe Arg Ala Arg Gln Arg Arg Gly Ser Pro Arg Tyr Thr Ile Tyr Leu
            420                 425                 430

Gly Phe Gly Gln Asp Leu Ser Ala Gly Arg Pro Lys Glu Lys Ser Leu
        435                 440                 445

Val Leu Val Lys Leu Glu Pro Trp Leu Cys Arg Val His Leu Glu Gly
    450                 455                 460

Thr Gln Arg Glu Gly Val Ser Ser Leu Asp Ser Ser Ser Leu Ser Leu
465                 470                 475                 480

Cys Leu Ser Ser Ala Asn Ser Leu Tyr Asp Asp Ile Glu Cys Phe Leu
                485                 490                 495

Met Glu Leu Glu Gln Pro Ala
            500

<210>  46
<211>  474
<212>  PRT
<213>  Homo sapiens

<400>  46

Met Ala Leu Ala Pro Glu Arg Ala Ala Pro Arg Val Leu Phe Gly Glu
1               5                   10                  15

Trp Leu Leu Gly Glu Ile Ser Ser Gly Cys Tyr Glu Gly Leu Gln Trp
            20                  25                  30

Leu Asp Glu Ala Arg Thr Cys Phe Arg Val Pro Trp Lys His Phe Ala
        35                  40                  45

Arg Lys Asp Leu Ser Glu Ala Asp Ala Arg Ile Phe Lys Ala Trp Ala
    50                  55                  60

Val Ala Arg Gly Arg Trp Pro Pro Ser Ser Arg Gly Gly Gly Pro Pro
65                  70                  75                  80

Pro Glu Ala Glu Thr Ala Glu Arg Ala Gly Trp Lys Thr Asn Phe Arg
                85                  90                  95

Cys Ala Leu Arg Ser Thr Arg Arg Phe Val Met Leu Arg Asp Asn Ser
            100                 105                 110

Gly Asp Pro Ala Asp Pro His Lys Val Tyr Ala Leu Ser Arg Glu Leu
        115                 120                 125

Cys Trp Arg Glu Gly Pro Gly Thr Asp Gln Thr Glu Ala Glu Ala Pro
    130                 135                 140

Ala Ala Val Pro Pro Pro Gln Gly Gly Pro Pro Gly Pro Phe Leu Ala
145                 150                 155                 160

His Thr His Ala Gly Leu Gln Ala Pro Gly Pro Leu Pro Ala Pro Ala
                165                 170                 175

Gly Asp Lys Gly Asp Leu Leu Leu Gln Ala Val Gln Gln Ser Cys Leu
            180                 185                 190

Ala Asp His Leu Leu Thr Ala Ser Trp Gly Ala Asp Pro Val Pro Thr
        195                 200                 205

Lys Ala Pro Gly Glu Gly Gln Glu Gly Leu Pro Leu Thr Gly Ala Cys
    210                 215                 220

Ala Gly Gly Glu Ala Ala Ala Pro Glu Ser Pro His Gln Ala Glu Pro
225                 230                 235                 240

Tyr Leu Ser Pro Ser Pro Ser Ala Cys Thr Ala Val Gln Glu Pro Ser
                245                 250                 255

Pro Gly Ala Leu Asp Val Thr Ile Met Tyr Lys Gly Arg Thr Val Leu
            260                 265                 270

Gln Lys Val Val Gly His Pro Ser Cys Thr Phe Leu Tyr Gly Pro Pro
        275                 280                 285

Asp Pro Ala Val Arg Ala Thr Asp Pro Gln Gln Val Ala Phe Pro Ser
    290                 295                 300

Pro Ala Glu Leu Pro Asp Gln Lys Gln Leu Arg Tyr Thr Glu Glu Leu
305                 310                 315                 320

Leu Arg His Val Ala Pro Gly Leu His Leu Glu Leu Arg Gly Pro Gln
                325                 330                 335

Leu Trp Ala Arg Arg Met Gly Lys Cys Lys Val Tyr Trp Glu Val Gly
            340                 345                 350

Gly Pro Pro Gly Ser Ala Ser Pro Ser Thr Pro Ala Cys Leu Leu Pro
        355                 360                 365

Arg Asn Cys Asp Thr Pro Ile Phe Asp Phe Arg Val Phe Phe Gln Glu
    370                 375                 380

Leu Val Glu Phe Arg Ala Arg Gln Arg Arg Gly Ser Pro Arg Tyr Thr
385                 390                 395                 400

Ile Tyr Leu Gly Phe Gly Gln Asp Leu Ser Ala Gly Arg Pro Lys Glu
                405                 410                 415

Lys Ser Leu Val Leu Val Lys Leu Glu Pro Trp Leu Cys Arg Val His
            420                 425                 430

Leu Glu Gly Thr Gln Arg Glu Gly Val Ser Ser Leu Asp Ser Ser Ser
        435                 440                 445

Leu Ser Leu Cys Leu Ser Ser Ala Asn Ser Leu Tyr Asp Asp Ile Glu
    450                 455                 460

Cys Phe Leu Met Glu Leu Glu Gln Pro Ala
465                 470

<210>  47
<211>  516
<212>  PRT
<213>  Homo sapiens

<400>  47

Met Pro Val Pro Glu Arg Pro Ala Ala Gly Pro Asp Ser Pro Arg Pro
1               5                   10                  15

Gly Thr Arg Arg Ala Ala Pro Arg Val Leu Phe Gly Glu Trp Leu Leu
            20                  25                  30

Gly Glu Ile Ser Ser Gly Cys Tyr Glu Gly Leu Gln Trp Leu Asp Glu
        35                  40                  45

Ala Arg Thr Cys Phe Arg Val Pro Trp Lys His Phe Ala Arg Lys Asp
    50                  55                  60

Leu Ser Glu Ala Asp Ala Arg Ile Phe Lys Ala Trp Ala Val Ala Arg
65                  70                  75                  80

Gly Arg Trp Pro Pro Ser Ser Arg Gly Gly Gly Pro Pro Pro Glu Ala
                85                  90                  95

Glu Thr Ala Glu Arg Ala Gly Trp Lys Thr Asn Phe Arg Cys Ala Leu
            100                 105                 110

Arg Ser Thr Arg Arg Phe Val Met Leu Arg Asp Asn Ser Gly Asp Pro
        115                 120                 125

Ala Asp Pro His Lys Val Tyr Ala Leu Ser Arg Glu Leu Cys Trp Arg
    130                 135                 140

Glu Gly Pro Gly Thr Asp Gln Thr Glu Ala Glu Ala Pro Ala Ala Val
145                 150                 155                 160

Pro Pro Pro Gln Gly Gly Pro Pro Gly Pro Phe Leu Ala His Thr His
                165                 170                 175

Ala Gly Leu Gln Ala Pro Gly Pro Leu Pro Ala Pro Ala Gly Asp Lys
            180                 185                 190

Gly Asp Leu Leu Leu Gln Ala Val Gln Gln Ser Cys Leu Ala Asp His
        195                 200                 205

Leu Leu Thr Ala Ser Trp Gly Ala Asp Pro Val Pro Thr Lys Ala Pro
    210                 215                 220

Gly Glu Gly Gln Glu Gly Leu Pro Leu Thr Gly Ala Cys Ala Gly Gly
225                 230                 235                 240

Pro Gly Leu Pro Ala Gly Glu Leu Tyr Gly Trp Ala Val Glu Thr Thr
                245                 250                 255

Pro Ser Pro Gly Pro Gln Pro Ala Ala Leu Thr Thr Gly Glu Ala Ala
            260                 265                 270

Ala Pro Glu Ser Pro His Gln Ala Glu Pro Tyr Leu Ser Pro Ser Pro
        275                 280                 285

Ser Ala Cys Thr Ala Val Gln Glu Pro Ser Pro Gly Ala Leu Asp Val
    290                 295                 300

Thr Ile Met Tyr Lys Gly Arg Thr Val Leu Gln Lys Val Val Gly His
305                 310                 315                 320

Pro Ser Cys Thr Phe Leu Tyr Gly Pro Pro Asp Pro Ala Val Arg Ala
                325                 330                 335

Thr Asp Pro Gln Gln Val Ala Phe Pro Ser Pro Ala Glu Leu Pro Asp
            340                 345                 350

Gln Lys Gln Leu Arg Tyr Thr Glu Glu Leu Leu Arg His Val Ala Pro
        355                 360                 365

Gly Leu His Leu Glu Leu Arg Gly Pro Gln Leu Trp Ala Arg Arg Met
    370                 375                 380

Gly Lys Cys Lys Val Tyr Trp Glu Val Gly Gly Pro Pro Gly Ser Ala
385                 390                 395                 400

Ser Pro Ser Thr Pro Ala Cys Leu Leu Pro Arg Asn Cys Asp Thr Pro
                405                 410                 415

Ile Phe Asp Phe Arg Val Phe Phe Gln Glu Leu Val Glu Phe Arg Ala
            420                 425                 430

Arg Gln Arg Arg Gly Ser Pro Arg Tyr Thr Ile Tyr Leu Gly Phe Gly
        435                 440                 445

Gln Asp Leu Ser Ala Gly Arg Pro Lys Glu Lys Ser Leu Val Leu Val
    450                 455                 460

Lys Leu Glu Pro Trp Leu Cys Arg Val His Leu Glu Gly Thr Gln Arg
465                 470                 475                 480

Glu Gly Val Ser Ser Leu Asp Ser Ser Ser Leu Ser Leu Cys Leu Ser
                485                 490                 495

Ser Ala Asn Ser Leu Tyr Asp Asp Ile Glu Cys Phe Leu Met Glu Leu
            500                 505                 510

Glu Gln Pro Ala
        515

<210>  48
<211>  2862
<212>  DNA
<213>  Homo sapiens

<400>  48
agattcctac ttcttacgcc ccccacatca cccgcctcga gacctcaagg gtagaggtgg      60

gcacccccgc ctccgcactt ttgctcgggg ctccagattg tagggcaggg cggcgcttct     120

cggaaagcga aagccggcgg ggcggggcgg gtgccgcagg agaaagagga agcgctggca     180

gacaatgcga cccgaccgcg ctgaggctcc aggaccgccc gccatggctg caggaggtcc     240

cggcgcgggg tctgcggccc cggtctcctc cacatcctcc cttcccctgg ctgctctcaa     300

catgcgagtg cggcgccgcc tgtctctgtt cttgaacgtg cggacacagg tggcggccga     360

ctggaccgcg ctggcggagg agatggactt tgagtacttg gagatccggc aactggagac     420

acaagcggac cccactggca ggctgctgga cgcctggcag ggacgccctg gcgcctctgt     480

aggccgactg ctcgagctgc ttaccaagct gggccgcgac gacgtgctgc tggagctggg     540

acccagcatt gaggaggatt gccaaaagta tatcttgaag cagcagcagg aggaggctga     600

gaagccttta caggtggccg ctgtagacag cagtgtccca cggacagcag agctggcggg 660

catcaccaca cttgatgacc ccctggggca tatgcctgag cgtttcgatg ccttcatctg 720

ctattgcccc agcgacatcc agtttgtgca ggagatgatc cggcaactgg aacagacaaa 780

ctatcgactg aagttgtgtg tgtctgaccg cgatgtcctg cctggcacct gtgtctggtc 840

tattgctagt gagctcatcg aaaagaggtg ccgccggatg gtggtggttg tctctgatga 900

ttacctgcag agcaaggaat gtgacttcca gaccaaattt gcactcagcc tctctccagg 960

tgcccatcag aagcgactga tccccatcaa gtacaaggca atgaagaaag agttccccag 1020

catcctgagg ttcatcactg tctgcgacta caccaacccc tgcaccaaat cttggttctg 1080

gactcgcctt gccaaggcct tgtccctgcc ctgaagactg ttctgaggcc ctgggtgtgt 1140

gtgtatctgt ctgcctgtcc atgtacttct gccctgcctc ctcctttcgt tgtaggagga 1200

atctgtgctc tacttacctc tcaattcctg gagatgccaa cttcacagac acgtctgcag 1260

cagctggaca tcacatttca tgtcctgcat ggaaccagtg gctgtgagtg gcatgtccac 1320

ttgctggatt atcagccagg acactataga acaggaccag ctgagactaa gaaggaccag 1380

cagagccagc tcagctctga gccattcaca catcttcacc ctcagtttcc tcacttgagg 1440

agtgggatgg ggagaacaga gagtagctgt gtttgaatcc ctgtaggaaa tggtgaagca 1500

tagctctggg tctcctgggg gagaccaggc ttggctgcgg gagagctggc tgttgctgga 1560

ctacatgctg gccactgctg tgaccacgac actgctgggg cagcttcttc cacagtgatg 1620

cctactgatg cttcagtgcc tctgcacacc gcccattcca cttcctcctt ccccacaggg 1680

caggtgggga agcagtttgg cccagcccaa ggagacccca ccttgagcct tatttcctaa 1740

tgggtccacc tctcatctgc atctttcaca cctcccagct tctgcccaac cttcagcagt 1800

gacaagtccc caagagactc gcctgagcag cttgggctgc ttttcatttc cacctgtcag 1860

gatgcctgtg gtcatgctct cagctccacc tggcatgaga agggatcctg gcctctggca 1920

tattcatcaa gtatgagttc tggggatgag tcactgtaat gatgtgagca gggagccttc 1980

ctccctgggc cacctgcaga gagctttccc accaactttg taccttgatt gccttacaaa 2040

gttatttgtt tacaaacagc gaccatataa aagcctcctg ccccaaagct tgtgggcaca 2100

tgggcacata cagactcaca tacagacaca cacatatatg tacagacatg tactctcaca 2160

cacacaggca ccagcataca cacgtttttc taggtacagc tcccaggaac agctaggtgg 2220

gaaagtccca tcactgaggg agcctaacca tgtccctgaa caaaaattgg gcactcatct 2280

attccttttc tcttgtgtcc ctactcattg aaaccaaact ctggaaagga cccaatgtac 2340

cagtatttat acctctaatg aagcacagag agaggaagag agctgcttaa actcacacaa 2400

caatgaactg cagacacagc tgttctctcc ctctctcctt cccagagcaa tttatacttt 2460

accctcaggc tgtcctctgg ggagaaggtg ccatggtctt aggtgtctgt gccccaggac 2520

agaccctagg accctaaatc caatagaaaa tgcatatctt tgctccactt tcagccaggc 2580

tggagcaagg taccttttct taggatcttg ggagggaatg gatgcccctc tctgcatgat 2640

cttgttgagg catttagctg ccatgcacct gtcccccttt aatactgggc attttaaagc 2700

catctcaaga ggcatcttct acatgttttg tacgcattaa aataatttca aagatatctg 2760

agaaaagccg atatttgcca ttcttcctat atcctggaat atatcttgca tcctgagttt 2820

ataataataa ataatattct accttggaaa aaaaaaaaaa aa 2862

<210> 49
<211> 2720
<212> DNA
<213> Homo sapiens

<400> 49
gcggggtgga gccagcgccc tcagcgcgct acggtccgcg ggcaactccg cagaagcccc 60

agcccccagg accccaggac ccagtggcgc agccggcagc cccggatccc tgatctgctt 120

gggcagctcc tgcagaacct ggaacagtga atgggtaggg gacactgggc gtgcagaagg 180

cggggggcag tgtggaacat gccttcacca cctccagctt ctgctgccgg aggctgcacc 240

cacctgtgcc catggcctgc acaggcccat cacttcctag cgccttcgac attctaggtg 300

cagcaggcca ggacaagctc ttgtatctga agcacaaact gaagacccca cgcccaggct 360

gccaggggca ggacctcctg catgccatgg ttctcctgaa gctgggccag gaaactgagg 420

ccaggatctc tctagaggca ttgaaggccg atgcggtggc ccggctggtg gcccgccagt 480

gggctggcgt ggacagcacc gaggacccag aggagccccc agatgtgtcc tgggctgtgg 540

cccgcttgta ccacctgctg gctgaggaga agctgtgccc cgcctcgctg cgggacgtgg 600

cctaccagga agccgtccgc accctcagct ccagggacga ccaccggctg ggggaacttc 660

aggatgaggc ccgaaaccgg tgtgggtggg acattgctgg ggatccaggg agcatccgga 720

cgctccagtc caatctgggc tgcctcccac catcctcggc tttgccctct gggaccagga 780

gcctcccacg ccccattgac ggtgtttcgg actggagcca agggtgctcc ctgcgatcca 840

ctggcagccc tgcctccctg gccagcaact tggaaatcag ccagtcccct accatgccct 900

tcctcagcct gcaccgcagc ccacatgggc ccagcaagct ctgtgacgac ccccaggcca 960

gcttggtgcc cgagcctgtc cccggtggct gccaggagcc tgaggagatg agctggccgc 1020

catcggggga gattgccagc ccaccagagc tgccaagcag cccacctcct gggcttcccg 1080

aagtggcccc agatgcaacc tccactggcc tccctgatac ccccgcagct ccagaaacca 1140

gcaccaacta cccagtggag tgcaccgagg ggtctgcagg cccccagtct ctccccttgc 1200

ctattctgga gccggtcaaa aacccctgct ctgtcaaaga ccagacgcca ctccaacttt 1260

ctgtagaaga taccacctct ccaaatacca agccgtgccc acctactccc accaccccag 1320

aaacatcccc tcctcctcct cctcctcctc cttcatctac tccttgttca gctcacctga 1380

ccccctcctc cctgttccct tcctccctgg aatcatcatc ggaacagaaa ttctataact 1440

ttgtgatcct ccacgccagg gcagacgaac acatcgccct gcgggttcgg gagaagctgg 1500

aggcccttgg cgtgcccgac ggggccacct tctgcgagga tttccaggtg ccggggcgcg 1560

gggagctgag ctgcctgcag gacgccatag accactcagc tttcatcatc ctacttctca 1620

cctccaactt cgactgtcgc ctgagcctgc accaggtgaa ccaagccatg atgagcaacc 1680

tcacgcgaca ggggtcgcca gactgtgtca tccccttcct gcccctggag agctccccgg 1740

cccagctcag ctccgacacg gccagcctgc tctccgggct ggtgcggctg gacgaacact 1800

cccagatctt cgccaggaag gtggccaaca ccttcaagcc ccacaggctt caggcccgaa 1860

aggccatgtg gaggaaggaa caggacaccc gagccctgcg ggaacagagc caacacctgg 1920

acggtgagcg gatgcaggcg gcggcactga acgcagccta ctcagcctac ctccagagct 1980

acttgtccta ccaggcacag atggagcagc tccaggtggc ttttgggagc cacatgtcat 2040

ttgggactgg ggcgccctat ggggctcgaa tgccctttgg gggccaggtg cccctgggag 2100

ccccgccacc ctttcccact tggccggggt gcccgcagcc gccacccctg cacgcatggc 2160

aggctggcac ccccccaccg ccctccccac agccagcagc ctttccacag tcactgccct 2220

tcccgcagtc cccagccttc cctacggcct cacccgcacc ccctcagagc ccagggctgc 2280

aacccctcat tatccaccac gcacagatgg tacagctggg gctgaacaac cacatgtgga 2340

accagagagg gtcccaggcg cccgaggaca agacgcagga ggcagaatga ccgcgtgtcc 2400

ttgcctgacc acctggggaa cacccctgga cccaggcatc ggccaggacc ccatagagca 2460

ccccggtctg ccctgtgccc tgtggacagt ggaagatgag gtcatctgcc actttcagga 2520

cattgtccgg gagcccttca tttaggacaa aacgggcgcg atgatgccct ggctttcagg 2580

gtggtcagaa ctggatacgg tgtttacaat tccaatctct ctatttctgg gtgaagggtc 2640

ttggtggtgg gggtattgct acggtctttt aattataata aatatttatt gaatgcttcc 2700

gcaaaaaaaa aaaaaaaaaa 2720

<210> 50
<211> 4992
<212> DNA
<213> Homo sapiens

<400> 50
gaagactcca gatataggat cactccatgc catcaagaaa gttgatgcta ttgggcccat 60

ctcaagctga tcttggcacc tctcatgctc tgctctcttc aaccagacct ctacattcca 120

ttttggaaga agactaaaaa tggtgtttcc aatgtggaca ctgaagagac aaattcttat 180

cctttttaac ataatcctaa tttccaaact ccttggggct agatggtttc ctaaaactct 240
gccctgtgat gtcactctgg atgttccaaa gaaccatgtg atcgtggact gcacagacaa 300
gcatttgaca gaaattcctg gaggtattcc cacgaacacc acgaacctca ccctcaccat 360
taaccacata ccagacatct ccccagcgtc ctttcacaga ctggaccatc tggtagagat 420
cgatttcaga tgcaactgtg tacctattcc actggggtca aaaaacaaca tgtgcatcaa 480
gaggctgcag attaaaccca gaagctttag tggactcact tatttaaaat ccctttacct 540
ggatggaaac cagctactag agataccgca gggcctcccg cctagcttac agcttctcag 600
ccttgaggcc aacaacatct tttccatcag aaaagagaat ctaacagaac tggccaacat 660
agaaatactc tacctgggcc aaaactgtta ttatcgaaat ccttgttatg tttcatattc 720
aatagagaaa gatgccttcc taaacttgac aaagttaaaa gtgctctccc tgaaagataa 780
caatgtcaca gccgtcccta ctgttttgcc atctacttta acagaactat atctctacaa 840
caacatgatt gcaaaaatcc aagaagatga ttttaataac ctcaaccaat tacaaattct 900
tgacctaagt ggaaattgcc ctcgttgtta taatgcccca tttccttgtg cgccgtgtaa 960
aaataattct cccctacaga tccctgtaaa tgcttttgat gcgctgacag aattaaaagt 1020
tttacgtcta cacagtaact ctcttcagca tgtgccccca agatggttta agaacatcaa 1080
caaactccag gaactggatc tgtcccaaaa cttcttggcc aaagaaattg gggatgctaa 1140
atttctgcat tttctcccca gcctcatcca attggatctg tctttcaatt ttgaacttca 1200
ggtctatcgt gcatctatga atctatcaca agcatttctt tcactgaaaa gcctgaaaat 1260
tctgcggatc agaggatatg tctttaaaga gttgaaaagc tttaacctct cgccattaca 1320
taatcttcaa aatcttgaag ttcttgatct tggcactaac tttataaaaa ttgctaacct 1380
cagcatgttt aaacaattta aaagactgaa agtcatagat ctttcagtga ataaaatatc 1440
accttcagga gattcaagtg aagttggctt ctgctcaaat gccagaactt ctgtagaaag 1500
ttatgaaccc caggtcctgg aacaattaca ttatttcaga tatgataagt atgcaaggag 1560
ttgcagattc aaaaacaaag aggcttcttt catgtctgtt aatgaaagct gctacaagta 1620
tgggcagacc ttggatctaa gtaaaaatag tatatttttt gtcaagtcct ctgattttca 1680
gcatctttct ttcctcaaat gcctgaatct gtcaggaaat ctcattagcc aaactcttaa 1740
tggcagtgaa ttccaacctt tagcagagct gagatatttg gacttctcca acaaccggct 1800
tgatttactc cattcaacag catttgaaga gcttcacaaa ctggaagttc tggatataag 1860
cagtaatagc cattattttc aatcagaagg aattactcat atgctaaact ttaccaagaa 1920
cctaaaggtt ctgcagaaac tgatgatgaa cgacaatgac atctcttcct ccaccagcag 1980
gaccatggag agtgagtctc ttagaactct ggaattcaga ggaaatcact tagatgtttt 2040
atggagagaa ggtgataaca gatacttaca attattcaag aatctgctaa aattagagga 2100

attagacatc tctaaaaatt ccctaagttt cttgccttct ggagtttttg atggtatgcc 2160

tccaaatcta aagaatctct ctttggccaa aaatgggctc aaatctttca gttggaagaa 2220

actccagtgt ctaaagaacc tggaaacttt ggacctcagc cacaaccaac tgaccactgt 2280

ccctgagaga ttatccaact gttccagaag cctcaagaat ctgattctta agaataatca 2340

aatcaggagt ctgacgaagt attttctaca agatgccttc cagttgcgat atctggatct 2400

cagctcaaat aaaatccaga tgatccaaaa gaccagcttc ccagaaaatg tcctcaacaa 2460

tctgaagatg ttgcttttgc atcataatcg gtttctgtgc acctgtgatg ctgtgtggtt 2520

tgtctggtgg gttaaccata cggaggtgac tattccttac ctggccacag atgtgacttg 2580

tgtggggcca ggagcacaca agggccaaag tgtgatctcc ctggatctgt acacctgtga 2640

gttagatctg actaacctga ttctgttctc actttccata tctgtatctc tctttctcat 2700

ggtgatgatg acagcaagtc acctctattt ctgggatgtg tggtatattt accatttctg 2760

taaggccaag ataaaggggt atcagcgtct aatatcacca gactgttgct atgatgcttt 2820

tattgtgtat gacactaaag acccagctgt gaccgagtgg gttttggctg agctggtggc 2880

caaactggaa gacccaagag agaaacattt taatttatgt ctcgaggaaa gggactggtt 2940

accagggcag ccagttctgg aaaacctttc ccagagcata cagcttagca aaaagacagt 3000

gtttgtgatg acagacaagt atgcaaagac tgaaaatttt aagatagcat tttacttgtc 3060

ccatcagagg ctcatggatg aaaaagttga tgtgattatc ttgatatttc ttgagaagcc 3120

ctttcagaag tccaagttcc tccagctccg gaaaaggctc tgtgggagtt ctgtccttga 3180

gtggccaaca aacccgcaag ctcacccata cttctggcag tgtctaaaga acgccctggc 3240

cacagacaat catgtggcct atagtcaggt gttcaaggaa acggtctagc ccttctttgc 3300

aaaacacaac tgcctagttt accaaggaga ggcctggctg tttaaattgt tttcatatat 3360

atcacaccaa aagcgtgttt tgaaattctt caagaaatga gattgcccat atttcagggg 3420

agccaccaac gtctgtcaca ggagttggaa agatggggtt tatataatgc atcaagtctt 3480

ctttcttatc tctctgtgtc tctatttgca cttgagtctc tcacctcagc tcctgtaaaa 3540

gagtggcaag taaaaaacat ggggctctga ttctcctgta attgtgataa ttaaatatac 3600

acacaatcat gacattgaga agaactgcat ttctaccctt aaaaagtact ggtatataca 3660

gaaatagggt taaaaaaaac tcaagctctc tctatatgag accaaaatgt actagagtta 3720

gtttagtgaa ataaaaaacc agtcagctgg ccgggcatgg tggctcatgc ttgtaatccc 3780

agcactttgg gaggccgagg caggtggatc acgaggtcag gagtttgaga ccagtctggc 3840

caacatggtg aaaccccgtc tgtactaaaa atacaaaaat tagctgggcg tggtggtggg 3900

tgcctgtaat cccagctact tgggaggctg aggcaggaga atcgcttgaa cccgggaggt 3960

ggaggtggca gtgagccgag atcacgccac tgcaatgcag cccgggcaac agagctagac 4020

tgtctcaaaa gaacaaaaaa aaaaaaacac aaaaaaactc agtcagcttc ttaaccaatt 4080

gcttccgtgt catccagggc cccattctgt gcagattgag tgtgggcacc acacaggtgg 4140

ttgctgcttc agtgcttcct gctctttttc cttgggcctg cttctgggtt ccatagggaa 4200

acagtaagaa agaaagacac atccttacca taaatgcata tggtccacct acaaatagaa 4260

aaatatttaa atgatctgcc tttatacaaa gtgatattct ctacctttga taatttacct 4320

gcttaaatgt ttttatctgc actgcaaagt actgtatcca aagtaaaatt tcctcatcca 4380

atatctttca aactgttttg ttaactaatg ccatatattt gtaagtatct gcacacttga 4440

tacagcaacg ttagatggtt ttgatggtaa accctaaagg aggactccaa gagtgtgtat 4500

ttatttatag ttttatcaga gatgacaatt atttgaatgc caattatatg gattcctttc 4560

attttttgct ggaggatggg agaagaaacc aaagtttata gaccttcaca ttgagaaagc 4620

ttcagttttg aacttcagct atcagattca aaaacaacag aaagaaccaa gacattctta 4680

agatgcctgt actttcagct gggtataaat tcatgagttc aaagattgaa acctgaccaa 4740

tttgctttat ttcatggaag aagtgatcta caaaggtgtt tgtgccattt ggaaaacagc 4800

gtgcatgtgt tcaagcctta gattggcgat gtcgtatttt cctcacgtgt ggcaatgcca 4860

aaggctttac tttacctgtg agtacacact atatgaatta tttccaacgt acatttaatc 4920

aataagggtc acaaattccc aaatcaatct ctggaataaa tagagaggta attaaattgc 4980

tggagccaac ta 4992

<210> 51
<211> 3196
<212> DNA
<213> Homo sapiens

<400> 51
agtgcggggt ctgacagagg aggctccgtg tctgcagcta gtgtgtcaac tcagcgtttc 60

tcctctcgtc cctggtgagg tgtagcggcg gcacgcggct ggagatcccc tgtggcctcc 120

agtttaggaa gggtccagca tcccaaggga ggggtgtgtg ggcgaggggt ctctgggccc 180

ggggtcgcgg ctgtgaggag aggatgcccg cgcggcggca tctcaggcac ctggaggagg 240

ccgcgctttc tcctcaggga accggcgcct tggcagcccc cggcgacgcc gcccccttcg 300

cggcctaggt tggtctggtg agccgggaag cgggcgtcgt tcgcagcgcc gctgtgacca 360

ccgcgtcccg ggcggagctg ggctcagtgc cggcctgggc ctagagtccg agcctcgagc 420

tgccggcgtg gggggtcgcg agtggcctaa tgcggcctcg aagccgaagg acccgagtcc 480

gagctcgcac tccgacccgc tggtgctgtg gaaaactcag gtggccttcc gctttcgtag 540

cctctaaagt ggggaccaag actttcacct cttaggattg tagtcgggat taaaagattt 600

tcccggaaga gctaaagatg gctgaatttc tagatgacca ggaaactcga ctgtgtgaca 660
actgcaaaaa agaaattcct gtgtttaact ttaccatcca tgagatccac tgtcaaagga 720
acattggtat gtgtcctacc tgtaaggaac catttcccaa atctgacatg gagactcaca 780
tggctgcaga acactgtcag gtgacctgca aatgtaacaa gaagttggag aagaggctgt 840
taaagaagca tgaggagact gagtgccctt tgcggcttgc tgtctgccag cactgtgatt 900
tagaactttc cattctcaaa ctgaaggaac atgaagatta ttgtggtgcc cggacggaac 960
tatgtggcaa ctgtggtcgc aatgtccttg tgaaagatct gaagactcac cctgaagttt 1020
gtgggagaga gggggaggaa aagagaaatg aggttgccat acctcctaat gcatatgatg 1080
aatcttgggg tcaggatgga atctggattg catcccaact cctcagacaa attgaggctc 1140
tggacccacc catgaggctg ccgcgaaggc ccctgagagc ctttgaatca gatgttttcc 1200
acaatagaac taccaaccaa aggaacatta cagcccaggt ttcaattcag aataatctgt 1260
ttgaagaaca agagaggcag gaaaggaata gaggccaaca gccccccaaa gagggtggtg 1320
aagagagtgc aaacttggac ttcatgttgg ccctaagtct gcaaaatgaa ggccaagcct 1380
ccagtgtggc agagcaggac ttctggaggg ccgtatgtga ggccgaccag tctcatggcg 1440
gtcccaggtc tctcagtgac ataaagggtg cagctgacga gatcatgttg ccttgtgaat 1500
tttgtgagga gctctaccca gaggaactgc tgattgacca tcagacaagc tgtaaccctt 1560
cacgtgcctt accttcactc aatactggca gctcttcccc cagaggggtg gaggaacctg 1620
atgtcatctt ccagaacttc ttgcaacagg ctgcaagtaa ccagttagac tctttgatgg 1680
gcctgagcaa ttcacaccct gtggaggaga gcatcattat cccatgtgaa ttctgtgggg 1740
tacagctgga agaggaggtg ctgttccatc accaggacca gtgtgaccaa cgcccagcca 1800
ctgcaaccaa ccatgtgaca gaggggattc ctagactgga ttcccagcct caagagacct 1860
caccagagct gcccaggagg cgtgtcagac accagggaga cctgtcttct ggttacctgg 1920
atgatactaa gcaggaaaca gctaatgggc ccacctcctg tctgcctccc agccgaccca 1980
ttaacaatat gacagctacc tataaccagc tatcgagatc aacatcaggc cccagacctg 2040
ggtgccagcc cagctctcct tgtgtgccga agctcagcaa ctcagacagc caggacatcc 2100
aggggcggaa tcgagacagc cagaatgggg ccatagcccc tgggcacgtt tcagtgattc 2160
gccctcctca aaatctctac ccagaaaaca ttgtgccctc tttctcccct gggccttcag 2220
ggagatacgg agctagtggt aggagtgaag gtggcaggaa ttcccgggtc acccctgcag 2280
ctgccaacta ccgcagcaga actgcaaagg caaagccttc caagcaacag ggagctgggg 2340
atgcagaaga ggaagaggag gagtaatggt gtctccagag actttacatc ggttcctgtc 2400
ttctgtgcac agcagcactt gccgctgtgc aggcccacct ctttggctct ttgggtggga 2460
gagtttttcc agattttaga tttttctagg ttatggccat tttgtgtctt ttgaggttgt 2520

gctgtggggg tttgggtttg agggaaggga gcagggtggc ggttgaggaa cgcttcagcc 2580

ttagctgcta cctttcggca gcagtgaaat acaagctgca gcctcggctg ccagggctcc 2640

cttttgactt attgtcgcca ctgccccttg gtgctgtgtg gtcccagtgg aaggagggga 2700

agattttgga aacctggtag ccaccagtaa ggtgattctc tgccctgttg gggcctaaat 2760

ttgggggctt ttgggcaacc tctccgtgta ctgcgtctgt ccacactcga ttgggcccca 2820

ggtgtgtatg aggcgctctg gtaaggtgct caggccagtt gcaatgtctg tcagtaacga 2880

ggcttttgat gtgttgagct ggaggtgagt ggaccggggg ctgtgtttta agctgcttcc 2940

ttggcatttg gcatcactgc cttctgttcc cgggggagca tggatctttt gtcctcactg 3000

ctttctaatg gggagggctg agggctccct gtccccacag caggtatgtt gggctctgcc 3060

ccagccccac acttgctctg aaaaccaagt gtcagagccc cttccccttg tttttatttt 3120

actgttataa taattattaa cttccttgta atagaaataa agtttgtact tggagttcaa 3180

aaaaaaaaaa aaaaaa 3196

<210> 52
<211> 2665
<212> DNA
<213> Homo sapiens

<400> 52
agtgcggggt ctgacagagg aggctccgtg tctgcagcta gtgtgtcaac tcagcgtttc 60

tcctctcgtc cctggaagag ctaaagatgg ctgaatttct agatgaccag gaaactcgac 120

tgtgtgacaa ctgcaaaaaa gaaattcctg tgtttaactt taccatccat gagatccact 180

gtcaaaggaa cattggtatg tgtcctacct gtaaggaacc atttcccaaa tctgacatgg 240

agactcacat ggctgcagaa cactgtcagg tgacctgcaa atgtaacaag aagttggaga 300

agaggctgtt aaagaagcat gaggagactg agtgcccttt gcggcttgct gtctgccagc 360

actgtgattt agaactttcc attctcaaac tgaaggaaca tgaagattat tgtggtgccc 420

ggacggaact atgtggcaac tgtggtcgca atgtccttgt gaaagatctg aagactcacc 480

ctgaagtttg tgggagagag ggggaggaaa agagaaatga ggttgccata cctcctaatg 540

catatgatga atcttggggt caggatggaa tctggattgc atcccaactc ctcagacaaa 600

ttgaggctct ggacccaccc atgaggctgc cgcgaaggcc cctgagagcc tttgaatcag 660

atgttttcca caatagaact accaaccaaa ggaacattac agcccaggtt tcaattcaga 720

ataatctgtt tgaagaacaa gagaggcagg aaaggaatag aggccaacag ccccccaaag 780

agggtggtga agagagtgca aacttggact tcatgttggc cctaagtctg caaaatgaag 840

gccaagcctc cagtgtggca gagcaggact tctggagggc cgtatgtgag gccgaccagt 900

ctcatggcgg tcccaggtct ctcagtgaca taaagggtgc agctgacgag atcatgttgc 960

cttgtgaatt ttgtgaggag ctctacccag aggaactgct gattgaccat cagacaagct 1020

gtaacccttc acgtgcctta ccttcactca atactggcag ctcttccccc agaggggtgg 1080

aggaacctga tgtcatcttc cagaacttct tgcaacaggc tgcaagtaac cagttagact 1140

ctttgatggg cctgagcaat tcacaccctg tggaggagag catcattatc ccatgtgaat 1200

tctgtggggt acagctggaa gaggaggtgc tgttccatca ccaggaccag tgtgaccaac 1260

gcccagccac tgcaaccaac catgtgacag aggggattcc tagactggat tcccagcctc 1320

aagagacctc accagagctg cccaggaggc gtgtcagaca ccagggagac ctgtcttctg 1380

gttacctgga tgatactaag caggaaacag ctaatgggcc cacctcctgt ctgcctccca 1440

gccgacccat taacaatatg acagctacct ataaccagct atcgagatca acatcaggcc 1500

ccagacctgg gtgccagccc agctctcctt gtgtgccgaa gctcagcaac tcagacagcc 1560

aggacatcca ggggcggaat cgagacagcc agaatggggc catagcccct gggcacgttt 1620

cagtgattcg ccctcctcaa aatctctacc cagaaaacat tgtgccctct ttctcccctg 1680

ggccttcagg gagatacgga gctagtggta ggagtgaagg tggcaggaat tcccgggtca 1740

cccctgcagc tgccaactac cgcagcagaa ctgcaaaggc aaagccttcc aagcaacagg 1800

gagctgggga tgcagaagag gaagaggagg agtaatggtg tctccagaga ctttacatcg 1860

gttcctgtct tctgtgcaca gcagcacttg ccgctgtgca ggcccacctc tttggctctt 1920

tgggtgggag agtttttcca gattttagat ttttctaggt tatggccatt ttgtgtcttt 1980

tgaggttgtg ctgtgggggt ttgggtttga gggaagggag cagggtggcg gttgaggaac 2040

gcttcagcct tagctgctac ctttcggcag cagtgaaata caagctgcag cctcggctgc 2100

cagggctccc ttttgactta ttgtcgccac tgccccttgg tgctgtgtgg tcccagtgga 2160

aggaggggaa gattttggaa acctggtagc caccagtaag gtgattctct gccctgttgg 2220

ggcctaaatt tgggggcttt tgggcaacct ctccgtgtac tgcgtctgtc cacactcgat 2280

tgggccccag gtgtgtatga ggcgctctgg taaggtgctc aggccagttg caatgtctgt 2340

cagtaacgag gcttttgatg tgttgagctg gaggtgagtg gaccgggggc tgtgttttaa 2400

gctgcttcct tggcatttgg catcactgcc ttctgttccc gggggagcat ggatcttttg 2460

tcctcactgc tttctaatgg ggagggctga gggctccctg tccccacagc aggtatgttg 2520

ggctctgccc cagccccaca cttgctctga aaaccaagtg tcagagcccc ttccccttgt 2580

ttttatttta ctgttataat aattattaac ttccttgtaa tagaaataaa gtttgtactt 2640

ggagttcaaa aaaaaaaaaa aaaaa 2665

<210> 53
<211> 1972
<212> DNA

<213>  Homo sapiens

<400>  53
gaaactcccg cctggccacc ataaaagcgc cggccctccg cttccccgcg agacgaaact      60

tcccgtcccg gcggctctgg cacccagggt ccggcctgcg ccttcccgcc aggcctggac     120

actggttcaa cacctgtgac ttcatgtgtg cgcgccggcc acacctgcag tcacacctgt     180

agccccctct gccaagagat ccataccgag gcagcgtcgg tggctacaag ccctcagtcc     240

acacctgtgg acacctgtga cacctggcca cacgacctgt ggccgcggcc tggcgtctgc     300

tgcgacagga gcccttacct cccctgttat aacacctgac cgccacctaa ctgcccctgc     360

agaaggagca atggccttgg ctcctgagag ggcagcccca cgcgtgctgt tcggagagtg     420

gctccttgga gagatcagca gcggctgcta tgaggggctg cagtggctgg acgaggcccg     480

cacctgtttc cgcgtgccct ggaagcactt cgcgcgcaag gacctgagcg aggccgacgc     540

gcgcatcttc aaggcctggg ctgtggcccg cggcaggtgg ccgcctagca gcaggggagg     600

tggcccgccc cccgaggctg agactgcgga gcgcgccggc tggaaaacca acttccgctg     660

cgcactgcgc agcacgcgtc gcttcgtgat gctgcgggat aactcggggg acccggccga     720

cccgcacaag gtgtacgcgc tcagccggga gctgtgctgg cgagaaggcc caggcacgga     780

ccagactgag gcagaggccc ccgcagctgt cccaccacca cagggtgggc ccccagggcc     840

attcctggca cacacacatg ctggactcca agccccaggc cccctccctg ccccagctgg     900

tgacaagggg gacctcctgc tccaggcagt gcaacagagc tgcctggcag accatctgct     960

gacagcgtca tggggggcag atccagtccc aaccaaggct cctggagagg gacaagaagg    1020

gcttcccctg actggggcct gtgctggagg cccagggctc cctgctgggg agctgtacgg    1080

gtgggcagta gagacgaccc ccagccccgg gccccagccc gcggcactaa cgacaggcga    1140

ggccgcggcc ccagagtccc cgcaccaggc agagccgtac ctgtcaccct ccccaagcgc    1200

ctgcaccgcg gtgcaagagc ccagcccagg ggcgctggac gtgaccatca tgtacaaggg    1260

ccgcacggtg ctgcagaagg tggtgggaca cccgagctgc acgttcctat acggcccccc    1320

agacccagct gtccgggcca cagaccccca gcaggtagca ttccccagcc ctgccgagct    1380

cccggaccag aagcagctgc gctacacgga ggaactgctg cggcacgtgg cccctgggtt    1440

gcacctggag cttcgggggc cacagctgtg ggcccggcgc atgggcaagt gcaaggtgta    1500

ctgggaggtg ggcggacccc caggctccgc cagcccctcc accccagcct gcctgctgcc    1560

tcggaactgt gacaccccca tcttcgactt cagagtcttc ttccaagagc tggtggaatt    1620

ccgggcacgg cagcgccgtg gctccccacg ctataccatc tacctgggct tcgggcagga    1680

cctgtcagct gggaggccca aggagaagag cctggtcctg gtgaagctgg aaccctggct    1740

gtgccgagtg cacctagagg gcacgcagcg tgagggtgtg tcttccctgg atagcagcag    1800

cctcagcctc tgcctgtcca gcgccaacag cctctatgac gacatcgagt gcttccttat 1860

ggagctggag cagcccgcct agaacccagt ctaatgagaa ctccagaaag ctggagcagc 1920

ccacctagag ctggccgcgg ccgcccagtc taataaaaag aactccagaa ca 1972

<210> 54
<211> 1885
<212> DNA
<213> Homo sapiens

<400> 54
gaaactcccg cctggccacc ataaaagcgc cggccctccg cttccccgcg agacgaaact 60

tcccgtcccg gcggctctgg cacccagggt ccggcctgcg ccttcccgcc aggcctggac 120

actggttcaa cacctgtgac ttcatgtgtg cgcgccggcc acacctgcag tcacacctgt 180

agccccctct gccaagagat ccataccgag gcagcgtcgg tggctacaag ccctcagtcc 240

acacctgtgg acacctgtga cacctggcca cacgacctgt ggccgcggcc tggcgtctgc 300

tgcgacagga gcccttacct cccctgttat aacacctgac cgccacctaa ctgcccctgc 360

agaaggagca atggccttgg ctcctgagag ggcagcccca cgcgtgctgt tcggagagtg 420

gctccttgga gagatcagca gcggctgcta tgaggggctg cagtggctgg acgaggcccg 480

cacctgtttc cgcgtgccct ggaagcactt cgcgcgcaag gacctgagcg aggccgacgc 540

gcgcatcttc aaggcctggg ctgtggcccg cggcaggtgg ccgcctagca gcaggggagg 600

tggcccgccc cccgaggctg agactgcgga gcgcgccggc tggaaaacca acttccgctg 660

cgcactgcgc agcacgcgtc gcttcgtgat gctgcgggat aactcggggg acccggccga 720

cccgcacaag gtgtacgcgc tcagccggga gctgtgctgg cgagaaggcc caggcacgga 780

ccagactgag gcagaggccc ccgcagctgt cccaccacca cagggtgggc ccccagggcc 840

attcctggca cacacacatg ctggactcca agccccaggc cccctccctg ccccagctgg 900

tgacaagggg gacctcctgc tccaggcagt gcaacagagc tgcctggcag accatctgct 960

gacagcgtca tggggggcag atccagtccc aaccaaggct cctggagagg gacaagaagg 1020

gcttcccctg actggggcct gtgctggagg cgaggccgcg gccccagagt ccccgcacca 1080

ggcagagccg tacctgtcac cctccccaag cgcctgcacc gcggtgcaag agcccagccc 1140

aggggcgctg gacgtgacca tcatgtacaa gggccgcacg gtgctgcaga aggtggtggg 1200

acacccgagc tgcacgttcc tatacggccc cccagaccca gctgtccggg ccacagaccc 1260

ccagcaggta gcattcccca gccctgccga gctcccggac cagaagcagc tgcgctacac 1320

ggaggaactg ctgcggcacg tggcccctgg gttgcacctg gagcttcggg ggccacagct 1380

gtgggcccgg cgcatgggca agtgcaaggt gtactgggag gtgggcggac ccccaggctc 1440

cgccagcccc tccaccccag cctgcctgct gcctcggaac tgtgacaccc ccatcttcga 1500

cttcagagtc ttcttccaag agctggtgga attccgggca cggcagcgcc gtggctcccc 1560

acgctatacc atctacctgg gcttcgggca ggacctgtca gctgggaggc ccaaggagaa 1620

gagcctggtc ctggtgaagc tggaaccctg gctgtgccga gtgcacctag agggcacgca 1680

gcgtgagggt gtgtcttccc tggatagcag cagcctcagc ctctgcctgt ccagcgccaa 1740

cagcctctat gacgacatcg agtgcttcct tatggagctg gagcagcccg cctagaaccc 1800

agtctaatga gaactccaga aagctggagc agcccaccta gagctggccg cggccgccca 1860

gtctaataaa aagaactcca gaaca 1885

<210> 55
<211> 2051
<212> DNA
<213> Homo sapiens

<400> 55
agggtgcgaa gcgccactgt ttaggtttcg ctttcccggg agcctgaccc gcccctgacg 60

tcgcctttcc cgtctccgca gggtccggcc tgcgccttcc cgccaggcct ggacactggt 120

tcaacacctg tgacttcatg tgtgcgcgcc ggccacacct gcagtcacac ctgtagcccc 180

ctctgccaag agatccatac cgaggcagcg tcggtggcta caagccctca gtccacacct 240

gtggacacct gtgacacctg gccacacgac ctgtggccgc ggcctggcgt ctgctgcgac 300

aggagccctt acctcccctg ttataacacc tgaccgccac ctaactgccc ctgcagaagg 360

agcaatggcc ttggctcctg agaggtaaga gcccggccca ccctctccag atgccagtcc 420

ccgagcgccc tgcagccggc cctgactctc cgcggccggg cacccgcagg gcagccccac 480

gcgtgctgtt cggagagtgg ctccttggag agatcagcag cggctgctat gaggggctgc 540

agtggctgga cgaggcccgc acctgtttcc gcgtgccctg gaagcacttc gcgcgcaagg 600

acctgagcga ggccgacgcg cgcatcttca aggcctgggc tgtggcccgc ggcaggtggc 660

cgcctagcag caggggaggt ggcccgcccc ccgaggctga gactgcggag cgcgccggct 720

ggaaaaccaa cttccgctgc gcactgcgca gcacgcgtcg cttcgtgatg ctgcgggata 780

actcggggga cccggccgac ccgcacaagg tgtacgcgct cagccgggag ctgtgctggc 840

gagaaggccc aggcacggac cagactgagg cagaggcccc cgcagctgtc ccaccaccac 900

agggtgggcc cccagggcca ttcctggcac acacacatgc tggactccaa gccccaggcc 960

ccctccctgc cccagctggt gacaaggggg acctcctgct ccaggcagtg caacagagct 1020

gcctggcaga ccatctgctg acagcgtcat gggggcagaa tccagtccca accaaggctc 1080

ctggagaggg acaagaaggg cttcccctga ctggggcctg tgctggaggc ccagggctcc 1140

ctgctgggga gctgtacggg tgggcagtag agacgacccc cagccccggg ccccagcccg 1200

cggcactaac gacaggcgag gccgcggccc cagagtcccc gcaccaggca gagccgtacc 1260

tgtcaccctc cccaagcgcc tgcaccgcgg tgcaagagcc cagcccaggg gcgctggacg 1320

tgaccatcat gtacaagggc cgcacggtgc tgcagaaggt ggtgggacac ccgagctgca 1380

cgttcctata cggcccccca gacccagctg tccgggccac agacccccag caggtagcat 1440

tccccagccc tgccgagctc ccggaccaga agcagctgcg ctacacggag gaactgctgc 1500

ggcacgtggc ccctgggttg cacctggagc ttcgggggcc acagctgtgg gcccggcgca 1560

tgggcaagtg caaggtgtac tgggaggtgg gcggaccccc aggctccgcc agcccctcca 1620

ccccagcctg cctgctgcct cggaactgtg acacccccat cttcgacttc agagtcttct 1680

tccaagagct ggtggaattc cgggcacggc agcgccgtgg ctccccacgc tataccatct 1740

acctgggctt cgggcaggac ctgtcagctg ggaggcccaa ggagaagagc ctggtcctgg 1800

tgaagctgga accctggctg tgccgagtgc acctagaggg cacgcagcgt gagggtgtgt 1860

cttccctgga tagcagcagc ctcagcctct gcctgtccag cgccaacagc ctctatgacg 1920

acatcgagtg cttccttatg gagctggagc agcccgccta gaacccagtc taatgagaac 1980

tccagaaagc tggagcagcc cacctagagc tggccgcggc cgcccagtct aataaaaaga 2040

actccagaac a 2051

<210> 56
<211> 515
<212> DNA
<213> Homo sapiens

<400> 56
gttgaggatc tcttactctc taagccacgg aattaacccg agcaggcatg gaggcctctg 60

ctctcacctc atcagcagtg accagtgtgg ccaaagtggt cagggtggcc tctggctctg 120

ccgtagtttt gcccctggcc aggattgcta cagttgtgat tggaggagtt gtggccatgg 180

cggctgtgcc catggtgctc agtgccatgg gcttcactgc ggcgggaatc gcctcgtcct 240

ccatagcagc caagatgatg tccgcggcgg ccattgccaa tgggggtgga gttgcctcgg 300

gcagccttgt gggtactctg cagtcactgg gagcaactgg actctccgga ttgaccaagt 360

tcatcctggg ctccattggg tctgccattg cggctgtcat tgcgaggttc tactagctcc 420

ctgcccctcg ccctgcagag aagagaacca tgccagggga gaaggcaccc agccatcctg 480

acccagcgag gagccaacta tcccaaatat acctg 515

<210> 57
<211> 544
<212> DNA
<213> Homo sapiens

<400> 57
taactctacc ctggcactat aatgtaagct ctactgaggt gctatgttct tagtggatgt 60

tctgaccctg cttcaaatat ttccctcacc tttcccatct tccaagggta ctaaggaatc 120

tttctgcttt ggggtttatc agaattctca gaatctcaaa taactaaaag gtatgcaatc 180

aaatctgctt tttaaagaat gctctttact tcatggactt ccactgccat cctcccaagg 240

ggcccaaatt ctttcagtgg ctacctacat acaattccaa acacatacag gaaggtagaa 300

atatctgaaa atgtatgtgt aagtattctt atttaatgaa agactgtaca aagtataagt 360

cttagatgta tatatttcct atattgtttt cagtgtacat ggaataacat gtaattaagt 420

actatgtatc aatgagtaac aggaaaattt taaaaataca gatagatata tgctctgcat 480

gttacataag ataaatgtgc tgaatggttt tcaaataaaa atgaggtact ctcctggaaa 540

tatt 544

<210> 58
<211> 482
<212> DNA
<213> Homo sapiens

<400> 58
atttctttgg cagttttcgt gctaatgttt ttgctaagga agataagctc tgaaccatta 60

aaggacgagt ttaaaaacac aggatcaggt ctcctgggca tggctaacat tgacctggaa 120

aagagcagga ctggtgatga aattattctt ccgagaggcc tcgagtacac ggtggaagaa 180

tgcacctgtg aagactgcat caagagcaaa ccgaaggtcg actctgacca ttgctttcca 240

ctcccagcta tggaggaagg cgcaaccatt cttgtcacca cgaaaacgaa tgactattgc 300

aagagcctgc cagctgcttt gagtgctacg gagatagaga aatcaatttc tgctaggtaa 360

ttaaccattt cgactcgagc agtgccactt taaaaatctt ttgtcagaat agatgatgtg 420

tcagatctct ttaggatgac tgtatttttc agttgccgat acagcttttt gtcctctaac 480

tg 482

<210> 59
<211> 534
<212> DNA
<213> Homo sapiens

<400> 59
gaacattcca ggcagacttt cctctgggcc ctgccaccca cggagggacc tacagatgct 60

tcggctcttt ccgtgccctg ccctgcgtgt ggtcaaactc aagtgaccca ctgcttgttt 120

ctgtcacagg aaacccttca agtagttggc cttcacccac agaaccaagc tccaaatctg 180

gtatctgcag acacctgcat gttctgattg ggacctcagt ggtcatcttc ctcttcatcc 240

tcctcctctt ctttctcctt tatcgctggt gctccaacaa aaagaatgct gctgtaatgg 300

accaagagcc tgcgggggac agaacagtga ataggcagga ctctgatgaa caagaccctc 360

aggaggtgac gtacgcacag ttggatcact gcgttttcat acagagaaaa atcagtcgcc 420

cttctcagag gcccaagaca cccctaacag ataccagcgt gtacacggaa cttccaaatg 480

ctgagcccag atccaaagtt gtctcctgcc cacgagcacc acagtcaggt cttg 534

<210> 60
<211> 534
<212> DNA
<213> Homo sapiens

<400> 60
ggaacttcca aatgctgagc ccagatccaa agttgtctcc tgcccacgag caccacagtc 60

aggtcttgag gggtttttct agggagacaa cagccctgtc tcaaaaccag gttgccagat 120

ccaatgaacc agcagctgga atctgaaggc atcagtctgc atcttagggg atcgctcttc 180

ctcacaccac gaatctgaac atgcctctct cttgcttaca aatgcctaag gtcgccactg 240

cctgctgcag agaaaacaca ctcctttgct tagcccacaa ggtatctatt tcacttgacc 300

cctgcccacc tctccaacct aactggctta cttcctagtc ctacttgagg ctgcaatcac 360

actgaggaac tcacaattcc aaacatacaa gaggctccct cttaacacgg cacttacaca 420

cttgctgttc caccttccct catgctgttc cacctcccct cagactatct ttcagccttc 480

tgtcatcagt aaaatttata aatttttttt ataacttcag tgtagctctc tcct 534

<210> 61
<211> 456
<212> DNA
<213> Homo sapiens

<400> 61
   taatggacca agagcctgcg ggggacagaa cagtgaatag gcaggactct gatgaacaag 60
   accctcagga ggtgacgtac gcacagttgg atcactgcgt tttcatacag agaaaaatca 120
   gtcgcccttc tcagaggccc aagacacccc taacagatac cagcgtgtac acggaacttc 180
   caaatgctga gcccagatcc aaagttgtct cctgcccacg agcaccacag tcaggtcttg 240
   agggggtttt ctagggagac aacagccctg tctcaaaacc aggttgccag atccaatgaa 300
   ccagcagctg gaatctgaag gcatcagtct gcatcttagg ggatcgctct tcctcacacc 360
   acgaatctga acatgcctct ctcttgctta caaatgccta aggtcgccac tgcctgctgc 420
   agagaaaaca cactcctttg cttagcccac aagtat 456

<210> 62
<211> 547
<212> DNA
<213> Homo sapiens

<400> 62
acctgcatgt tctgattggg acctcagtgg tcatcttcct cttcatcctc ctcctcttct 60

ttctccttta tcgctggtgc tccaacaaaa agaatgctgc tgtaatggac caagagcctg 120

cgggggacag aacagtgaat aggcaggact ctgatgaaca agaccctcag gaggtgacgt 180

acgcacagtt ggatcactgc gttttcatac agagaaaaat cagtcgccct tctcagaggc 240

ccaagacacc cctaacagat accagcgtgt acacggaact tccaaatgct gagcccagat 300

ccaaagttgt ctcctgccca cgagcaccac agtcaggtct tgagggggtt ttctagggag 360

acaacagccc tgtctcaaaa ccaggttgcc agatccaatg aaccagcagc tggaatctga 420

aggcatcagt ctgcatctta ggggatcgct cttcctcaca ccacgaatct gaacatgcct 480

ctctcttgct tacaaatgcc taaggtcgcc actgcctgct gcagagaaaa cacactcctt 540

tgcttag 547

<210> 63
<211> 488
<212> DNA
<213> Homo sapiens

<400> 63
gaacagcgaa caggcaggac tctgatgaac aagaccctca ggaggtgaca tacacacagt 60

tgaatcactg cgttttcaca cagagaaaaa tcactcgccc ttctcagagg cccaagacac 120

ccccaacaga tatcatcgtg tacgcggaac ttccaaatgc tgagtccaga tccaaagttg 180

tctcctgccc atgagcacca cagtcaggcc ttgagggcat cttctaggga gacaacagcc 240

ctgtctcaaa accgggttgc cagctcccat gtaccagcag ctggaatctg aaggcatgag 300

tctgcatctt agggcatcgc tcttcctcac accacaaatc tgaatgtgcc tctcacttgc 360

ttacaaatgt ctaaggtccc cactgcctgc tggagaaaaa acacactcct ttgcttagcc 420

cacagttctc catttcactt gacccctgcc cacctctcca acctaactgg cttacttcct 480

agtctact 488

<210> 64
<211> 475
<212> DNA
<213> Homo sapiens

<400> 64
tcagtgtaat ccttgacttt gctcctcacc atcagggcaa acttgccttc ttccctccta 60

agctccagta aataaacaga acagctttca ccaaagtggg tagtatagtc ctcaaatatc 120

ggataaatat atgcgttttt gtaccccaga aaaacttttc ctccctcttc atcaacatag 180

taaaataagt caaacaaaat gagaacacca aattttgggg gaataaattt ttatttaaca 240

ctgcaaagga aagagagaga aaacaagcaa agataggtag gacagaaagg aagacagcca 300

gatccagtga ttgacttggc atgaaaatga gaaaatgcag acagacctca acattcaaca 360

ttcaacaaca tccatacagc actgctggag gaagaggaag atttgtgcag accaagagca 420

ccacagacta caactgccca gcttcatcta aatacttgtt aacctctttg gtcat 475

<210>  65
<211>  526
<212>  DNA
<213>  Homo sapiens

<400>  65
tgcagggccc aaggtcaacg gaacattcca ggccgacttt cctctgggcc ctgccaccca      60

cggagggacc tacagatgct tcggctcttt ccgtgactct ccatacgagt ggtcaaactc     120

gagtgaccca ctgcttgttt ctgtcacagg aaacccttca aatagttggc cttcacccac     180

tgaaccaagc tccgaaaccg gtaaccccag acacctgcat gttctgattg ggacctcagt     240

ggtcatcatc ctcttcatcc tcctcctctt ctttctcctt catcgctggt gctgcaacaa     300

aaaaaatgct gttgtaatgg accaagagcc tgcagggaac agaacagtga acagggagga     360

ctctgatgaa caagaccctc aggaggtgac atatgcacag ttgaatcact gcgttttcac     420

acagagaaaa atcactcacc cttctcagag gcccaagaca cccccaacag atatcatcgt     480

gtacacggaa cttccaaatg ctgagccctg atccaaagtt gtctcc                    526


<210>  66
<211>  542
<212>  DNA
<213>  Homo sapiens

<400>  66
actggccctt ctcagaggag caagagaccc tcaacagata ccagcgtgtg tatagaactt      60

ccaaatgctg agcccagagc gttatctcct gcgcatgagc accacagtca ggccttgatg     120

ggatcttcta gggagacaac agccctgtct caaacccagc ttgccagctc ccatgtacca     180

gcagctggaa tctgaaggcg tgagtctcca tcttagagca tcactcttcc tcacaccaca     240

aatctggtgc ctgtctcttg cttaccaatg tctaaggtcc ccactgcctg ctgcagagaa     300

aacacactcc tttgcttagc ccacaattct ctatttcact tgacccctgc ccacctctcc     360

aacctaactg gcttacttcc tagtctactt gaggctgcaa tcacactgag gaactcacaa     420

ttccaaacat acaagaggct ctctcttaac acggcactta gacacgtgct gttccacctt     480

ccctcgtgct gttccacctt tcctcagact atttttcagc cttctggcat cagcaaacct     540

ta                                                                    542


<210>  67
<211>  225
<212>  DNA
<213>  Homo sapiens

<400>  67
aatctactaa ttccacacct tttattgaca cagaaaatgt tgagaatccc aaatttgatt      60

gatttgaaga acatgtgaga ggtttgacta gatgatggat gccaatatta aatctgctgg     120

agtttcatgt acaagatgaa ggagaggcaa catccaaaat agttaagaca tgatttcctt 180

gaatgtggct tgagaaatat ggacacttaa tactaccttg aaaat 225

<210> 68
<211> 388
<212> DNA
<213> Homo sapiens

<400> 68
ctgggattac aggcttgagc ccccgcgccc agccatcaaa atgcttttta tttctgcata 60

tgtttgaata ctttttacaa tttaaaaaaa tgatctgttt tgaaggcaaa attgcaaatc 120

ttgaaattaa gaaggcaaaa tgtaaaggag tcaaactata aatcaagtat ttgggaagtg 180

aagactggaa gctaatttgc ataaattcac aaacttttat actctttctg tatatacatt 240

ttttttcttt aaaaaacaac tatggatcag aatagccaca tttagaacac tttttgttat 300

cagtcaatat ttttagatag ttagaacctg gtcctaagcc taaaagtggg cttgattctg 360

cagtaaatct tttacaactg cctcgaca 388

<210> 69
<211> 399
<212> DNA
<213> Homo sapiens

<400> 69
tcacaaactt ttatactctt tctgtatata catttttttt ctttaaaaaa caactatgga 60

tcagaatagc cacatttaga acactttttg ttatcagtca atatttttag atagttagaa 120

cctggtccta agcctaaaag tgggcttgat tctgcagtaa atcttttaca actgcctcga 180

cacacataaa cctttttaaa aatagacact ccccgaagtc ttttgttcgc atggtcacac 240

actgatgctt agatgttcca gtaatctaat atggccacag tagtcttgat gaccaaagtc 300

ctttttttcc atctttagaa aactacatgg gaacaaacag atcgaacagt tttgaagcta 360

ctgtgtgtgt gaatgaacac tcttgcttta ttccagaat 399

<210> 70
<211> 459
<212> DNA
<213> Homo sapiens


<220>
<221> misc_feature
<222> (32)..(32)
<223> n is a, c, g, or t

<220>
<221> misc_feature
<222> (78)..(78)
<223> n is a, c, g, or t

<220>
<221>  misc_feature
<222>  (82)..(82)
<223>  n is a, c, g, or t

<220>
<221>  misc_feature
<222>  (242)..(242)
<223>  n is a, c, g, or t

<400>  70
tcacaaactt ttatactctt tctgtatata cntttttttt ctttaaaaaa caactatgga      60

tcagaatagc cacatttnga anactttttg ttatcagtca atatttttag atagttagaa     120

cctggtccta agcctaaaag tgggcttgat tctgcagtaa atcttttaca actgcctcga     180

cacacataaa cctttttaaa aatagacact ccccgaagtc ttttgttcgc atggtcacac     240

anctgatgct tagatgttcc agtaatctaa tatggccaca gtagtcttga tgaccaaagt     300

cctttttttc catctttaga aaactacatg ggaacaaaca gatcgaacag ttttgaagct     360

actgtgtgtg tgaatgaaca ctcttgcttt attccagaat gctgtacatc tattttggat      420

tgtatattgt gtttgtgtat ttacgctttg attcatagt                            459

<210>  71
<211>  346
<212>  DNA
<213>  Homo sapiens

<400>  71
cgcgcaccga cggaggggac atgggcagag caatggtggc caggctcggg ctggggctgc      60

tgctgctggc actgctccta cccacgcaga tttattccag tgaaacaaca actggaactt     120

caagtaactc ctcccagagt acttccaact ctgggttggc cccaaatcca actaatgcca     180

ccaccaaggt ggctggtggt gccctgcagt caacagccag tctcttcgtg gtctcactct     240

ctcttctgca tctctactct taagagactc aggccaagaa acgtcttcta aatttcccca     300

tcttctaaac ccaatccaaa tggcgtctgg aagtccaatg tggcaa                    346

<210>  72
<211>  478
<212>  DNA
<213>  Homo sapiens

<400>  72
ttcacaaact tttatactct ttctgtatat acattttttt tctttaaaaa acaactatgg      60

atcagaatag caacatttag aacacttttt gttatcagtc aatattttta gatagttaga     120

acctggtcct aagcctaaaa gtgggcttga ttctgcagta aatcttttac aactgcctcg     180

acacacataa acctttttaa aaatagacac tccccgaagt cttttgtttg tatggtcaca     240

cactgatgct tagatgttcc agtaatctaa tatggccaca gtagtcttga tgaccaaagt     300

cctttttttc catctttaga aaactacatg ggaacaaaca gatcgaacag ttttgaagct 360

actgtgtgtg tgaatgaaca ctcttgcttt attccagaat gctgtacatc tattttggat 420

tgtatattgt ggttgtgtat ttacgctttg attcatagta acttcttatg gaattgat 478

<210> 73
<211> 517
<212> DNA
<213> Homo sapiens

<400> 73
gtggcggagc agctgagagc ctacctggag ggcgagtgcg tggagtggct ccgcagatac 60

ctggagaacg ggaaggagac gctgcagcgc gcggaccccc caaagacaca cgtgacccac 120

caccccatct ctgaccatga ggccaccctg aggtgctggg ccctgggctt ctaccctgcg 180

gagatcacac tgacctggca gcgggatggc gaggaccaaa ctcaggacac tgagcttgtg 240

gagaccagac cagcaggaga tagaaccttc cagaagtggg cagctgtggt ggtgccttct 300

ggagaagagc agagatacac atgccatgta cagcatgagg ggctgccgaa gcccctcacc 360

ctgagatggg agccgtcttc ccagtccacc gtccccatcg tgggcattgt tgctggcctg 420

gctgtcctag cagttgtggt catcggagct gtggtcgctg ctgtgatgtg taggaggaag 480

agctcaggtg gaaaaggagg gagctactct caggctg 517

<210> 74
<211> 509
<212> DNA
<213> Homo sapiens

<400> 74
gacctggcag cgggatggcg aggaccaaac tcaggacacc gagcttgtgg agaccagacc 60

agcaggagac agaaccttcc agaagtgggc agctgtggtg gtgccttctg gagaagagca 120

gagatacaca tgccatgtac agcatgaggg gctgccgaag cccctcaccc tgagatggga 180

gccatcttcc cagtccaccg tccccatcgt gggcattgtt gctggcctgg ctgtcctagc 240

agttgtggtc atcggagctg tggtcgctgc tgtgatgtgt aggaggaaga gttcaggtgg 300

aaaaggaggg agctactctc aggctgcgtc cagcgacagt gcccagggct ctgatgtgtc 360

tctcacagct tgaaaagcct gagacagctg tcttgtgagg gactgagatg caggatttct 420

tcacgcctcc cctttgtgac ttcaagagcc tctggcatct ctttctgcaa aggcacctga 480

atgtgtctgc gtccctgtta gcataatgt 509

<210> 75
<211> 505
<212> DNA
<213> Homo sapiens

<400>  75
   ctgagagcct acctggaggg cctgtgcgtg gagtggctcc gcagatacct ggagaacggg      60
   aaggagacgc tgcagcgcgc ggacccccca aagacacatg tgacccacca ccccatctct      120
   gaccatgagg ccaccctgag gtgctgggcc ctgggcttct accctgcgga gatcacactg      180
   acctggcagc gggatggcga ggaccaaact caggacaccg agcttgtgga gaccagacca      240
   gcaggagata gaaccttcca gaagtgggca gctgtggtgg tgccttctgg agaagagcag      300
   agatacacat gccatgtaca gcatgagggg ctgccgaagc ccctcaccct gagatgggag      360
   ccatcttccc agtccaccat ccccatcgtg ggcattgttg ctggcctggc tgtcctagca      420
   gttgtggtca tcggagctgt ggtcgctact gtgatgtgta ggaggaagag ctcaggtgga      480
   aaaggaggga gctactctca ggctg                                            505


<210>  76
<211>  425
<212>  DNA
<213>  Homo sapiens

<400>  76
gctgtcctag gagctgtggt ggctgttgtt atgtgtagga ggaagagctc aggtggaaaa      60

ggagggagct gctctcaggc tgcgtccagc aacagtgccc agggctctga tgagtctctc      120

atcgcttgta aagcctgaga cagctgcctg tgtgggactg agatgcagga tttcttcaca      180

cctctccttt gtgacttcaa gagcctctgg catctctttc tgcaaaggca tctgaatgcg      240

tctgcgttcc tgttagcata atgtgaggag gtggagagac agcccacccc cgtgtccacc      300

gtgacccctg tccccacact gacctgtgtt ccctccccga tcatctttcc tgttccagag      360

aagtgggctg gatgtctcca tctctgtctc aactttacgt gtactgagct gcaacttctt      420

acttc                                                                  425


<210>  77
<211>  230
<212>  DNA
<213>  Homo sapiens

<400>  77
aggtctagtc agagcgtcgt acaccctgat ggaaacacct agttgtgatg gactcagcag      60

cggccaggcc atcttccaag agtccttata cacaagattt gtaaccgttg ctttggggtc      120

ccaaaacgag ttagcggcgg gggcccaggg acagatttca cactgaaaat cagcaggctg      180

gaagcggggg atgtcggggt tcattaatac atgcgagata cacaatttcc                230


<210>  78
<211>  591
<212>  DNA
<213>  Homo sapiens

<400>  78
gaggcggagc agctgagagc ctacctggag ggcgagtgcg tggagtggct ccgcggatac      60

ctggagaacg ggaaggagac gctgcagcgc gcggaacgcc caaagacaca cgtgacccac     120

catcccgtct ctgaccatga ggccaccctg aggtgctggg ccctgggctt ctaccctgcg     180

gagatcacac tgacctggca gcgggatggg gaggaccaaa ctcaggacac cgagcttgtg     240

gagaccaggc cagcaggaga tggaaccttc cagaagtggg cagctgtggt ggtgccttct     300

ggacaagaac agagatacac gtgccatgtg cagcacgagg ggctgcagga gccctgcacc     360

ctgagatgga agccgtcttc ccagcccacc atccccaact tgggcatcgt ttctggccca     420

gctgtcctgg ctgtcctggc tgtcctggct gtcctagctg tcctaggagc tgtggtcgct     480

gctgtgatac ataggaggaa gagctcaggt ggaaaaggag ggagctgctc tcaggctgcg     540

tccagcaaca gtgcccaggg ctctgatgag tctctcatcg cttgtaaagc c              591


<210>  79
<211>  524
<212>  DNA
<213>  Homo sapiens


<220>
<221>  misc_feature
<222>  (425)..(427)
<223>  n is a, c, g, or t

<220>
<221>  misc_feature
<222>  (436)..(436)
<223>  n is a, c, g, or t

<220>
<221>  misc_feature
<222>  (450)..(456)
<223>  n is a, c, g, or t

<400>  79
agctgtgatg gtgccttctg gagaagagca gagatacacg tgccatgtgc agcacgaggg      60

gctgccggag cccctcaccc tgagatggga gccgtcttcc cagcccacca tccccatcgt     120

gggcatcgtt gctggcctgg ctgtcctggc tgtcctagct gtcctaggag ctgtggtggc     180

tgttgtgatg tgtaggagga agagctcagg tggaaaagga gggagctgct ctcaggctgc     240

gtccagcaac agtgcccagg gctctgatga gtctctcatc gcttgtaaag cctgagacag     300

ctgcctgtgt gggactgaga tgcaggattt cttcacacct ctcctttgtg acttcaagag     360

cctctggcat ctctttctgc aaaggcatct gaatgtgtct gcgttcctgt tagcataatg     420

tgagnnngtg gagagnacag cccacccccn nnnnnncacc gtgacccctg tccccaacac     480

atgacctgtg ttccctcccc gatcatcttt cctgttccag agaa                      524

<210>  80
<211>  379
<212>  DNA
<213>  Homo sapiens

<400>  80
ccatcatggg catcgttgct ggcctggctg tcctggttgt cctagctgtc cttggagctg      60

tggtcaccgc tatgatgtgt aggaggaaga gctcaggtgg tgtgtgggac tgagatgcag     120

gatttcttca cacctctcct ttgtgacttc aagagcctct ggcatctctt tctgcaaagg     180

cgtctgaatg tgtctgcgtt cctgttagca taatgtgagg aggtggagag acagcccacc     240

cccgtgtcca ccgtgacccc tgtccccaca ctgacctgtg ttccctcccc gatcatcttt     300

cctgttccag agaggtgggg ctggatgtct ccatctctgt ctcaaattca tggtgcactg     360

agctgcaact tcttacttc                                                  379


<210>  81
<211>  493
<212>  DNA
<213>  Homo sapiens

<400>  81
tagctttggg gcttctgact gcagttcttc tgagtgtgct gctataccag tggatcctgt      60

gccagggctc caactactcc acttgtgcca gctgtcctag ctgcccagac cgctggatga     120

aatatggtaa ccattgttat tatttctcag tggaggaaaa ggactggaat tctagtctgg     180

aattctgcct agccagagac tcacacctcc ttgtgataac ggacaatcag gaaatgagcc     240

tgctccaagt tttcctcagt gaggcctttt gctggattgg tctgaggaac aattctggct     300

ggaggtggga agatggatca cctctaaact tctcaaggat ttcttctaat agctttgtgc     360

agacatgcgg tgccatcaac aaaaatggtc ttcaagcctc aagctgtgaa gttcctttac     420

actgggtgtg taagaaggtc agactttgat agatgaccac tctgtcctga ccctcagatc     480

tgtcatgtat ccc                                                        493


<210>  82
<211>  503
<212>  DNA
<213>  Homo sapiens

<400>  82
tggaccgcag cggacactgc ggctcagatc tccaagcgca agtgtgaggc ggccaatgtg      60

gctgaacaaa ggagagccta cctggagggc acgtgcgtgg agtggctcca cagatacctg     120

gagaacggga aggagatgct gcagcgcgcg gaccccccca agacacacgt gacccaccac     180

cctgtctttg actatgaggc caccctgagg tgctgggccc tgggcttcta ccctgcggag     240

atcatactga cctggcagcg ggatggggag gaccagaccc aggacgtgga gctcgtggag     300

accaggcctg caggggatgg aaccttccag aagtgggcag ctgtggtggt gccttctgga     360

gaggagcaga gatacacgtg ccatgtgcag catgaggggc tgccggagcc cctcatgctg 420

agatggaagc agtcttccct gcccaccatc cccatcatgg gtatcgttgc tggcctggtt 480

gtccttgcag ctgtagtcac tgg 503

<210> 83
<211> 434
<212> DNA
<213> Homo sapiens

<400> 83
caccaccctg tctttgacta tgaggccacc ctgaggtgct gggccctggg cttctaccct 60

gcggagatca tactgacctg gcagcgggat ggggaggacc agacccagga cgtggagctc 120

gtggagacca ggcctgcagg ggatggaacc ttccagaagt gggcagctgt ggtggtgcct 180

tctggagagg agcagagata cacgtgccat gtgcagcatg aggggctgcc ggagcccctc 240

atgctgagat ggaagcagtc ttccctgccc accatcccca tcatgggtat cgttgctggc 300

ctggttgtcc ttgcagctgt agtcactgga gctgcggtcg ctgctgtgct gtggagaaag 360

aagagctcag attgaaaagg agggagctac tctcaggctg caatgtgaaa cagctgccct 420

gtgtgggact gagt 434

<210> 84
<211> 482
<212> DNA
<213> Homo sapiens

<400> 84
caccaccctg tctttgacta tgaggccacc ctgaggtgct gggccctggg cttctaccct 60

gcggagatca tactgacctg gcagcgggat ggggaggacc agacccagga cgtggagctc 120

gtggagacca ggcctgcagg ggatggaacc ttccagaagt gggcagctgt ggtggtgcct 180

tctggagagg agcagagata cacgtgccat gtgcagcatg aggggctgcc ggagcccctc 240

atgctgagat ggaagcagtc ttccctgccc accatcccca tcatgggtat cgttgctggc 300

ctggttgtcc ttgcagctgt agtcactgga gctgcggtcg ctgctgtgct gtggagaaag 360

aagagctcag attgaaaagg agggagctac tctcaggctg caagcagtga cagtgcccag 420

ggctctaatg tgtctctcac ggcttgtaaa ttgtgaaaca gctgccctgt gtgggactga 480

gt 482

<210> 85
<211> 469
<212> DNA
<213> Homo sapiens

<400> 85
gctcccactc catgaggtat ttcagcgccg ccgtgtcccg gcccggccgc ggggagcccc 60

gcttcatcgc catgggctac gtggacgaca cgcagttcgt gcggttcgac agcgactcgg 120

cgtgtccgag gatggagccg cgggcgccgt gggtggagca ggaggggccg gagtattggg 180

aagaggagac acggaacacc aaggcccacg cacagactga cagaatgaac ctgcagaccc 240

tgcgcggcta ctacaaccag agcgaggcca agcagtcttc cctgcccacc atccccatca 300

tgggtatcgt tgctggcctg gttgtccttg cagctgtagt cactggagct gcggtcgctg 360

ctgtgctgtg gagaaagaag agctcagatt gaaaaggagg gagctactct caggctgcaa 420

tgtgaaacag ctgccctgtg tgggactgag tggcaagtcc ctttgtgac 469

<210> 86
<211> 402
<212> DNA
<213> Homo sapiens

<400> 86
ggagctccta tgacatgtac catctatcca gggagggggg agcccatgaa cgtaggctcc 60

ctgcagtgcg caaggtcaac agaacattcc aggcagattt ccctctgggc cctgccaccc 120

acggagggac ctacagatgc ttcggctctt tccgtcactc tccctacgag tggtcagacc 180

cgagtgaccc actgcttgtt tctgtcacag gaaacccttc aagtagttgg ccttcaccca 240

cagaaccaag ctccaaatct ggtaacctca gacacctgca cattctgatt gggacctcag 300

tggtcaaaat ccctttcacc atcctcctct tctttctcct tcatcgctgg tgctccaaca 360

aaaaaaaatg ctgctgtaat ggaccaagag cctgcaggga ac 402

<210> 87
<211> 528
<212> DNA
<213> Homo sapiens

<400> 87
tgggacctca gtggtcatca tcctcttcat cctcctcctc ttctttctcc ttcatctctg 60

gtgctccaac aaaaaaaatg ctgctgtaat ggaccaagag cctgcaggga acagaacagc 120

caacagcgag gactctgatg aacaagaccc tcaggaggtg acatatgcac agttggatca 180

ctgcgttttc acacagagaa aaatcactcg cccttctcag aggcccaaga caccccctac 240

agataccatc ttgtacacgg aacttccaaa tgctaagccc agatccaaag ttgtctcctg 300

cccatgagca ccacagtcag gccttgaggg cgtcttctag ggagacaaca gccctgtctc 360

aaaaccgagt tgccagctcc catgtaccag cagctggaat ctgaaggcgt gagtcttcat 420

cttagggcat cgctcctcct cacgccacaa atctggtgcc tctctcttgc ttacaaatgt 480

ctaggtcccc actgcctgct ggaaagaaaa cacactcctt tgcttagc 528

<210> 88

<211>  558
<212>  DNA
<213>  Homo sapiens

<220>
<221>  misc_feature
<222>  (126)..(127)
<223>  n is a, c, g, or t

<220>
<221>  misc_feature
<222>  (130)..(130)
<223>  n is a, c, g, or t

<220>
<221>  misc_feature
<222>  (139)..(139)
<223>  n is a, c, g, or t

<220>
<221>  misc_feature
<222>  (144)..(144)
<223>  n is a, c, g, or t

<220>
<221>  misc_feature
<222>  (148)..(148)
<223>  n is a, c, g, or t

<220>
<221>  misc_feature
<222>  (151)..(151)
<223>  n is a, c, g, or t

<220>
<221>  misc_feature
<222>  (168)..(168)
<223>  n is a, c, g, or t

<220>
<221>  misc_feature
<222>  (176)..(177)
<223>  n is a, c, g, or t

<220>
<221>  misc_feature
<222>  (220)..(221)
<223>  n is a, c, g, or t

<220>
<221>  misc_feature
<222>  (251)..(251)
<223>  n is a, c, g, or t

<220>
<221>  misc_feature
<222>  (392)..(392)
<223>  n is a, c, g, or t

<220>
<221>  misc_feature
<222>  (426)..(426)

<223>  n is a, c, g, or t

<400>  88
gacagacctc aggagggcta ttggtccagg acccacacct gctttcttca tgtttcctga      60

tcccgccctg ggtctgcagt cacacatttc tggaaacttc tctggggtcc aagactagga     120

ggttcnnctn ggaccttang gccntggntc ntttctggta tctcacangg acattnnctt     180

ctcacagata gaaaaggagg gagttacact caggctgcan ncagtgacag tgcccaggct     240

ctgatgtgtc nctcacagct tgtaaagtgt gagacagctg ccttgtgtgg gactgagagg     300

caagagttgt tcctgccctt ccctttgtga cttgaagaac cctgactttg tttctgcaaa     360

ggcacctgca tgtgtctgtg ttcgtgtagg cntaatgtga ggaggtgggg agaccacccc     420

accccnatgt ccaccatgac cctcttccca cgctgacctg tgctccctcc ccaatcatct     480

ttcctgttcc agagaggtgg ggctgaggtg tctccatctc tgtctcaact tcatggtgca     540

ctgagctgta acttcttc                                                   558


<210>  89
<211>  516
<212>  DNA
<213>  Homo sapiens


<220>
<221>  misc_feature
<222>  (64)..(64)
<223>  n is a, c, g, or t

<220>
<221>  misc_feature
<222>  (281)..(282)
<223>  n is a, c, g, or t

<220>
<221>  misc_feature
<222>  (322)..(322)
<223>  n is a, c, g, or t

<400>  89
tgagatggga gctgtcttcc cagcccacca tccccatcgt gggcatcatt gctggcctgg      60

ttcnccttgg agctgtgatc actggagctg tggtcgctgc cgtgatgtgg aggaggaaga     120

gctcaggtgg agaaggggtg aaagatagaa aaggagggag ttacactcag gctgcaagca     180

gtgacagtgc ccagggctct gatgtgtctc tcacagcttg taaagtgtga gacagctgcc     240

ttgtgtggga ctgagaggca agagttgttc ctgcccttcc nnttgtgact tgaagaaccc     300

tgactttgtt tctgcaaagg cncctgcatg tgtctgtgtt cgtgtaggca taatgtgagg     360

aggtggggag accaccccac cccgatgtcc accatgaccc tcttcccacg ctgacctgtg     420

ctccctctcc aatcatcttt cctgttccag agaggtgggg ctgaggtgtc tccatctctg     480

tctcaacttc atggtgcact gagctgtaac ttcttc                               516

<210>  90
<211>  550
<212>  DNA
<213>  Homo sapiens

<400>  90
ccttcatcgc tggtgtgcca acaaaaagaa tgctgttgta atggaccaag agcctgcagg      60

gaacagaaca gtgaacaggg aggactctga tgaacaagac cctcaggagg tgacatacgc     120

acagttgaat cactgcgttt tcacacagag aaaaatcact cgcccttctc agaggcccaa     180

gacaccccca acagatacca gcgtgtaaca cggaacttcc aaatgctgag cgcagatcca     240

aagttgtctt ctgtccacta gcaccacagt caggccttga tgggatcttc tagggagaca     300

atagccctgt ctcaaaaccg ggttgccagc tcccatgtac cagcagctgg actctgaagg     360

cgtgagtctg catcttaggg catcgctctt cctcacacca cgaatctgaa catgcctctc     420

tcttgcttac aaatgtctaa ggtccccact gcctgctgga gagaaaacac acttgcttag     480

cccacaattc tccatttcac ttgacccctg cccacctctc caacctaact ggcttacttc     540

ctagtctact                                                            550


<210>  91
<211>  122
<212>  PRT
<213>  Homo sapiens

<400>  91

Met Glu Ala Ser Ala Leu Thr Ser Ser Ala Val Thr Ser Val Ala Lys
1               5                   10                  15


Val Val Arg Val Ala Ser Gly Ser Ala Val Val Leu Pro Leu Ala Arg
            20                  25                  30


Ile Ala Thr Val Val Ile Gly Gly Val Val Ala Met Ala Ala Val Pro
        35                  40                  45


Met Val Leu Ser Ala Met Gly Phe Thr Ala Ala Gly Ile Ala Ser Ser
    50                  55                  60


Ser Ile Ala Ala Lys Met Met Ser Ala Ala Ala Ile Ala Asn Gly Gly
65                  70                  75                  80


Gly Val Ala Ser Gly Ser Leu Val Ala Thr Leu Gln Ser Leu Gly Ala
                85                  90                  95


Thr Gly Leu Ser Gly Leu Thr Lys Phe Ile Leu Gly Ser Ile Gly Ser
            100                 105                 110

Ala Ile Ala Ala Val Ile Ala Arg Phe Tyr
        115                 120

<210>  92
<211>  98
<212>  PRT
<213>  Homo sapiens

<400>  92

Met Asn Gln Thr Ala Ile Leu Ile Cys Cys Leu Ile Phe Leu Thr Leu
1               5                   10                  15

Ser Gly Ile Gln Gly Val Pro Leu Ser Arg Thr Val Arg Cys Thr Cys
            20                  25                  30

Ile Ser Ile Ser Asn Gln Pro Val Asn Pro Arg Ser Leu Glu Lys Leu
        35                  40                  45

Glu Ile Ile Pro Ala Ser Gln Phe Cys Pro Arg Val Glu Ile Ile Ala
    50                  55                  60

Thr Met Lys Lys Lys Gly Glu Lys Arg Cys Leu Asn Pro Glu Ser Lys
65                  70                  75                  80

Ala Ile Lys Asn Leu Leu Lys Ala Val Ser Lys Glu Arg Ser Lys Arg
                85                  90                  95

Ser Pro

<210>  93
<211>  184
<212>  PRT
<213>  Homo sapiens

<400>  93

Met Leu Gln Met Ala Gly Gln Cys Ser Gln Asn Glu Tyr Phe Asp Ser
1               5                   10                  15

Leu Leu His Ala Cys Ile Pro Cys Gln Leu Arg Cys Ser Ser Asn Thr
            20                  25                  30

Pro Pro Leu Thr Cys Gln Arg Tyr Cys Asn Ala Ser Val Thr Asn Ser
        35                  40                  45

Val Lys Gly Thr Asn Ala Ile Leu Trp Thr Cys Leu Gly Leu Ser Leu
    50                  55                  60

Ile Ile Ser Leu Ala Val Phe Val Leu Met Phe Leu Leu Arg Lys Ile
65                  70                  75                  80

Asn Ser Glu Pro Leu Lys Asp Glu Phe Lys Asn Thr Gly Ser Gly Leu
                85                  90                  95

Leu Gly Met Ala Asn Ile Asp Leu Glu Lys Ser Arg Thr Gly Asp Glu
            100                 105                 110

Ile Ile Leu Pro Arg Gly Leu Glu Tyr Thr Val Glu Glu Cys Thr Cys
        115                 120                 125

Glu Asp Cys Ile Lys Ser Lys Pro Lys Val Asp Ser Asp His Cys Phe
    130                 135                 140

Pro Leu Pro Ala Met Glu Glu Gly Ala Thr Ile Leu Val Thr Thr Lys
145                 150                 155                 160

Thr Asn Asp Tyr Cys Lys Ser Leu Pro Ala Ala Leu Ser Ala Thr Glu
                165                 170                 175

Ile Glu Lys Ser Ile Ser Ala Arg
            180

<210>  94
<211>  455
<212>  PRT
<213>  Homo sapiens

<400>  94

Met Ser Leu Thr Val Val Ser Met Ala Cys Val Gly Phe Phe Leu Leu
1               5                   10                  15

Gln Gly Ala Trp Pro Leu Met Gly Gly Gln Asp Lys Pro Phe Leu Ser
            20                  25                  30

Ala Arg Pro Ser Thr Val Val Pro Arg Gly Gly His Val Ala Leu Gln
        35                  40                  45

Cys His Tyr Arg Arg Gly Phe Asn Asn Phe Met Leu Tyr Lys Glu Asp
    50                  55                  60

Arg Ser His Val Pro Ile Phe His Gly Arg Ile Phe Gln Glu Ser Phe
65                  70                  75                  80

Ile Met Gly Pro Val Thr Pro Ala His Ala Gly Thr Tyr Arg Cys Arg
                85                  90                  95

Gly Ser Arg Pro His Ser Leu Thr Gly Trp Ser Ala Pro Ser Asn Pro
        100                 105                 110

Leu Val Ile Met Val Thr Gly Asn His Arg Lys Pro Ser Leu Leu Ala
    115                 120                 125

His Pro Gly Pro Leu Leu Lys Ser Gly Glu Thr Val Ile Leu Gln Cys
    130                 135                 140

Trp Ser Asp Val Met Phe Glu His Phe Phe Leu His Arg Glu Gly Ile
145                 150                 155                 160

Ser Glu Asp Pro Ser Arg Leu Val Gly Gln Ile His Asp Gly Val Ser
                165                 170                 175

Lys Ala Asn Phe Ser Ile Gly Pro Leu Met Pro Val Leu Ala Gly Thr
            180                 185                 190

Tyr Arg Cys Tyr Gly Ser Val Pro His Ser Pro Tyr Gln Leu Ser Ala
        195                 200                 205

Pro Ser Asp Pro Leu Asp Ile Val Ile Thr Gly Leu Tyr Glu Lys Pro
    210                 215                 220

Ser Leu Ser Ala Gln Pro Gly Pro Thr Val Gln Ala Gly Glu Asn Val
225                 230                 235                 240

Thr Leu Ser Cys Ser Ser Trp Ser Ser Tyr Asp Ile Tyr His Leu Ser
                245                 250                 255

Arg Glu Gly Glu Ala His Glu Arg Arg Leu Arg Ala Val Pro Lys Val
            260                 265                 270

Asn Arg Thr Phe Gln Ala Asp Phe Pro Leu Gly Pro Ala Thr His Gly
        275                 280                 285

Gly Thr Tyr Arg Cys Phe Gly Ser Phe Arg Ala Leu Pro Cys Val Trp
    290                 295                 300

Ser Asn Ser Ser Asp Pro Leu Leu Val Ser Val Thr Gly Asn Pro Ser
305                 310                 315                 320

Ser Ser Trp Pro Ser Pro Thr Glu Pro Ser Ser Lys Ser Gly Ile Cys
                325                 330                 335

Arg His Leu His Val Leu Ile Gly Thr Ser Val Val Ile Phe Leu Phe
            340                 345                 350

Ile Leu Leu Leu Phe Phe Leu Leu Tyr Arg Trp Cys Ser Asn Lys Lys
        355                 360                 365

Asn Ala Ala Val Met Asp Gln Glu Pro Ala Gly Asp Arg Thr Val Asn
    370                 375                 380

Arg Gln Asp Ser Asp Glu Gln Asp Pro Gln Glu Val Thr Tyr Ala Gln
385                 390                 395                 400

Leu Asp His Cys Val Phe Ile Gln Arg Lys Ile Ser Arg Pro Ser Gln
                405                 410                 415

Arg Pro Lys Thr Pro Leu Thr Asp Thr Ser Val Tyr Thr Glu Leu Pro
            420                 425                 430

Asn Ala Glu Pro Arg Ser Lys Val Val Ser Cys Pro Arg Ala Pro Gln
        435                 440                 445

Ser Gly Leu Glu Gly Val Phe
    450                 455

<210>  95
<211>  342
<212>  PRT
<213>  Homo sapiens

<400>  95

Met Ser Leu Thr Val Val Ser Met Ala Cys Val Gly Phe Phe Leu Leu
1               5                   10                  15

Gln Gly Ala Trp Pro Leu Met Gly Gly Gln Asp Lys Pro Phe Leu Ser
            20                  25                  30

Ala Arg Pro Ser Thr Val Val Pro Arg Gly Gly His Val Ala Leu Gln
        35                  40                  45

Cys His Tyr Arg Arg Gly Phe Asn Asn Phe Met Leu Tyr Lys Glu Asp
    50                  55                  60

Arg Ser His Val Pro Ile Phe His Gly Arg Ile Phe Gln Glu Ser Phe
65                  70                  75                  80

Ile Met Gly Pro Val Thr Pro Ala His Ala Gly Thr Tyr Arg Cys Arg
                85                  90                  95

Gly Ser Arg Pro His Ser Leu Thr Gly Trp Ser Thr Pro Ser Asn Pro
            100                 105                 110

Leu Val Ile Met Val Thr Gly Asn His Arg Lys Pro Ser Leu Leu Ala
        115                 120                 125

His Pro Gly Pro Leu Leu Lys Ser Gly Glu Thr Val Ile Leu Gln Cys
    130                 135                 140

Trp Ser Asp Val Met Phe Glu His Phe Phe Leu His Arg Glu Gly Ile
145                 150                 155                 160

Ser Glu Asp Pro Ser Arg Leu Val Gly Gln Ile His Asp Gly Val Ser
                165                 170                 175

Lys Ala Asn Phe Ser Ile Gly Pro Leu Met Pro Val Leu Ala Gly Thr
            180                 185                 190

Tyr Arg Cys Tyr Gly Ser Val Pro His Ser Pro Tyr Gln Leu Ser Ala
        195                 200                 205

Pro Ser Asp Pro Leu Asp Ile Val Ile Thr Gly Leu Tyr Glu Lys Pro
    210                 215                 220

Ser Leu Ser Ala Gln Pro Gly Pro Thr Val Gln Ala Gly Glu Asn Val
225                 230                 235                 240

Thr Leu Ser Cys Ser Ser Trp Ser Ser Tyr Asp Ile Tyr His Leu Ser
                245                 250                 255

Arg Glu Gly Glu Ala His Glu Arg Arg Leu Arg Ala Val Pro Lys Val
            260                 265                 270

Asn Arg Thr Phe Gln Ala Asp Phe Pro Leu Gly Pro Ala Thr His Gly
        275                 280                 285

Gly Thr Tyr Arg Cys Phe Gly Ser Phe Arg Ala Leu Pro Cys Val Trp
    290                 295                 300

Ser Asn Ser Ser Asp Pro Leu Leu Val Ser Val Thr Gly Asn Pro Ser
305                 310                 315                 320

Ser Ser Trp Pro Ser Pro Thr Glu Pro Ser Ser Lys Ser Gly Glu Thr
                325                 330                 335

Ser Tyr Lys Leu Glu Glu
            340

<210>  96
<211>  348

<212>  PRT
<213>  Homo sapiens

<400>  96

Met Ser Leu Met Val Val Ser Met Ala Cys Val Gly Phe Phe Leu Leu
1               5                   10                  15

Gln Gly Ala Trp Pro His Glu Gly Val His Arg Lys Pro Ser Leu Leu
            20                  25                  30

Ala His Pro Gly Arg Leu Val Lys Ser Glu Glu Thr Val Ile Leu Gln
        35                  40                  45

Cys Trp Ser Asp Val Arg Phe Glu His Phe Leu Leu His Arg Glu Gly
    50                  55                  60

Lys Phe Lys Asp Thr Leu His Leu Ile Gly Glu His His Asp Gly Val
65                  70                  75                  80

Ser Lys Ala Asn Phe Ser Ile Gly Pro Met Met Gln Asp Leu Ala Gly
                85                  90                  95

Thr Tyr Arg Cys Tyr Gly Ser Val Thr His Ser Pro Tyr Gln Leu Ser
            100                 105                 110

Ala Pro Ser Asp Pro Leu Asp Ile Val Ile Thr Gly Leu Tyr Glu Lys
        115                 120                 125

Pro Ser Leu Ser Ala Gln Pro Gly Pro Thr Val Leu Ala Gly Glu Ser
    130                 135                 140

Val Thr Leu Ser Cys Ser Ser Arg Ser Ser Tyr Asp Met Tyr His Leu
145                 150                 155                 160

Ser Arg Glu Gly Glu Ala His Glu Cys Arg Phe Ser Ala Gly Pro Lys
                165                 170                 175

Val Asn Gly Thr Phe Gln Ala Asp Phe Pro Leu Gly Pro Ala Thr His
            180                 185                 190

Gly Gly Thr Tyr Arg Cys Phe Gly Ser Phe Arg Asp Ser Pro Tyr Glu
        195                 200                 205

Trp Ser Asn Ser Ser Asp Pro Leu Leu Val Ser Val Thr Gly Asn Pro
    210                 215                 220

Ser Asn Ser Trp Pro Ser Pro Thr Glu Pro Ser Ser Lys Thr Gly Asn
225                 230                 235                 240

Pro Arg His Leu His Ile Leu Ile Gly Thr Ser Val Val Ile Ile Leu
                245                 250                 255

Phe Ile Leu Leu Phe Phe Leu Leu His Arg Trp Cys Ser Asn Lys Lys
            260                 265                 270

Asn Ala Ala Val Met Asp Gln Glu Ser Ala Gly Asn Arg Thr Ala Asn
        275                 280                 285

Ser Glu Asp Ser Asp Glu Gln Asp Pro Gln Glu Val Thr Tyr Thr Gln
    290                 295                 300

Leu Asn His Cys Val Phe Thr Gln Arg Lys Ile Thr Arg Pro Ser Gln
305                 310                 315                 320

Arg Pro Lys Thr Pro Pro Thr Asp Ile Ile Val Tyr Thr Glu Leu Pro
                325                 330                 335

Asn Ala Glu Ser Arg Ser Lys Val Val Ser Cys Pro
            340                 345

<210>  97
<211>  181
<212>  PRT
<213>  Homo sapiens

<400>  97

Met Leu Leu Glu Pro Gly Arg Gly Cys Cys Ala Leu Ala Ile Leu Leu
1               5                   10                  15

Ala Ile Val Asp Ile Gln Ser Gly Gly Cys Ile Asn Ile Thr Ser Ser
            20                  25                  30

Ala Ser Gln Glu Gly Thr Arg Leu Asn Leu Ile Cys Thr Val Trp His
        35                  40                  45

Lys Lys Glu Glu Ala Glu Gly Phe Val Val Phe Leu Cys Lys Asp Arg
    50                  55                  60

Ser Gly Asp Cys Ser Pro Glu Thr Ser Leu Lys Gln Leu Arg Leu Lys
65                  70                  75                  80

Arg Asp Pro Gly Ile Asp Gly Val Gly Glu Ile Ser Ser Gln Leu Met
                85                  90                  95

Phe Thr Ile Ser Gln Val Thr Pro Leu His Ser Gly Thr Tyr Gln Cys
            100                 105                 110

Cys Ala Arg Ser Gln Lys Ser Gly Ile Arg Leu Gln Gly His Phe Phe
        115                 120                 125

Ser Ile Leu Phe Thr Glu Thr Gly Asn Tyr Thr Val Thr Gly Leu Lys
    130                 135                 140

Gln Arg Gln His Leu Glu Phe Ser His Asn Glu Gly Thr Leu Ser Ser
145                 150                 155                 160

Gly Phe Leu Gln Glu Lys Val Trp Val Met Leu Val Thr Ser Leu Val
                165                 170                 175

Ala Leu Gln Ala Leu
            180

<210>  98
<211>  243
<212>  PRT
<213>  Homo sapiens

<400>  98

Met Ser Leu Met Val Val Ser Met Val Cys Val Gly Phe Phe Leu Leu
1               5                   10                  15

Gln Gly Ala Trp Pro His Glu Gly Val His Arg Lys Pro Ser Leu Leu
            20                  25                  30

Ala His Pro Gly Pro Leu Val Lys Ser Glu Glu Thr Val Ile Leu Gln
        35                  40                  45

Cys Trp Ser Asp Val Arg Phe Gln His Phe Leu Leu His Arg Glu Gly
    50                  55                  60

Lys Phe Lys Asp Thr Leu His Leu Ile Gly Glu His His Asp Gly Val
65                  70                  75                  80

Ser Lys Ala Asn Phe Ser Ile Gly Pro Met Met Gln Asp Leu Ala Gly
                85                  90                  95

Thr Tyr Arg Cys Tyr Gly Ser Val Thr His Ser Pro Tyr Gln Leu Ser
            100                 105                 110

Ala Pro Ser Asp Pro Leu Leu Val Ser Val Thr Gly Asn Pro Ser Asn
        115                 120                 125

Ser Trp Pro Ser Pro Thr Glu Pro Ser Ser Glu Thr Gly Asn Pro Arg
    130                 135                 140

His Leu His Val Leu Ile Gly Thr Ser Val Val Ile Ile Leu Phe Ile
145                 150                 155                 160

Leu Leu Leu Phe Phe Leu Leu His Arg Trp Cys Cys Asn Lys Lys Asn
                165                 170                 175

Ala Val Val Met Asp Gln Glu Pro Ala Gly Asn Arg Thr Val Asn Arg
            180                 185                 190

Glu Asp Ser Asp Glu Gln Asp Pro Gln Glu Val Thr Tyr Ala Gln Leu
        195                 200                 205

Asn His Cys Val Phe Thr Gln Arg Lys Ile Thr Arg Pro Ser Gln Arg
    210                 215                 220

Pro Lys Thr Pro Pro Thr Asp Ile Ile Val Tyr Thr Glu Leu Pro Asn
225                 230                 235                 240

Ala Glu Pro

<210>  99
<211>  341
<212>  PRT
<213>  Homo sapiens

<400>  99

Met Ser Leu Met Val Val Ser Met Val Cys Val Gly Phe Phe Leu Leu
1               5                   10                  15

Gln Gly Ala Trp Pro His Glu Gly Val His Arg Lys Pro Ser Leu Leu
            20                  25                  30

Ala His Pro Gly Pro Leu Val Lys Ser Glu Glu Thr Val Ile Leu Gln
        35                  40                  45

Cys Trp Ser Asp Val Arg Phe Gln His Phe Leu Leu His Arg Glu Gly
    50                  55                  60

Lys Phe Lys Asp Thr Leu His Leu Ile Gly Glu His His Asp Gly Val
65                  70                  75                  80

Ser Lys Ala Asn Phe Ser Ile Gly Pro Met Met Gln Asp Leu Ala Gly
                85                  90                  95

Thr Tyr Arg Cys Tyr Gly Ser Val Thr His Ser Pro Tyr Gln Leu Ser

100                 105                 110

Ala Pro Ser Asp Pro Leu Asp Ile Val Ile Thr Gly Leu Tyr Glu Lys
        115                 120                 125

Pro Ser Leu Ser Ala Gln Pro Gly Pro Thr Val Leu Ala Gly Glu Ser
    130                 135                 140

Val Thr Leu Ser Cys Ser Ser Arg Ser Ser Tyr Asp Met Tyr His Leu
145                 150                 155                 160

Ser Arg Glu Gly Glu Ala His Glu Arg Arg Phe Ser Ala Gly Pro Lys
                165                 170                 175

Val Asn Gly Thr Phe Gln Ala Asp Phe Pro Leu Gly Pro Ala Thr His
            180                 185                 190

Gly Gly Thr Tyr Arg Cys Phe Gly Ser Phe Arg Asp Ser Pro Tyr Glu
        195                 200                 205

Trp Ser Asn Ser Ser Asp Pro Leu Leu Val Ser Val Thr Gly Asn Pro
    210                 215                 220

Ser Asn Ser Trp Pro Ser Pro Thr Glu Pro Ser Ser Glu Thr Gly Asn
225                 230                 235                 240

Pro Arg His Leu His Val Leu Ile Gly Thr Ser Val Val Ile Ile Leu
                245                 250                 255

Phe Ile Leu Leu Leu Phe Phe Leu Leu His Arg Trp Cys Cys Asn Lys
            260                 265                 270

Lys Asn Ala Val Val Met Asp Gln Glu Pro Ala Gly Asn Arg Thr Val
        275                 280                 285

Asn Arg Glu Asp Ser Asp Glu Gln Asp Pro Gln Glu Val Thr Tyr Ala
    290                 295                 300

Gln Leu Asn His Cys Val Phe Thr Gln Arg Lys Ile Thr Arg Pro Ser
305                 310                 315                 320

Gln Arg Pro Lys Thr Pro Pro Thr Asp Ile Ile Val Tyr Thr Glu Leu
                325                 330                 335

Pro Asn Ala Glu Pro
            340

<210>  100
<211>  342
<212>  PRT
<213>  Homo sapiens

<400>  100

Met Ser Met Ser Pro Thr Val Ile Ile Leu Ala Cys Leu Gly Phe Phe
1               5                   10                  15

Leu Asp Gln Ser Val Trp Ala His Val Gly Gly Gln Asp Lys Pro Phe
            20                  25                  30

Cys Ser Ala Trp Pro Ser Ala Val Val Pro Gln Gly Gly His Val Thr
        35                  40                  45

Leu Arg Cys His Tyr Arg Arg Gly Phe Asn Ile Phe Thr Leu Tyr Lys
    50                  55                  60

Lys Asp Gly Val Pro Val Pro Glu Leu Tyr Asn Arg Ile Phe Trp Asn
65                  70                  75                  80

Ser Phe Leu Ile Ser Pro Val Thr Pro Ala His Ala Gly Thr Tyr Arg
                85                  90                  95

Cys Arg Gly Phe His Pro His Ser Pro Thr Glu Trp Ser Ala Pro Ser
            100                 105                 110

Asn Pro Leu Val Ile Met Val Thr Gly Leu Tyr Glu Lys Pro Ser Leu
        115                 120                 125

Thr Ala Arg Pro Gly Pro Thr Val Arg Ala Gly Glu Asn Val Thr Leu
    130                 135                 140

Ser Cys Ser Ser Gln Ser Ser Phe Asp Ile Tyr His Leu Ser Arg Glu
145                 150                 155                 160

Gly Glu Ala His Glu Leu Arg Leu Pro Ala Val Pro Ser Ile Asn Gly
                165                 170                 175

Thr Phe Gln Ala Asp Phe Pro Leu Gly Pro Ala Thr His Gly Glu Thr
            180                 185                 190

Tyr Arg Cys Phe Gly Ser Phe His Gly Ser Pro Tyr Glu Trp Ser Asp
        195                 200                 205

Pro Ser Asp Pro Leu Pro Val Ser Val Thr Gly Asn Pro Ser Ser Ser
    210                 215                 220

Trp Pro Ser Pro Thr Glu Pro Ser Phe Lys Thr Asp Ala Ala Val Met
225                 230                 235                 240

Asn Gln Glu Pro Ala Gly His Arg Thr Val Asn Arg Glu Asp Ser Asp
                245                 250                 255

Glu Gln Asp Pro Gln Glu Val Thr Tyr Ala Gln Leu Asp His Cys Ile
            260                 265                 270

Phe Thr Gln Arg Lys Ile Thr Gly Pro Ser Gln Arg Ser Lys Arg Pro
        275                 280                 285

Ser Thr Asp Thr Ser Val Cys Ile Glu Leu Pro Asn Ala Glu Pro Arg
    290                 295                 300

Ala Leu Ser Pro Ala His Glu His His Ser Gln Ala Leu Met Gly Ser
305                 310                 315                 320

Ser Arg Glu Thr Thr Ala Leu Ser Gln Thr Gln Leu Ala Ser Ser Asn
                325                 330                 335

Val Pro Ala Ala Gly Ile
            340

<210>  101
<211>  273
<212>  PRT
<213>  Homo sapiens

<400>  101

Met Ser Met Ser Pro Thr Val Ile Ile Leu Ala Cys Leu Gly Phe Phe
1               5                   10                  15

Leu Asp Gln Ser Val Trp Ala His Val Gly Gly Gln Asp Lys Pro Phe
            20                  25                  30

Cys Ser Ala Trp Pro Ser Ala Val Val Pro Gln Gly Gly His Val Thr
        35                  40                  45

Leu Arg Cys His Tyr Arg Arg Gly Phe Asn Ile Phe Thr Leu Tyr Lys
    50                  55                  60

Lys Asp Gly Val Pro Val Pro Glu Leu Tyr Asn Arg Ile Phe Trp Asn
65                  70                  75                  80

Ser Phe Leu Ile Ser Pro Val Thr Pro Ala His Ala Gly Thr Tyr Arg
                85                  90                  95

Cys Arg Gly Phe His Pro His Ser Pro Thr Glu Trp Ser Ala Pro Ser
            100                 105                 110

Asn Pro Leu Val Ile Met Val Thr Gly Leu Tyr Glu Lys Pro Ser Leu
        115                 120                 125

Thr Ala Arg Pro Gly Pro Thr Val Arg Ala Gly Glu Asn Val Thr Leu
    130                 135                 140

Ser Cys Ser Ser Gln Ser Ser Phe Asp Ile Tyr His Leu Ser Arg Glu
145                 150                 155                 160

Gly Glu Ala His Glu Leu Arg Leu Pro Ala Val Pro Ser Ile Asn Gly
                165                 170                 175

Thr Phe Gln Ala Asp Phe Pro Leu Gly Pro Ala Thr His Gly Glu Thr
            180                 185                 190

Tyr Arg Cys Phe Gly Ser Phe His Gly Ser Pro Tyr Glu Trp Ser Asp
        195                 200                 205

Pro Ser Asp Pro Leu Pro Val Ser Val Thr Gly Asn Pro Ser Ser Ser
    210                 215                 220

Trp Pro Ser Pro Thr Glu Pro Ser Phe Lys Thr Gly Ile Ala Arg His
225                 230                 235                 240

Leu His Ala Val Ile Arg Tyr Ser Val Ala Ile Ile Leu Phe Thr Ile
                245                 250                 255

Leu Pro Phe Phe Leu Leu His Arg Trp Cys Ser Lys Lys Lys Met Leu
            260                 265                 270

Leu

<210>  102
<211>  377
<212>  PRT
<213>  Homo sapiens

<400>  102

Met Ser Met Ser Pro Thr Val Ile Ile Leu Ala Cys Leu Gly Phe Phe
1               5                   10                  15

Leu Asp Gln Ser Val Trp Ala His Val Gly Gly Gln Asp Lys Pro Phe
            20                  25                  30

Cys Ser Ala Trp Pro Ser Ala Val Val Pro Gln Gly Gly His Val Thr
        35                  40                  45

Leu Arg Cys His Tyr Arg Arg Gly Phe Asn Ile Phe Thr Leu Tyr Lys
    50                  55                  60

Lys Asp Gly Val Pro Val Pro Glu Leu Tyr Asn Arg Ile Phe Trp Asn
65                  70                  75                  80

Ser Phe Leu Ile Ser Pro Val Thr Pro Ala His Ala Gly Thr Tyr Arg
                85                  90                  95

Cys Arg Gly Phe His Pro His Ser Pro Thr Glu Trp Ser Ala Pro Ser
            100                 105                 110

Asn Pro Leu Val Ile Met Val Thr Gly Leu Tyr Glu Lys Pro Ser Leu
        115                 120                 125

Thr Ala Arg Pro Gly Pro Thr Val Arg Ala Gly Glu Asn Val Thr Leu
    130                 135                 140

Ser Cys Ser Ser Gln Ser Ser Phe Asp Ile Tyr His Leu Ser Arg Glu
145                 150                 155                 160

Gly Glu Ala His Glu Leu Arg Leu Pro Ala Val Pro Ser Ile Asn Gly
                165                 170                 175

Thr Phe Gln Ala Asp Phe Pro Leu Gly Pro Ala Thr His Gly Glu Thr
            180                 185                 190

Tyr Arg Cys Phe Gly Ser Phe His Gly Ser Pro Tyr Glu Trp Ser Asp
        195                 200                 205

Pro Ser Asp Pro Leu Pro Val Ser Val Thr Gly Asn Pro Ser Ser Ser
    210                 215                 220

Trp Pro Ser Pro Thr Glu Pro Ser Phe Lys Thr Gly Ile Ala Arg His
225                 230                 235                 240

Leu His Ala Val Ile Arg Tyr Ser Val Ala Ile Ile Leu Phe Thr Ile
                245                 250                 255

Leu Pro Phe Phe Leu Leu His Arg Trp Cys Ser Lys Lys Lys Asn Ala
            260                 265                 270

Ala Val Met Asn Gln Glu Pro Ala Gly His Arg Thr Val Asn Arg Glu
        275                 280                 285

Asp Ser Asp Glu Gln Asp Pro Gln Glu Val Thr Tyr Ala Gln Leu Asp
    290                 295                 300

His Cys Ile Phe Thr Gln Arg Lys Ile Thr Gly Pro Ser Gln Arg Ser
305                 310                 315                 320

Lys Arg Pro Ser Thr Asp Thr Ser Val Cys Ile Glu Leu Pro Asn Ala
                325                 330                 335

Glu Pro Arg Ala Leu Ser Pro Ala His Glu His His Ser Gln Ala Leu
            340                 345                 350

Met Gly Ser Ser Arg Glu Thr Thr Ala Leu Ser Gln Thr Gln Leu Ala
        355                 360                 365

Ser Ser Asn Val Pro Ala Ala Gly Ile
    370                 375

<210>  103
<211>  304
<212>  PRT
<213>  Homo sapiens

<400>  103

Met Ser Leu Met Val Ile Ser Met Ala Cys Val Ala Phe Phe Leu Leu
1               5                   10                  15

Gln Gly Ala Trp Pro His Glu Gly Phe Arg Arg Lys Pro Ser Leu Leu
            20                  25                  30

Ala His Pro Gly Pro Leu Val Lys Ser Glu Glu Thr Val Ile Leu Gln
        35                  40                  45

Cys Trp Ser Asp Val Met Phe Glu His Phe Leu Leu His Arg Glu Gly
    50                  55                  60

Thr Phe Asn His Thr Leu Arg Leu Ile Gly Glu His Ile Asp Gly Val
65                  70                  75                  80

Ser Lys Gly Asn Phe Ser Ile Gly Arg Met Thr Gln Asp Leu Ala Gly
                85                  90                  95

Thr Tyr Arg Cys Tyr Gly Ser Val Thr His Ser Pro Tyr Gln Leu Ser
            100                 105                 110

Ala Pro Ser Asp Pro Leu Asp Ile Val Ile Thr Gly Leu Tyr Glu Lys

115                 120                 125

Pro Ser Leu Ser Ala Gln Pro Gly Pro Thr Val Leu Ala Gly Glu Ser
    130                 135                 140

Val Thr Leu Ser Cys Ser Ser Arg Ser Ser Tyr Asp Met Tyr His Leu
145                 150                 155                 160

Ser Arg Glu Gly Glu Ala His Glu Arg Arg Leu Pro Ala Gly Pro Lys
                165                 170                 175

Val Asn Arg Thr Phe Gln Ala Asp Phe Pro Leu Asp Pro Ala Thr His
            180                 185                 190

Gly Gly Thr Tyr Arg Cys Phe Gly Ser Phe Arg Asp Ser Pro Tyr Glu
        195                 200                 205

Trp Ser Lys Ser Ser Asp Pro Leu Leu Val Ser Val Thr Gly Asn Ser
    210                 215                 220

Ser Asn Ser Trp Pro Ser Pro Thr Glu Pro Ser Ser Glu Thr Gly Asn
225                 230                 235                 240

Pro Arg His Leu His Val Leu Ile Gly Thr Ser Val Val Lys Leu Pro
                245                 250                 255

Phe Thr Ile Leu Leu Phe Phe Leu Leu His Arg Trp Cys Ser Asn Lys
            260                 265                 270

Lys Asn Ala Ser Val Met Asp Gln Gly Pro Ala Gly Asn Arg Thr Val
        275                 280                 285

Asn Arg Glu Asp Ser Asp Glu Gln Asp His Gln Glu Val Ser Tyr Ala
    290                 295                 300

<210>  104
<211>  233
<212>  PRT
<213>  Homo sapiens

<400>  104

Gly Gly Gln Asp Lys Pro Leu Leu Ser Thr Trp Pro Ser Leu Val Val
1               5                   10                  15

Pro Pro Glu His Val Thr Leu Arg Cys His Ser Asn Leu Gly Phe Asn
            20                  25                  30

Asn Phe Ser Leu Tyr Lys Asp Asp Gly Val Pro Val Pro Glu Leu Tyr

35                  40                  45

Asn Arg Ile Phe Trp Lys Ser Leu Phe Met Gly Pro Val Thr Pro Ser
    50                  55                  60

His Thr Gly Thr Tyr Arg Cys Arg Gly Ser His Thr His Ser Pro Ser
65                  70                  75                  80

Gly Gly Ser Ala Pro Ser Asn Pro Leu Val Ile Val Val Thr Gly Phe
                85                  90                  95

Arg Arg Lys Pro Ser Leu Leu Ala His Pro Gly Arg Leu Val Lys Ser
            100                 105                 110

Glu Glu Thr Val Ile Leu Gln Cys Trp Ser Asp Val Met Phe Glu His
        115                 120                 125

Phe Leu Leu His Arg Glu Gly Thr Phe Asn Asp Thr Leu Arg Leu Ile
    130                 135                 140

Gly Glu His Ile Asp Gly Val Ser Lys Ala Asn Phe Ser Ile Gly Arg
145                 150                 155                 160

Met Arg Gln Asp Leu Ala Gly Thr Tyr Arg Cys Tyr Gly Ser Val Pro
                165                 170                 175

His Ser Pro Tyr Gln Phe Ser Ala Pro Ser Asp Pro Leu Asp Ile Val
            180                 185                 190

Ile Thr Gly Glu Ser Val Gln Thr Phe Phe Ser Leu Ser Phe Gly His
        195                 200                 205

Arg Val Asn Asp Pro Gly Leu Gly Gly Pro Gly Gly Cys Lys Glu Asp
    210                 215                 220

Glu Leu Gly Ile Leu Met Glu Arg Asp
225                 230

<210>  105
<211>  375
<212>  PRT
<213>  Homo sapiens

<400>  105

Met Ser Leu Met Val Ile Ser Met Ala Cys Val Gly Phe Phe Leu Leu
1               5                   10                  15

Gln Gly Ala Trp Thr His Glu Gly Gly Gln Asp Lys Pro Leu Leu Ser

20                  25                  30

Ala Trp Pro Ser Ala Val Val Pro Arg Gly Gly His Val Thr Leu Leu
        35                  40                  45

Cys Arg Ser Arg Leu Gly Phe Thr Ile Phe Ser Leu Tyr Lys Glu Asp
    50                  55                  60

Gly Val Pro Val Pro Glu Leu Tyr Asn Lys Ile Phe Trp Lys Ser Ile
65                  70                  75                  80

Leu Met Gly Pro Val Thr Pro Ala His Ala Gly Thr Tyr Arg Cys Arg
                85                  90                  95

Gly Ser His Pro Arg Ser Pro Ile Glu Trp Ser Ala Pro Ser Asn Pro
            100                 105                 110

Leu Val Ile Val Val Thr Gly Leu Phe Gly Lys Pro Ser Leu Ser Ala
        115                 120                 125

Gln Pro Gly Pro Thr Val Arg Thr Gly Glu Asn Val Thr Leu Ser Cys
    130                 135                 140

Ser Ser Arg Ser Ser Phe Asp Met Tyr His Leu Ser Arg Glu Gly Arg
145                 150                 155                 160

Ala His Glu Pro Arg Leu Pro Ala Val Pro Ser Val Asn Gly Thr Phe
                165                 170                 175

Gln Ala Asp Phe Pro Leu Gly Pro Ala Thr His Gly Gly Thr Tyr Thr
            180                 185                 190

Cys Phe Gly Ser Leu His Asp Ser Pro Tyr Glu Trp Ser Asp Pro Ser
        195                 200                 205

Asp Pro Leu Leu Val Ser Val Thr Gly Asn Ser Ser Ser Ser Ser Ser
    210                 215                 220

Ser Pro Thr Glu Pro Ser Ser Lys Thr Gly Ile Arg Arg His Leu His
225                 230                 235                 240

Ile Leu Ile Gly Thr Ser Val Ala Ile Ile Leu Phe Ile Ile Leu Phe
                245                 250                 255

Phe Phe Leu Leu His Cys Cys Cys Ser Asn Lys Lys Asn Ala Ala Val
            260                 265                 270

Met Asp Gln Glu Pro Ala Gly Asp Arg Thr Val Asn Arg Glu Asp Ser
        275                 280                 285

Asp Asp Gln Asp Pro Gln Glu Val Thr Tyr Ala Gln Leu Asp His Cys
    290                 295                 300

Val Phe Thr Gln Thr Lys Ile Thr Ser Pro Ser Gln Arg Pro Lys Thr
305                 310                 315                 320

Pro Pro Thr Asp Thr Thr Met Tyr Met Glu Leu Pro Asn Ala Lys Pro
                325                 330                 335

Arg Ser Leu Ser Pro Ala His Lys His His Ser Gln Ala Leu Arg Gly
            340                 345                 350

Ser Ser Arg Glu Thr Thr Ala Leu Ser Gln Asn Arg Val Ala Ser Ser
        355                 360                 365

His Val Pro Ala Ala Gly Ile
    370                 375

<210>  106
<211>  80
<212>  PRT
<213>  Homo sapiens

<400>  106

Met Gly Arg Ala Met Val Ala Arg Leu Gly Leu Gly Leu Leu Leu Leu
1               5                   10                  15

Ala Leu Leu Leu Pro Thr Gln Ile Tyr Ser Ser Glu Thr Thr Thr Gly
            20                  25                  30

Thr Ser Ser Asn Ser Ser Gln Ser Thr Ser Asn Ser Gly Leu Ala Pro
        35                  40                  45

Asn Pro Thr Asn Ala Thr Thr Lys Ala Ala Gly Gly Ala Leu Gln Ser
    50                  55                  60

Thr Ala Ser Leu Phe Val Val Ser Leu Ser Leu Leu His Leu Tyr Ser
65                  70                  75                  80

<210>  107
<211>  362
<212>  PRT
<213>  Homo sapiens

<400>  107
   Met Leu Val Met Ala Pro Arg Thr Val Leu Leu Leu Leu Ser Ala Ala
   1               5                   10                  15
   Leu Ala Leu Thr Glu Thr Trp Ala Gly Ser His Ser Met Arg Tyr Phe
               20                  25                  30
   Tyr Thr Ser Val Ser Arg Pro Gly Arg Gly Glu Pro Arg Phe Ile Ser
           35                  40                  45
   Val Gly Tyr Val Asp Asp Thr Gln Phe Val Arg Phe Asp Ser Asp Ala
       50                  55                  60
   Ala Ser Pro Arg Glu Glu Pro Arg Ala Pro Trp Ile Glu Gln Glu Gly
   65                  70                  75                  80
   Pro Glu Tyr Trp Asp Arg Asn Thr Gln Ile Tyr Lys Ala Gln Ala Gln
                   85                  90                  95
   Thr Asp Arg Glu Ser Leu Arg Asn Leu Arg Gly Tyr Tyr Asn Gln Ser
               100                 105                 110
   Glu Ala Gly Ser His Thr Leu Gln Ser Met Tyr Gly Cys Asp Val Gly
           115                 120                 125
   Pro Asp Gly Arg Leu Leu Arg Gly His Asp Gln Tyr Ala Tyr Asp Gly
       130                 135                 140
   Lys Asp Tyr Ile Ala Leu Asn Glu Asp Leu Arg Ser Trp Thr Ala Ala
   145                 150                 155                 160
   Asp Thr Ala Ala Gln Ile Thr Gln Arg Lys Trp Glu Ala Ala Arg Glu
                   165                 170                 175
   Ala Glu Gln Arg Arg Ala Tyr Leu Glu Gly Glu Cys Val Glu Trp Leu
               180                 185                 190
   Arg Arg Tyr Leu Glu Asn Gly Lys Asp Lys Leu Glu Arg Ala Asp Pro
           195                 200                 205
   Pro Lys Thr His Val Thr His His Pro Ile Ser Asp His Glu Ala Thr
       210                 215                 220
   Leu Arg Cys Trp Ala Leu Gly Phe Tyr Pro Ala Glu Ile Thr Leu Thr
   225                 230                 235                 240
   Trp Gln Arg Asp Gly Glu Asp Gln Thr Gln Asp Thr Glu Leu Val Glu
                   245                 250                 255
   Thr Arg Pro Ala Gly Asp Arg Thr Phe Gln Lys Trp Ala Ala Val Val
               260                 265                 270
   Val Pro Ser Gly Glu Glu Gln Arg Tyr Thr Cys His Val Gln His Glu
           275                 280                 285
   Gly Leu Pro Lys Pro Leu Thr Leu Arg Trp Glu Pro Ser Ser Gln Ser
       290                 295                 300
   Thr Val Pro Ile Val Gly Ile Val Ala Gly Leu Ala Val Leu Ala Val
   305                 310                 315                 320
   Val Val Ile Gly Ala Val Val Ala Ala Val Met Cys Arg Arg Lys Ser
                   325                 330                 335
   Ser Gly Gly Lys Gly Gly Ser Tyr Ser Gln Ala Ala Cys Ser Asp Ser
               340                 345                 350
   Ala Gln Gly Ser Asp Val Ser Leu Thr Ala
           355                 360

<210>  108
<211>  366
<212>  PRT
<213>  Homo sapiens

<400>  108

Met Arg Val Met Ala Pro Arg Ala Leu Leu Leu Leu Leu Ser Gly Gly
1               5                   10                  15

Leu Ala Leu Thr Glu Thr Trp Ala Cys Ser His Ser Met Arg Tyr Phe
            20                  25                  30

Asp Thr Ala Val Ser Arg Pro Gly Arg Gly Glu Pro Arg Phe Ile Ser
        35                  40                  45

Val Gly Tyr Val Asp Asp Thr Gln Phe Val Arg Phe Asp Ser Asp Ala
    50                  55                  60

Ala Ser Pro Arg Gly Glu Pro Arg Ala Pro Trp Val Glu Gln Glu Gly
65                  70                  75                  80

Pro Glu Tyr Trp Asp Arg Glu Thr Gln Lys Tyr Lys Arg Gln Ala Gln
                85                  90                  95

Ala Asp Arg Val Ser Leu Arg Asn Leu Arg Gly Tyr Tyr Asn Gln Ser
            100                 105                 110

Glu Asp Gly Ser His Thr Leu Gln Arg Met Ser Gly Cys Asp Leu Gly
        115                 120                 125

Pro Asp Gly Arg Leu Leu Arg Gly Tyr Asp Gln Ser Ala Tyr Asp Gly
    130                 135                 140

Lys Asp Tyr Ile Ala Leu Asn Glu Asp Leu Arg Ser Trp Thr Ala Ala
145                 150                 155                 160

Asp Thr Ala Ala Gln Ile Thr Gln Arg Lys Leu Glu Ala Ala Arg Ala
                165                 170                 175

Ala Glu Gln Leu Arg Ala Tyr Leu Glu Gly Thr Cys Val Glu Trp Leu
            180                 185                 190

Arg Arg Tyr Leu Glu Asn Gly Lys Glu Thr Leu Gln Arg Ala Glu Pro
        195                 200                 205

Pro Lys Thr His Val Thr His His Pro Leu Ser Asp His Glu Ala Thr
    210                 215                 220

Leu Arg Cys Trp Ala Leu Gly Phe Tyr Pro Ala Glu Ile Thr Leu Thr
225                 230                 235                 240

Trp Gln Arg Asp Gly Glu Asp Gln Thr Gln Asp Thr Glu Leu Val Glu
                245                 250                 255

Thr Arg Pro Ala Gly Asp Gly Thr Phe Gln Lys Trp Ala Ala Val Val
            260                 265                 270

Val Pro Ser Gly Gln Glu Gln Arg Tyr Thr Cys His Met Gln His Glu
        275                 280                 285

Gly Leu Gln Glu Pro Leu Thr Leu Ser Trp Glu Pro Ser Ser Gln Pro
    290                 295                 300

Thr Ile Pro Ile Met Gly Ile Val Ala Gly Leu Ala Val Leu Val Val
305                 310                 315                 320

Leu Ala Val Leu Gly Ala Val Val Thr Ala Met Met Cys Arg Arg Lys
                325                 330                 335

Ser Ser Gly Gly Lys Gly Gly Ser Cys Ser Gln Ala Ala Cys Ser Asn
            340                 345                 350

Ser Ala Gln Gly Ser Asp Glu Ser Leu Ile Thr Cys Lys Ala

355                 360                 365

<210>  109
<211>  189
<212>  PRT
<213>  Homo sapiens

<400>  109

Met Thr Asp Ser Val Ile Tyr Ser Met Leu Glu Leu Pro Thr Ala Thr
1               5                   10                  15

Gln Ala Gln Asn Asp Tyr Gly Pro Gln Gln Lys Ser Ser Ser Ser Arg
            20                  25                  30

Pro Ser Cys Ser Cys Leu Val Ala Ile Ala Leu Gly Leu Leu Thr Ala
        35                  40                  45

Val Leu Leu Ser Val Leu Leu Tyr Gln Trp Ile Leu Cys Gln Gly Ser
    50                  55                  60

Asn Tyr Ser Thr Cys Ala Ser Cys Pro Ser Cys Pro Asp Arg Trp Met
65                  70                  75                  80

Lys Tyr Gly Asn His Cys Tyr Tyr Phe Ser Val Glu Glu Lys Asp Trp
                85                  90                  95

Asn Ser Ser Leu Glu Phe Cys Leu Ala Arg Asp Ser His Leu Leu Val
            100                 105                 110

Ile Thr Asp Asn Gln Glu Met Ser Leu Leu Gln Val Phe Leu Ser Glu
        115                 120                 125

Ala Phe Cys Trp Ile Gly Leu Arg Asn Asn Ser Gly Trp Arg Trp Glu
    130                 135                 140

Asp Gly Ser Pro Leu Asn Phe Ser Arg Ile Ser Ser Asn Ser Phe Val
145                 150                 155                 160

Gln Thr Cys Gly Ala Ile Asn Lys Asn Gly Leu Gln Ala Ser Ser Cys
                165                 170                 175

Glu Val Pro Leu His Trp Val Cys Lys Lys Val Arg Leu
            180                 185

<210>  110
<211>  338
<212>  PRT
<213>  Homo sapiens

<400>  110

Met Val Val Met Ala Pro Arg Thr Leu Phe Leu Leu Leu Ser Gly Ala
1               5                   10                  15

Leu Thr Leu Thr Glu Thr Trp Ala Gly Ser His Ser Met Arg Tyr Phe
            20                  25                  30

Ser Ala Ala Val Ser Arg Pro Gly Arg Gly Glu Pro Arg Phe Ile Ala
        35                  40                  45

Met Gly Tyr Val Asp Asp Thr Gln Phe Val Arg Phe Asp Ser Asp Ser
    50                  55                  60

Ala Cys Pro Arg Met Glu Pro Arg Ala Pro Trp Val Glu Gln Glu Gly
65                  70                  75                  80

Pro Glu Tyr Trp Glu Glu Glu Thr Arg Asn Thr Lys Ala His Ala Gln
                85                  90                  95

Thr Asp Arg Met Asn Leu Gln Thr Leu Arg Gly Tyr Tyr Asn Gln Ser
            100                 105                 110

Glu Ala Ser Ser His Thr Leu Gln Trp Met Ile Gly Cys Asp Leu Gly
        115                 120                 125

Ser Asp Gly Arg Leu Leu Arg Gly Tyr Glu Gln Tyr Ala Tyr Asp Gly
    130                 135                 140

Lys Asp Tyr Leu Ala Leu Asn Glu Asp Leu Arg Ser Trp Thr Ala Ala
145                 150                 155                 160

Asp Thr Ala Ala Gln Ile Ser Lys Arg Lys Cys Glu Ala Ala Asn Val
                165                 170                 175

Ala Glu Gln Arg Arg Ala Tyr Leu Glu Gly Thr Cys Val Glu Trp Leu
            180                 185                 190

His Arg Tyr Leu Glu Asn Gly Lys Glu Met Leu Gln Arg Ala Asp Pro
        195                 200                 205

Pro Lys Thr His Val Thr His His Pro Val Phe Asp Tyr Glu Ala Thr
    210                 215                 220

Leu Arg Cys Trp Ala Leu Gly Phe Tyr Pro Ala Glu Ile Ile Leu Thr
225                 230                 235                 240

Trp Gln Arg Asp Gly Glu Asp Gln Thr Gln Asp Val Glu Leu Val Glu

245                 250                 255

Thr Arg Pro Ala Gly Asp Gly Thr Phe Gln Lys Trp Ala Ala Val Val
            260                 265                 270

Val Pro Ser Gly Glu Glu Gln Arg Tyr Thr Cys His Val Gln His Glu
        275                 280                 285

Gly Leu Pro Glu Pro Leu Met Leu Arg Trp Lys Gln Ser Ser Leu Pro
    290                 295                 300

Thr Ile Pro Ile Met Gly Ile Val Ala Gly Leu Val Val Leu Ala Ala
305                 310                 315                 320

Val Val Thr Gly Ala Ala Val Ala Ala Val Leu Trp Arg Lys Lys Ser
                325                 330                 335

Ser Asp

<210>  111
<211>  382
<212>  PRT
<213>  Homo sapiens

<400>  111

Met Leu Leu Met Val Val Ser Met Ala Cys Val Gly Leu Phe Leu Val
1               5                   10                  15

Gln Arg Ala Gly Pro His Met Gly Gly Gln Asp Lys Pro Phe Leu Ser
            20                  25                  30

Ala Trp Pro Ser Ala Val Val Pro Arg Gly Gly His Val Thr Leu Arg
        35                  40                  45

Cys His Tyr Arg His Arg Phe Asn Asn Phe Met Leu Tyr Lys Glu Asp
    50                  55                  60

Arg Ile His Val Pro Ile Phe His Gly Arg Ile Phe Gln Glu Gly Phe
65                  70                  75                  80

Asn Met Ser Pro Val Thr Thr Ala His Ala Gly Asn Tyr Thr Cys Arg
                85                  90                  95

Gly Ser His Pro His Ser Pro Thr Gly Trp Ser Ala Pro Ser Asn Pro
            100                 105                 110

Met Val Ile Met Val Thr Gly Asn His Arg Lys Pro Ser Leu Leu Ala
        115                 120                 125

His Pro Gly Pro Leu Val Lys Ser Gly Glu Arg Val Ile Leu Gln Cys
    130                 135                 140

Trp Ser Asp Ile Met Phe Glu His Phe Phe Leu His Lys Glu Trp Ile
145                 150                 155                 160

Ser Lys Asp Pro Ser Arg Leu Val Gly Gln Ile His Asp Gly Val Ser
                165                 170                 175

Lys Ala Asn Phe Ser Ile Gly Ser Met Met Arg Ala Leu Ala Gly Thr
            180                 185                 190

Tyr Arg Cys Tyr Gly Ser Val Thr His Thr Pro Tyr Gln Leu Ser Ala
        195                 200                 205

Pro Ser Asp Pro Leu Asp Ile Val Val Thr Gly Leu Tyr Glu Lys Pro
    210                 215                 220

Ser Leu Ser Ala Gln Pro Gly Pro Lys Val Gln Ala Gly Glu Ser Val
225                 230                 235                 240

Thr Leu Ser Cys Ser Ser Arg Ser Ser Tyr Asp Met Tyr His Leu Ser
                245                 250                 255

Arg Glu Gly Gly Ala His Glu Arg Arg Leu Pro Ala Val Arg Lys Val
            260                 265                 270

Asn Arg Thr Phe Gln Ala Asp Phe Pro Leu Gly Pro Ala Thr His Gly
        275                 280                 285

Gly Thr Tyr Arg Cys Phe Gly Ser Phe Arg His Ser Pro Tyr Glu Trp
    290                 295                 300

Ser Asp Pro Ser Asp Pro Leu Leu Val Ser Val Thr Gly Asn Pro Ser
305                 310                 315                 320

Ser Ser Trp Pro Ser Pro Thr Glu Pro Ser Ser Lys Ser Gly Asn Leu
                325                 330                 335

Arg His Leu His Ile Leu Ile Gly Thr Ser Val Val Lys Ile Pro Phe
            340                 345                 350

Thr Ile Leu Leu Phe Phe Leu Leu His Arg Trp Cys Ser Asn Lys Lys
        355                 360                 365

Lys Cys Cys Cys Asn Gly Pro Arg Ala Cys Arg Glu Gln Lys
    370                 375                 380

<210>  112
<211>  365
<212>  PRT
<213>  Homo sapiens

<400>  112

Met Ala Val Met Ala Pro Arg Thr Leu Leu Leu Leu Leu Ser Gly Ala
1               5                   10                  15

Leu Ala Leu Thr Gln Thr Trp Ala Gly Ser His Ser Met Arg Tyr Phe
            20                  25                  30

Phe Thr Ser Val Ser Arg Pro Gly Arg Gly Glu Pro Arg Phe Ile Ala
        35                  40                  45

Val Gly Tyr Val Asp Asp Thr Gln Phe Val Arg Phe Asp Ser Asp Ala
    50                  55                  60

Ala Ser Gln Arg Met Glu Pro Arg Ala Pro Trp Ile Glu Gln Glu Gly
65                  70                  75                  80

Pro Glu Tyr Trp Asp Gln Glu Thr Arg Asn Val Lys Ala Gln Ser Gln
                85                  90                  95

Thr Asp Arg Val Asp Leu Gly Thr Leu Arg Gly Tyr Tyr Asn Gln Ser
            100                 105                 110

Glu Ala Gly Ser His Thr Ile Gln Ile Met Tyr Gly Cys Asp Val Gly
        115                 120                 125

Ser Asp Gly Arg Phe Leu Arg Gly Tyr Arg Gln Asp Ala Tyr Asp Gly
    130                 135                 140

Lys Asp Tyr Ile Ala Leu Asn Glu Asp Leu Arg Ser Trp Thr Ala Ala
145                 150                 155                 160

Asp Met Ala Ala Gln Ile Thr Lys Arg Lys Trp Glu Ala Ala His Glu
                165                 170                 175

Ala Glu Gln Leu Arg Ala Tyr Leu Asp Gly Thr Cys Val Glu Trp Leu
            180                 185                 190

Arg Arg Tyr Leu Glu Asn Gly Lys Glu Thr Leu Gln Arg Thr Asp Pro
        195                 200                 205

Pro Lys Thr His Met Thr His His Pro Ile Ser Asp His Glu Ala Thr
    210                 215                 220

Leu Arg Cys Trp Ala Leu Gly Phe Tyr Pro Ala Glu Ile Thr Leu Thr
225                 230                 235                 240

Trp Gln Arg Asp Gly Glu Asp Gln Thr Gln Asp Thr Glu Leu Val Glu
                245                 250                 255

Thr Arg Pro Ala Gly Asp Gly Thr Phe Gln Lys Trp Ala Ala Val Val
            260                 265                 270

Val Pro Ser Gly Glu Glu Gln Arg Tyr Thr Cys His Val Gln His Glu
        275                 280                 285

Gly Leu Pro Lys Pro Leu Thr Leu Arg Trp Glu Leu Ser Ser Gln Pro
    290                 295                 300

Thr Ile Pro Ile Val Gly Ile Ile Ala Gly Leu Val Leu Leu Gly Ala
305                 310                 315                 320

Val Ile Thr Gly Ala Val Val Ala Ala Val Met Trp Arg Arg Lys Ser
                325                 330                 335

Ser Asp Arg Lys Gly Gly Ser Tyr Thr Gln Ala Ala Ser Ser Asp Ser
            340                 345                 350

Ala Gln Gly Ser Asp Val Ser Leu Thr Ala Cys Lys Val
        355                 360                 365

<210>  113
<211>  410
<212>  PRT
<213>  Homo sapiens

<400>  113

Met Ser Leu Met Val Val Ser Met Ala Cys Val Gly Phe Phe Leu Leu
1               5                   10                  15

Glu Gly Pro Trp Pro His Val Gly Gly Gln Asp Lys Pro Phe Leu Ser
            20                  25                  30

Ala Trp Pro Gly Thr Val Val Ser Glu Gly Gln His Val Thr Leu Gln
        35                  40                  45

Cys Arg Ser Arg Leu Gly Phe Asn Glu Phe Ser Leu Ser Lys Glu Asp
    50                  55                  60

Gly Met Pro Val Pro Glu Leu Tyr Asn Arg Ile Phe Arg Asn Ser Phe
65                  70                  75                  80

Leu Met Gly Pro Val Thr Pro Ala His Ala Gly Thr Tyr Arg Cys Cys
                85                  90                  95

Ser Ser His Pro His Ser Pro Thr Gly Trp Ser Ala Pro Ser Asn Pro
            100                 105                 110

Val Val Ile Met Val Thr Gly Val His Arg Lys Pro Ser Leu Leu Ala
        115                 120                 125

His Pro Gly Pro Leu Val Lys Ser Gly Glu Thr Val Ile Leu Gln Cys
    130                 135                 140

Trp Ser Asp Val Arg Phe Glu Arg Phe Leu Leu His Arg Glu Gly Ile
145                 150                 155                 160

Thr Glu Asp Pro Leu Arg Leu Val Gly Gln Leu His Asp Ala Gly Ser
                165                 170                 175

Gln Val Asn Tyr Ser Met Gly Pro Met Thr Pro Ala Leu Ala Gly Thr
            180                 185                 190

Tyr Arg Cys Phe Gly Ser Val Thr His Leu Pro Tyr Glu Leu Ser Ala
        195                 200                 205

Pro Ser Asp Pro Leu Asp Ile Val Val Val Gly Leu Tyr Gly Lys Pro
    210                 215                 220

Ser Leu Ser Ala Gln Pro Gly Pro Thr Val Gln Ala Gly Glu Asn Val
225                 230                 235                 240

Thr Leu Ser Cys Ser Ser Arg Ser Leu Phe Asp Ile Tyr His Leu Ser
                245                 250                 255

Arg Glu Ala Glu Ala Gly Glu Leu Arg Leu Thr Ala Val Leu Arg Val
            260                 265                 270

Asn Gly Thr Phe Gln Ala Asn Phe Pro Leu Gly Pro Val Thr His Gly
        275                 280                 285

Gly Asn Tyr Arg Cys Phe Gly Ser Phe Arg Ala Leu Pro His Ala Trp
    290                 295                 300

Ser Asp Pro Ser Asp Pro Leu Pro Val Ser Val Thr Gly Asn Ser Arg

305                 310                 315                 320

His Leu His Val Leu Ile Gly Thr Ser Val Val Ile Ile Pro Phe Ala
                325                 330                 335

Ile Leu Leu Phe Phe Leu Leu His Arg Trp Cys Ala Asn Lys Lys Asn
            340                 345                 350

Ala Val Val Met Asp Gln Glu Pro Ala Gly Asn Arg Thr Val Asn Arg
        355                 360                 365

Glu Asp Ser Asp Glu Gln Asp Pro Gln Glu Val Thr Tyr Ala Gln Leu
    370                 375                 380

Asn His Cys Val Phe Thr Gln Arg Lys Ile Thr Arg Pro Ser Gln Arg
385                 390                 395                 400

Pro Lys Thr Pro Pro Thr Asp Thr Ser Val
                405                 410


<210>  114
<211>  328
<212>  PRT
<213>  Homo sapiens

<400>  114

Met Ser Leu Met Val Ile Ser Met Ala Cys Val Gly Phe Phe Leu Leu
1               5                   10                  15

Gln Gly Ala Trp Thr His Glu Gly Gly Gln Asp Lys Pro Phe Leu Ser
            20                  25                  30

Ala Trp Pro Ser Pro Val Val Ser Glu Gly Glu His Val Ala Leu Gln
        35                  40                  45

Cys Arg Ser Arg Leu Gly Phe Asn Glu Phe Ser Leu Ser Lys Glu Asp
    50                  55                  60

Gly Met Pro Val Pro Glu Leu Tyr Asn Arg Val Phe Arg Asn Thr Val
65                  70                  75                  80

Phe Ile Gly Pro Val Thr Pro Ala His Ala Gly Thr Tyr Arg Cys Arg
                85                  90                  95

Gly Ser His Pro His Phe Leu Thr Gly Trp Ser Ala Pro Ser Asn Pro
            100                 105                 110

Leu Val Ile Met Val Thr Gly Val His Arg Lys Pro Ser Leu Leu Ala

115                 120                 125

His Pro Gly Pro Leu Val Lys Ser Glu Glu Thr Val Ile Leu Gln Cys
    130                 135                 140

Trp Ser Asp Val Met Phe Glu His Phe Leu Leu His Arg Glu Gly Lys
145                 150                 155                 160

Phe Asn Asp Thr Leu Arg Leu Thr Gly Glu Leu His Asp Gly Val Ser
                165                 170                 175

Lys Ala Asn Phe Ser Ile Gly Arg Met Thr Gln Asp Leu Ala Gly Thr
            180                 185                 190

Tyr Arg Cys Tyr Gly Ser Val Pro His Ser Pro Tyr Gln Leu Ser Ala
        195                 200                 205

Pro Ser Asp Pro Leu Asp Ile Val Ile Thr Gly Leu Cys Gly Lys Pro
    210                 215                 220

Ser Leu Ser Ala Gln Pro Arg Pro Met Val Lys Ala Gly Glu Ser Val
225                 230                 235                 240

Thr Leu Ser Cys Ser Ser Arg Ser Ser Tyr Asp Ile Tyr His Leu Ser
                245                 250                 255

Arg Glu Gly Glu Ala His Glu Leu Arg Phe Pro Ala Val Pro Lys Val
            260                 265                 270

Asn Gly Thr Phe Gln Ala Asn Phe Pro Leu Gly Pro Ala Thr His Gly
        275                 280                 285

Gly Thr Tyr Arg Cys Phe Gly Ser Phe Arg Asp Ser Pro Tyr Glu Trp
    290                 295                 300

Ser Asp Leu Ser Asp Pro Leu Leu Val Ser Val Thr Asp Ser Met Lys
305                 310                 315                 320

Glu Lys Gly Lys Asp Val Ile Leu
                325

<210>  115
<211>  304
<212>  PRT
<213>  Homo sapiens

<400>  115

Met Ser Leu Met Val Val Ser Met Ala Cys Val Gly Phe Phe Leu Leu

1               5                   10                  15

Gln Gly Ala Trp Pro His Glu Gly Val His Arg Lys Pro Ser Leu Leu
            20                  25                  30

Ala His Pro Gly Pro Leu Val Lys Ser Glu Glu Thr Val Ile Leu Gln
        35                  40                  45

Cys Trp Ser Asp Val Arg Phe Glu His Phe Leu Leu His Arg Glu Gly
    50                  55                  60

Lys Tyr Lys Asp Thr Leu His Leu Ile Gly Glu His His Asp Gly Val
65                  70                  75                  80

Ser Lys Ala Asn Phe Ser Ile Gly Pro Met Met Gln Asp Leu Ala Gly
                85                  90                  95

Thr Tyr Arg Cys Tyr Gly Ser Val Thr His Ser Pro Tyr Gln Leu Ser
            100                 105                 110

Ala Pro Ser Asp Pro Leu Asp Ile Val Ile Thr Gly Leu Tyr Glu Lys
        115                 120                 125

Pro Ser Leu Ser Ala Gln Pro Gly Pro Thr Val Leu Ala Gly Glu Ser
    130                 135                 140

Val Thr Leu Ser Cys Ser Ser Arg Ser Ser Tyr Asp Met Tyr His Leu
145                 150                 155                 160

Ser Arg Glu Gly Glu Ala His Glu Arg Arg Phe Ser Ala Gly Pro Lys
                165                 170                 175

Val Asn Gly Thr Phe Gln Ala Asp Phe Pro Leu Gly Pro Ala Thr His
            180                 185                 190

Gly Gly Thr Tyr Arg Cys Phe Gly Ser Phe Arg Asp Ser Pro Tyr Glu
        195                 200                 205

Trp Ser Asn Ser Ser Asp Pro Leu Leu Val Ser Val Thr Gly Asn Pro
    210                 215                 220

Ser Asn Ser Trp Pro Ser Pro Thr Glu Pro Ser Ser Lys Thr Gly Asn
225                 230                 235                 240

Pro Arg His Leu His Val Leu Ile Gly Thr Ser Val Val Lys Ile Pro
                245                 250                 255

Phe Thr Ile Leu Leu Phe Phe Leu Leu His Arg Trp Cys Ser Asn Lys
            260                 265                 270

Lys Asn Ala Ala Val Met Asp Gln Glu Pro Ala Gly Asn Arg Thr Val
        275                 280                 285

Asn Ser Glu Asp Ser Asp Glu Gln Asp His Gln Glu Val Ser Tyr Ala
    290                 295                 300

<210> 116
<211> 304
<212> PRT
<213> Homo sapiens

<400> 116

Met Ser Leu Met Val Ile Ser Met Ala Cys Val Gly Phe Phe Trp Leu
1               5                   10                  15

Gln Gly Ala Trp Pro His Glu Gly Phe Arg Arg Lys Pro Ser Leu Leu
            20                  25                  30

Ala His Pro Gly Arg Leu Val Lys Ser Glu Glu Thr Val Ile Leu Gln
        35                  40                  45

Cys Trp Ser Asp Val Met Phe Glu His Phe Leu Leu His Arg Glu Gly
    50                  55                  60

Thr Phe Asn Asp Thr Leu Arg Leu Ile Gly Glu His Ile Asp Gly Val
65                  70                  75                  80

Ser Lys Ala Asn Phe Ser Ile Gly Arg Met Arg Gln Asp Leu Ala Gly
                85                  90                  95

Thr Tyr Arg Cys Tyr Gly Ser Val Pro His Ser Pro Tyr Gln Phe Ser
            100                 105                 110

Ala Pro Ser Asp Pro Leu Asp Ile Val Ile Thr Gly Leu Tyr Glu Lys
        115                 120                 125

Pro Ser Leu Ser Ala Gln Pro Gly Pro Thr Val Leu Ala Gly Glu Ser
    130                 135                 140

Val Thr Leu Ser Cys Ser Ser Trp Ser Ser Tyr Asp Met Tyr His Leu
145                 150                 155                 160

Ser Thr Glu Gly Glu Ala His Glu Arg Arg Phe Ser Ala Gly Pro Lys
                165                 170                 175

Val Asn Gly Thr Phe Gln Ala Asp Phe Pro Leu Gly Pro Ala Thr Gln
            180                 185                 190

Gly Gly Thr Tyr Arg Cys Phe Gly Ser Phe His Asp Ser Pro Tyr Glu
        195                 200                 205

Trp Ser Lys Ser Ser Asp Pro Leu Leu Val Ser Val Thr Gly Asn Pro
    210                 215                 220

Ser Asn Ser Trp Pro Ser Pro Thr Glu Pro Ser Ser Lys Thr Gly Asn
225                 230                 235                 240

Pro Arg His Leu His Val Leu Ile Gly Thr Ser Val Val Lys Leu Pro
                245                 250                 255

Phe Thr Ile Leu Leu Phe Phe Leu Leu His Arg Trp Cys Ser Asp Lys
            260                 265                 270

Lys Asn Ala Ser Val Met Asp Gln Gly Pro Ala Gly Asn Arg Thr Val
        275                 280                 285

Asn Arg Glu Asp Ser Asp Glu Gln Asp His Gln Glu Val Ser Tyr Ala
    290                 295                 300

<210> 117
<211> 304
<212> PRT
<213> Homo sapiens

<400> 117

Met Ser Leu Met Val Ile Ile Met Ala Cys Val Gly Phe Phe Leu Leu
1               5                   10                  15

Gln Gly Ala Trp Pro Gln Glu Gly Val His Arg Lys Pro Ser Phe Leu
            20                  25                  30

Ala Leu Pro Gly His Leu Val Lys Ser Glu Glu Thr Val Ile Leu Gln
        35                  40                  45

Cys Trp Ser Asp Val Met Phe Glu His Phe Leu Leu His Arg Glu Gly
    50                  55                  60

Lys Phe Asn Asn Thr Leu His Leu Ile Gly Glu His His Asp Gly Val
65                  70                  75                  80

Ser Lys Ala Asn Phe Ser Ile Gly Pro Met Met Pro Val Leu Ala Gly
                85                  90                  95

Thr Tyr Arg Cys Tyr Gly Ser Val Pro His Ser Pro Tyr Gln Leu Ser
            100                 105                 110

Ala Pro Ser Asp Pro Leu Asp Met Val Ile Ile Gly Leu Tyr Glu Lys
        115                 120                 125

Pro Ser Leu Ser Ala Gln Pro Gly Pro Thr Val Gln Ala Gly Glu Asn
    130                 135                 140

Val Thr Leu Ser Cys Ser Ser Arg Ser Ser Tyr Asp Met Tyr His Leu
145                 150                 155                 160

Ser Arg Glu Gly Glu Ala His Glu Arg Arg Leu Pro Ala Val Arg Ser
                165                 170                 175

Ile Asn Gly Thr Phe Gln Ala Asp Phe Pro Leu Gly Pro Ala Thr His
            180                 185                 190

Gly Gly Thr Tyr Arg Cys Phe Gly Ser Phe Arg Asp Ala Pro Tyr Glu
        195                 200                 205

Trp Ser Asn Ser Ser Asp Pro Leu Leu Val Ser Val Thr Gly Asn Pro
    210                 215                 220

Ser Asn Ser Trp Pro Ser Pro Thr Glu Pro Ser Ser Lys Thr Gly Asn
225                 230                 235                 240

Pro Arg His Leu His Val Leu Ile Gly Thr Ser Val Val Lys Ile Pro
                245                 250                 255

Phe Thr Ile Leu Leu Phe Phe Leu Leu His Arg Trp Cys Ser Asp Lys
            260                 265                 270

Lys Asn Ala Ala Val Met Asp Gln Glu Pro Ala Gly Asn Arg Thr Val
        275                 280                 285

Asn Ser Glu Asp Ser Asp Glu Gln Asp His Gln Glu Val Ser Tyr Ala
    290                 295                 300

<210>  118
<211>  348
<212>  PRT
<213>  Homo sapiens

<400>  118

Met Ser Leu Leu Val Val Ser Met Ala Cys Val Gly Phe Phe Leu Leu
1               5                   10                  15

Gln Gly Ala Trp Pro His Glu Gly Val His Arg Lys Pro Ser Leu Leu
            20                  25                  30

Ala His Pro Gly Arg Leu Val Lys Ser Glu Glu Thr Val Ile Leu Gln
        35                  40                  45

Cys Trp Ser Asp Val Met Phe Glu His Phe Leu Leu His Arg Glu Gly
    50                  55                  60

Met Phe Asn Asp Thr Leu Arg Leu Ile Gly Glu His His Asp Gly Val
65                  70                  75                  80

Ser Lys Ala Asn Phe Ser Ile Ser Arg Met Thr Gln Asp Leu Ala Gly
                85                  90                  95

Thr Tyr Arg Cys Tyr Gly Ser Val Thr His Ser Pro Tyr Gln Val Ser
            100                 105                 110

Ala Pro Ser Asp Pro Leu Asp Ile Val Ile Ile Gly Leu Tyr Glu Lys
        115                 120                 125

Pro Ser Leu Ser Ala Gln Leu Gly Pro Thr Val Leu Ala Gly Glu Asn
    130                 135                 140

Val Thr Leu Ser Cys Ser Ser Arg Ser Ser Tyr Asp Met Tyr His Leu
145                 150                 155                 160

Ser Arg Glu Gly Glu Ala His Glu Arg Arg Leu Pro Ala Gly Pro Lys
                165                 170                 175

Val Asn Gly Thr Phe Gln Ala Asp Phe Pro Leu Gly Pro Ala Thr His
            180                 185                 190

Gly Gly Thr Tyr Arg Cys Phe Gly Ser Phe His Asp Ser Pro Tyr Glu
        195                 200                 205

Trp Ser Lys Ser Ser Asp Pro Leu Leu Val Ser Val Thr Gly Asn Pro
    210                 215                 220

Ser Asn Ser Trp Pro Ser Pro Thr Glu Pro Ser Ser Lys Thr Gly Asn
225                 230                 235                 240

Pro Arg His Leu His Ile Leu Ile Gly Thr Ser Val Val Ile Ile Leu
                245                 250                 255

Phe Ile Leu Leu Phe Phe Leu Leu His Arg Trp Cys Ser Asn Lys Lys
            260                 265                 270

Asn Ala Ala Val Met Asp Gln Glu Ser Ala Gly Asn Arg Thr Ala Asn
        275                 280                 285

Ser Glu Asp Ser Asp Glu Gln Asp Pro Gln Glu Val Thr Tyr Thr Gln
    290                 295                 300

Leu Asn His Cys Val Phe Thr Gln Arg Lys Ile Thr Arg Pro Ser Gln
305                 310                 315                 320

Arg Pro Lys Thr Pro Pro Thr Asp Ile Ile Val Tyr Thr Glu Leu Pro
                325                 330                 335

Asn Ala Glu Ser Arg Ser Lys Val Val Ser Cys Pro
            340                 345

<210>  119
<211>  304
<212>  PRT
<213>  Homo sapiens

<400>  119

Met Ser Leu Thr Val Val Ser Met Ala Cys Val Gly Phe Phe Leu Leu
1               5                   10                  15

Gln Gly Ala Trp Pro His Glu Gly Val His Arg Lys Pro Ser Leu Leu
            20                  25                  30

Ala His Pro Gly Arg Leu Val Lys Ser Glu Glu Thr Val Ile Leu Gln
        35                  40                  45

Cys Trp Ser Asp Val Met Phe Glu His Phe Leu Leu His Arg Glu Gly
    50                  55                  60

Met Phe Asn Asp Thr Leu Arg Leu Ile Gly Glu His His Asp Gly Val
65                  70                  75                  80

Ser Lys Ala Asn Phe Ser Ile Ser Arg Met Lys Gln Asp Leu Ala Gly
                85                  90                  95

Thr Tyr Arg Cys Tyr Gly Ser Val Thr His Ser Pro Tyr Gln Leu Ser
            100                 105                 110

Ala Pro Ser Asp Pro Leu Asp Ile Val Ile Ile Gly Leu Tyr Glu Lys
        115                 120                 125

Pro Ser Leu Ser Ala Gln Pro Gly Pro Thr Val Leu Ala Gly Glu Asn
    130                 135                 140

Val Thr Leu Ser Cys Ser Ser Arg Ser Ser Tyr Asp Met Tyr His Leu
145                 150                 155                 160

Ser Arg Glu Gly Glu Ala His Glu Arg Arg Leu Pro Ala Gly Thr Lys
                165                 170                 175

Val Asn Gly Thr Phe Gln Ala Asn Phe Pro Leu Gly Pro Ala Thr His
            180                 185                 190

Gly Gly Thr Tyr Arg Cys Phe Gly Ser Phe Arg Asp Ser Pro Tyr Glu
        195                 200                 205

Trp Ser Lys Ser Ser Asp Pro Leu Leu Val Ser Val Thr Gly Asn Pro
    210                 215                 220

Ser Asn Ser Trp Pro Ser Pro Thr Glu Pro Ser Ser Glu Thr Gly Asn
225                 230                 235                 240

Pro Arg His Leu His Val Leu Ile Gly Thr Ser Val Val Lys Ile Pro
                245                 250                 255

Phe Thr Ile Leu Leu Phe Phe Leu Leu His Arg Trp Cys Ser Asp Lys
            260                 265                 270

Lys Asn Ala Ala Val Met Asp Gln Glu Pro Ala Gly Asn Arg Thr Val
        275                 280                 285

Asn Ser Glu Asp Ser Asp Glu Gln Asp His Gln Glu Val Ser Tyr Ala
    290                 295                 300


<210>  120
<211>  254
<212>  PRT
<213>  Homo sapiens

<400>  120

Met Ala Pro Arg Ser Leu Leu Leu Leu Leu Ser Gly Ala Leu Ala Leu
1               5                   10                  15

Thr Asp Thr Trp Ala Gly Ser His Ser Leu Arg Tyr Phe Ser Thr Ala
            20                  25                  30

Val Ser Arg Pro Gly Arg Gly Glu Pro Arg Tyr Ile Ala Val Glu Tyr
        35                  40                  45

Val Asp Asp Thr Gln Phe Leu Arg Phe Asp Ser Asp Ala Ala Ile Pro
    50                  55                  60

Arg Met Glu Pro Arg Glu Pro Trp Val Glu Gln Glu Gly Pro Gln Tyr
65                  70                  75                  80

Trp Glu Trp Thr Thr Gly Tyr Ala Lys Ala Asn Ala Gln Thr Asp Arg
                85                  90                  95

Val Ala Leu Arg Asn Leu Leu Arg Arg Tyr Asn Gln Ser Glu Ala Gly
            100                 105                 110

Ser His Thr Leu Gln Gly Met Asn Gly Cys Asp Met Gly Pro Asp Gly
        115                 120                 125

Arg Leu Leu Arg Gly Tyr His Gln His Ala Tyr Asp Gly Lys Asp Tyr
    130                 135                 140

Ile Ser Leu Asn Glu Asp Leu Arg Ser Trp Thr Ala Ala Asp Thr Val
145                 150                 155                 160

Ala Gln Ile Thr Gln Arg Phe Tyr Glu Ala Glu Glu Tyr Ala Glu Glu
                165                 170                 175

Phe Arg Thr Tyr Leu Glu Gly Glu Cys Leu Glu Leu Leu Arg Arg Tyr
            180                 185                 190

Leu Glu Asn Gly Lys Glu Thr Leu Gln Arg Ala Glu Gln Ser Pro Gln
        195                 200                 205

Pro Thr Ile Pro Ile Val Gly Ile Val Ala Gly Leu Val Val Leu Gly
    210                 215                 220

Ala Val Val Thr Gly Ala Val Val Ala Ala Val Met Trp Arg Lys Lys
225                 230                 235                 240

Ser Ser Asp Arg Asn Arg Gly Ser Tyr Ser Gln Ala Ala Val
                245                 250

<210>  121
<211>  442
<212>  PRT
<213>  Homo sapiens

<400>  121

Met Ala Pro Arg Ser Leu Leu Leu Leu Leu Ser Gly Ala Leu Ala Leu
1               5                   10                  15

Thr Asp Thr Trp Ala Gly Ser His Ser Leu Arg Tyr Phe Ser Thr Ala
            20                  25                  30

Val Ser Arg Pro Gly Arg Gly Glu Pro Arg Tyr Ile Ala Val Glu Tyr
        35                  40                  45

Val Asp Asp Thr Gln Phe Leu Arg Phe Asp Ser Asp Ala Ala Ile Pro
    50                  55                  60

Arg Met Glu Pro Arg Glu Pro Trp Val Glu Gln Glu Gly Pro Gln Tyr
65                  70                  75                  80

Trp Glu Trp Thr Thr Gly Tyr Ala Lys Ala Asn Ala Gln Thr Asp Arg
                85                  90                  95

Val Ala Leu Arg Asn Leu Leu Arg Arg Tyr Asn Gln Ser Glu Ala Gly
            100                 105                 110

Ser His Thr Leu Gln Gly Met Asn Gly Cys Asp Met Gly Pro Asp Gly
        115                 120                 125

Arg Leu Leu Arg Gly Tyr His Gln His Ala Tyr Asp Gly Lys Asp Tyr
    130                 135                 140

Ile Ser Leu Asn Glu Asp Leu Arg Ser Trp Thr Ala Ala Asp Thr Val
145                 150                 155                 160

Ala Gln Ile Thr Gln Arg Phe Tyr Glu Ala Glu Glu Tyr Ala Glu Glu
                165                 170                 175

Phe Arg Thr Tyr Leu Glu Gly Glu Cys Leu Glu Leu Leu Arg Arg Tyr
            180                 185                 190

Leu Glu Asn Gly Lys Glu Thr Leu Gln Arg Ala Asp Pro Pro Lys Ala
        195                 200                 205

His Val Ala His His Pro Ile Ser Asp His Glu Ala Thr Leu Arg Cys
    210                 215                 220

Trp Ala Leu Gly Phe Tyr Pro Ala Glu Ile Thr Leu Thr Trp Gln Arg
225                 230                 235                 240

Asp Gly Glu Glu Gln Thr Gln Asp Thr Glu Leu Val Glu Thr Arg Pro
                245                 250                 255

Ala Gly Asp Gly Thr Phe Gln Lys Trp Ala Ala Val Val Val Pro Pro
            260                 265                 270

Gly Glu Glu Gln Arg Tyr Thr Cys His Val Gln His Glu Gly Leu Pro

275                 280                 285

Gln Pro Leu Ile Leu Arg Trp Glu Gln Ser Pro Gln Pro Thr Ile Pro
    290                 295                 300

Ile Val Gly Ile Val Ala Gly Leu Val Val Leu Gly Ala Val Val Thr
305                 310                 315                 320

Gly Ala Val Val Ala Ala Val Met Trp Arg Lys Lys Ser Ser Asp Arg
                325                 330                 335

Asn Arg Gly Ser Tyr Ser Gln Ala Ala Ala Tyr Ser Val Val Ser Gly
            340                 345                 350

Asn Leu Met Ile Thr Trp Trp Ser Ser Leu Phe Leu Leu Gly Val Leu
        355                 360                 365

Phe Gln Gly Tyr Leu Gly Cys Leu Arg Ser His Ser Val Leu Gly Arg
    370                 375                 380

Arg Lys Val Gly Asp Met Trp Ile Leu Phe Phe Leu Trp Leu Trp Thr
385                 390                 395                 400

Ser Phe Asn Thr Ala Phe Leu Ala Leu Gln Ser Leu Arg Phe Gly Phe
                405                 410                 415

Gly Phe Arg Arg Gly Arg Ser Phe Leu Leu Arg Ser Trp His His Leu
            420                 425                 430

Met Lys Arg Val Gln Ile Lys Ile Phe Asp
        435                 440

<210>  122
<211>  346
<212>  PRT
<213>  Homo sapiens

<400>  122

Met Ala Pro Arg Ser Leu Leu Leu Leu Leu Ser Gly Ala Leu Ala Leu
1               5                   10                  15

Thr Asp Thr Trp Ala Gly Ser His Ser Leu Arg Tyr Phe Ser Thr Ala
            20                  25                  30

Val Ser Arg Pro Gly Arg Gly Glu Pro Arg Tyr Ile Ala Val Glu Tyr
        35                  40                  45

Val Asp Asp Thr Gln Phe Leu Arg Phe Asp Ser Asp Ala Ala Ile Pro

50                  55                  60

Arg Met Glu Pro Arg Glu Pro Trp Val Glu Gln Glu Gly Pro Gln Tyr
65                  70                  75                  80

Trp Glu Trp Thr Thr Gly Tyr Ala Lys Ala Asn Ala Gln Thr Asp Arg
                85                  90                  95

Val Ala Leu Arg Asn Leu Leu Arg Arg Tyr Asn Gln Ser Glu Ala Gly
            100                 105                 110

Ser His Thr Leu Gln Gly Met Asn Gly Cys Asp Met Gly Pro Asp Gly
        115                 120                 125

Arg Leu Leu Arg Gly Tyr His Gln His Ala Tyr Asp Gly Lys Asp Tyr
    130                 135                 140

Ile Ser Leu Asn Glu Asp Leu Arg Ser Trp Thr Ala Ala Asp Thr Val
145                 150                 155                 160

Ala Gln Ile Thr Gln Arg Phe Tyr Glu Ala Glu Glu Tyr Ala Glu Glu
                165                 170                 175

Phe Arg Thr Tyr Leu Glu Gly Glu Cys Leu Glu Leu Leu Arg Arg Tyr
            180                 185                 190

Leu Glu Asn Gly Lys Glu Thr Leu Gln Arg Ala Asp Pro Pro Lys Ala
        195                 200                 205

His Val Ala His His Pro Ile Ser Asp His Glu Ala Thr Leu Arg Cys
    210                 215                 220

Trp Ala Leu Gly Phe Tyr Pro Ala Glu Ile Thr Leu Thr Trp Gln Arg
225                 230                 235                 240

Asp Gly Glu Glu Gln Thr Gln Asp Thr Glu Leu Val Glu Thr Arg Pro
                245                 250                 255

Ala Gly Asp Gly Thr Phe Gln Lys Trp Ala Ala Val Val Val Pro Pro
            260                 265                 270

Gly Glu Glu Gln Arg Tyr Thr Cys His Val Gln His Glu Gly Leu Pro
        275                 280                 285

Gln Pro Leu Ile Leu Arg Trp Glu Gln Ser Pro Gln Pro Thr Ile Pro
    290                 295                 300

Ile Val Gly Ile Val Ala Gly Leu Val Val Leu Gly Ala Val Val Thr
305                 310                 315                 320

Gly Ala Val Val Ala Ala Val Met Trp Arg Lys Lys Ser Ser Asp Arg
                325                 330                 335

Asn Arg Gly Ser Tyr Ser Gln Ala Ala Val
            340                 345

<210>  123
<211>  665
<212>  DNA
<213>  Homo sapiens

<400>  123
gggaacacat ccaagcttaa gacggtgagg tcagcttcac attctcagga actctccttc      60

tttgggtctg gctgaagttg aggatctctt actctctagg ccacggaatt aacccgagca     120

ggcatggagg cctctgctct cacctcatca gcagtgacca gtgtggccaa agtggtcagg     180

gtggcctctg gctctgccgt agttttgccc ctggccagga ttgctacagt tgtgattgga     240

ggagttgtgg ccatggcggc tgtgcccatg gtgctcagtg ccatgggctt cactgcggcg     300

ggaatcgcct cgtcctccat agcagccaag atgatgtccg cggcggccat tgccaatggg     360

ggtggagttg cctcgggcag ccttgtggct actctgcagt cactgggagc aactggactc     420

tccggattga ccaagttcat cctgggctcc attgggtctg ccattgcggc tgtcattgcg     480

aggttctact agctccctgc ccctcgccct gcagagaaga gaaccatgcc aggggagaag     540

gcacccagcc atcctgaccc agcgaggagc caactatccc aaatatacct ggggtgaaat     600

ataccaaatt ctgcatctcc agaggaaaat aagaaataaa gatgaattgt tgcaactctt     660

caaaa                                                                 665

<210>  124
<211>  656
<212>  DNA
<213>  Homo sapiens

<400>  124
gggaacacat ccaagcttaa gacggtgagg tcagcttcac attctcagga actctccttc      60

tttgggtctg gctgaagttg aggatctctt actctctagg ccacggaatt aacccgagca     120

ggcatggagg cctctgctct cacctcatca gcagtgacca gtgtggccaa agtggtcagg     180

gtggcctctg gctctgccgt agttttgccc ctggccagga ttgctacagt tgtgattgga     240

ggagttgtgg ctgtgcccat ggtgctcagt gccatgggct tcactgcggc gggaatcgcc     300

tcgtcctcca tagcagccaa gatgatgtcc gcggcggcca ttgccaatgg gggtggagtt     360

gcctcgggca gccttgtggc tactctgcag tcactgggag caactggact ctccggattg     420

accaagttca tcctgggctc cattgggtct gccattgcgg ctgtcattgc gaggttctac 480

tagctccctg cccctcgccc tgcagagaag agaaccatgc caggggagaa ggcacccagc 540

catcctgacc cagcgaggag ccaactatcc caaatatacc tggggtgaaa tataccaaat 600

tctgcatctc cagaggaaaa taagaaataa agatgaattg ttgcaactct tcaaaa 656

<210> 125
<211> 1184
<212> DNA
<213> Homo sapiens

<400> 125
gggggagaca ttcctcaatt gcttagacat attctgagcc tacagcagag gaacctccag 60

tctcagcacc atgaatcaaa ctgccattct gatttgctgc cttatctttc tgactctaag 120

tggcattcaa ggagtacctc tctctagaac tgtacgctgt acctgcatca gcattagtaa 180

tcaacctgtt aatccaaggt ctttagaaaa acttgaaatt attcctgcaa gccaattttg 240

tccacgtgtt gagatcattg ctacaatgaa aaagaagggt gagaagagat gtctgaatcc 300

agaatcgaag gccatcaaga atttactgaa agcagttagc aaggaaaggt ctaaaagatc 360

tccttaaaac cagaggggag caaaatcgat gcagtgcttc caaggatgga ccacacagag 420

gctgcctctc ccatcacttc cctacatgga gtatatgtca agccataatt gttcttagtt 480

tgcagttaca ctaaaaggtg accaatgatg gtcaccaaat cagctgctac tactcctgta 540

ggaaggttaa tgttcatcat cctaagctat tcagtaataa ctctaccctg gcactataat 600

gtaagctcta ctgaggtgct atgttcttag tggatgttct gaccctgctt caaatatttc 660

cctcaccttt cccatcttcc aagggtacta aggaatcttt ctgctttggg gtttatcaga 720

attctcagaa tctcaaataa ctaaaaggta tgcaatcaaa tctgcttttt aaagaatgct 780

ctttacttca tggacttcca ctgccatcct cccaaggggc ccaaattctt tcagtggcta 840

cctacataca attccaaaca catacaggaa ggtagaaata tctgaaaatg tatgtgtaag 900

tattcttatt taatgaaaga ctgtacaaag tagaagtctt agatgtatat atttcctata 960

ttgttttcag tgtacatgga ataacatgta attaagtact atgtatcaat gagtaacagg 1020

aaaattttaa aaatacagat agatatatgc tctgcatgtt acataagata aatgtgctga 1080

atggttttca aaataaaaat gaggtactct cctggaaata ttaagaaaga ctatctaaat 1140

gttgaaagat caaaaggtta ataaagtaat tataactaaa aaaa 1184

<210> 126
<211> 994
<212> DNA
<213> Homo sapiens

<400> 126

aagactcaaa cttagaaact tgaattagat gtggtattca aatccttagc tgccgcgaag 60

acacagacag cccccgtaag aacccacgaa gcaggcgaag ttcattgttc tcaacattct 120

agctgctctt gctgcatttg ctctggaatt cttgtagaga tattacttgt ccttccaggc 180

tgttctttct gtagctccct tgttttcttt ttgtgatcat gttgcagatg gctgggcagt 240

gctcccaaaa tgaatatttt gacagtttgt tgcatgcttg cataccttgt caacttcgat 300

gttcttctaa tactcctcct ctaacatgtc agcgttattg taatgcaagt gtgaccaatt 360

cagtgaaagg aacgaatgcg attctctgga cctgtttggg actgagctta ataatttctt 420

tggcagtttt cgtgctaatg tttttgctaa ggaagataaa ctctgaacca ttaaaggacg 480

agtttaaaaa cacaggatca ggtctcctgg gcatggctaa cattgacctg gaaaagagca 540

ggactggtga tgaaattatt cttccgagag gcctcgagta cacggtggaa gaatgcacct 600

gtgaagactg catcaagagc aaaccgaagg tcgactctga ccattgcttt ccactcccag 660

ctatggagga aggcgcaacc attcttgtca ccacgaaaac gaatgactat tgcaagagcc 720

tgccagctgc tttgagtgct acggagatag agaaatcaat ttctgctagg taattaacca 780

tttcgactcg agcagtgcca ctttaaaaat cttttgtcag aatagatgat gtgtcagatc 840

tctttaggat gactgtattt ttcagttgcc gatacagctt tttgtcctct aactgtggaa 900

actctttatg ttagatatat ttctctaggt tactgttggg agcttaatgg tagaaacttc 960

cttggtttca tgattaaact cttttttttc ctga 994

<210> 127
<211> 1877
<212> DNA
<213> Homo sapiens

<400> 127
ggggcgcggc ctcctgtctg caccggcagc accatgtcgc tcacggtcgt cagcatggcg 60

tgcgttgggt tcttcttgct gcagggggcc tggccactca tgggtggtca ggacaaaccc 120

ttcctgtctg cccggcccag cactgtggtg cctcgaggag gacacgtggc tcttcagtgt 180

cactatcgtc gtgggtttaa caatttcatg ctgtacaaag aagacagaag ccacgttccc 240

atcttccacg gcagaatatt ccaggagagc ttcatcatgg gccctgtgac cccagcacat 300

gcagggacct acagatgtcg gggttcacgc ccacactccc tcactgggtg gtcggcaccc 360

agcaaccccc tggtgatcat ggtcacagga aaccacagaa aaccttccct cctggcccac 420

ccagggcccc tgctgaaatc aggagagaca gtcatcctgc aatgttggtc agatgtcatg 480

tttgagcact tctttctgca cagagagggg atctctgagg acccctcacg cctcgttgga 540

cagatccatg atggggtctc caaggccaac ttctccatcg gtcccttgat gcctgtcctt 600

gcaggaacct acagatgtta tggttctgtt cctcactccc cctatcagtt gtcagctccc 660

agtgaccccc tggacatcgt gatcacaggt ctatatgaga aaccttctct ctcagcccag 720

ccgggcccca cggttcaggc aggagagaac gtgaccttgt cctgtagctc ctggagctcc 780

tatgacatct accatctgtc cagggaaggg gaggcccatg aacgtaggct ccgtgcagtg 840

cccaaggtca acagaacatt ccaggcagac tttcctctgg gccctgccac ccacggaggg 900

acctacagat gcttcggctc tttccgtgcc ctgccctgcg tgtggtcaaa ctcaagtgac 960

ccactgcttg tttctgtcac aggaaaccct tcaagtagtt ggccttcacc cacagaacca 1020

agctccaaat ctggtatctg cagacacctg catgttctga ttgggacctc agtggtcatc 1080

ttcctcttca tcctcctcct cttctttctc ctttatcgct ggtgctccaa caaaaagaat 1140

gctgctgtaa tggaccaaga gcctgcgggg gacagaacag tgaataggca ggactctgat 1200

gaacaagacc ctcaggaggt gacgtacgca cagttggatc actgcgtttt catacagaga 1260

aaaatcagtc gcccttctca gaggcccaag acacccctaa cagataccag cgtgtacacg 1320

gaacttccaa atgctgagcc cagatccaaa gttgtctcct gcccacgagc accacagtca 1380

ggtcttgagg gggttttcta gggagacaac agccctgtct caaaaccagg ttgccagatc 1440

caatgaacca gcagctggaa tctgaaggca tcagtctgca tcttagggga tcgctcttcc 1500

tcacaccacg aatctgaaca tgcctctctc ttgcttacaa atgcctaagg tcgccactgc 1560

ctgctgcaga gaaaacacac tcctttgctt agcccacaag tatctatttc acttgacccc 1620

tgcccacctc tccaacctaa ctggcttact tcctagtcct acttgaggct gcaatcacac 1680

tgaggaactc acaattccaa acatacaaga ggctccctct taacacggca cttacacact 1740

tgctgttcca ccttccctca tgctgttcca cctcccctca gactatcttt cagccttctg 1800

tcatcagtaa aatttataaa ttttttttat aacttcagtg tagctctctc ctcttcaaat 1860

aaacatgtct gccctca 1877

<210> 128
<211> 1029
<212> DNA
<213> Homo sapiens

<400> 128
atgtcgctca ctgtcgtcag catggcgtgc gttgggttct tcttgctgca gggggcctgg 60

ccactcatgg gtggtcagga caaacccttc ctgtctgccc ggcccagcac tgtggtgcct 120

cgaggaggac acgtggctct tcagtgtcac tatcgtcgtg ggtttaacaa tttcatgctg 180

tacaaagaag acagaagcca cgttcccatc ttccacggca gaatattcca ggagagcttc 240

atcatgggcc ctgtgacccc agcacatgca gggacctaca gatgtcgggg ttcacgccca 300

cactccctca ctgggtggtc gacacccagc aaccccctgg tgatcatggt cacaggaaac 360

cacagaaaac cttccctcct ggcccaccca gggcccctgc tgaaatcagg agagacagtc 420

atcctgcaat gttggtcaga tgtcatgttt gagcacttct ttctgcacag agaggggatc 480

tctgaggacc cctcacgcct cgttggacag atccatgatg gggtctccaa ggccaacttc 540

tccatcggtc ccttgatgcc tgtccttgca ggaacctaca gatgttatgg ttctgttcct 600

cactccccct atcagttgtc agctcccagt gaccccctgg acatcgtgat cacaggtcta 660

tatgagaaac cttctctctc agcccagccg ggccccacgg ttcaggcagg agagaacgtg 720

accttgtcct gtagctcctg gagctcctat gacatctacc atctgtccag ggaaggggag 780

gcccatgaac gtaggctccg tgcagtgccc aaggtcaaca gaacattcca ggcagacttt 840

cctctgggcc ctgccaccca cggagggacc tacagatgct tcggctcttt ccgtgccctg 900

ccctgcgtgt ggtcaaactc aagtgaccca ctgcttgttt ctgtcacagg aaacccttca 960

agtagttggc cttcacccac agaaccaagc tccaaatctg gtgagacctc ctacaagcta 1020

gaagaataa 1029

<210> 129
<211> 1587
<212> DNA
<213> Homo sapiens

<400> 129
cgcggccgcc tgtctgcaca gacagcacca tgtcgctcat ggtcgtcagc atggcgtgtg 60

ttgggttctt cttgctgcag ggggcctggc cacatgaggg agtccacaga aaaccttccc 120

tcctggccca cccaggtcgc ctggtgaaat cagaagagac agtcatcctg caatgttggt 180

cagatgtcag gtttgagcac ttccttctgc acagagaagg gaagtttaag gacactttgc 240

acctcattgg agagcaccat gatggggtct ccaaagccaa cttctccatc ggtcccatga 300

tgcaagacct tgcagggacc tacagatgct acggttctgt tactcactcc ccctatcagt 360

tgtcagctcc cagtgaccct ctggacatcg tcatcacagg tctatatgag aaaccttctc 420

tctcagccca gccgggcccc acggttctgg caggagagag cgtgaccttg tcctgcagct 480

cccggagctc ctatgacatg taccatctat ccagggaggg ggaggcccat gaatgtaggt 540

tctctgcagg gcccaaggtc aacggaacat tccaggccga ctttcctctg ggccctgcca 600

cccacggagg aacctacaga tgcttcggct ctttccgtga ctctccatac gagtggtcaa 660

actcgagtga cccactgctt gtttctgtca caggaaaccc ttcaaatagt tggccttcac 720

ccactgaacc aagctctaaa accggtaacc cccgacacct gcacattctg attgggacct 780

cagtggtcat catcctcttc atcctcctct tctttctcct tcatcgctgg tgctccaaca 840

aaaaaaatgc tgcggtaatg gaccaagagt ctgcagggaa cagaacagcg aatagcgagg 900

actctgatga acaagaccct caggaggtga catacacaca gttgaatcac tgcgttttca 960

cacagagaaa aatcactcgc ccttctcaga ggcccaagac acccccaaca gatatcatcg 1020

tgtacacgga acttccaaat gctgagtcca gatccaaagt tgtctcctgc ccatgagcac 1080

cacagtcagg ccttgagggc gtcttctagg gagacaacag ccctgtctca aaaccgggtt 1140

gccagctccc atgtaccagc agctggaatc tgaaggcatg agtctgcatc ttagggcatc 1200

gctcttcctc acaccacaaa tctgaatgtg cctctcactt gcttacaaat gtctaaggtc 1260

cccactgcct gctggagaaa aaacacactc ctttgcttag cccacagttc tccatttcac 1320

ttgacccctg cccacctctc caacctaact ggcttacttc ctagtctact tgaggctgca 1380

atcacactga ggaactcaca attccaaaca tacaagaggc tccctcttaa cgcagcactt 1440

agacacgtgt tgttccacct tccctcatgc tgttccacct cccctcagac tagctttcag 1500

tcttctgtca gcagtaaaac ttatatattt tttaaaataa cttcaatgta gttttccatc 1560

cttcaaataa acatgtctgc ccccatg 1587

<210> 130
<211> 1559
<212> DNA
<213> Homo sapiens

<400> 130
ccccagtctg agaacaagaa agaagaactt ctgtctcgag ggtctcactg tcaaccaggc 60

cagagtgcag tgaagatcat acctcactac atccgtgaac tcccgggctc ctcccaccta 120

agtctcttga gtagctggga cttcaggaga ctgaagccaa ggataccagc agagccaaca 180

tttgcttcaa gttcctgggc ctgctgacag cgtgcaggat gctgttggaa cccggcagag 240

gctgctgtgc cctggccatc ctgctggcaa ttgtggacat ccagtctggt ggatgcatta 300

acatcaccag ctcagcttcc caggaaggaa cgcgactaaa cttaatctgt actgtatggc 360

ataagaaaga agaggctgag gggtttgtag tgtttttgtg caaggacagg tctggagact 420

gttctcctga gaccagttta aaacagctga gacttaaaag ggatcctggg atagatggtg 480

ttggtgaaat atcatctcag ttgatgttca ccataagcca agtcacaccg ttgcacagtg 540

ggacctacca gtgttgtgcc agaagccaga agtcaggtat ccgccttcag ggccattttt 600

tctccattct attcacagag acagggaact acacagtgac gggattgaaa caaagacaac 660

accttgagtt cagccataat gaaggcactc tcagttcagg cttcctacaa gaaaaggtct 720

gggtaatgct ggtcaccagc cttgtggccc ttcaagcttt gtaagccttg tgccaaaaga 780

aacttttaaa acagctacag caagatgagt ctgactatgg cttagtatct ttctcattac 840

aataggcaca gagaagaatg caacagggca caggggaaga gatgctaaat ataccaagaa 900

tctgtggaaa tataagctgg ggcaaatcag tgtaatcctt gactttgctc ctcaccatca 960

gggcaaactt gccttcttcc ctcctaagct ccagtaaata aacagaacag ctttcaccaa 1020

agtgggtagt atagtcctca aatatcggat aaatatatgc gtttttgtac cccagaaaaa 1080

cttttcctcc ctcttcatca acatagtaaa ataagtcaaa caaaatgaga acaccaaatt 1140

ttgggggaat aaatttttat ttaacactgc aaaggaaaga gagagaaaac aagcaaagat 1200

aggtaggaca gaaaggaaga cagccagatc cagtgattga cttggcatga aaatgagaaa 1260

atgcagacag acctcaacat tcaacaacat ccatacagca ctgctggagg aagaggaaga 1320

tttgtgcaga ccaagagcac cacagactac aactgcccag cttcatctaa atacttgtta 1380

acctctttgg tcatttctct ttttaaataa atgcccatag cagtatttgg agtattttct 1440

tttctcctaa atccacaaac tctcttcttt ctctttggac agatgacctc ttgtcatagt 1500

taagcagaga gtgggcagga tattcctgat aggaggaact acatgaataa aggggtaag 1559

<210> 131
<211> 1302
<212> DNA
<213> Homo sapiens

<400> 131
agctggggcg cggccgcctg tctgcacaga cagcaccatg tcgctcatgg tcgtcagcat 60

ggtgtgtgtt gggttcttct tgctgcaggg ggcctggcca catgagggag tccacagaaa 120

accttccctc ctggcccacc caggtcccct ggtgaaatca gaagagacag tcatcctgca 180

atgttggtca gatgtcaggt ttcagcactt ccttctgcac agagaaggga agtttaagga 240

cactttgcac ctcattggag agcaccatga tggggtctcc aaggccaact tctccatcgg 300

tcccatgatg caagaccttg cagggaccta cagatgctac ggttctgtta ctcactcccc 360

ctatcagttg tcagctccga gtgacccact gcttgtttct gtcacaggaa acccttcaaa 420

tagttggcct tcacccactg aaccaagctc cgaaaccggt aaccccagac acctgcatgt 480

tctgattggg acctcagtgg tcatcatcct cttcatcctc ctcctcttct ttctccttca 540

tcgctggtgc tgcaacaaaa aaaatgctgt tgtaatggac caagagcctg cagggaacag 600

aacagtgaac agggaggact ctgatgaaca agaccctcag gaggtgacat atgcacagtt 660

gaatcactgc gttttcacac agagaaaaat cactcgccct tctcagaggc ccaagacacc 720

cccaacagat atcatcgtgt acacggaact tccaaatgct gagccctgat ccaaagttgt 780

ctcctgccca tgagcaccac agtcaggcct tgaggggatc ttctagggag acaacagccc 840

tgtctcaaaa ctgggttgcc agctccaatg taccagcagc tggaatctga aggcgtgagt 900

ctgcatctta gggcatcgct cttcctcaca ccacaaatct gaacgtgcct ctcccttgct 960

tacaaatgtc taaggtcccc actgcctgct ggagagaaaa cacactcctt tgcttagccc 1020

acaattctcc atttcacttg acccctgccc acctctccaa cctaactggc ttacttccta 1080

gtctacttga ggctgcaatc acactgagga actcacaatt ccaaacatac aagaggctcc 1140

ctcttaacac ggcacttaga cacgtgctgt tccaccttcc ctcatgctgt tccacctccc 1200

ctcagactag ctttcagcct tctgtcagca gtaaaactta tatatttttt aaaataattt 1260

caatgtagtt ttccctcctt caaataaaca tgtctgccct ca 1302

<210> 132
<211> 1596
<212> DNA
<213> Homo sapiens

<400> 132
   agctggggcg cggccgcctg tctgcacaga cagcaccatg tcgctcatgg tcgtcagcat 60
   ggtgtgtgtt gggttcttct tgctgcaggg ggcctggcca catgagggag tccacagaaa 120
   accttccctc ctggcccacc caggtcccct ggtgaaatca gaagagacag tcatcctgca 180
   atgttggtca gatgtcaggt ttcagcactt ccttctgcac agagaaggga agtttaagga 240
   cactttgcac ctcattggag agcaccatga tggggtctcc aaggccaact tctccatcgg 300
   tcccatgatg caagaccttg cagggaccta cagatgctac ggttctgtta ctcactcccc 360
   ctatcagttg tcagctccca gtgaccctct ggacatcgtc atcacaggtc tatatgagaa 420
   accttctctc tcagcccagc cgggccccac ggttctggca ggagagagcg tgaccttgtc 480
   ctgcagctcc cggagctcct atgacatgta ccatctatcc agggaggggg aggcccatga 540
   acgtaggttc tctgcagggc ccaaggtcaa cggaacattc caggccgact ttcctctggg 600
   ccctgccacc cacggaggaa cctacagatg cttcggctct ttccgtgact ctccatacga 660
   gtggtcaaac tcgagtgacc cactgcttgt ttctgtcaca ggaaaccctt caaatagttg 720
   gccttcaccc actgaaccaa gctccgaaac cggtaacccc agacacctgc atgttctgat 780
   tgggacctca gtggtcatca tcctcttcat cctcctcctc ttctttctcc ttcatcgctg 840
   gtgctgcaac aaaaaaaatg ctgttgtaat ggaccaagag cctgcaggga acagaacagt 900
   gaacagggag gactctgatg aacaagaccc tcaggaggtg acatatgcac agttgaatca 960
   ctgcgttttc acacagagaa aaatcactcg cccttctcag aggcccaaga cacccccaac 1020
   agatatcatc gtgtacacgg aacttccaaa tgctgagccc tgatccaaag ttgtctcctg 1080
   cccatgagca ccacagtcag gccttgaggg gatcttctag ggagacaaca gccctgtctc 1140
   aaaactgggt tgccagctcc aatgtaccag cagctggaat ctgaaggcgt gagtctgcat 1200
   cttagggcat cgctcttcct cacaccacaa atctgaacgt gcctctccct tgcttacaaa 1260
   tgtctaaggt ccccactgcc tgctggagag aaaacacact cctttgctta gcccacaatt 1320
   ctccatttca cttgacccct gcccacctct ccaacctaac tggcttactt cctagtctac 1380
   ttgaggctgc aatcacactg aggaactcac aattccaaac atacaagagg ctccctctta 1440
   acacggcact tagacacgtg ctgttccacc ttccctcatg ctgttccacc tcccctcaga 1500
   ctagctttca gccttctgtc agcagtaaaa cttatatatt ttttaaaata atttcaatgt 1560
   agttttccct ccttcaaata aacatgtctg ccctca 1596

<210> 133
<211> 1505
<212> DNA
<213> Homo sapiens

<400> 133
tcgagccgag tcactgcgtc ctggcagcag aagctgcacc atgtccatgt cacccacggt 60

catcatcctg gcatgtcttg ggttcttctt ggaccagagt gtgtgggcac acgtgggtgg 120

tcaggacaag cccttctgct ctgcctggcc cagcgctgtg gtgcctcaag gaggacacgt 180

gactcttcgg tgtcactatc gtcgtgggtt taacatcttc acgctgtaca agaaagatgg 240

ggtccctgtc cctgagctct acaacagaat attctggaac agtttcctca ttagccctgt 300

gaccccagca cacgcaggga cctacagatg tcgaggtttt cacccgcact cccccactga 360

gtggtcggca cccagcaacc ccctggtgat catggtcaca ggtctatatg agaaaccttc 420

gcttacagcc cggccgggcc ccacggttcg cgcaggagag aacgtgacct tgtcctgcag 480

ctcccagagc tcctttgaca tctaccatct atccagggag ggggaagccc atgaacttag 540

gctccctgca gtgcccagca tcaatggaac attccaggcc gacttccctc tgggtcctgc 600

cacccacgga gagacctaca gatgcttcgg ctctttccat ggatctccct acgagtggtc 660

agacccgagt gacccactgc ctgtttctgt cacaggaaac ccttctagta gttggccttc 720

acccactgaa ccaagcttca aaactgatgc tgctgtaatg aaccaagagc ctgcgggaca 780

cagaacagtg aacagggagg actctgatga acaagaccct caggaggtga catacgcaca 840

gttggatcac tgcattttca cacagagaaa aatcactggc ccttctcaga ggagcaagag 900

accctcaaca gataccagcg tgtgtataga acttccaaat gctgagccca gagcgttgtc 960

tcctgcccat gagcaccaca gtcaggcctt gatgggatct tctagggaga caacagccct 1020

gtctcaaacc cagcttgcca gctctaatgt accagcagct ggaatctgaa ggcgtgagtc 1080

tccatcttag agcatcactc ttcctcacac cacaaatctg gtgcctgtct cttgcttacc 1140

aatgtctaag gtccccactg cctgctgcag agaaaacaca ctcctttgct tagcccacaa 1200

ttctctattt cacttgaccc ctgcccacct ctccaaccta actggcttac ttcctagtct 1260

acttgaggct gcaatcacac tgaggaactc acaattccaa acatacaaga ggctctctct 1320

taacacggca cttagacacg tgctgttcca ccttccctcg tgctgttcca cctttcctca 1380

gactattttt cagccttctg gcatcagcaa accttataaa atttttttga tttcagtgta 1440

gttctctcct cttcaaataa acatgtctgc cttcaaaaaa aaaaaaaaaa aaaaaaaaaa 1500

aaaaa 1505

<210> 134

<211>  1609
<212>  DNA
<213>  Homo sapiens

<400>  134
tcgagccgag tcactgcgtc ctggcagcag aagctgcacc atgtccatgt cacccacggt      60

catcatcctg gcatgtcttg ggttcttctt ggaccagagt gtgtgggcac acgtgggtgg     120

tcaggacaag cccttctgct ctgcctggcc cagcgctgtg gtgcctcaag gaggacacgt     180

gactcttcgg tgtcactatc gtcgtgggtt taacatcttc acgctgtaca agaaagatgg     240

ggtccctgtc cctgagctct acaacagaat attctggaac agtttcctca ttagccctgt     300

gaccccagca cacgcaggga cctacagatg tcgaggtttt cacccgcact cccccactga     360

gtggtcggca cccagcaacc ccctggtgat catggtcaca ggtctatatg agaaaccttc     420

gcttacagcc cggccgggcc ccacggttcg cgcaggagag aacgtgacct tgtcctgcag     480

ctcccagagc tcctttgaca tctaccatct atccagggag ggggaagccc atgaacttag     540

gctccctgca gtgcccagca tcaatggaac attccaggcc gacttccctc tgggtcctgc     600

cacccacgga gagacctaca gatgcttcgg ctctttccat ggatctccct acgagtggtc     660

agacccgagt gacccactgc ctgtttctgt cacaggaaac ccttctagta gttggccttc     720

acccactgaa ccaagcttca aaactggtat cgccagacac ctgcatgctg tgattaggta     780

ctcagtggcc atcatcctct ttaccatcct tcccttcttt ctccttcatc gctggtgctc     840

caaaaaaaaa atgctgctgt aatgaaccaa gagcctgcgg gacacagaac agtgaacagg     900

gaggactctg atgaacaaga ccctcaggag gtgacatacg cacagttgga tcactgcatt     960

ttcacacaga gaaaaatcac tggcccttct cagaggagca agagaccctc aacagatacc    1020

agcgtgtgta tagaacttcc aaatgctgag cccagagcgt tgtctcctgc ccatgagcac    1080

cacagtcagg ccttgatggg atcttctagg gagacaacag ccctgtctca aacccagctt    1140

gccagctcta atgtaccagc agctggaatc tgaaggcgtg agtctccatc ttagagcatc    1200

actcttcctc acaccacaaa tctggtgcct gtctcttgct taccaatgtc taaggtcccc    1260

actgcctgct gcagagaaaa cacactcctt tgcttagccc acaattctct atttcacttg    1320

acccctgccc acctctccaa cctaactggc ttacttccta gtctacttga ggctgcaatc    1380

acactgagga actcacaatt ccaaacatac aagaggctct ctcttaacac ggcacttaga    1440

cacgtgctgt tccaccttcc ctcgtgctgt tccacctttc ctcagactat ttttcagcct     1500

tctggcatca gcaaacctta taaaattttt ttgatttcag tgtagttctc tcctcttcaa    1560

ataaacatgt ctgccttcaa aaaaaaaaaa aaaaaaaaaa aaaaaaaaa               1609

<210>  135
<211>  1610
<212>  DNA

<213>  Homo sapiens

<400>  135
tcgagccgag tcactgcgtc ctggcagcag aagctgcacc atgtccatgt cacccacggt      60

catcatcctg gcatgtcttg ggttcttctt ggaccagagt gtgtgggcac acgtgggtgg     120

tcaggacaag cccttctgct ctgcctggcc cagcgctgtg gtgcctcaag gaggacacgt     180

gactcttcgg tgtcactatc gtcgtgggtt taacatcttc acgctgtaca agaaagatgg     240

ggtccctgtc cctgagctct acaacagaat attctggaac agtttcctca ttagccctgt     300

gaccccagca cacgcaggga cctacagatg tcgaggtttt cacccgcact cccccactga     360

gtggtcggca cccagcaacc ccctggtgat catggtcaca ggtctatatg agaaaccttc     420

gcttacagcc cggccgggcc ccacggttcg cgcaggagag aacgtgacct tgtcctgcag     480

ctcccagagc tcctttgaca tctaccatct atccagggag ggggaagccc atgaacttag     540

gctccctgca gtgcccagca tcaatggaac attccaggcc gacttccctc tgggtcctgc     600

cacccacgga gagacctaca gatgcttcgg ctctttccat ggatctccct acgagtggtc     660

agacccgagt gacccactgc ctgtttctgt cacaggaaac ccttctagta gttggccttc     720

acccactgaa ccaagcttca aaactggtat cgccagacac ctgcatgctg tgattaggta     780

ctcagtggcc atcatcctct ttaccatcct tcccttcttt ctccttcatc gctggtgctc     840

caaaaaaaaa aatgctgctg taatgaacca agagcctgcg ggacacagaa cagtgaacag     900

ggaggactct gatgaacaag accctcagga ggtgacatac gcacagttgg atcactgcat     960

tttcacacag agaaaaatca ctggcccttc tcagaggagc aagagaccct caacagatac    1020

cagcgtgtgt atagaacttc caaatgctga gcccagagcg ttgtctcctg cccatgagca    1080

ccacagtcag gccttgatgg gatcttctag ggagacaaca gccctgtctc aaacccagct    1140

tgccagctct aatgtaccag cagctggaat ctgaaggcgt gagtctccat cttagagcat    1200

cactcttcct cacaccacaa atctggtgcc tgtctcttgc ttaccaatgt ctaaggtccc    1260

cactgcctgc tgcagagaaa acacactcct ttgcttagcc cacaattctc tatttcactt    1320

gacccctgcc cacctctcca acctaactgg cttacttcct agtctacttg aggctgcaat    1380

cacactgagg aactcacaat tccaaacata caagaggctc tctcttaaca cggcacttag    1440

acacgtgctg ttccaccttc cctcgtgctg ttccaccttt cctcagacta tttttcagcc    1500

ttctggcatc agcaaacctt ataaaatttt tttgatttca gtgtagttct ctcctcttca    1560

aataaacatg tctgccttca aaaaaaaaaa aaaaaaaaaa aaaaaaaaaa               1610


<210>  136
<211>  1596
<212>  DNA
<213>  Homo sapiens

<400> 136
ctgtgcgctg ctgagctgag ctggggcgcg gccgcctgtc tgcaccggca gcaccatgtc 60

gctcatggtc atcagcatgg cgtgtgttgg gttcttcttg ctgcaggggg cctggacaca 120

tgagggtggt caggacaagc ccttgctgtc tgcctggccc agcgctgtgg tgcctcgagg 180

aggacatgtg actcttctgt gtcgctctcg tcttgggttt accatcttca gtctgtacaa 240

agaagatggg gtgcctgtcc ctgagctcta caacaaaata ttctggaaga gcatcctcat 300

gggccctgtg acccctgcac acgcagggac ctacagatgt cggggttcac acccacgctc 360

ccccattgag tggtcagcac ccagcaaccc cctggtgatc gtggtcacag gtctatttgg 420

gaaaccttca ctctcagccc agccgggccc cacggttcgc acaggagaga acgtgacctt 480

gtcctgcagc tccaggagct catttgacat gtaccatcta tccagggagg ggagggccca 540

tgaacctagg ctccctgcag tgcccagcgt caatggaaca ttccaggctg actttcctct 600

gggccctgcc acccacggag ggacctacac atgcttcggc tctctccatg actcacccta 660

tgagtggtca gacccgagtg acccactgct tgtttctgtc acaggaaact cttcaagtag 720

ttcatcttca cccactgaac caagctccaa aactggtatc cgcagacacc tgcacattct 780

gattgggacc tcagtggcta tcatcctctt catcatcctc ttcttctttc tccttcattg 840

ctgctgctcc aacaaaaaga atgctgctgt aatggaccaa gagcctgccg gggacagaac 900

agtgaacagg gaggactctg atgatcaaga ccctcaggag gtgacatatg cacagttgga 960

tcactgcgtt ttcacacaga caaaaatcac ttccccttct cagaggccca agacacctcc 1020

aacagatacc accatgtaca tggaacttcc aaatgctaag ccaagatcat tgtctcctgc 1080

ccataagcac cacagtcagg ccttgagggg atcttctagg gagacaacag ccctgtctca 1140

aaaccgggtt gctagctccc atgtaccagc agctggaatc tgaaggcatc agtcttcatc 1200

ttaggggatc gctcttcctc acaccacaaa tctgaacatg cctctctctt gcttacaaat 1260

gtctaaggtc cccactgcct gctggagaga agacacactc ctttgcttag cccacaattc 1320

tctatttcac ttgacccctg cccacctctc caactgaact ggcttacttc ctagtctact 1380

tgaggctgca atcacactga ggaactcaca attccagaca tacaagaggc tccctcttaa 1440

catggcactg agacacgtgc tgttccacct tccctcatgc tgtttcacct ttcctcagac 1500

tattttccag ccttctgtca gtcagcagtg aaacttataa aattttttgt gatttcaatg 1560

tagctgtctc cttttcaaat aaacatgtct gccctc 1596

<210> 137
<211> 2194
<212> DNA
<213> Homo sapiens

<400> 137
gggtctcgcc ggctcgccgc gctccccacc ttgcctgcgc ccgcccggag ccagcggttc 60

tccaagcacc cagcatcctg ctagacgcgc cgcgcaccga cggaggggac atgggcagag 120

caatggtggc caggctcggg ctggggctgc tgctgctggc actgctccta cccacgcaga 180

tttattccag tgaaacaaca actggaactt caagtaactc ctcccagagt acttccaact 240

ctgggttggc cccaaatcca actaatgcca ccaccaaggc ggctggtggt gccctgcagt 300

caacagccag tctcttcgtg gtctcactct ctcttctgca tctctactct taagagactc 360

aggccaagaa acgtcttcta aatttcccca tcttctaaac ccaatccaaa tggcgtctgg 420

aagtccaatg tggcaaggaa aaacaggtct tcatcgaatc tactaattcc acacctttta 480

ttgacacaga aaatgttgag aatcccaaat ttgattgatt tgaagaacat gtgagaggtt 540

tgactagatg atggatgcca atattaaatc tgctggagtt tcatgtacaa gatgaaggag 600

aggcaacatc caaaatagtt aagacatgat ttccttgaat gtggcttgag aaatatggac 660

acttaatact accttgaaaa taagaataga aataaaggat gggattgtgg aatggagatt 720

cagttttcat ttggttcatt aattctataa ggccataaaa caggtaatat aaaaagcttc 780

catgattcta tttatatgta catgagaagg aacttccagg tgttactgta attcctcaac 840

gtattgtttc gacagcacta atttaatgcc gatatactct agatgaagtt ttacattgtt 900

gagctattgc tgttctcttg ggaactgaac tcactttcct cctgaggctt tggatttgac 960

attgcatttg accttttatg tagtaattga catgtgccag ggcaatgatg aatgagaatc 1020

tacccccaga tccaagcatc ctgagcaact cttgattatc catattgagt caaatggtag 1080

gcatttccta tcacctgttt ccattcaaca agagcactac attcatttag ctaaacggat 1140

tccaaagagt agaattgcat tgaccacgac taatttcaaa atgcttttta ttattattat 1200

tttttagaca gtctcacttt gtcgcccagg ccggagtgca gtggtgcgat ctcagatcag 1260

tgtaccattt gcctcccggg ctcaagcgat tctcctgcct cagcctccca agtagctggg 1320

attacaggca cctgccacca tgcccggcta atttttgtaa ttttagtaga gacagggttt 1380

caccatgttg cccaggctgg tttcgaactc ctgacctcag gtgatccacc cgcctcggcc 1440

tcccaaagtg ctgggattac aggcttgagc ccccgcgccc agccatcaaa atgcttttta 1500

tttctgcata tgttgaatac tttttacaat ttaaaaaaat gatctgtttt gaaggcaaaa 1560

ttgcaaatct tgaaattaag aaggcaaaaa tgtaaaggag tcaaaactat aaatcaagta 1620

tttgggaagt gaagactgga agctaatttg cattaaattc acaaactttt atactctttc 1680

tgtatataca ttttttttct ttaaaaaaca actatggatc agaatagcca catttagaac 1740

actttttgtt atcagtcaat atttttagat agttagaacc tggtcctaag cctaaaagtg 1800

ggcttgattc tgcagtaaat cttttacaac tgcctcgaca cacataaacc tttttaaaaa 1860

tagacactcc ccgaagtctt ttgttcgcat ggtcacacac tgatgcttag atgttccagt 1920

aatctaatat ggccacagta gtcttgatga ccaaagtcct ttttttccat ctttagaaaa 1980

ctacatggga acaaacagat cgaacagttt tgaagctact gtgtgtgtga atgaacactc 2040

ttgctttatt ccagaatgct gtacatctat tttggattgt atattgtgtt tgtgtattta 2100

cgctttgatt catagtaact tcttatggaa ttgatttgca ttgaacacaa actgtaaata 2160

aaaagaaatg gctgaaagag caaaaaaaaa aaaa 2194

<210> 138
<211> 1578
<212> DNA
<213> Homo sapiens

<400> 138
agttctaaag tccccacgca cccacccgga ctcagagtct cctcagacgc cgagatgctg 60

gtcatggcgc cccgaaccgt cctcctgctg ctctcggcgg ccctggccct gaccgagacc 120

tgggccggct cccactccat gaggtatttc tacacctccg tgtcccggcc cggccgcggg 180

gagccccgct tcatctcagt gggctacgtg gacgacaccc agttcgtgag gttcgacagc 240

gacgccgcga gtccgagaga ggagccgcgg gcgccgtgga tagagcagga ggggccggag 300

tattgggacc ggaacacaca gatctacaag gcccaggcac agactgaccg agagagcctg 360

cggaacctgc gcggctacta caaccagagc gaggccgggt ctcacaccct ccagagcatg 420

tacggctgcg acgtggggcc ggacgggcgc ctcctccgcg ggcatgacca gtacgcctac 480

gacggcaagg attacatcgc cctgaacgag gacctgcgct cctggaccgc cgcggacacg 540

gcggctcaga tcacccagcg caagtgggag gcggcccgtg aggcggagca gcggagagcc 600

tacctggagg gcgagtgcgt ggagtggctc cgcagatacc tggagaacgg gaaggacaag 660

ctggagcgcg ctgacccccc aaagacacac gtgacccacc accccatctc tgaccatgag 720

gccaccctga ggtgctgggc cctgggtttc taccctgcgg agatcacact gacctggcag 780

cgggatggcg aggaccaaac tcaggacact gagcttgtgg agaccagacc agcaggagat 840

agaaccttcc agaagtgggc agctgtggtg gtgccttctg gagaagagca gagatacaca 900

tgccatgtac agcatgaggg gctgccgaag cccctcaccc tgagatggga gccgtcttcc 960

cagtccaccg tccccatcgt gggcattgtt gctggcctgg ctgtcctagc agttgtggtc 1020

atcggagctg tggtcgctgc tgtgatgtgt aggaggaaga gttcaggtgg aaaaggaggg 1080

agctactctc aggctgcgtg cagcgacagt gcccagggct ctgatgtgtc tctcacagct 1140

tgaaaagcct gagacagctg tcttgtgagg gactgagatg caggatttct tcacgcctcc 1200

cctttgtgac ttcaagagcc tctggcatct ctttctgcaa aggcacctga atgtgtctgc 1260

gtccctgtta gcataatgtg aggaggtgga gagacagccc acccttgtgt ccactgtgac 1320

ccctgttccc atgctgacct gtgtttcctc cccagtcatc tttcttgttc cagagaggtg 1380

gggctggatg tctccatctc tgtctcaact ttacgtgcac tgagctgcaa cttcttactt 1440

ccctactgaa aataagaatc tgaatataaa tttgttttct caaatatttg ctatgagagg 1500

ttgatggatt aattaaataa gtcaattcct ggaatttgag agagcaaata aagacctgag 1560

aaccttccag aaaaaaaa 1578

<210> 139
<211> 1543
<212> DNA
<213> Homo sapiens

<400> 139
ggccgagatg cgggtcatgg cgccccgagc cctcctcctg ctgctctcgg gaggcctggc 60

cctgaccgag acctgggcct gctcccactc catgaggtat ttcgacaccg ccgtgtcccg 120

gcccggccgc ggagagcccc gcttcatctc agtgggctac gtggacgaca cgcagttcgt 180

gcggttcgac agcgacgccg cgagtccgag aggggagccg cgggcgccgt gggtggagca 240

ggaggggccg gagtattggg accgggagac acagaagtac aagcgccagg cacaggctga 300

ccgagtgagc ctgcggaacc tgcgcggcta ctacaaccag agcgaggacg ggtctcacac 360

cctccagagg atgtctggct gcgacctggg gcccgacggg cgcctcctcc gcgggtatga 420

ccagtccgcc tacgacggca aggattacat cgccctgaac gaggacctgc gctcctggac 480

cgccgcggac accgcggctc agatcaccca gcgcaagttg gaggcggccc gtgcggcgga 540

gcagctgaga gcctacctgg agggcacgtg cgtggagtgg ctccgcagat acctggagaa 600

cgggaaggag acgctgcagc gcgcagaacc cccaaagaca cacgtgaccc accaccccct 660

ctctgaccat gaggccaccc tgaggtgctg ggccctgggc ttctaccctg cggagatcac 720

actgacctgg cagcgggatg gggaggacca gacccaggac accgagcttg tggagaccag 780

gccagcagga gatggaacct tccagaagtg ggcagctgtg gtggtgcctt ctggacaaga 840

gcagagatac acgtgccata tgcagcacga ggggctgcaa gagcccctca ccctgagctg 900

ggagccatct tcccagccca ccatccccat catgggcatc gttgctggcc tggctgtcct 960

ggttgtccta gctgtccttg gagctgtggt caccgctatg atgtgtagga ggaagagctc 1020

aggtggaaaa ggagggagct gctctcaggc tgcgtgcagc aacagtgccc agggctctga 1080

tgagtctctc atcacttgta aagcctgaga cagctgcctg tgtgggactg agatgcagga 1140

tttcttcaca cctctccttt gtgacttcaa gagcctctgg catctctttc tgcaaaggca 1200

cctgaatgtg tctgcgttcc tgttagcata atgtgaggag gtggagagac agcccacccc 1260

cgtgtccacc gtgacccctg tccccacact gacctgtgtt ccctccccga tcatctttcc 1320

tgttccagag aggtggggct ggatgtctcc atctctgtct caaattcatg gtgcactgag 1380

ctgcaacttc ttacttccct aatgaagtta agaacctgaa tataaatttg tgttctcaaa 1440

tatttgctat gaagcgttga tggattaatt aaataagtca attcctagaa gttgagagag 1500

caaataaaga cctgagaacc ttccaaaaaa aaaaaaaaaa aaa 1543

<210> 140
<211> 1815
<212> DNA
<213> Homo sapiens

<400> 140
cttagctgaa gatgactgac agtgttattt attccatgtt agagttgcct acggcaaccc 60

aagcccagaa tgactatgga ccacagcaaa aatcttcctc ttccaggcct tcttgttctt 120

gccttgtggc aatagctttg gggcttctga ctgcagttct tctgagtgtg ctgctatacc 180

agtggatcct gtgccagggc tccaactact ccacttgtgc cagctgtcct agctgcccag 240

accgctggat gaaatatggt aaccattgtt attatttctc agtggaggaa aaggactgga 300

attctagtct ggaattctgc ctagccagag actcacacct ccttgtgata acggacaatc 360

aggaaatgag cctgctccaa gttttcctca gtgaggcctt ttgctggatt ggtctgagga 420

acaattctgg ctggaggtgg gaagatggat cacctctaaa cttctcaagg atttcttcta 480

atagctttgt gcagacatgc ggtgccatca acaaaaatgg tcttcaagcc tcaagctgtg 540

aagttccttt acactgggtg tgtaagaagg tcagactttg atagatgacc actctgtcct 600

gaccctcaga tctgtcatgt atccctaaaa ggagggagct ggccactggc tgttgggaaa 660

gccatgagta tatagttagc aaatactgaa ctttctcaga tatggcatta gatgcaagac 720

aacctcctag ggattgatgc ctaactgatg gattctcttt gagactattt agatattatg 780

tgagcaattt aaagaccaga tctaagcaaa ttttgaaata gatgtttgtt ttttgtattt 840

ctcagtatgg aaactaatgc tgccactctc atccccgtcc caaccatctc tgtcaaaaat 900

ataccttttt catatgatat tctgagctaa tctgataaaa tctatgccaa tatatactat 960

tgcttgtgta ctagagaagt acattattgc tgtactcctc tgtacattac tgatccctga 1020

tggtatattt ctatcctaac agtgtccctt tgcagatcaa gctttattct gaagaataaa 1080

cctagctggc atgctggtgt gtacctgtag tcctagctat ttgggaagct gaggtgggag 1140

ggtcgcttga gcccaggagt ttgaggctgc agtgagctat gattgtgcca ctgtactcca 1200

gcctgggaga tagagcaaga ctccatctct aaaaaaaaaa aaaaatgcta atgtgagaat 1260

ataaattgtg ggaaatgagt gagggcaagg tggtacttcc tccttctgag ctcttcacac 1320

gtaatgcaaa aacccggtct taactgattt tgtttttttt ctgagtatgc atatatgtgg 1380

ttgaatgaac caatgtgtga ttgtatcttt tccattatgt gactgtttga cctgcatatt 1440

aatttcaaga tagcagtcaa ttcgataagg cattttcata gaggaaagtt tacagaaaca 1500

gtttatgtgg ttggatcacc aaattatctt aggtactaag gcctcaaaaa taagaaaaac 1560

tttattattt ctcctcagta gagtttggac atacataagg agagaaggta cagtgatgaa 1620

ggagaccata attctgtagt gttgatgatc ctggattata atctttttct ctttatcttt 1680

catagttttt ttaaaaacat ggactgtatc ttatctacca ctatatccca aatacctaag 1740

atagtgctta cgttcagtga ctattaaata aataaatgga tgaattaaaa aaaaaaaaaa 1800

aaaaaaaaaa aaaaa 1815

<210> 141
<211> 1578
<212> DNA
<213> Homo sapiens

<400> 141
agtgtggtac tttgtcttga ggagatgtcc tggactcaca cggaaactta gggctacgga 60

atgaagttct cactcccatt aggtgacagg tttttagaga agccaatcag cgtcgccgcg 120

gtcctggttc taaagtcctc gctcacccac ccggactcat tctccccaga cgccaaggat 180

ggtggtcatg gcgccccgaa ccctcttcct gctgctctcg ggggccctga ccctgaccga 240

gacctgggcg ggctcccact ccatgaggta tttcagcgcc gccgtgtccc ggcccggccg 300

cggggagccc cgcttcatcg ccatgggcta cgtggacgac acgcagttcg tgcggttcga 360

cagcgactcg gcgtgtccga ggatggagcc gcgggcgccg tgggtggagc aggaggggcc 420

ggagtattgg gaagaggaga cacggaacac caaggcccac gcacagactg acagaatgaa 480

cctgcagacc ctgcgcggct actacaacca gagcgaggcc agttctcaca ccctccagtg 540

gatgattggc tgcgacctgg ggtccgacgg acgcctcctc cgcgggtatg aacagtatgc 600

ctacgatggc aaggattacc tcgccctgaa cgaggacctg cgctcctgga ccgcagcgga 660

cactgcggct cagatctcca agcgcaagtg tgaggcggcc aatgtggctg aacaaaggag 720

agcctacctg gagggcacgt gcgtggagtg gctccacaga tacctggaga acgggaagga 780

gatgctgcag cgcgcggacc cccccaagac acacgtgacc caccaccctg tctttgacta 840

tgaggccacc ctgaggtgct gggccctggg cttctaccct gcggagatca tactgacctg 900

gcagcgggat ggggaggacc agacccagga cgtggagctc gtggagacca ggcctgcagg 960

ggatggaacc ttccagaagt gggcagctgt ggtggtgcct tctggagagg agcagagata 1020

cacgtgccat gtgcagcatg aggggctgcc ggagcccctc atgctgagat ggaagcagtc 1080

ttccctgccc accatcccca tcatgggtat cgttgctggc ctggttgtcc ttgcagctgt 1140

agtcactgga gctgcggtcg ctgctgtgct gtggagaaag aagagctcag attgaaaagg 1200

agggagctac tctcaggctg caatgtgaaa cagctgccct gtgtgggact gagtggcaag 1260

tccctttgtg acttcaagaa ccctgactcc tctttgtgca gagaccagcc cacccctgtg 1320

cccaccatga ccctcttcct catgctgaac tgcattcctt ccccaatcac ctttcctgtt 1380

ccagaaaagg ggctgggatg tctccgtctc tgtctcaaat ttgtggtcca ctgagctata 1440

acttacttct gtattaaaat tagaatctga gtataaattt actttttcaa attatttcca 1500

agagagattg atgggttaat taaaggagaa gattcctgaa atttgagaga caaaataaat 1560

ggaagacatg agaacttt 1578

<210> 142
<211> 1741
<212> DNA
<213> Homo sapiens

<400> 142
ccggcagcac catgttgctc atggtcgtca gcatggcgtg tgttgggttg ttcttggtcc 60

agagggccgg tccacacatg ggtggtcagg acaagccctt cctgtctgcc tggcccagcg 120

ctgtggtgcc tcgcggagga cacgtgactc ttcggtgtca ctatcgtcat aggtttaaca 180

atttcatgct atacaaagaa gacagaatcc acgttcccat cttccatggc agaatattcc 240

aggagggctt caacatgagc cctgtgacca cagcacatgc agggaactac acatgtcggg 300

gttcacaccc acactccccc actgggtggt cggcacccag caaccccatg gtgatcatgg 360

tcacaggaaa ccacagaaaa ccttccctcc tggcccaccc aggtcccctg gtgaaatcag 420

gagagagagt catcctgcaa tgttggtcag atatcatgtt tgagcacttc tttctgcaca 480

aagagtggat ctctaaggac ccctcacgcc tcgttggaca gatccatgat ggggtctcca 540

aggccaattt ctccatcggt tccatgatgc gtgcccttgc agggacctac agatgctacg 600

gttctgttac tcacaccccc tatcagttgt cagctcccag tgatcccctg gacatcgtgg 660

tcacaggtct atatgagaaa ccttctctct cagcccagcc gggccccaag gttcaggcag 720

gagagagcgt gaccttgtcc tgtagctccc ggagctccta tgacatgtac catctatcca 780

gggagggggg agcccatgaa cgtaggctcc ctgcagtgcg caaggtcaac agaacattcc 840

aggcagattt ccctctgggc cctgccaccc acggagggac ctacagatgc ttcggctctt 900

tccgtcactc tccctacgag tggtcagacc cgagtgaccc actgcttgtt tctgtcacag 960

gaaacccttc aagtagttgg ccttcaccca cagaaccaag ctccaaatct ggtaacctca 1020

gacacctgca cattctgatt gggacctcag tggtcaaaat ccctttcacc atcctcctct 1080

tctttctcct tcatcgctgg tgctccaaca aaaaaaaatg ctgctgtaat ggaccaagag 1140

cctgcaggga acagaagtga acagcgagga ttctgatgaa caagaccatc aggaggtgtc 1200

atacgcataa ttggaacact gtgttttcac acagagaaaa atcactcgcc cttctcagag 1260

gcccaagaca cccccaacag ataccagcat gtacatagaa cttccaaatg ctgagcccag 1320

atccaaagtt gtcttctgtc cacgagcacc acagtcaggc cttgagggga tcttctaggg 1380

agacaacagc cctgtctcaa aactgggttg ccagctccca tgtaccagca gctggaatct 1440

gaaggcatca gtcttcatct tagggcatcg ctcttcctca caccacaaat ctgaatgtgc 1500

ctctcacttg cttacaaatg tctaaggtcc ccactgcctg ctggagaaaa aacacactcc 1560

tttgcttagc ccacagttct ccatttcact tgacccctgc ccacctctcc aacctaactg 1620

gcttacttcc tagtctactt gaggctgcaa tcacactgag gaactcacaa ttccacacat 1680

acaagaggct ccgtcttaac gcagcactta gacacgtgct gttccacctt ccctcatgct 1740

g 1741

<210> 143
<211> 1986
<212> DNA
<213> Homo sapiens

<400> 143
ataacatcct gtgcgctgct gagctgagct ggggcgcagc cgcctgtctg caccggcagc 60

accatgtcgc tcatggtcgt cagcatggcg tgtgttgggt tgttcttggt ccagagggcc 120

ggtccacaca tgggtggtca ggacaaaccc ttcctgtctg cctggcccag cgctgtggtg 180

cctcgaggag gacacgtgac tcttcggtgt cactatcgtc ataggtttaa caatttcatg 240

ctatacaaag aagacagaat ccacattccc atcttccatg gcagaatatt ccaggagagc 300

ttcaacatga gccctgtgac cacagcacat gcagggaact acacatgtcg gggttcacac 360

ccacactccc ccactgggtg gtcggcaccc agcaaccccg tggtgatcat ggtcacagga 420

aaccacagaa aaccttccct cctggcccac ccaggtcccc tggtgaaatc aggagagaga 480

gtcatcctgc aatgttggtc agatatcatg tttgagcact tctttctgca caaagagggg 540

atctctaagg acccctcacg cctcgttgga cagatccatg atggggtctc caaggccaat 600

ttctccatcg gtcccatgat gcttgccctt gcagggacct acagatgcta cggttctgtt 660

actcacaccc cctatcagtt gtcagctccc agtgatcccc tggacatcgt ggtcacaggt 720

ccatatgaga aaccttctct ctcagcccag ccgggcccca aggttcaggc aggagagagc 780

gtgaccttgt cctgtagctc ccggagctcc tatgacatgt accatctatc cagggagggg 840

ggagcccatg aacgtaggct ccctgcagtg cgcaaggtca acagaacatt ccaggcagat 900

ttccctctgg gccctgccac ccacggaggg acctacagat gcttcggctc tttccgtcac 960

tctccctacg agtggtcaga cccgagtgac ccactgcttg tttctgtcac aggaaaccct 1020

tcaagtagtt ggccttcacc cacagaacca agctccaaat ctggtaaccc cagacacctg 1080

cacattctga ttgggacctc agtggtcatc atcctcttca tcctcctcct cttctttctc 1140

cttcatctct ggtgctccaa caaaaaaaat gctgctgtaa tggaccaaga gcctgcaggg 1200

aacagaacag ccaacagcga ggactctgat gaacaagacc ctgaggaggt gacatacgca 1260

cagttggatc actgcgtttt cacacagaga aaaatcactc gcccttctca gaggcccaag 1320

acacccccta cagataccat cttgtacacg gaacttccaa atgctaagcc cagatccaaa 1380

gttgtctcct gcccatgagc accacagtca ggccttgagg acgtcttcta gggagacaac 1440

agccctgtct caaaaccgag ttgccagctc ccatgtacca gcagctggaa tctgaaggcg 1500

tgagtcttca tcttagggca tcgctcctcc tcacgccaca aatctggtgc ctctctcttg 1560

cttacaaatg tctaggtccc cactgcctgc tggaaagaaa acacactcct ttgcttagcc 1620

cacagttctc catttcactt gacccctgcc cacctctcca acctaactgg cttacttcct 1680

agtctacttg aggctgcaat cacactgagg aactcacaat tccaaacata caagaggctc 1740

cctcttgacg tggcacttac ccacgtgctg ttccaccttc cctcatgctg tttcaccttt 1800

cttcggacta ttttccagcc ttctgtcagc agtgaaactt ataaaatttt ttgtgatttc 1860

aatgtagctg tctcctcttc aaataaacat gtctgccctc aaaaaaaaaa aaaaaaaaaa 1920

aaaaaaaaaa aaaaaaaaaa aaaaaaaaaa aaaaaaaaaa aaaaaaaaaa aaaaaaaaaa 1980

aaaaaa 1986

<210> 144
<211> 1574
<212> DNA
<213> Homo sapiens

<400> 144
agattctccc cagacgccga ggatggccgt catggcgccc cgaaccctcc tcctgctact 60

ctcgggggcc ctggccctga cccagacctg ggcgggctcc cactccatga ggtatttctt 120

cacatccgtg tcccggcccg gccgcgggga gccccgcttc atcgccgtgg gctacgtgga 180

cgacacgcag ttcgtgcggt tcgacagcga cgccgcgagc cagaggatgg agccgcgggc 240

gccgtggata gagcaggagg ggccggagta ttgggaccag gagacacgga atgtgaaggc 300

ccagtcacag actgaccgag tggacctggg gaccctgcgc ggctactaca accagagcga 360

ggccggttct cacaccatcc agataatgta tggctgcgac gtggggtcgg acgggcgctt 420

cctccgcggg taccggcagg acgcctacga cggcaaggat tacatcgccc tgaacgagga 480

cctgcgctct tggaccgcgg cggacatggc ggctcagatc accaagcgca agtgggaggc 540

ggcccatgag gcggagcagt tgagagccta cctggatggc acgtgcgtgg agtggctccg 600

cagatacctg gagaacggga aggagacgct gcagcgcacg gaccccccca agacacatat 660

gacccaccac cccatctctg accatgaggc caccctgagg tgctgggccc tgggcttcta 720

ccctgcggag atcacactga cctggcagcg ggatggggag gaccagaccc aggacacgga 780

gctcgtggag accaggcctg caggggatgg aaccttccag aagtgggcgg ctgtggtggt 840

gccttctgga gaggagcaga gatacacctg ccatgtgcag catgagggtc tgcccaagcc 900

cctcaccctg agatgggagc tgtcttccca gcccaccatc cccatcgtgg gcatcattgc 960

tggcctggtt ctccttggag ctgtgatcac tggagctgtg gtcgctgccg tgatgtggag 1020

gaggaagagc tcagatagaa aaggagggag ttacactcag gctgcaagca gtgacagtgc 1080

ccagggctct gatgtgtccc tcacagcttg taaagtgtga gacagctgcc ttgtgtggga 1140

ctgagaggca agagttgttc ctgcccttcc ctttgtgact tgaagaaccc tgactttgtt 1200

tctgcaaagg cacctgcatg tgtctgtgtt cgtgtaggca taatgtgagg aggtggggag 1260

accaccccac ccccatgtcc accatgaccc tcttcccacg ctgacctgtg ctccctcccc 1320

aatcatcttt cctgttccag agaggtgggg ctgaggtgtc tccatctctg tctcaacttc 1380

atggtgcact gagctgtaac ttcttccttc cctattaaaa ttagaacctt agtataaatt 1440

tactttctca aattcttgcc atgagaggtt gatgagttaa ttaaaggaga agattcctaa 1500

aatttgagag acaaaataaa tggaagacat gagaaccttc cagagtccaa aaaaaaaaaa 1560

aaaaaaaaaa aaaa 1574

<210> 145
<211> 444
<212> PRT
<213> Homo sapiens

<400> 145

Met Ser Leu Met Val Val Ser Met Ala Cys Val Gly Leu Phe Leu Val
1               5                   10                  15

Gln Arg Ala Gly Pro His Met Gly Gly Gln Asp Lys Pro Phe Leu Ser
            20                  25                  30

Ala Trp Pro Ser Ala Val Val Pro Arg Gly Gly His Val Thr Leu Arg
        35                  40                  45

Cys His Tyr Arg His Arg Phe Asn Asn Phe Met Leu Tyr Lys Glu Asp
    50                  55                  60

Arg Ile His Ile Pro Ile Phe His Gly Arg Ile Phe Gln Glu Ser Phe
65                  70                  75                  80

Asn Met Ser Pro Val Thr Thr Ala His Ala Gly Asn Tyr Thr Cys Arg
                85                  90                  95

Gly Ser His Pro His Ser Pro Thr Gly Trp Ser Ala Pro Ser Asn Pro
            100                 105                 110

Val Val Ile Met Val Thr Gly Asn His Arg Lys Pro Ser Leu Leu Ala
        115                 120                 125

His Pro Gly Pro Leu Val Lys Ser Gly Glu Arg Val Ile Leu Gln Cys

130                 135                 140

Trp Ser Asp Ile Met Phe Glu His Phe Phe Leu His Lys Glu Gly Ile
145                 150                 155                 160

Ser Lys Asp Pro Ser Arg Leu Val Gly Gln Ile His Asp Gly Val Ser
                165                 170                 175

Lys Ala Asn Phe Ser Ile Gly Pro Met Met Leu Ala Leu Ala Gly Thr
            180                 185                 190

Tyr Arg Cys Tyr Gly Ser Val Thr His Thr Pro Tyr Gln Leu Ser Ala
        195                 200                 205

Pro Ser Asp Pro Leu Asp Ile Val Val Thr Gly Pro Tyr Glu Lys Pro
    210                 215                 220

Ser Leu Ser Ala Gln Pro Gly Pro Lys Val Gln Ala Gly Glu Ser Val
225                 230                 235                 240

Thr Leu Ser Cys Ser Ser Arg Ser Ser Tyr Asp Met Tyr His Leu Ser
                245                 250                 255

Arg Glu Gly Gly Ala His Glu Arg Arg Leu Pro Ala Val Arg Lys Val
            260                 265                 270

Asn Arg Thr Phe Gln Ala Asp Phe Pro Leu Gly Pro Ala Thr His Gly
        275                 280                 285

Gly Thr Tyr Arg Cys Phe Gly Ser Phe Arg His Ser Pro Tyr Glu Trp
    290                 295                 300

Ser Asp Pro Ser Asp Pro Leu Leu Val Ser Val Thr Gly Asn Pro Ser
305                 310                 315                 320

Ser Ser Trp Pro Ser Pro Thr Glu Pro Ser Ser Lys Ser Gly Asn Pro
                325                 330                 335

Arg His Leu His Ile Leu Ile Gly Thr Ser Val Val Ile Ile Leu Phe
            340                 345                 350

Ile Leu Leu Leu Phe Phe Leu Leu His Leu Trp Cys Ser Asn Lys Lys
        355                 360                 365

Asn Ala Ala Val Met Asp Gln Glu Pro Ala Gly Asn Arg Thr Ala Asn
    370                 375                 380

Ser Glu Asp Ser Asp Glu Gln Asp Pro Glu Glu Val Thr Tyr Ala Gln
385                 390                 395                 400

Leu Asp His Cys Val Phe Thr Gln Arg Lys Ile Thr Arg Pro Ser Gln
                405                 410                 415

Arg Pro Lys Thr Pro Pro Thr Asp Thr Ile Leu Tyr Thr Glu Leu Pro
            420                 425                 430

Asn Ala Lys Pro Arg Ser Lys Val Val Ser Cys Pro
        435                 440

<210>  146
<211>  1691
<212>  DNA
<213>  Homo sapiens

<400>  146
gtctgcaccg gcagcaccat gtcgctcatg gtcgtcagca tggcgtgtgt tgggttcttc      60

ttgctggagg ggccctggcc acatgtgggt ggtcaggaca agcccttcct ctctgcctgg     120

cccggcactg tggtgtctga aggacaacat gtgactcttc agtgtcgctc tcgtcttggg     180

tttaacgaat tcagtctgtc caaagaagac gggatgcctg tccctgagct ctacaacaga     240

atattccgga acagctttct catgggccct gtgaccccag cacatgcagg gacctacaga     300

tgttgcagtt cacacccaca ctcccccact gggtggtcgg cacccagcaa ccctgtggtg     360

atcatggtca caggagtcca cagaaaacct tccctcctgg cccacccagg tcccctggtg     420

aaatcgggag agacggtcat cctgcaatgt tggtcagatg tcaggtttga gcgcttcctt     480

ctgcacagag aggggatcac tgaggacccc ttgcgcctcg ttggacagct ccacgatgcg     540

ggttcccagg tcaactattc catgggtccc atgacacctg cccttgcagg gacctacaga     600

tgctttggtt ctgtcactca cttaccctat gagttgtcgg ctcccagtga ccctctggac     660

atcgtggtcg taggtctata tgggaaacct tctctctcag cccagccggg ccccacggtt     720

caggcaggag agaatgtgac cttgtcctgc agctcccgga gcttgtttga catttaccat     780

ctatccaggg aggcagaggc cggtgaactt aggctcactg cggtgctgag ggtcaatgga     840

acattccagg ccaacttccc tctgggccct gtgacccacg gagggaacta cagatgcttc     900

ggctctttcc gtgccctgcc ccacgcgtgg tcagacccga gtgacccact gcccgtttct     960

gtcacaggta actccagaca cctgcacgtt ctgattggga cctcagtggt catcatcccc    1020

tttgctatcc tcctcttctt tctccttcat cgctggtgtg ccaacaaaaa gaatgctgtt    1080

gtaatggacc aagagcctgc agggaacaga acagtgaaca gggaggactc tgatgaacaa    1140

gaccctcagg aggtgacata cgcacagttg aatcactgcg ttttcacaca gagaaaaatc    1200

actcgccctt ctcagaggcc caagacaccc ccaacagata ccagcgtgta acacggaact 1260

tccaaatgct gagcgcagat ccaaagttgt cttctgtcca ctagcaccac agtcaggcct 1320

tgatgggatc ttctagggag acaatagccc tgtctcaaaa ccgggttgcc agctcccatg 1380

taccagcagc tggactctga aggcgtgagt ctgcatctta gggcatcgct cttcctcaca 1440

ccacgaatct gaacatgcct ctctcttgct tacaaatgtc taaggtcccc actgcctgct 1500

ggagagaaaa cacacttgct tagcccacaa ttctccattt cacttgaccc ctgcccacct 1560

ctccaaccta actggcttac ttcctagtct acttgaggct gcgatcacac tgaggaactc 1620

acaattccaa acatataaga ggctccctct taacacggca cttagatacg tgctattcca 1680

cctttcctca g 1691

<210> 147
<211> 1294
<212> DNA
<213> Homo sapiens

<400> 147
catgtcgctc atggtcatca gcatggcgtg tgttgggttc ttcttgctgc agggggcctg 60

gacacatgag ggtggtcagg acaagccctt cctctctgcc tggcccagcc ctgtggtgtc 120

tgaaggagaa catgtggctc ttcagtgtcg ctctcgtctt gggtttaacg aattcagtct 180

gtccaaagaa gacgggatgc ctgtccctga gctctacaac agagtattcc gaaacaccgt 240

tttcataggc cctgtgaccc cagcacatgc agggacctac agatgtcggg gttcacaccc 300

acacttcctc actgggtggt cagcacccag caaccccctg gtgatcatgg tcacaggagt 360

ccacagaaaa ccttccctcc tggcccaccc aggtcccctg gtgaaatcag aagagacagt 420

catcctgcaa tgttggtcag atgtcatgtt tgagcacttc cttctgcaca gagaggggaa 480

gtttaatgac actttgcgcc tcactggaga gctccatgat ggggtctcca aggccaactt 540

ctccatcggt cgcatgacgc aagaccttgc agggacctac agatgctacg gttctgttcc 600

tcattccccc tatcagttgt cagctcccag tgaccctctg gacatcgtga ttacaggtct 660

atgtgggaaa ccttctctct cagcccagcc gcgccccatg gttaaggcag gagagagcgt 720

gaccttgtcc tgcagctccc ggagctccta tgacatctac catctatcaa gggaggggga 780

ggctcatgaa cttaggttcc ctgcagtgcc caaggtcaat ggaaccttcc aggccaactt 840

tcctctgggc cctgccaccc acggagggac ctacagatgc ttcggctctt tccgtgactc 900

tccctacgag tggtcagacc ttagtgaccc actgcttgtt tctgtcacag attctatgaa 960

ggagaaagga aaagatgtga tactgtaatt ttgctccatt tgtctaaaat gagtaggctg 1020

caactcctct tgaagtgata ccttttctag ctcttgttgg aggtgtctca ggactcatta 1080

cttcggggaa cctgcaactg tgtcagtctg gggaaactgc aaatattctt gtcttacatt 1140

tgtctccagc caattgtgat ggactccagt gacctgcaat tgctgttatt gcaggtaaaa 1200

tgtacctgag tcaggccaca gttctcctgg actatgagcc cctggccatg ttcctgaggc 1260

aattctgttc atctaaatat aataataata acac 1294

<210> 148
<211> 375
<212> PRT
<213> Homo sapiens

<400> 148
   Met Ser Leu Met Val Ile Ser Met Ala Cys Val Gly Phe Phe Leu Leu
   1 5 10 15
   Gln Gly Ala Trp Thr His Glu Gly Gly Gln Asp Lys Pro Leu Leu Ser
   20 25 30
   Ala Trp Pro Ser Ala Val Val Pro Arg Gly Gly His Val Thr Leu Leu
   35 40 45
   Cys Arg Ser Arg Leu Gly Phe Thr Ile Phe Ser Leu Tyr Lys Glu Asp
   50 55 60
   Gly Val Pro Val Pro Glu Leu Tyr Asn Lys Ile Phe Trp Lys Ser Ile
   65 70 75 80
   Leu Met Gly Pro Val Thr Pro Ala His Ala Gly Thr Tyr Arg Cys Arg
   85 90 95
   Gly Ser His Pro Arg Ser Pro Ile Glu Trp Ser Ala Pro Ser Asn Pro
   100 105 110
   Leu Val Ile Val Val Thr Gly Leu Phe Gly Lys Pro Ser Leu Ser Ala
   115 120 125
   Gln Pro Gly Pro Thr Val Arg Thr Gly Glu Asn Val Ala Leu Ser Cys
   130 135 140
   Ser Ser Arg Ser Ser Phe Asp Met Tyr His Leu Ser Arg Glu Gly Arg
   145 150 155 160
   Ala His Glu Pro Arg Leu Pro Ala Val Pro Ser Val Asp Gly Thr Phe
   165 170 175
   Gln Ala Asp Phe Pro Leu Gly Pro Ala Thr His Gly Gly Thr Tyr Thr
   180 185 190
   Cys Phe Ser Ser Leu His Asp Ser Pro Tyr Glu Trp Ser Asp Pro Ser
   195                 200                 205
   Asp Pro Leu Leu Val Ser Val Thr Gly Asn Ser Ser Ser Ser Ser Ser
       210                 215                 220
   Ser Pro Thr Glu Pro Ser Ser Lys Thr Gly Ile Arg Arg His Leu His
   225                 230                 235                 240
   Ile Leu Ile Gly Thr Ser Val Ala Ile Ile Leu Phe Ile Ile Leu Phe
                   245                 250                 255
   Phe Phe Leu Leu His Cys Cys Cys Ser Asn Lys Lys Asn Ala Ala Val
               260                 265                 270
   Met Asp Gln Gly Pro Ala Gly Asp Arg Thr Val Asn Arg Glu Asp Ser
           275                 280                 285
   Asp Asp Gln Asp Pro Gln Glu Val Thr Tyr Ala Gln Leu Asp His Cys
       290                 295                 300
   Val Phe Thr Gln Thr Lys Ile Thr Ser Pro Ser Gln Arg Pro Lys Ala
   305                 310                 315                 320
   Pro Pro Thr Asp Thr Thr Met Tyr Met Glu Leu Pro Asn Ala Lys Pro
                   325                 330                 335
   Arg Ser Leu Ser Pro Ala His Lys His His Ser Gln Ala Leu Arg Gly
               340                 345                 350
   Ser Ser Arg Glu Thr Thr Ala Leu Ser Gln Asn Arg Val Ala Ser Ser
           355                 360                 365
   His Val Pro Ala Ala Gly Ile
       370                 375

<210>  149
<211>  1632
<212>  DNA
<213>  Homo sapiens

<400>  149
atgtcgctca tggtcatcag catggcgtgt gttgggttct tcttgctgca gggggcctgg      60

acacatgagg gtggacagga caagcccttg ctgtctgcct ggcccagcgc tgtggtgcct     120

cgaggaggac atgtgactct tctgtgtcgc tctcgtcttg ggtttaccat cttcagtctg     180

tacaaagaag atggggtgcc tgtccctgag ctctacaaca aaatattctg gaagagcatc     240

ctcatgggcc ctgtgacccc tgcacacgca gggacctaca gatgtcgggg ttcacacccg     300

cgctccccca ttgagtggtc ggcacccagc aaccccctgg tgatcgtggt cacaggtcta 360

tttgggaaac cttcactctc agcccagccg ggccccacgg ttcgcacagg agagaacgtg 420

gccttgtcct gcagctccag gagctcattt gacatgtacc atctatccag ggaggggagg 480

gcccatgaac ctaggctccc tgcagtgccc agcgtcgatg gaacattcca ggctgacttt 540

cctctgggcc ctgccaccca cggagggacc tacacatgct tcagctctct ccatgactca 600

ccctatgagt ggtcagaccc gagtgaccca ctgcttgttt ctgtcacagg aaactcttca 660

agtagttcat cttcacccac tgaaccaagc tccaaaactg gtatccgcag acacctgcac 720

attctgattg ggacctcagt ggctatcatc ctcttcatca tcctcttctt ctttctcctt 780

cattgctgct gctccaacaa aaagaatgct gctgtaatgg accaaggccc tgccggggac 840

agaacagtga acagggagga ctctgatgat caagaccctc aggaggtgac atatgcacag 900

ttggatcact gcgttttcac acagacaaaa atcacttccc cttctcagag gcccaaggca 960

cctccaacag ataccaccat gtacatggaa cttccaaatg ctaagccaag atcattgtct 1020

cctgcccata agcaccacag tcaggccttg aggggatctt ctagggagac aacagccctg 1080

tctcaaaacc gggttgctag ctcccatgta ccagcagctg gaatctgaag gcatcagtct 1140

tcatcttagg ggatcgctct tcctcacacc acaaatctga acatgcctct ctcttgctta 1200

caaatgtcta aggtccccac tgcctgctgg agagaagaca cacacctttg cttagcccac 1260

aattctctat ttcacttgac ccctgcccac ctctccaact gaactggctt acttcctagt 1320

ctacttgagg ctgcaatcac actgaggaac tcacaattcc agacatacaa gaggctccct 1380

cttaacatgg cactgagaca cgtgctgttc caccctccct catgctgttt cacctttcct 1440

cagactattt tccagccttc tgtcagtcag cagtgaaact tataaaattt tttgtgattt 1500

caatgtagct gtctcctttt caaataaaca tgtctgccct cattgcttta ggtaatgtga 1560

cactattcgc tgaaagaaac cgctgttatc attaccatgt ccacataacc ccatctgtta 1620

tccactgggt tc 1632

<210> 150
<211> 1559
<212> DNA
<213> Homo sapiens

<400> 150
tgtctgcaca gacagcacca tgtcgctcat ggtcgtcagc atggcgtgtg ttgggttctt 60

cttgctgcag ggggcctggc cacatgaggg agtccacaga aaaccttccc tcctggccca 120

cccaggtccc ctggtgaaat cagaagagac agtcatcctg caatgttggt cagatgtcag 180

gtttgagcac ttccttctgc acagagaggg gaagtataag gacactttgc acctcattgg 240

agagcaccat gatggggtct ccaaggccaa cttctccatc ggtcccatga tgcaagacct 300

tgcagggacc tacagatgct acggttctgt tactcactcc ccctatcagt tgtcagctcc 360

cagtgaccct ctggacatcg tcatcacagg tctatatgag aaaccttctc tctcagccca 420

gccgggcccc acggttttgg caggagagag cgtgaccttg tcctgcagct cccggagctc 480

ctatgacatg taccatctat ccagggaggg ggaggcccat gaacgtaggt tctctgcagg 540

gcccaaggtc aacggaacat tccaggccga ctttcctctg ggccctgcca cccacggagg 600

aacctacaga tgcttcggct ctttccgtga ctctccctat gagtggtcaa actcgagtga 660

cccactgctt gtttctgtca caggaaaccc ttcaaatagt tggccttcac ccactgaacc 720

aagctccaaa accggtaacc ccagacacct gcatgttctg attgggacct cagtggtcaa 780

aatccctttc accatcctcc tcttctttct ccttcatcgc tggtgctcca acaaaaaaaa 840

tgctgctgta atggaccaag agcctgcagg gaacagaaca gtgaacagcg aggattctga 900

tgaacaagac catcaggagg tgtcatacgc ataattggat cactgtgttt tcacacagag 960

aaaaatcact cgcccttctg agaggcccaa gacaccccca acagatacca gcatgtacat 1020

agaacttcca aatgctgagc ccagatccaa agttgtcttc tgtccacgag caccacagtc 1080

aggccttgag gggatcttct agggagacaa cagccctgtc tcaaaaccgg gttgccagct 1140

cccatgtacc agcagctgga atctgaaggc atcagtcttc atcttagggc atcgctcttc 1200

ctcacaccac gaatctgaac atgcctctct cttgcttaca aatgtctaag gtccccactg 1260

cctgctggag agaaaacaca ctcctttgct tagcccacaa ttctccattt cacttgaccc 1320

ctgcccacct ctccaaccta actggcttac ttcctagtct acctgaggct gcaatcacac 1380

tgaggaactc acaattccaa acatacaaga ggctgcctct taacacagca cttagacacg 1440

tgctgttcca cctcccttca gactatcttt cagccttctg ccagcagtaa aacttataaa 1500

ttttttaaat aatttcaatg tagttttccc gccttcaaat aaacatgtct gccctcatg 1559

<210> 151
<211> 1113
<212> DNA
<213> Homo sapiens

<400> 151
ccggcagcac catgtcgctc atggtcatca gcatggcatg tgttgggttc ttctggctgc 60

agggggcctg gccacatgag ggattccgca gaaaaccttc cctcctggcc cacccaggtc 120

gcctggtgaa atcagaagag acagtcatcc tgcaatgttg gtcagatgtc atgtttgagc 180

acttccttct gcacagagag gggacgttta acgacacttt gcgcctcatt ggagagcaca 240

ttgatggggt ctccaaggcc aacttctcca tcggtcgcat gaggcaagac ctggcaggga 300

cctacagatg ctacggttct gttcctcact ccccctatca gttttcagct cccagtgacc 360

ctctggacat cgtgatcaca ggtctatatg agaaaccttc tctctcagcc cagccgggcc 420

ccacggttct ggcaggagag agcgtgacct tgtcctgcag ctcctggagc tcctatgaca 480

tgtaccatct atccacggag ggggaggccc atgaacgtag gttctctgca gggcccaagg 540

tcaacggaac attccaggcc gactttcctc tgggccctgc cacccaagga ggaacctaca 600

gatgcttcgg ctctttccat gactctccct acgagtggtc aaagtcaagt gacccactgc 660

ttgtttctgt cacaggaaac ccttcaaata gttggccttc acccactgaa ccaagctcca 720

aaaccggtaa ccccagacac ctacacgttc tgattgggac ctcagtggtc aaactccctt 780

tcaccatcct cctcttcttt ctccttcatc gctggtgctc cgacaaaaaa aatgcatctg 840

taatggacca agggcctgcg gggaacagaa cagtgaacag ggaggattct gacgaacagg 900

accatcagga ggtgtcatac gcataattgg atcactgtgt tttcacacag agaaaaatca 960

ctcccccttc tcagaggccc aagacacccc caacagatag cagcatgtac atagaacttc 1020

caaatgctga gtccagatcc aaagctgtct tctgtccacg agcaccacag tcaggccttg 1080

aggggatctt ctagggagac aacagccctg tct 1113

<210> 152
<211> 1586
<212> DNA
<213> Homo sapiens

<400> 152
gctgagctga gctggggcgc ggccgcctgt ctgcaccggc agcaccatgt cgctcatggt 60

catcatcatg gcgtgtgttg ggttcttctt gctgcagggg gcctggccac aggagggagt 120

ccacagaaaa ccttccttcc tggccctccc aggtcacctg gtgaaatcag aagagacagt 180

catcctgcaa tgttggtcgg atgtcatgtt tgagcacttc cttctgcaca gagaggggaa 240

gtttaacaac actttgcacc tcattggaga gcaccatgat ggggtttcca aggccaactt 300

ctccattggt cccatgatgc ctgtccttgc aggaacctac agatgctacg gttctgttcc 360

tcactccccc tatcagttgt cagctcccag tgaccctctg gacatggtga tcataggtct 420

atatgagaaa ccttctctct cagcccagcc gggccccacg gttcaggcag gagagaatgt 480

gaccttgtcc tgcagctccc ggagctccta tgacatgtac catctatcca gggaagggga 540

ggcccatgaa cgtaggctcc ctgcagtgcg cagcatcaac ggaacattcc aggccgactt 600

tcctctgggc cctgccaccc acggagggac ctacagatgc ttcggctctt tccgtgacgc 660

tccctacgag tggtcaaact cgagtgatcc actgcttgtt tccgtcacag gaaacccttc 720

aaatagttgg ccttcaccca ctgaaccaag ctccaaaacc ggtaacccca gacacctaca 780

tgttctgatt gggacctcag tggtcaaaat ccctttcacc atcctcctct tctttctcct 840

tcatcgctgg tgctccgaca aaaaaaatgc tgctgtaatg gaccaagagc ctgcagggaa 900

cagaacagtg aacagcgagg attctgatga acaagaccat caggaggtgt catacgcata 960

attggatcac tgtgttttca cacagagaaa aatcactcgc ccttctgaga ggcccaagac 1020

acccccaaca gataccagca tgtacataga acttccaaat gctgagccca gatccaaagt 1080

tgtcttctgt ccacgagcac cacagtcagg ccttgagggg atcttctagg gagacaacag 1140

ccctgtctca aaaccgggtt gccagctccc atgtaccagc agctggaatc tgaaggcatc 1200

agtcttcatc ttagggcatc gctcttcctc acaccacgaa tctgaacatg cctctctctt 1260

gcttacaaat gtctaaggtc cccactgcct gctggagaga aaacacactc ctttgcttag 1320

cccacaattc tccatttcac ttgacccctg cccacctctc caacctaact ggcttacttc 1380

ctagtctacc tgaggctgca atcacactga ggaactcaca attccaaaca tacaagaggc 1440

tgcctcttaa cacagcactt agacacgtgc tgttccacct cccttcagac tatctttcag 1500

ccttctgcca gcagtaaaac ttataaattt tttaaataat ttcaatgtag ttttcccgcc 1560

ttcaaataaa catgtctgcc ctcatg 1586

<210> 153
<211> 1598
<212> DNA
<213> Homo sapiens

<400> 153
gagctcggtc gcggctgcct gtctgctccg gcagcaccat gtcgctcttg gtcgtcagca 60

tggcgtgtgt tgggttcttc ttgctgcagg gggcctggcc acatgaggga gtccacagaa 120

aaccttccct cctggcccac ccaggtcgcc tggtgaaatc agaagagaca gtcatcctgc 180

agtgttggtc agatgtcatg tttgaacact tccttctgca cagagagggg atgtttaacg 240

acactttgcg cctcattgga gaacaccatg atggggtctc caaggccaac ttctccatca 300

gtcgcatgac gcaagacctg gcagggacct acagatgcta cggttctgtt actcactccc 360

cctatcaggt gtcagctccc agtgaccctc tggacatcgt gatcataggt ctatatgaga 420

aaccttctct ctcagcccag ctgggcccca cggttctggc aggagagaat gtgaccttgt 480

cctgcagctc ccggagctcc tatgacatgt accatctatc cagggaaggg gaggcccatg 540

aacgtaggct ccctgcaggg cccaaggtca acggaacatt ccaggctgac tttcctctgg 600

gccctgccac ccacggaggg acctacagat gcttcggctc tttccatgac tctccatacg 660

agtggtcaaa gtcaagtgac ccactgcttg tttctgtcac aggaaaccct tcaaatagtt 720

ggccttcacc cactgaacca agctccaaaa ccggtaaccc ccgacacctg cacattctga 780

ttgggacctc agtggtcatc atcctcttca tcctcctctt ctttctcctt catcgctggt 840

gctccaacaa aaaaaatgct gcggtaatgg accaagagtc tgcaggaaac agaacagcga 900

atagcgagga ctctgatgaa caagaccctc aggaggtgac atacacacag ttgaatcact 960

gcgttttcac acagagaaaa atcactcgcc cttctcagag gcccaagaca cccccaacag 1020

atatcatcgt gtacacggaa cttccaaatg ctgagtccag atccaaagtt gtctcctgcc 1080

catgagcacc acagtcaggc cttgagggcg tcttctaggg agacaacagc cctgtctcaa 1140

aaccgggttg ccagctccca tgtaccagca gctggaatct gaaggcgtga gtctgcatct 1200

tagggcatcg atcttcctca caccacaaat ctgaatgtgc ctctctcttg cttacaaatg 1260

tctaaggtcc ccactgcctg ctggagaaaa aacacactcc tttgcttaac ccacagttct 1320

ccatttcact tgacccctgc ccacctctcc aacctaactg gcttacttcc tagtctactt 1380

gaggctgcaa tcacactgag gaactcacaa ttccaaacat acaagaggct ccctcttaac 1440

gcagcactta gacacgtgtt gttccacctt ccctcatgct gttccacctc ccctcagact 1500

agctttcagt cttctgtcag cagtaaaact tatatatttt ttaaaataac ttcaatgtag 1560

ttttccatcc ttcaaataaa catgtctgcc cccatggt 1598

<210> 154
<211> 1101
<212> DNA
<213> Homo sapiens

<400> 154
caccggcagc accatgtcgc tcacggtcgt cagcatggcg tgtgttgggt tcttcttgct 60

gcagggggcc tggccacatg agggagtcca cagaaaacct tccctcctgg cccacccagg 120

tcgcctggtg aaatcagaag agacagtcat cctgcaatgt tggtcagatg tcatgtttga 180

acacttcctt ctgcacagag aggggatgtt taacgacact ttgcgcctca ttggagaaca 240

ccatgatggg gtctccaagg ccaacttctc catcagtcgc atgaagcaag acctggcagg 300

gacctacaga tgctacggtt ctgttactca ctccccctat cagttgtcag ctcccagtga 360

ccctctggac atcgtgatca taggtctata tgagaaacct tctctctcag cccagccggg 420

ccccacggtt ctggcaggag agaatgtgac cttgtcctgc agctcccgga gctcctatga 480

catgtaccat ctatccaggg aaggggaggc ccatgaacgt aggctccctg cagggaccaa 540

ggtcaacgga acattccagg ccaactttcc tctgggccct gccacccatg gagggaccta 600

cagatgcttc ggctctttcc gtgactctcc atacgagtgg tcaaagtcaa gtgacccact 660

gcttgtttct gtcacaggaa acccttcaaa tagttggcct tcacccactg aaccaagctc 720

cgaaaccggt aaccccagac acctacatgt tctgattggg acctcagtgg tcaaaatccc 780

tttcaccatc ctcctcttct ttctccttca tcgctggtgc tccgacaaaa aaaatgctgc 840

tgtaatggac caagagcctg cagggaacag aacagtgaac agcgaggatt ctgatgaaca 900

agaccatcag gaggtgtcat acgcataatt ggatcactgt gttttcacac agagaaaaat 960

cactcgccct tctgagaggc ccaagacacc cccaacagat accagcatgt acatagaact 1020

tccaaatgct gagcccagat ccaaagttgt cttctgtcca cgagcaccac agtcaggcct 1080

tgaggggatc ttctagggag a 1101

<210> 155
<211> 1602
<212> DNA
<213> Homo sapiens

<400> 155
aggggtgcgg ccgcctgtct gcaccggcag caccatgtcg ctcatggtca tcagcatggc 60

gtgtgttgcg ttcttcttgc tgcagggggc ctggccacat gagggattcc gcagaaaacc 120

ttccctcctg gcccacccag gtcccctggt gaaatcagaa gagacagtca tcctgcaatg 180

ttggtcagat gtcatgtttg agcacttcct tctgcacaga gaggggacgt ttaaccacac 240

tttgcgcctc attggagagc acattgatgg ggtctccaag ggcaacttct ccatcggtcg 300

catgacacaa gacctggcag ggacctacag atgctacggt tctgttactc actcccccta 360

tcagttgtca gcgcccagtg accctctgga catcgtgatc acaggtctat atgagaaacc 420

ttctctctca gcccagccgg gccccacggt tctggcagga gagagcgtga ccttgtcctg 480

cagctcccgg agctcctatg acatgtacca tctatccagg gaaggggagg cccatgaacg 540

taggctccct gcagggccca aggtcaacag aacattccag gccgactttc ctctggaccc 600

tgccacccac ggagggacct acagatgctt cggctctttc cgtgactctc catacgagtg 660

gtcaaagtca agtgacccac tgcttgtttc tgtcacagga aactcttcaa atagttggcc 720

ttcacccact gaaccaagct ccgaaaccgg taaccccaga cacctacacg ttctgattgg 780

gacctcagtg gtcaaactcc ctttcaccat cctcctcttc tttctccttc atcgctggtg 840

ctccaacaaa aaaaatgcat ctgtaatgga ccaagggcct gcggggaaca gaacagtgaa 900

cagggaggat tctgatgaac aggaccatca ggaggtgtca tacgcataat tggatcactg 960

tgttttcaca cagagaaaaa tcactccccc ttctcagagg cccaagacac ccccaacaga 1020

taccagcatg tacatagaac ttccaaatgc tgagtccaga tccaaagctg tcttctgtcc 1080

acgagcacca cagtcaggcc ttgaggggat cttctaggga gacaacagcc ctgtctcaaa 1140

accgggttgc cagctcccat gtaccagcag ctggaatctg aaggcatcag tcttcatctt 1200

aggggatcgc tcttcctcaa accacgaatc tgaacatgcc tctctcttgc ttacaaatgt 1260

ctaaggtccc cactgcctgc tggagagaaa acacactcct ttgcttagcc cacaattctc 1320

catttcactt gacccctgcc cacctctcca acctaactgg cttacttcct agtctacttg 1380

aggctgcaat cacactgagg aactcacaat tccaaacata caagaggctc cctcttaaca 1440

cagcacttag acacgtgctg ttccaccttc tctcatgcag ttccacctcc cctcagacta 1500

tctttcagcc ttctgtcagc agtaaaactt ataaattgtt tttagtaatt tcaatgtagt 1560

tttccctcct tcaaataaac atgtctgccc tcatggtttc ga 1602

<210>  156
<211>  702
<212>  DNA
<213>  Homo sapiens

<400>  156
ggtggtcagg acaagccctt gctttctacc tggcccagcc ttgtggtgcc tccagaacat      60

gtgactcttc ggtgtcactc taatcttggg tttaacaact tcagtctgta caaggatgat     120

ggggtgcctg tccctgagct ctacaacaga atattctgga aagccttttt catgggccct     180

gtgaccccgt cacacacagg gacctataga tgccggggtt cacacacaca ctcccccagt     240

ggggggtcgg cacccagcaa ccccctggtg atcgtggtca caggattccg cagaaaacct     300

tccctcctgg cccacccagg tcgcctggtg aaatcagaag agacagtcat cctgcaatgt     360

tggtcagatg tcatgtttga gcacttcctt ctgcacagag aggggacgtt taacgacact     420

ttgcgcctca ttggagagca cattgatggg gtctccaagg ccaacttctc catcggtcgc     480

atgaggcaag acctggcagg gacctacaga tgctacggtt ctgttcctca ctccccctat     540

cagttttcag ctcccagtga ccctctggac atcgtgatca caggtgagag tgtccagaca     600

ttcttctcat tgtcattcgg acacagagtg aatgatccag gacttggagg cccaggtggt     660

tgtaaggaag atgagcttgg tattcttatg gagagagact ga                        702

<210>  157
<211>  1025
<212>  DNA
<213>  Homo sapiens

<400>  157
tttctcactc ccattgggcg tcgcgtttct agagaagcca atcagtgtcg ccgcagttcc      60

caggttctaa agtcccacgc accccgcggg actcatattt ttcccagacg cggaggttgg     120

ggtcatggcg ccccgaagcc tcctcctgct gctctcaggg gccctggccc tgaccgatac     180

ttgggcgggc tcccactcct tgaggtattt cagcaccgct gtgtcgcggc ccggccgcgg     240

ggagccccgc tacatcgccg tggagtacgt agacgacacg caattcctgc ggttcgacag     300

cgacgccgcg attccgagga tggagccgcg ggagccgtgg gtggagcaag aggggccgca     360

gtattgggag tggaccacag ggtacgccaa ggccaacgca cagactgacc gagtggccct     420

gaggaacctg ctccgccgct acaaccagag cgaggctggg tctcacaccc tccagggaat     480

gaatggctgc gacatggggc ccgacggacg cctcctccgc gggtatcacc agcacgcgta     540

cgacggcaag gattacatct ccctgaacga ggacctgcgc tcctggaccg cggcggacac     600

cgtggctcag atcacccagc gcttctatga ggcagaggaa tatgcagagg agttcaggac     660

ctacctggag ggcgagtgcc tggagttgct ccgcagatac ttggagaatg ggaaggagac     720

gctacagcgc gcagagcagt ctccccagcc caccatcccc atcgtgggca tcgttgctgg     780

ccttgttgtc cttggagctg tggtcactgg agctgtggtc gctgctgtga tgtggaggaa 840

gaagagctca gatagaaaca gagggagcta ctctcaggct gcagtgtgag acagcttcct 900

tgtgtgggac tgagaagcaa gatatcaatg tagcagaatt gcacttgtgc ctcacgaaca 960

tacataaatt ttaaaaataa agaataaaaa tatatctttt tatagataaa aaaaaaaaaa 1020

aaaaa 1025

<210> 158
<211> 1591
<212> DNA
<213> Homo sapiens

<400> 158
tttctcactc ccattgggcg tcgcgtttct agagaagcca atcagtgtcg ccgcagttcc 60

caggttctaa agtcccacgc accccgcggg actcatattt ttcccagacg cggaggttgg 120

ggtcatggcg ccccgaagcc tcctcctgct gctctcaggg gccctggccc tgaccgatac 180

ttgggcgggc tcccactcct tgaggtattt cagcaccgct gtgtcgcggc ccggccgcgg 240

ggagccccgc tacatcgccg tggagtacgt agacgacacg caattcctgc ggttcgacag 300

cgacgccgcg attccgagga tggagccgcg ggagccgtgg gtggagcaag aggggccgca 360

gtattgggag tggaccacag ggtacgccaa ggccaacgca cagactgacc gagtggccct 420

gaggaacctg ctccgccgct acaaccagag cgaggctggg tctcacaccc tccagggaat 480

gaatggctgc gacatggggc ccgacggacg cctcctccgc gggtatcacc agcacgcgta 540

cgacggcaag gattacatct ccctgaacga ggacctgcgc tcctggaccg cggcggacac 600

cgtggctcag atcacccagc gcttctatga ggcagaggaa tatgcagagg agttcaggac 660

ctacctggag ggcgagtgcc tggagttgct ccgcagatac ttggagaatg ggaaggagac 720

gctacagcgc gcagatcctc caaaggcaca cgttgcccac caccccatct ctgaccatga 780

ggccaccctg aggtgctggg ccctgggctt ctaccctgcg gagatcacgc tgacctggca 840

gcgggatggg gaggaacaga cccaggacac agagcttgtg gagaccaggc ctgcagggga 900

tggaaccttc cagaagtggg ccgctgtggt ggtgcctcct ggagaggaac agagatacac 960

atgccatgtg cagcacgagg ggctgcccca gcccctcatc ctgagatggg agcagtctcc 1020

ccagcccacc atccccatcg tgggcatcgt tgctggcctt gttgtccttg gagctgtggt 1080

cactggagct gtggtcgctg ctgtgatgtg gaggaagaag agctcagata gaaacagagg 1140

gagctactct caggctgcag cctactcagt ggtcagcgga aacttgatga taacatggtg 1200

gtcaagctta tttctcctgg gggtgctctt ccaaggatat ttgggctgcc tccggagtca 1260

cagtgtcttg ggccgccgga aggtgggtga catgtggatc ttgttttttt tgtggctgtg 1320

gacatctttc aacactgcct tcttggcctt gcaaagcctt cgctttggct tcggctttag 1380

gaggggcagg agcttccttc ttcgttcttg gcaccatctt atgaaaaggg tccagattaa 1440

gatttttgac tgagtcattc taaagtaagt tgcaagaccc atgatactag accactaaat 1500

acttcatcac acacctccta agaataagaa ccaacattat cacaccaaag aaaataaata 1560

attccataat attaaaaaaa aaaaaaaaaa a 1591

<210> 159
<211> 1301
<212> DNA
<213> Homo sapiens

<400> 159
tttctcactc ccattgggcg tcgcgtttct agagaagcca atcagtgtcg ccgcagttcc 60

caggttctaa agtcccacgc accccgcggg actcatattt ttcccagacg cggaggttgg 120

ggtcatggcg ccccgaagcc tcctcctgct gctctcaggg gccctggccc tgaccgatac 180

ttgggcgggc tcccactcct tgaggtattt cagcaccgct gtgtcgcggc ccggccgcgg 240

ggagccccgc tacatcgccg tggagtacgt agacgacacg caattcctgc ggttcgacag 300

cgacgccgcg attccgagga tggagccgcg ggagccgtgg gtggagcaag aggggccgca 360

gtattgggag tggaccacag ggtacgccaa ggccaacgca cagactgacc gagtggccct 420

gaggaacctg ctccgccgct acaaccagag cgaggctggg tctcacaccc tccagggaat 480

gaatggctgc gacatggggc ccgacggacg cctcctccgc gggtatcacc agcacgcgta 540

cgacggcaag gattacatct ccctgaacga ggacctgcgc tcctggaccg cggcggacac 600

cgtggctcag atcacccagc gcttctatga ggcagaggaa tatgcagagg agttcaggac 660

ctacctggag ggcgagtgcc tggagttgct ccgcagatac ttggagaatg ggaaggagac 720

gctacagcgc gcagatcctc caaaggcaca cgttgcccac caccccatct ctgaccatga 780

ggccaccctg aggtgctggg ccctgggctt ctaccctgcg gagatcacgc tgacctggca 840

gcgggatggg gaggaacaga cccaggacac agagcttgtg gagaccaggc ctgcagggga 900

tggaaccttc cagaagtggg ccgctgtggt ggtgcctcct ggagaggaac agagatacac 960

atgccatgtg cagcacgagg ggctgcccca gcccctcatc ctgagatggg agcagtctcc 1020

ccagcccacc atccccatcg tgggcatcgt tgctggcctt gttgtccttg gagctgtggt 1080

cactggagct gtggtcgctg ctgtgatgtg gaggaagaag agctcagata gaaacagagg 1140

gagctactct caggctgcag tgtgagacag cttccttgtg tgggactgag aagcaagata 1200

tcaatgtagc agaattgcac ttgtgcctca cgaacataca taaattttaa aaataaagaa 1260

taaaaatata tctttttata gataaaaaaa aaaaaaaaaa a 1301

<210> 160
<211> 244

<212>  DNA
<213>  Homo sapiens

<220>
<221>  misc_feature
<222>  (31)..(31)
<223>  n is a, c, g, or t

<220>
<221>  misc_feature
<222>  (54)..(54)
<223>  n is a, c, g, or t

<220>
<221>  misc_feature
<222>  (70)..(70)
<223>  n is a, c, g, or t

<220>
<221>  misc_feature
<222>  (96)..(96)
<223>  n is a, c, g, or t

<220>
<221>  misc_feature
<222>  (108)..(108)
<223>  n is a, c, g, or t

<220>
<221>  misc_feature
<222>  (124)..(124)
<223>  n is a, c, g, or t

<220>
<221>  misc_feature
<222>  (127)..(127)
<223>  n is a, c, g, or t

<220>
<221>  misc_feature
<222>  (130)..(130)
<223>  n is a, c, g, or t

<220>
<221>  misc_feature
<222>  (141)..(141)
<223>  n is a, c, g, or t

<220>
<221>  misc_feature
<222>  (145)..(145)
<223>  n is a, c, g, or t

<220>
<221>  misc_feature
<222>  (158)..(158)
<223>  n is a, c, g, or t

<220>
<221>  misc_feature
<222>  (170)..(170)
<223>  n is a, c, g, or t

<400>  160
ggcctggcca catgagggtg gtcaggacaa ncccttgctg tctgcctggc ccanccttgt      60

ggtgcctctn ggacatgtca ttcttcggtg tcactnttat cttgggtnta acaacttcag      120

tctntanaan gaaggtgggg ngccngtccc tgagctcnac aacagaatan tctggaacag      180

ccttttcatg ggccctgtga cccccgcact acagggacat acagatgtcg gggttcacac      240

acac                                                                  244


<210>  161
<211>  429
<212>  DNA
<213>  Homo sapiens


<220>
<221>  misc_feature
<222>  (129)..(129)
<223>  n is a, c, g, or t

<400>  161
tctaccctgc ggagatcacg ctgacctggc agcgggatgg ggaggaacag acccaggaca      60

cagagcttgt ggagaccagg cctgcagggg atggaacctt ccagaagtgg gccgctgtgg      120

tggtgcctnc tggagaggaa cagagataca catgccatgt gcagcacgag gggctgcccc      180

agcccctcat cctgagatgg gagcagtctc cccagcccac catccccatc gtgggcatcg      240

ttgctggcct tgttgtcctt ggagctgtgg tcactggagc tgtggtcgct gctgtgatgt      300

ggaggaagaa gagctcagat agaaacagag ggagctactc tcaggctgca gtgtgagaca      360

gcttccttgt gtgggactga gaagcaagat atcaatgtag cagaattgca cttgtgcctc      420

acgaacata                                                             429


<210>  162
<211>  392
<212>  DNA
<213>  Homo sapiens

<400>  162
ggacattttc ttcccataga tagaaacaga gggagctact ctcaggctgc aggtaagatg      60

aaggaggctg atccctgaga ttgttgggat attgtggtca ggagcctatg agggagctca      120

cccaccccac agttcctcta gccacatctg tgggctctga ccaggtcctg tttttgttct      180

accccaatca ctgacagtgc ccagggctct ggggtgtctc tcacagctaa taaaggtgac      240

actccagggc aggggccctg atgtgagtgg ggtgttgggg gggaacagag gggactcagc      300

tgtgctattg ggtttctttg acttggatgt cttgagcatg aaatgggcta tttagagtgt      360

tacctctcac tgtgactgat acgaatttgt tc                                   392

<210>  163
<211>  503
<212>  DNA
<213>  Homo sapiens

<400>  163
cagatcctcc aaaggcacac gttgcccacc accccatctc tgaccatgag gccaccctga      60

ggtgctgggc cctgggcttc taccctgcgg agatcacgct gacctggcag cgggatgggg     120

aggaacagac ccaggacaca gagcttgtgg agaccaggcc tgcaggggat ggaaccttcc     180

agaagtgggc cgctgtggtg gtgccttctg gagaggaaca gagatacaca tgccatgtgc     240

agcacgaggg gctgccccag cccctcatcc tgagatggga gcagtctccc cagcccacca     300

tccccatcgt gggcatcgtt gctggccttg ttgtccttgg agctgtggtc actggagctg     360

tggtcgctgc tgtgatgtgg aggaagaaga gctcagatag aaacagaggg agctactctc     420

aggctgcagt cactgacagt gcccagggct ctggggtgtc tctcacagct aataaagtgt     480

gagacagctt ccttgtgtgg gac                                             503

<210>  164
<211>  119
<212>  PRT
<213>  Homo sapiens

<400>  164

Met Glu Ala Ser Ala Leu Thr Ser Ser Ala Val Thr Ser Val Ala Lys
1               5                   10                  15

Val Val Arg Val Ala Ser Gly Ser Ala Val Val Leu Pro Leu Ala Arg
            20                  25                  30

Ile Ala Thr Val Val Ile Gly Gly Val Val Ala Val Pro Met Val Leu
        35                  40                  45

Ser Ala Met Gly Phe Thr Ala Ala Gly Ile Ala Ser Ser Ser Ile Ala
    50                  55                  60

Ala Lys Met Met Ser Ala Ala Ala Ile Ala Asn Gly Gly Gly Val Ala
65                  70                  75                  80

Ser Gly Ser Leu Val Ala Thr Leu Gln Ser Leu Gly Ala Thr Gly Leu
                85                  90                  95

Ser Gly Leu Thr Lys Phe Ile Leu Gly Ser Ile Gly Ser Ala Ile Ala
            100                 105                 110

Ala Val Ile Ala Arg Phe Tyr
        115

<210>  165
<211>  876
<212>  DNA
<213>  Homo sapiens

<400>  165
agaacctaga gcccaaggtt cagagtcacc catctcagca agcccagaag tatctgcaat      60

atctacgatg gcctcgccct ttgctttact gatggtcctg gtggtgctca gctgcaagtc     120

aagctgctct ctgggctgtg atctccctga gacccacagc ctggataaca ggaggacctt     180

gatgctcctg gcacaaatga gcagaatctc tccttcctcc tgtctgatgg acagacatga     240

ctttggattt ccccaggagg agtttgatgg caaccagttc cagaaggctc cagccatctc     300

tgtcctccat gagctgatcc agcagatctt caacctcttt accacaaaag attcatctgc     360

tgcttgggat gaggacctcc tagacaaatt ctgcaccgaa ctctaccagc agctgaatga     420

cttggaagcc tgtgtgatgc aggaggagag ggtgggagaa actcccctga tgaatgcgga     480

ctccatcttg gctgtgaaga aatacttccg aagaatcact ctctatctga cagagaagaa     540

atacagccct tgtgcctggg aggttgtcag agcagaaatc atgagatccc tctctttatc     600

aacaaacttg caagaaagat taaggaggaa ggaataacat ctggtccaac atgaaaacaa     660

ttcttattga ctcatacacc aggtcacgct ttcatgaatt ctgtcatttc aaagactctc     720

acccctgcta taactatgac catgctgata aactgattta tctatttaaa tatttattta     780

actattcata agatttaaat tatttttgtt catataacgt catgtgcacc tttacactgt     840

ggttagtgta ataaaacatg ttccttatat ttactc                               876

<210>  166
<211>  840
<212>  DNA
<213>  Homo sapiens

<400>  166
acattctaac tgcaaccttt cgaagccttt gctctggcac aacaggtagt aggcgacact      60

gttcgtgttg tcaacatgac caacaagtgt ctcctccaaa ttgctctcct gttgtgcttc     120

tccactacag ctctttccat gagctacaac ttgcttggat tcctacaaag aagcagcaat     180

tttcagtgtc agaagctcct gtggcaattg aatgggaggc ttgaatactg cctcaaggac     240

aggatgaact ttgacatccc tgaggagatt aagcagctgc agcagttcca gaaggaggac     300

gccgcattga ccatctatga gatgctccag aacatctttg ctattttcag acaagattca     360

tctagcactg gctggaatga gactattgtt gagaacctcc tggctaatgt ctatcatcag     420

ataaaccatc tgaagacagt cctggaagaa aaactggaga aagaagattt caccagggga     480

aaactcatga gcagtctgca cctgaaaaga tattatggga ggattctgca ttacctgaag     540

gccaaggagt acagtcactg tgcctggacc atagtcagag tggaaatcct aaggaacttt     600

tacttcatta acagacttac aggttacctc cgaaactgaa gatctcctag cctgtgcctc 660

tgggactgga caattgcttc aagcattctt caaccagcag atgctgttta agtgactgat 720

ggctaatgta ctgcatatga aaggacacta gaagattttg aaatttttat taaattatga 780

gttattttta tttatttaaa ttttattttg gaaaataaat tatttttggt gcaaaagtca 840

<210> 167
<211> 1514
<212> DNA
<213> Homo sapiens

<400> 167
gatctggtaa acctgaagca aatatagaaa cctatagggc ctgacttcct acataaagta 60

aggagggtaa aaatggaggc tagaataagg gttaaaattt tgcttctaga acagagaaaa 120

tgattttttt catatatata tgaatatata ttatatatac acatatatac atatattcac 180

tatagtgtgt atacataaat atataatata tatattgtta gtgtagtgtg tgtctgatta 240

tttacatgca tatagtatat acacttatga ctttagtacc cagacgtttt tcatttgatt 300

aagcattcat ttgtattgac acagctgaag tttactggag tttagctgaa gtctaatgca 360

aaattaatag attgttgtca tcctcttaag gtcataggga gaacacacaa atgaaaacag 420

taaaagaaac tgaaagtaca gagaaatgtt cagaaaatga aaaccatgtg tttcctatta 480

aaagccatgc atacaagcaa tgtcttcaga aaacctaggg tccaaggtta agccatatcc 540

cagctcagta aagccaggag catcctcatt tcccaatggc cctcctgttc cctctactgg 600

cagccctagt gatgaccagc tatagccctg ttggatctct gggctgtgat ctgcctcaga 660

accatggcct acttagcagg aacaccttgg tgcttctgca ccaaatgagg agaatctccc 720

ctttcttgtg tctcaaggac agaagagact tcaggttccc ccaggagatg gtaaaaggga 780

gccagttgca gaaggcccat gtcatgtctg tcctccatga gatgctgcag cagatcttca 840

gcctcttcca cacagagcgc tcctctgctg cctggaacat gaccctccta gaccaactcc 900

acactggact tcatcagcaa ctgcaacacc tggagacctg cttgctgcag gtagtgggag 960

aaggagaatc tgctggggca attagcagcc ctgcactgac cttgaggagg tacttccagg 1020

gaatccgtgt ctacctgaaa gagaagaaat acagcgactg tgcctgggaa gttgtcagaa 1080

tggaaatcat gaaatccttg ttcttatcaa caaacatgca agaaagactg agaagtaaag 1140

atagagacct gggctcatct tgaaatgatt ctcattgatt aatttgccat ataacacttg 1200

cacatgtgac tctggtcaat tcaaaagact cttatttcgg ctttaatcac agaattgact 1260

gaattagttc tgcaaatact ttgtcggtat attaagccag tatatgttaa aaagacttag 1320

gttcaggggc atcagtccct aagatgttat ttatttttac tcatttattt attcttacat 1380

tttatcatat ttatactatt tatattctta tataacaaat gtttgccttt acattgtatt 1440

aagataacaa aacatgttca gctttccatt tggttaaata ttgtattttg ttatttatta 1500

aattattttc aaac 1514

<210> 168
<211> 1240
<212> DNA
<213> Homo sapiens

<400> 168
cacattgttc tgatcatctg aagatcagct attagaagag aaagatcagt taagtccttt 60

ggacctgatc agcttgatac aagaactact gatttcaact tctttggctt aattctctcg 120

gaaacgatga aatatacaag ttatatcttg gcttttcagc tctgcatcgt tttgggttct 180

cttggctgtt actgccagga cccatatgta aaagaagcag aaaaccttaa gaaatatttt 240

aatgcaggtc attcagatgt agcggataat ggaactcttt tcttaggcat tttgaagaat 300

tggaaagagg agagtgacag aaaaataatg cagagccaaa ttgtctcctt ttacttcaaa 360

ctttttaaaa actttaaaga tgaccagagc atccaaaaga gtgtggagac catcaaggaa 420

gacatgaatg tcaagttttt caatagcaac aaaaagaaac gagatgactt cgaaaagctg 480

actaattatt cggtaactga cttgaatgtc caacgcaaag caatacatga actcatccaa 540

gtgatggctg aactgtcgcc agcagctaaa acagggaagc gaaaaaggag tcagatgctg 600

tttcgaggtc gaagagcatc ccagtaatgg ttgtcctgcc tgcaatattt gaattttaaa 660

tctaaatcta tttattaata tttaacatta tttatatggg gaatatattt ttagactcat 720

caatcaaata agtatttata atagcaactt ttgtgtaatg aaaatgaata tctattaata 780

tatgtattat ttataattcc tatatcctgt gactgtctca cttaatcctt tgttttctga 840

ctaattaggc aaggctatgt gattacaagg ctttatctca ggggccaact aggcagccaa 900

cctaagcaag atcccatggg ttgtgtgttt atttcacttg atgatacaat gaacacttat 960

aagtgaagtg atactatcca gttactgccg gtttgaaaat atgcctgcaa tctgagccag 1020

tgctttaatg gcatgtcaga cagaacttga atgtgtcagg tgaccctgat gaaaacatag 1080

catctcagga gatttcatgc ctggtgcttc caaatattgt tgacaactgt gactgtaccc 1140

aaatggaaag taactcattt gttaaaatta tcaatatcta atatatatga ataaagtgta 1200

agttcacaac aaaaaaaaaa aaaaaaaaaa aaaaaaaaaa 1240

<210> 169
<211> 189
<212> PRT
<213> Homo sapiens

<400> 169

Met Ala Ser Pro Phe Ala Leu Leu Met Val Leu Val Val Leu Ser Cys

1               5                   10                  15

Lys Ser Ser Cys Ser Leu Gly Cys Asp Leu Pro Glu Thr His Ser Leu
            20                  25                  30

Asp Asn Arg Arg Thr Leu Met Leu Leu Ala Gln Met Ser Arg Ile Ser
        35                  40                  45

Pro Ser Ser Cys Leu Met Asp Arg His Asp Phe Gly Phe Pro Gln Glu
    50                  55                  60

Glu Phe Asp Gly Asn Gln Phe Gln Lys Ala Pro Ala Ile Ser Val Leu
65                  70                  75                  80

His Glu Leu Ile Gln Gln Ile Phe Asn Leu Phe Thr Thr Lys Asp Ser
                85                  90                  95

Ser Ala Ala Trp Asp Glu Asp Leu Leu Asp Lys Phe Cys Thr Glu Leu
            100                 105                 110

Tyr Gln Gln Leu Asn Asp Leu Glu Ala Cys Val Met Gln Glu Glu Arg
        115                 120                 125

Val Gly Glu Thr Pro Leu Met Asn Ala Asp Ser Ile Leu Ala Val Lys
    130                 135                 140

Lys Tyr Phe Arg Arg Ile Thr Leu Tyr Leu Thr Glu Lys Lys Tyr Ser
145                 150                 155                 160

Pro Cys Ala Trp Glu Val Val Arg Ala Glu Ile Met Arg Ser Leu Ser
                165                 170                 175

Leu Ser Thr Asn Leu Gln Glu Arg Leu Arg Arg Lys Glu
            180                 185

<210>  170
<211>  187
<212>  PRT
<213>  Homo sapiens

<400>  170

Met Thr Asn Lys Cys Leu Leu Gln Ile Ala Leu Leu Leu Cys Phe Ser
1               5                   10                  15

Thr Thr Ala Leu Ser Met Ser Tyr Asn Leu Leu Gly Phe Leu Gln Arg
            20                  25                  30

Ser Ser Asn Phe Gln Cys Gln Lys Leu Leu Trp Gln Leu Asn Gly Arg

35                  40                  45

Leu Glu Tyr Cys Leu Lys Asp Arg Met Asn Phe Asp Ile Pro Glu Glu
    50                  55                  60

Ile Lys Gln Leu Gln Gln Phe Gln Lys Glu Asp Ala Ala Leu Thr Ile
65                  70                  75                  80

Tyr Glu Met Leu Gln Asn Ile Phe Ala Ile Phe Arg Gln Asp Ser Ser
                85                  90                  95

Ser Thr Gly Trp Asn Glu Thr Ile Val Glu Asn Leu Leu Ala Asn Val
            100                 105                 110

Tyr His Gln Ile Asn His Leu Lys Thr Val Leu Glu Glu Lys Leu Glu
        115                 120                 125

Lys Glu Asp Phe Thr Arg Gly Lys Leu Met Ser Ser Leu His Leu Lys
    130                 135                 140

Arg Tyr Tyr Gly Arg Ile Leu His Tyr Leu Lys Ala Lys Glu Tyr Ser
145                 150                 155                 160

His Cys Ala Trp Thr Ile Val Arg Val Glu Ile Leu Arg Asn Phe Tyr
                165                 170                 175

Phe Ile Asn Arg Leu Thr Gly Tyr Leu Arg Asn
            180                 185

<210>  171
<211>  195
<212>  PRT
<213>  Homo sapiens

<400>  171

Met Ala Leu Leu Phe Pro Leu Leu Ala Ala Leu Val Met Thr Ser Tyr
1               5                   10                  15

Ser Pro Val Gly Ser Leu Gly Cys Asp Leu Pro Gln Asn His Gly Leu
            20                  25                  30

Leu Ser Arg Asn Thr Leu Val Leu Leu His Gln Met Arg Arg Ile Ser
        35                  40                  45

Pro Phe Leu Cys Leu Lys Asp Arg Arg Asp Phe Arg Phe Pro Gln Glu
    50                  55                  60

Met Val Lys Gly Ser Gln Leu Gln Lys Ala His Val Met Ser Val Leu

65                  70                  75                  80

His Glu Met Leu Gln Gln Ile Phe Ser Leu Phe His Thr Glu Arg Ser
                85                  90                  95

Ser Ala Ala Trp Asn Met Thr Leu Leu Asp Gln Leu His Thr Gly Leu
            100                 105                 110

His Gln Gln Leu Gln His Leu Glu Thr Cys Leu Leu Gln Val Val Gly
        115                 120                 125

Glu Gly Glu Ser Ala Gly Ala Ile Ser Ser Pro Ala Leu Thr Leu Arg
    130                 135                 140

Arg Tyr Phe Gln Gly Ile Arg Val Tyr Leu Lys Glu Lys Lys Tyr Ser
145                 150                 155                 160

Asp Cys Ala Trp Glu Val Val Arg Met Glu Ile Met Lys Ser Leu Phe
                165                 170                 175

Leu Ser Thr Asn Met Gln Glu Arg Leu Arg Ser Lys Asp Arg Asp Leu
            180                 185                 190

Gly Ser Ser
        195

<210>  172
<211>  164
<212>  PRT
<213>  Homo sapiens

<400>  172

Met Lys Tyr Thr Ser Tyr Ile Leu Ala Phe Gln Leu Cys Ile Val Leu
1               5                   10                  15

Gly Ser Leu Gly Cys Tyr Cys Gln Asp Pro Tyr Val Lys Glu Ala Glu
            20                  25                  30

Asn Leu Lys Lys Tyr Phe Asn Ala Gly His Ser Asp Val Ala Asp Asn
        35                  40                  45

Gly Thr Leu Phe Leu Gly Ile Leu Lys Asn Trp Lys Glu Glu Ser Asp
    50                  55                  60

Arg Lys Ile Met Gln Ser Gln Ile Val Ser Phe Tyr Phe Lys Leu Phe
65                  70                  75                  80

Lys Asn Phe Lys Asp Asp Gln Ser Ile Gln Lys Ser Val Glu Thr Ile

85                  90                  95

Lys Glu Asp Met Asn Val Lys Phe Phe Asn Ser Asn Lys Lys Lys Arg
            100                 105                 110

Asp Asp Phe Glu Lys Leu Thr Asn Tyr Ser Val Thr Asp Leu Asn Val
        115                 120                 125

Gln Arg Lys Ala Ile His Glu Leu Ile Gln Val Met Ala Glu Leu Ser
    130                 135                 140

Pro Ala Ala Lys Thr Gly Lys Arg Lys Arg Ser Gln Met Leu Phe Arg
145                 150                 155                 160

Gly Arg Arg Ala

<210>  173
<211>  1143
<212>  DNA
<213>  Homo sapiens

<400>  173
gagaacctgg agcctaaggt ttaggctcac ccatttcaac cagtctagca gcatctgcaa      60

catctacaat ggccttgacc tttgctttac tggtggccct cctggtgctc agctgcaagt     120

caagctgctc tgtgggctgt gatctgcctc aaacccacag cctgggtagc aggaggacct     180

tgatgctcct ggcacagatg aggagaatct ctcttttctc ctgcttgaag gacagacatg     240

actttggatt tccccaggag gagtttggca accagttcca aaaggctgaa accatccctg     300

tcctccatga gatgatccag cagatcttca atctcttcag cacaaaggac tcatctgctg     360

cttgggatga gaccctccta gacaaattct acactgaact ctaccagcag ctgaatgacc     420

tggaagcctg tgtgatacag ggggtggggg tgacagagac tcccctgatg aaggaggact     480

ccattctggc tgtgaggaaa tacttccaaa gaatcactct ctatctgaaa gagaagaaat     540

acagcccttg tgcctgggag gttgtcagag cagaaatcat gagatctttt tctttgtcaa     600

caaacttgca agaaagttta agaagtaagg aatgaaaact ggttcaacat ggaaatgatt     660

ttcattgatt cgtatgccag ctcacctttt tatgatctgc catttcaaag actcatgttt     720

ctgctatgac catgacacga tttaaatctt ttcaaatgtt tttaggagta ttaatcaaca     780

ttgtattcag ctcttaaggc actagtccct tacagaggac catgctgact gatccattat     840

ctatttaaat atttttaaaa tattatttat ttaactattt ataaaacaac ttatttttgt     900

tcatattatg tcatgtgcac ctttgcacag tggttaatgt aataaaatat gttctttgta     960

tttggtaaat ttattttgtg ttgttcattg aacttttgct atggaaactt ttgtacttgt    1020

ttattcttta aaatgaaatt ccaagcctaa ttgtgcaacc tgattacaga ataactggta 1080

cacttcattt atccatcaat attatattca agatataagt aaaaataaac tttctgtaaa 1140

cca 1143

<210> 174
<211> 188
<212> PRT
<213> Homo sapiens

<400> 174

Met Ala Leu Thr Phe Ala Leu Leu Val Ala Leu Leu Val Leu Ser Cys
1               5                   10                  15

Lys Ser Ser Cys Ser Val Gly Cys Asp Leu Pro Gln Thr His Ser Leu
            20                  25                  30

Gly Ser Arg Arg Thr Leu Met Leu Leu Ala Gln Met Arg Arg Ile Ser
        35                  40                  45

Leu Phe Ser Cys Leu Lys Asp Arg His Asp Phe Gly Phe Pro Gln Glu
    50                  55                  60

Glu Phe Gly Asn Gln Phe Gln Lys Ala Glu Thr Ile Pro Val Leu His
65                  70                  75                  80

Glu Met Ile Gln Gln Ile Phe Asn Leu Phe Ser Thr Lys Asp Ser Ser
                85                  90                  95

Ala Ala Trp Asp Glu Thr Leu Leu Asp Lys Phe Tyr Thr Glu Leu Tyr
            100                 105                 110

Gln Gln Leu Asn Asp Leu Glu Ala Cys Val Ile Gln Gly Val Gly Val
        115                 120                 125

Thr Glu Thr Pro Leu Met Lys Glu Asp Ser Ile Leu Ala Val Arg Lys
    130                 135                 140

Tyr Phe Gln Arg Ile Thr Leu Tyr Leu Lys Glu Lys Lys Tyr Ser Pro
145                 150                 155                 160

Cys Ala Trp Glu Val Val Arg Ala Glu Ile Met Arg Ser Phe Ser Leu
                165                 170                 175

Ser Thr Asn Leu Gln Glu Ser Leu Arg Ser Lys Glu
            180                 185

<210> 175

<211>  19
<212>  DNA
<213>  Artificial sequence

<220>
<223>  Exemplary siRNA target sequence

<400>  175
gggcattgtt gctggcctg                                                    19


<210>  176
<211>  19
<212>  DNA
<213>  Artificial sequence

<220>
<223>  Exemplary siRNA target sequence

<400>  176
catcgtgggc attgttgct                                                    19


<210>  177
<211>  19
<212>  DNA
<213>  Artificial sequence

<220>
<223>  Exemplary siRNA target sequence

<400>  177
cattgttgct ggcctggct                                                    19


<210>  178
<211>  19
<212>  DNA
<213>  Artificial sequence

<220>
<223>  Exemplary siRNA target sequence

<400>  178
tgggcattgt tgctggcct                                                    19


<210>  179
<211>  19
<212>  DNA
<213>  Artificial sequence

<220>
<223>  Exemplary siRNA target sequence

<400>  179
tctatgaggc agaggaata                                                    19


<210>  180
<211>  19
<212>  DNA
<213>  Artificial sequence

<220>
<223>  Exemplary siRNA target sequence

<400>  180
aggaatatgc agaggagtt                                                    19


<210>  181
<211>  19
<212>  DNA
<213>  Artificial sequence

<220>
<223>  Exemplary siRNA target sequence

<400>  181
gctacatcgc cgtggagta                                                    19


<210>  182
<211>  19
<212>  DNA
<213>  Artificial sequence

<220>
<223>  Exemplary siRNA target sequence

<400>  182
ggaatgaatg gctgcgaca                                                    19


<210>  183
<211>  19
<212>  DNA
<213>  Artificial sequence

<220>
<223>  Exemplary siRNA target sequence

<400>  183
aggcagagga atatgcaga                                                    19


<210>  184
<211>  19
<212>  DNA
<213>  Artificial sequence

<220>
<223>  Exemplary siRNA target sequence

<400>  184
ccgcagatac ttggagaat                                                    19


<210>  185
<211>  19
<212>  DNA
<213>  Artificial sequence

<220>
<223>  Exemplary siRNA target sequence

<400>  185
gagctcagat agaaacaga                                                    19


<210>  186
<211>  19
<212>  DNA
<213>  Artificial sequence

<220>
<223>  Exemplary siRNA target sequence

<400>  186
cagtattggg agtggacca                                                    19


<210>  187
<211>  19
<212>  DNA
<213>  Artificial sequence

<220>
<223>  Exemplary siRNA target sequence

<400>  187
gcagaggagt tcaggacct                                                    19


<210>  188
<211>  19
<212>  DNA
<213>  Artificial sequence

<220>
<223>  Exemplary siRNA target sequence

<400>  188
gcagatactt ggagaatgg                                                    19


<210>  189
<211>  19
<212>  DNA
<213>  Artificial sequence

<220>
<223>  Exemplary siRNA target sequence

<400>  189
tcagatagaa acagaggga                                                    19


<210>  190
<211>  19
<212>  DNA
<213>  Artificial sequence

<220>
<223>  Exemplary siRNA target sequence

<400>  190
agatagaaac agagggagc                                                    19

<210>  191
<211>  19
<212>  DNA
<213>  Artificial sequence

<220>
<223>  Exemplary siRNA target sequence

<400>  191
cagggtacgc caaggccaa                                                    19


<210>  192
<211>  19
<212>  DNA
<213>  Artificial sequence

<220>
<223>  Exemplary siRNA target sequence

<400>  192
cttctatgag gcagaggaa                                                    19


<210>  193
<211>  19
<212>  DNA
<213>  Artificial sequence

<220>
<223>  Exemplary siRNA target sequence

<400>  193
gatacttgga gaatgggaa                                                    19


<210>  194
<211>  19
<212>  DNA
<213>  Artificial sequence

<220>
<223>  Exemplary siRNA target sequence

<400>  194
aggccaacgc acagactga                                                    19


<210>  195
<211>  19
<212>  DNA
<213>  Artificial sequence

<220>
<223>  Exemplary siRNA target sequence

<400>  195
gtacgtagac gacacgcaa                                                    19


<210>  196

<211>  19
<212>  DNA
<213>  Artificial sequence

<220>
<223>  Exemplary siRNA target sequence

<400>  196
acgtagacga cacgcaatt                                                    19


<210>  197
<211>  19
<212>  DNA
<213>  Artificial sequence

<220>
<223>  Exemplary siRNA target sequence

<400>  197
cagaggagtt caggaccta                                                    19


<210>  198
<211>  19
<212>  DNA
<213>  Artificial sequence

<220>
<223>  Exemplary siRNA target sequence

<400>  198
aggaagagct caggtggaa                                                    19


<210>  199
<211>  19
<212>  DNA
<213>  Artificial sequence

<220>
<223>  Exemplary siRNA target sequence

<400>  199
gaggaagagc tcaggtgga                                                    19


<210>  200
<211>  19
<212>  DNA
<213>  Artificial sequence

<220>
<223>  Exemplary siRNA target sequence

<400>  200
ggaagagctc aggtggaaa                                                    19


<210>  201
<211>  19
<212>  DNA
<213>  Artificial sequence

<220>
<223>  Exemplary siRNA target sequence

<400>  201
ggaaaaggag ggagctgct                                                    19


<210>  202
<211>  19
<212>  DNA
<213>  Artificial sequence

<220>
<223>  Exemplary siRNA target sequence

<400>  202
cagcaggaga tggaacctt                                                    19


<210>  203
<211>  19
<212>  DNA
<213>  Artificial sequence

<220>
<223>  Exemplary siRNA target sequence

<400>  203
ccagggctct gatgagtct                                                    19


<210>  204
<211>  19
<212>  DNA
<213>  Artificial sequence

<220>
<223>  Exemplary siRNA target sequence

<400>  204
ggccagcagg agatggaac                                                    19


<210>  205
<211>  19
<212>  DNA
<213>  Artificial sequence

<220>
<223>  Exemplary siRNA target sequence

<400>  205
agcaggagat ggaaccttc                                                    19


<210>  206
<211>  19
<212>  DNA
<213>  Artificial sequence

<220>
<223>  Exemplary siRNA target sequence

<400>  206
aggaggaaga gctcaggtg                                                    19


<210>  207
<211>  19
<212>  DNA
<213>  Artificial sequence

<220>
<223>  Exemplary siRNA target sequence

<400>  207
ggaggaagag ctcaggtgg                                                    19


<210>  208
<211>  19
<212>  DNA
<213>  Artificial sequence

<220>
<223>  Exemplary siRNA target sequence

<400>  208
ccatcttccc agcccacca                                                    19


<210>  209
<211>  19
<212>  DNA
<213>  Artificial sequence

<220>
<223>  Exemplary siRNA target sequence

<400>  209
gccagcagga gatggaacc                                                    19


<210>  210
<211>  19
<212>  DNA
<213>  Artificial sequence

<220>
<223>  Exemplary siRNA target sequence

<400>  210
ccagcaggag atggaacct                                                    19


<210>  211
<211>  19
<212>  DNA
<213>  Artificial sequence

<220>
<223>  Exemplary siRNA target sequence

<400>  211
ggaagagctc agatagaaa                                                    19

<210>  212
<211>  19
<212>  DNA
<213>  Artificial sequence

<220>
<223>  Exemplary siRNA target sequence

<400>  212
gaggaagagc tcagataga                                                    19


<210>  213
<211>  19
<212>  DNA
<213>  Artificial sequence

<220>
<223>  Exemplary siRNA target sequence

<400>  213
gtgatgtgga ggaggaaga                                                    19


<210>  214
<211>  19
<212>  DNA
<213>  Artificial sequence

<220>
<223>  Exemplary siRNA target sequence

<400>  214
aggaggaaga gctcagata                                                    19


<210>  215
<211>  19
<212>  DNA
<213>  Artificial sequence

<220>
<223>  Exemplary siRNA target sequence

<400>  215
gcttcatcgc cgtgggcta                                                    19


<210>  216
<211>  19
<212>  DNA
<213>  Artificial sequence

<220>
<223>  Exemplary siRNA target sequence

<400>  216
ggaccaggag acacggaat                                                    19


<210>  217

<211>  19
<212>  DNA
<213>  Artificial sequence

<220>
<223>  Exemplary siRNA target sequence

<400>  217
agatagaaaa ggagggagt                                                    19


<210>  218
<211>  19
<212>  DNA
<213>  Artificial sequence

<220>
<223>  Exemplary siRNA target sequence

<400>  218
aggaagagct cagatagaa                                                    19


<210>  219
<211>  19
<212>  DNA
<213>  Artificial sequence

<220>
<223>  Exemplary siRNA target sequence

<400>  219
caggagacac ggaatgtga                                                    19


<210>  220
<211>  19
<212>  DNA
<213>  Artificial sequence

<220>
<223>  Exemplary siRNA target sequence

<400>  220
gagtattggg accaggaga                                                    19


<210>  221
<211>  19
<212>  DNA
<213>  Artificial sequence

<220>
<223>  Exemplary siRNA target sequence

<400>  221
ggacatggcg gctcagatc                                                    19


<210>  222
<211>  19
<212>  DNA
<213>  Artificial sequence

<220>
<223>  Exemplary siRNA target sequence

<400>  222
cccatgaggc ggagcagtt                                                    19


<210>  223
<211>  19
<212>  DNA
<213>  Artificial sequence

<220>
<223>  Exemplary siRNA target sequence

<400>  223
atagaaaagg agggagtta                                                    19


<210>  224
<211>  19
<212>  DNA
<213>  Artificial sequence

<220>
<223>  Exemplary siRNA target sequence

<400>  224
agaaaaggag ggagttaca                                                    19


<210>  225
<211>  19
<212>  DNA
<213>  Artificial sequence

<220>
<223>  Exemplary siRNA target sequence

<400>  225
tcaccaagcg caagtggga                                                    19


<210>  226
<211>  19
<212>  DNA
<213>  Artificial sequence

<220>
<223>  Exemplary siRNA target sequence

<400>  226
gagcctacct ggatggcac                                                    19


<210>  227
<211>  19
<212>  DNA
<213>  Artificial sequence

<220>
<223>  Exemplary siRNA target sequence

<400>  227
gccgtgatgt ggaggagga                                                    19


<210>  228
<211>  19
<212>  DNA
<213>  Artificial sequence

<220>
<223>  Exemplary siRNA target sequence

<400>  228
tctcacacca tccagataa                                                    19


<210>  229
<211>  19
<212>  DNA
<213>  Artificial sequence

<220>
<223>  Exemplary siRNA target sequence

<400>  229
cctcacagct tgtaaagtg                                                    19


<210>  230
<211>  19
<212>  DNA
<213>  Artificial sequence

<220>
<223>  Exemplary siRNA target sequence

<400>  230
gggaccagga gacacggaa                                                    19


<210>  231
<211>  19
<212>  DNA
<213>  Artificial sequence

<220>
<223>  Exemplary siRNA target sequence

<400>  231
ccaagacaca tatgaccca                                                    19


<210>  232
<211>  19
<212>  DNA
<213>  Artificial sequence

<220>
<223>  Exemplary siRNA target sequence

<400>  232
agactgaccg agtggacct                                                    19

<210>  233
<211>  19
<212>  DNA
<213>  Artificial sequence

<220>
<223>  Exemplary siRNA target sequence

<400>  233
gaagagctca gatagaaaa                                                    19


<210>  234
<211>  19
<212>  DNA
<213>  Artificial sequence

<220>
<223>  Exemplary siRNA target sequence

<400>  234
gagctcagat agaaaagga                                                    19


<210>  235
<211>  19
<212>  DNA
<213>  Artificial sequence

<220>
<223>  Exemplary siRNA target sequence

<400>  235
gtattgggac caggagaca                                                    19


<210>  236
<211>  19
<212>  DNA
<213>  Artificial sequence

<220>
<223>  Exemplary siRNA target sequence

<400>  236
aggcggagca gttgagagc                                                    19


<210>  237
<211>  19
<212>  DNA
<213>  Artificial sequence

<220>
<223>  Exemplary siRNA target sequence

<400>  237
atgtgcagca tgagggtct                                                    19


<210>  238

<211>  19
<212>  DNA
<213>  Artificial sequence

<220>
<223>  Exemplary siRNA target sequence

<400>  238
ccgtgatgtg gaggaggaa                                                    19


<210>  239
<211>  19
<212>  DNA
<213>  Artificial sequence

<220>
<223>  Exemplary siRNA target sequence

<400>  239
cggagtattg ggaccagga                                                    19


<210>  240
<211>  19
<212>  DNA
<213>  Artificial sequence

<220>
<223>  Exemplary siRNA target sequence

<400>  240
ggagacacgg aatgtgaag                                                    19


<210>  241
<211>  19
<212>  DNA
<213>  Artificial sequence

<220>
<223>  Exemplary siRNA target sequence

<400>  241
cacggaatgt gaaggccca                                                    19


<210>  242
<211>  19
<212>  DNA
<213>  Artificial sequence

<220>
<223>  Exemplary siRNA target sequence

<400>  242
cggacatggc ggctcagat                                                    19


<210>  243
<211>  19
<212>  DNA
<213>  Artificial sequence

<220>
<223>  Exemplary siRNA target sequence

<400>  243
gttgagagcc tacctggat                                                    19


<210>  244
<211>  19
<212>  DNA
<213>  Artificial sequence

<220>
<223>  Exemplary siRNA target sequence

<400>  244
agaaagaaga gctcagatt                                                    19


<210>  245
<211>  19
<212>  DNA
<213>  Artificial sequence

<220>
<223>  Exemplary siRNA target sequence

<400>  245
gcacagactg acagaatga                                                    19


<210>  246
<211>  19
<212>  DNA
<213>  Artificial sequence

<220>
<223>  Exemplary siRNA target sequence

<400>  246
gggaagagga gacacggaa                                                    19


<210>  247
<211>  19
<212>  DNA
<213>  Artificial sequence

<220>
<223>  Exemplary siRNA target sequence

<400>  247
cctacgatgg caaggatta                                                    19


<210>  248
<211>  19
<212>  DNA
<213>  Artificial sequence

<220>
<223>  Exemplary siRNA target sequence

<400>  248
acgcacagac tgacagaat                                                    19


<210>  249
<211>  19
<212>  DNA
<213>  Artificial sequence

<220>
<223>  Exemplary siRNA target sequence

<400>  249
ggtatgaaca gtatgccta                                                    19


<210>  250
<211>  19
<212>  DNA
<213>  Artificial sequence

<220>
<223>  Exemplary siRNA target sequence

<400>  250
ctgctgtgct gtggagaaa                                                    19


<210>  251
<211>  19
<212>  DNA
<213>  Artificial sequence

<220>
<223>  Exemplary siRNA target sequence

<400>  251
aaagaagagc tcagattga                                                    19


<210>  252
<211>  19
<212>  DNA
<213>  Artificial sequence

<220>
<223>  Exemplary siRNA target sequence

<400>  252
gcttcatcgc catgggcta                                                    19


<210>  253
<211>  19
<212>  DNA
<213>  Artificial sequence

<220>
<223>  Exemplary siRNA target sequence

<400>  253
gaagaggaga cacggaaca                                                    19

<210>  254
<211>  19
<212>  DNA
<213>  Artificial sequence

<220>
<223>  Exemplary siRNA target sequence

<400>  254
gaatgaacct gcagaccct                                                    19


<210>  255
<211>  19
<212>  DNA
<213>  Artificial sequence

<220>
<223>  Exemplary siRNA target sequence

<400>  255
gctcagatct ccaagcgca                                                    19


<210>  256
<211>  19
<212>  DNA
<213>  Artificial sequence

<220>
<223>  Exemplary siRNA target sequence

<400>  256
gctgtgctgt ggagaaaga                                                    19


<210>  257
<211>  19
<212>  DNA
<213>  Artificial sequence

<220>
<223>  Exemplary siRNA target sequence

<400>  257
tggagaacgg gaaggagat                                                    19


<210>  258
<211>  19
<212>  DNA
<213>  Artificial sequence

<220>
<223>  Exemplary siRNA target sequence

<400>  258
gctgctgtgc tgtggagaa                                                    19


<210>  259

<211>  19
<212>  DNA
<213>  Artificial sequence

<220>
<223>  Exemplary siRNA target sequence

<400>  259
acaccaaggc ccacgcaca                                                    19


<210>  260
<211>  19
<212>  DNA
<213>  Artificial sequence

<220>
<223>  Exemplary siRNA target sequence

<400>  260
tctccaagcg caagtgtga                                                    19


<210>  261
<211>  19
<212>  DNA
<213>  Artificial sequence

<220>
<223>  Exemplary siRNA target sequence

<400>  261
gtgctgtgga gaaagaaga                                                    19


<210>  262
<211>  19
<212>  DNA
<213>  Artificial sequence

<220>
<223>  Exemplary siRNA target sequence

<400>  262
gaggagacac ggaacacca                                                    19


<210>  263
<211>  19
<212>  DNA
<213>  Artificial sequence

<220>
<223>  Exemplary siRNA target sequence

<400>  263
aggattacct cgccctgaa                                                    19


<210>  264
<211>  19
<212>  DNA
<213>  Artificial sequence

<220>
<223>  Exemplary siRNA target sequence

<400>  264
ccaagacaca cgtgaccca                                                    19


<210>  265
<211>  19
<212>  DNA
<213>  Artificial sequence

<220>
<223>  Exemplary siRNA target sequence

<400>  265
acgtggagct cgtggagac                                                    19


<210>  266
<211>  19
<212>  DNA
<213>  Artificial sequence

<220>
<223>  Exemplary siRNA target sequence

<400>  266
cggagtattg ggaagagga                                                    19


<210>  267
<211>  19
<212>  DNA
<213>  Artificial sequence

<220>
<223>  Exemplary siRNA target sequence

<400>  267
ccacagatac ctggagaac                                                    19


<210>  268
<211>  19
<212>  DNA
<213>  Artificial sequence

<220>
<223>  Exemplary siRNA target sequence

<400>  268
acagatacct ggagaacgg                                                    19


<210>  269
<211>  19
<212>  DNA
<213>  Artificial sequence

<220>
<223>  Exemplary siRNA target sequence

<400>  269
ggaagaggag acacggaac                                                    19


<210>  270
<211>  19
<212>  DNA
<213>  Artificial sequence

<220>
<223>  Exemplary siRNA target sequence

<400>  270
tgaacaaagg agagcctac                                                    19


<210>  271
<211>  19
<212>  DNA
<213>  Artificial sequence

<220>
<223>  Exemplary siRNA target sequence

<400>  271
ccatgggcta cgtggacga                                                    19


<210>  272
<211>  19
<212>  DNA
<213>  Artificial sequence

<220>
<223>  Exemplary siRNA target sequence

<400>  272
gagaaagaag agctcagat                                                    19


<210>  273
<211>  19
<212>  DNA
<213>  Artificial sequence

<220>
<223>  Exemplary siRNA target sequence

<400>  273
gagtattggg aagaggaga                                                    19


<210>  274
<211>  19
<212>  DNA
<213>  Artificial sequence

<220>
<223>  Exemplary siRNA target sequence

<400>  274
gggtatgaac agtatgcct                                                    19

<210>  275
<211>  19
<212>  DNA
<213>  Artificial sequence

<220>
<223>  Exemplary siRNA target sequence

<400>  275
ccaatgtggc tgaacaaag                                                    19


<210>  276
<211>  19
<212>  DNA
<213>  Artificial sequence

<220>
<223>  Exemplary siRNA target sequence

<400>  276
ccaccctgtc tttgactat                                                    19


<210>  277
<211>  19
<212>  DNA
<213>  Artificial sequence

<220>
<223>  Exemplary siRNA target sequence

<400>  277
ggttgtcctt gcagctgta                                                    19


<210>  278
<211>  19
<212>  DNA
<213>  Artificial sequence

<220>
<223>  Exemplary siRNA target sequence

<400>  278
gaaagaagag ctcagattg                                                    19


<210>  279
<211>  19
<212>  DNA
<213>  Artificial sequence

<220>
<223>  Exemplary siRNA target sequence

<400>  279
agtattggga agaggagac                                                    19


<210>  280

<211>  19
<212>  DNA
<213>  Artificial sequence

<220>
<223>  Exemplary siRNA target sequence

<400>  280
ctcagatctc caagcgcaa                                                    19


<210>  281
<211>  19
<212>  DNA
<213>  Artificial sequence

<220>
<223>  Exemplary siRNA target sequence

<400>  281
tggagaaaga agagctcag                                                    19


<210>  282
<211>  19
<212>  DNA
<213>  Artificial sequence

<220>
<223>  Exemplary siRNA target sequence

<400>  282
ggccggagta ttgggaaga                                                    19


<210>  283
<211>  19
<212>  DNA
<213>  Artificial sequence

<220>
<223>  Exemplary siRNA target sequence

<400>  283
ggagaaagaa gagctcaga                                                    19


<210>  284
<211>  19
<212>  DNA
<213>  Artificial sequence

<220>
<223>  Exemplary siRNA target sequence

<400>  284
gtattgggaa gaggagaca                                                    19


<210>  285
<211>  19
<212>  DNA
<213>  Artificial sequence

<220>
<223>  Exemplary siRNA target sequence

<400>  285
aggagacacg gaacaccaa                                                    19


<210>  286
<211>  19
<212>  DNA
<213>  Artificial sequence

<220>
<223>  Exemplary siRNA target sequence

<400>  286
aggcccacgc acagactga                                                    19


<210>  287
<211>  19
<212>  DNA
<213>  Artificial sequence

<220>
<223>  Exemplary siRNA target sequence

<400>  287
cgtcgtgggt ttaacaatt                                                    19


<210>  288
<211>  19
<212>  DNA
<213>  Artificial sequence

<220>
<223>  Exemplary siRNA target sequence

<400>  288
gcaggaacct acagatgtt                                                    19


<210>  289
<211>  19
<212>  DNA
<213>  Artificial sequence

<220>
<223>  Exemplary siRNA target sequence

<400>  289
ctacagatgt tatggttct                                                    19


<210>  290
<211>  19
<212>  DNA
<213>  Artificial sequence

<220>
<223>  Exemplary siRNA target sequence

<400>  290
gcaggactct gatgaacaa                                                    19


<210>  291
<211>  19
<212>  DNA
<213>  Artificial sequence

<220>
<223>  Exemplary siRNA target sequence

<400>  291
gggacagaac agtgaatag                                                    19


<210>  292
<211>  19
<212>  DNA
<213>  Artificial sequence

<220>
<223>  Exemplary siRNA target sequence

<400>  292
gaacattcca ggcagactt                                                    19


<210>  293
<211>  19
<212>  DNA
<213>  Artificial sequence

<220>
<223>  Exemplary siRNA target sequence

<400>  293
tgaaatcagg agagacagt                                                    19


<210>  294
<211>  19
<212>  DNA
<213>  Artificial sequence

<220>
<223>  Exemplary siRNA target sequence

<400>  294
ggcaggactc tgatgaaca                                                    19


<210>  295
<211>  19
<212>  DNA
<213>  Artificial sequence

<220>
<223>  Exemplary siRNA target sequence

<400>  295
acagaacagt gaataggca                                                    19

<210>  296
<211>  19
<212>  DNA
<213>  Artificial sequence

<220>
<223>  Exemplary siRNA target sequence

<400>  296
tcatgggtgg tcaggacaa                                                    19


<210>  297
<211>  19
<212>  DNA
<213>  Artificial sequence

<220>
<223>  Exemplary siRNA target sequence

<400>  297
catcttccac ggcagaata                                                    19


<210>  298
<211>  19
<212>  DNA
<213>  Artificial sequence

<220>
<223>  Exemplary siRNA target sequence

<400>  298
aaatcaggag agacagtca                                                    19


<210>  299
<211>  19
<212>  DNA
<213>  Artificial sequence

<220>
<223>  Exemplary siRNA target sequence

<400>  299
tgaataggca ggactctga                                                    19


<210>  300
<211>  19
<212>  DNA
<213>  Artificial sequence

<220>
<223>  Exemplary siRNA target sequence

<400>  300
tgcccaaggt caacagaac                                                    19


<210>  301

<211>  19
<212>  DNA
<213>  Artificial sequence

<220>
<223>  Exemplary siRNA target sequence

<400>  301
cccacgagca ccacagtca                                                    19


<210>  302
<211>  19
<212>  DNA
<213>  Artificial sequence

<220>
<223>  Exemplary siRNA target sequence

<400>  302
ggagcttgtt tgacattta                                                    19


<210>  303
<211>  19
<212>  DNA
<213>  Artificial sequence

<220>
<223>  Exemplary siRNA target sequence

<400>  303
cgtaggtcta tatgggaaa                                                    19


<210>  304
<211>  19
<212>  DNA
<213>  Artificial sequence

<220>
<223>  Exemplary siRNA target sequence

<400>  304
tgacatacgc acagttgaa                                                    19


<210>  305
<211>  19
<212>  DNA
<213>  Artificial sequence

<220>
<223>  Exemplary siRNA target sequence

<400>  305
gctgagggtc aatggaaca                                                    19


<210>  306
<211>  19
<212>  DNA
<213>  Artificial sequence

<220>
<223>  Exemplary siRNA target sequence

<400>  306
gcagggacct acagatgtt                                                    19


<210>  307
<211>  19
<212>  DNA
<213>  Artificial sequence

<220>
<223>  Exemplary siRNA target sequence

<400>  307
ctacagatgt tgcagttca                                                    19


<210>  308
<211>  19
<212>  DNA
<213>  Artificial sequence

<220>
<223>  Exemplary siRNA target sequence

<400>  308
catcgtggtc gtaggtcta                                                    19


<210>  309
<211>  19
<212>  DNA
<213>  Artificial sequence

<220>
<223>  Exemplary siRNA target sequence

<400>  309
tcgtaggtct atatgggaa                                                    19


<210>  310
<211>  19
<212>  DNA
<213>  Artificial sequence

<220>
<223>  Exemplary siRNA target sequence

<400>  310
ctacagatgc tttggttct                                                    19


<210>  311
<211>  19
<212>  DNA
<213>  Artificial sequence

<220>
<223>  Exemplary siRNA target sequence

<400>  311
ccagcaaccc tgtggtgat                                                    19


<210>  312
<211>  19
<212>  DNA
<213>  Artificial sequence

<220>
<223>  Exemplary siRNA target sequence

<400>  312
aaagaatgct gttgtaatg                                                    19


<210>  313
<211>  19
<212>  DNA
<213>  Artificial sequence

<220>
<223>  Exemplary siRNA target sequence

<400>  313
gtgctgaggg tcaatggaa                                                    19


<210>  314
<211>  19
<212>  DNA
<213>  Artificial sequence

<220>
<223>  Exemplary siRNA target sequence

<400>  314
aacagatacc agcgtgtaa                                                    19


<210>  315
<211>  19
<212>  DNA
<213>  Artificial sequence

<220>
<223>  Exemplary siRNA target sequence

<400>  315
ccaacagata ccagcgtgt                                                    19


<210>  316
<211>  19
<212>  DNA
<213>  Artificial sequence

<220>
<223>  Exemplary siRNA target sequence

<400>  316
ggtctatatg ggaaacctt                                                    19

<210>  317
<211>  19
<212>  DNA
<213>  Artificial sequence

<220>
<223>  Exemplary siRNA target sequence

<400>  317
tcacttaccc tatgagttg                                                    19


<210>  318
<211>  19
<212>  DNA
<213>  Artificial sequence

<220>
<223>  Exemplary siRNA target sequence

<400>  318
catacgcaca gttgaatca                                                    19


<210>  319
<211>  19
<212>  DNA
<213>  Artificial sequence

<220>
<223>  Exemplary siRNA target sequence

<400>  319
acatcgtggt cgtaggtct                                                    19


<210>  320
<211>  19
<212>  DNA
<213>  Artificial sequence

<220>
<223>  Exemplary siRNA target sequence

<400>  320
acctacagat gttgcagtt                                                    19


<210>  321
<211>  19
<212>  DNA
<213>  Artificial sequence

<220>
<223>  Exemplary siRNA target sequence

<400>  321
tcccggagct tgtttgaca                                                    19


<210>  322

<211>  19
<212>  DNA
<213>  Artificial sequence

<220>
<223>  Exemplary siRNA target sequence

<400>  322
tgaaggacaa catgtgact                                                    19


<210>  323
<211>  19
<212>  DNA
<213>  Artificial sequence

<220>
<223>  Exemplary siRNA target sequence

<400>  323
aaggacaaca tgtgactct                                                    19


<210>  324
<211>  19
<212>  DNA
<213>  Artificial sequence

<220>
<223>  Exemplary siRNA target sequence

<400>  324
gcaaccctgt ggtgatcat                                                    19


<210>  325
<211>  19
<212>  DNA
<213>  Artificial sequence

<220>
<223>  Exemplary siRNA target sequence

<400>  325
cgtcataggt ttaacaatt                                                    19


<210>  326
<211>  19
<212>  DNA
<213>  Artificial sequence

<220>
<223>  Exemplary siRNA target sequence

<400>  326
ctgcaatgtt ggtcagata                                                    19


<210>  327
<211>  19
<212>  DNA
<213>  Artificial sequence

<220>
<223>  Exemplary siRNA target sequence

<400>  327
ggtcagatat catgtttga                                                    19


<210>  328
<211>  19
<212>  DNA
<213>  Artificial sequence

<220>
<223>  Exemplary siRNA target sequence

<400>  328
gaacattcca ggcagattt                                                    19


<210>  329
<211>  19
<212>  DNA
<213>  Artificial sequence

<220>
<223>  Exemplary siRNA target sequence

<400>  329
gcgcaaggtc aacagaaca                                                    19


<210>  330
<211>  19
<212>  DNA
<213>  Artificial sequence

<220>
<223>  Exemplary siRNA target sequence

<400>  330
ccacagcaca tgcagggaa                                                    19


<210>  331
<211>  19
<212>  DNA
<213>  Artificial sequence

<220>
<223>  Exemplary siRNA target sequence

<400>  331
cggtgtcact atcgtcata                                                    19


<210>  332
<211>  19
<212>  DNA
<213>  Artificial sequence

<220>
<223>  Exemplary siRNA target sequence

<400>  332
gctatacaaa gaagacaga                                                    19


<210>  333
<211>  19
<212>  DNA
<213>  Artificial sequence

<220>
<223>  Exemplary siRNA target sequence

<400>  333
cagcacatgc agggaacta                                                    19


<210>  334
<211>  19
<212>  DNA
<213>  Artificial sequence

<220>
<223>  Exemplary siRNA target sequence

<400>  334
atatcatgtt tgagcactt                                                    19


<210>  335
<211>  19
<212>  DNA
<213>  Artificial sequence

<220>
<223>  Exemplary siRNA target sequence

<400>  335
gcacatgcag ggaactaca                                                    19


<210>  336
<211>  19
<212>  DNA
<213>  Artificial sequence

<220>
<223>  Exemplary siRNA target sequence

<400>  336
cgcaaggtca acagaacat                                                    19


<210>  337
<211>  19
<212>  DNA
<213>  Artificial sequence

<220>
<223>  Exemplary siRNA target sequence

<400>  337
gcagggaaca gaacagcca                                                    19

<210>  338
<211>  19
<212>  DNA
<213>  Artificial sequence

<220>
<223>  Exemplary siRNA target sequence

<400>  338
ctggtgaaat caggagaga                                                    19


<210>  339
<211>  19
<212>  DNA
<213>  Artificial sequence

<220>
<223>  Exemplary siRNA target sequence

<400>  339
ggttcaggca ggagagagc                                                    19


<210>  340
<211>  19
<212>  DNA
<213>  Artificial sequence

<220>
<223>  Exemplary siRNA target sequence

<400>  340
atacaaagaa gacagaatc                                                    19


<210>  341
<211>  19
<212>  DNA
<213>  Artificial sequence

<220>
<223>  Exemplary siRNA target sequence

<400>  341
ccaaggttca ggcaggaga                                                    19


<210>  342
<211>  19
<212>  DNA
<213>  Artificial sequence

<220>
<223>  Exemplary siRNA target sequence

<400>  342
tgctaagccc agatccaaa                                                    19


<210>  343

<211>  19
<212>  DNA
<213>  Artificial sequence

<220>
<223>  Exemplary siRNA target sequence

<400>  343
cagatatcat gtttgagca                                                    19


<210>  344
<211>  19
<212>  DNA
<213>  Artificial sequence

<220>
<223>  Exemplary siRNA target sequence

<400>  344
caaagaagac agaatccac                                                    19


<210>  345
<211>  19
<212>  DNA
<213>  Artificial sequence

<220>
<223>  Exemplary siRNA target sequence

<400>  345
caacatgagc cctgtgacc                                                    19


<210>  346
<211>  19
<212>  DNA
<213>  Artificial sequence

<220>
<223>  Exemplary siRNA target sequence

<400>  346
agcacttctt tctgcacaa                                                    19


<210>  347
<211>  19
<212>  DNA
<213>  Artificial sequence

<220>
<223>  Exemplary siRNA target sequence

<400>  347
gtgcgcaagg tcaacagaa                                                    19


<210>  348
<211>  19
<212>  DNA
<213>  Artificial sequence

<220>
<223>  Exemplary siRNA target sequence

<400>  348
ccaaatgcta agcccagat                                                    19


<210>  349
<211>  19
<212>  DNA
<213>  Artificial sequence

<220>
<223>  Exemplary siRNA target sequence

<400>  349
aatgctaagc ccagatcca                                                    19


<210>  350
<211>  19
<212>  DNA
<213>  Artificial sequence

<220>
<223>  Exemplary siRNA target sequence

<400>  350
gcaatgttgg tcagatatc                                                    19


<210>  351
<211>  19
<212>  DNA
<213>  Artificial sequence

<220>
<223>  Exemplary siRNA target sequence

<400>  351
gcacttcttt ctgcacaaa                                                    19


<210>  352
<211>  19
<212>  DNA
<213>  Artificial sequence

<220>
<223>  Exemplary siRNA target sequence

<400>  352
agaacattcc aggcagatt                                                    19


<210>  353
<211>  19
<212>  DNA
<213>  Artificial sequence

<220>
<223>  Exemplary siRNA target sequence

<400>  353
gcagggaact acacatgtc                                                    19


<210>  354
<211>  19
<212>  DNA
<213>  Artificial sequence

<220>
<223>  Exemplary siRNA target sequence

<400>  354
acaaagaaga cagaatcca                                                    19


<210>  355
<211>  19
<212>  DNA
<213>  Artificial sequence

<220>
<223>  Exemplary siRNA target sequence

<400>  355
caacagatat catcgtgta                                                    19


<210>  356
<211>  19
<212>  DNA
<213>  Artificial sequence

<220>
<223>  Exemplary siRNA target sequence

<400>  356
atgagggagt ccacagaaa                                                    19


<210>  357
<211>  19
<212>  DNA
<213>  Artificial sequence

<220>
<223>  Exemplary siRNA target sequence

<400>  357
catcgctggt gctgcaaca                                                    19


<210>  358
<211>  19
<212>  DNA
<213>  Artificial sequence

<220>
<223>  Exemplary siRNA target sequence

<400>  358
cagatatcat cgtgtacac                                                    19

<210>  359
<211>  19
<212>  DNA
<213>  Artificial sequence

<220>
<223>  Exemplary siRNA target sequence

<400>  359
tgagggagtc cacagaaaa                                                    19


<210>  360
<211>  19
<212>  DNA
<213>  Artificial sequence

<220>
<223>  Exemplary siRNA target sequence

<400>  360
tcgctggtgc tgcaacaaa                                                    19


<210>  361
<211>  19
<212>  DNA
<213>  Artificial sequence

<220>
<223>  Exemplary siRNA target sequence

<400>  361
cgctggtgct gcaacaaaa                                                    19


<210>  362
<211>  19
<212>  DNA
<213>  Artificial sequence

<220>
<223>  Exemplary siRNA target sequence

<400>  362
ccagacacct gcatgttct                                                    19


<210>  363
<211>  19
<212>  DNA
<213>  Artificial sequence

<220>
<223>  Exemplary siRNA target sequence

<400>  363
ccaacagata tcatcgtgt                                                    19


<210>  364

<211>  19
<212>  DNA
<213>  Artificial sequence

<220>
<223>  Exemplary siRNA target sequence

<400>  364
acatgaggga gtccacaga                                                    19


<210>  365
<211>  19
<212>  DNA
<213>  Artificial sequence

<220>
<223>  Exemplary siRNA target sequence

<400>  365
catgagggag tccacagaa                                                    19


<210>  366
<211>  19
<212>  DNA
<213>  Artificial sequence

<220>
<223>  Exemplary siRNA target sequence

<400>  366
cctggccaca tgagggagt                                                    19


<210>  367
<211>  19
<212>  DNA
<213>  Artificial sequence

<220>
<223>  Exemplary siRNA target sequence

<400>  367
acagatatca tcgtgtaca                                                    19


<210>  368
<211>  19
<212>  DNA
<213>  Artificial sequence

<220>
<223>  Exemplary siRNA target sequence

<400>  368
ccacatgagg gagtccaca                                                    19


<210>  369
<211>  19
<212>  DNA
<213>  Artificial sequence

<220>
<223>  Exemplary siRNA target sequence

<400>  369
gatagatggt gttggtgaa                                                    19


<210>  370
<211>  19
<212>  DNA
<213>  Artificial sequence

<220>
<223>  Exemplary siRNA target sequence

<400>  370
caagaaaagg tctgggtaa                                                    19


<210>  371
<211>  19
<212>  DNA
<213>  Artificial sequence

<220>
<223>  Exemplary siRNA target sequence

<400>  371
cgactaaact taatctgta                                                    19


<210>  372
<211>  19
<212>  DNA
<213>  Artificial sequence

<220>
<223>  Exemplary siRNA target sequence

<400>  372
gtgacgggat tgaaacaaa                                                    19


<210>  373
<211>  19
<212>  DNA
<213>  Artificial sequence

<220>
<223>  Exemplary siRNA target sequence

<400>  373
caggaaggaa cgcgactaa                                                    19


<210>  374
<211>  19
<212>  DNA
<213>  Artificial sequence

<220>
<223>  Exemplary siRNA target sequence

<400>  374
ataagaaaga agaggctga                                                    19


<210>  375
<211>  19
<212>  DNA
<213>  Artificial sequence

<220>
<223>  Exemplary siRNA target sequence

<400>  375
ggatagatgg tgttggtga                                                    19


<210>  376
<211>  19
<212>  DNA
<213>  Artificial sequence

<220>
<223>  Exemplary siRNA target sequence

<400>  376
gggattgaaa caaagacaa                                                    19


<210>  377
<211>  19
<212>  DNA
<213>  Artificial sequence

<220>
<223>  Exemplary siRNA target sequence

<400>  377
aagaagaggc tgaggggtt                                                    19


<210>  378
<211>  19
<212>  DNA
<213>  Artificial sequence

<220>
<223>  Exemplary siRNA target sequence

<400>  378
cagttgatgt tcaccataa                                                    19


<210>  379
<211>  19
<212>  DNA
<213>  Artificial sequence

<220>
<223>  Exemplary siRNA target sequence

<400>  379
tcacagagac agggaacta                                                    19

<210>  380
<211>  19
<212>  DNA
<213>  Artificial sequence

<220>
<223>  Exemplary siRNA target sequence

<400>  380
gcataagaaa gaagaggct                                                    19


<210>  381
<211>  19
<212>  DNA
<213>  Artificial sequence

<220>
<223>  Exemplary siRNA target sequence

<400>  381
ttaaaacagc tgagactta                                                    19


<210>  382
<211>  19
<212>  DNA
<213>  Artificial sequence

<220>
<223>  Exemplary siRNA target sequence

<400>  382
gaaacaaaga caacacctt                                                    19


<210>  383
<211>  19
<212>  DNA
<213>  Artificial sequence

<220>
<223>  Exemplary siRNA target sequence

<400>  383
aagaaaaggt ctgggtaat                                                    19


<210>  384
<211>  19
<212>  DNA
<213>  Artificial sequence

<220>
<223>  Exemplary siRNA target sequence

<400>  384
cattctattc acagagaca                                                    19


<210>  385

<211>  19
<212>  DNA
<213>  Artificial sequence

<220>
<223>  Exemplary siRNA target sequence

<400>  385
acagggaact acacagtga                                                    19


<210>  386
<211>  19
<212>  DNA
<213>  Artificial sequence

<220>
<223>  Exemplary siRNA target sequence

<400>  386
cgggattgaa acaaagaca                                                    19


<210>  387
<211>  19
<212>  DNA
<213>  Artificial sequence

<220>
<223>  Exemplary siRNA target sequence

<400>  387
ggattgaaac aaagacaac                                                    19


<210>  388
<211>  19
<212>  DNA
<213>  Artificial sequence

<220>
<223>  Exemplary siRNA target sequence

<400>  388
ctaccagtgt tgtgccaga                                                    19


<210>  389
<211>  19
<212>  DNA
<213>  Artificial sequence

<220>
<223>  Exemplary siRNA target sequence

<400>  389
ggaaggaacg cgactaaac                                                    19


<210>  390
<211>  19
<212>  DNA
<213>  Artificial sequence

<220>
<223>  Exemplary siRNA target sequence

<400>  390
aggaacgcga ctaaactta                                                    19


<210>  391
<211>  19
<212>  DNA
<213>  Artificial sequence

<220>
<223>  Exemplary siRNA target sequence

<400>  391
actacacagt gacgggatt                                                    19


<210>  392
<211>  19
<212>  DNA
<213>  Artificial sequence

<220>
<223>  Exemplary siRNA target sequence

<400>  392
cagtctggtg gatgcatta                                                    19


<210>  393
<211>  19
<212>  DNA
<213>  Artificial sequence

<220>
<223>  Exemplary siRNA target sequence

<400>  393
ggaacgcgac taaacttaa                                                    19


<210>  394
<211>  19
<212>  DNA
<213>  Artificial sequence

<220>
<223>  Exemplary siRNA target sequence

<400>  394
cagccataat gaaggcact                                                    19


<210>  395
<211>  19
<212>  DNA
<213>  Artificial sequence

<220>
<223>  Exemplary siRNA target sequence

<400>  395
gttcaggctt cctacaaga                                                    19


<210>  396
<211>  19
<212>  DNA
<213>  Artificial sequence

<220>
<223>  Exemplary siRNA target sequence

<400>  396
aatctgtact gtatggcat                                                    19


<210>  397
<211>  19
<212>  DNA
<213>  Artificial sequence

<220>
<223>  Exemplary siRNA target sequence

<400>  397
cacagtgacg ggattgaaa                                                    19


<210>  398
<211>  19
<212>  DNA
<213>  Artificial sequence

<220>
<223>  Exemplary siRNA target sequence

<400>  398
cagtgacggg attgaaaca                                                    19


<210>  399
<211>  19
<212>  DNA
<213>  Artificial sequence

<220>
<223>  Exemplary siRNA target sequence

<400>  399
gcaaggacag gtctggaga                                                    19


<210>  400
<211>  19
<212>  DNA
<213>  Artificial sequence

<220>
<223>  Exemplary siRNA target sequence

<400>  400
tgggatagat ggtgttggt                                                    19

<210>  401
<211>  19
<212>  DNA
<213>  Artificial sequence

<220>
<223>  Exemplary siRNA target sequence

<400>  401
aagacaacac cttgagttc                                                    19


<210>  402
<211>  19
<212>  DNA
<213>  Artificial sequence

<220>
<223>  Exemplary siRNA target sequence

<400>  402
gaacgcgact aaacttaat                                                    19


<210>  403
<211>  19
<212>  DNA
<213>  Artificial sequence

<220>
<223>  Exemplary siRNA target sequence

<400>  403
agaagaggct gaggggttt                                                    19


<210>  404
<211>  19
<212>  DNA
<213>  Artificial sequence

<220>
<223>  Exemplary siRNA target sequence

<400>  404
caggcttcct acaagaaaa                                                    19


<210>  405
<211>  19
<212>  DNA
<213>  Artificial sequence

<220>
<223>  Exemplary siRNA target sequence

<400>  405
agacagggaa ctacacagt                                                    19


<210>  406

<211>  19
<212>  DNA
<213>  Artificial sequence

<220>
<223>  Exemplary siRNA target sequence

<400>  406
gcgactaaac ttaatctgt                                                    19


<210>  407
<211>  19
<212>  DNA
<213>  Artificial sequence

<220>
<223>  Exemplary siRNA target sequence

<400>  407
gatgttcacc ataagccaa                                                    19


<210>  408
<211>  19
<212>  DNA
<213>  Artificial sequence

<220>
<223>  Exemplary siRNA target sequence

<400>  408
acagagacag ggaactaca                                                    19


<210>  409
<211>  19
<212>  DNA
<213>  Artificial sequence

<220>
<223>  Exemplary siRNA target sequence

<400>  409
ccaggaagga acgcgacta                                                    19


<210>  410
<211>  19
<212>  DNA
<213>  Artificial sequence

<220>
<223>  Exemplary siRNA target sequence

<400>  410
aggaaggaac gcgactaaa                                                    19


<210>  411
<211>  19
<212>  DNA
<213>  Artificial sequence

<220>
<223>  Exemplary siRNA target sequence

<400>  411
gtatccgcct tcagggcca                                                    19


<210>  412
<211>  19
<212>  DNA
<213>  Artificial sequence

<220>
<223>  Exemplary siRNA target sequence

<400>  412
gcattaacat caccagctc                                                    19


<210>  413
<211>  19
<212>  DNA
<213>  Artificial sequence

<220>
<223>  Exemplary siRNA target sequence

<400>  413
gaagccagaa gtcaggtat                                                    19


<210>  414
<211>  19
<212>  DNA
<213>  Artificial sequence

<220>
<223>  Exemplary siRNA target sequence

<400>  414
attcacagag acagggaac                                                    19


<210>  415
<211>  19
<212>  DNA
<213>  Artificial sequence

<220>
<223>  Exemplary siRNA target sequence

<400>  415
gactaaactt aatctgtac                                                    19


<210>  416
<211>  19
<212>  DNA
<213>  Artificial sequence

<220>
<223>  Exemplary siRNA target sequence

<400>  416
gattgaaaca aagacaaca                                                    19


<210>  417
<211>  19
<212>  DNA
<213>  Artificial sequence

<220>
<223>  Exemplary siRNA target sequence

<400>  417
gctgttggaa cccggcaga                                                    19


<210>  418
<211>  19
<212>  DNA
<213>  Artificial sequence

<220>
<223>  Exemplary siRNA target sequence

<400>  418
gccagaagcc agaagtcag                                                    19


<210>  419
<211>  19
<212>  DNA
<213>  Artificial sequence

<220>
<223>  Exemplary siRNA target sequence

<400>  419
ctaaacttct caaggattt                                                    19


<210>  420
<211>  19
<212>  DNA
<213>  Artificial sequence

<220>
<223>  Exemplary siRNA target sequence

<400>  420
tgtaagaagg tcagacttt                                                    19


<210>  421
<211>  19
<212>  DNA
<213>  Artificial sequence

<220>
<223>  Exemplary siRNA target sequence

<400>  421
gaggaaaagg actggaatt                                                    19

<210>  422
<211>  19
<212>  DNA
<213>  Artificial sequence

<220>
<223>  Exemplary siRNA target sequence

<400>  422
gaaaaggact ggaattcta                                                    19


<210>  423
<211>  19
<212>  DNA
<213>  Artificial sequence

<220>
<223>  Exemplary siRNA target sequence

<400>  423
gctggatgaa atatggtaa                                                    19


<210>  424
<211>  19
<212>  DNA
<213>  Artificial sequence

<220>
<223>  Exemplary siRNA target sequence

<400>  424
ctgcctagcc agagactca                                                    19


<210>  425
<211>  19
<212>  DNA
<213>  Artificial sequence

<220>
<223>  Exemplary siRNA target sequence

<400>  425
tggattggtc tgaggaaca                                                    19


<210>  426
<211>  19
<212>  DNA
<213>  Artificial sequence

<220>
<223>  Exemplary siRNA target sequence

<400>  426
caacccaagc ccagaatga                                                    19


<210>  427

<211>  19
<212>  DNA
<213>  Artificial sequence

<220>
<223>  Exemplary siRNA target sequence

<400>  427
ccagaatgac tatggacca                                                    19


<210>  428
<211>  19
<212>  DNA
<213>  Artificial sequence

<220>
<223>  Exemplary siRNA target sequence

<400>  428
tgactatgga ccacagcaa                                                    19


<210>  429
<211>  19
<212>  DNA
<213>  Artificial sequence

<220>
<223>  Exemplary siRNA target sequence

<400>  429
taacggacaa tcaggaaat                                                    19


<210>  430
<211>  19
<212>  DNA
<213>  Artificial sequence

<220>
<223>  Exemplary siRNA target sequence

<400>  430
cctctaaact tctcaagga                                                    19


<210>  431
<211>  19
<212>  DNA
<213>  Artificial sequence

<220>
<223>  Exemplary siRNA target sequence

<400>  431
ggaagatgga tcacctcta                                                    19


<210>  432
<211>  19
<212>  DNA
<213>  Artificial sequence

<220>
<223>  Exemplary siRNA target sequence

<400>  432
ctggaattct agtctggaa                                                    19


<210>  433
<211>  19
<212>  DNA
<213>  Artificial sequence

<220>
<223>  Exemplary siRNA target sequence

<400>  433
ggattggtct gaggaacaa                                                    19


<210>  434
<211>  19
<212>  DNA
<213>  Artificial sequence

<220>
<223>  Exemplary siRNA target sequence

<400>  434
ctgcagttct tctgagtgt                                                    19


<210>  435
<211>  19
<212>  DNA
<213>  Artificial sequence

<220>
<223>  Exemplary siRNA target sequence

<400>  435
tctcaaggat ttcttctaa                                                    19


<210>  436
<211>  19
<212>  DNA
<213>  Artificial sequence

<220>
<223>  Exemplary siRNA target sequence

<400>  436
actatggacc acagcaaaa                                                    19


<210>  437
<211>  19
<212>  DNA
<213>  Artificial sequence

<220>
<223>  Exemplary siRNA target sequence

<400>  437
ggcaatagct ttggggctt                                                    19


<210>  438
<211>  19
<212>  DNA
<213>  Artificial sequence

<220>
<223>  Exemplary siRNA target sequence

<400>  438
ataacggaca atcaggaaa                                                    19


<210>  439
<211>  19
<212>  DNA
<213>  Artificial sequence

<220>
<223>  Exemplary siRNA target sequence

<400>  439
tctaatagct ttgtgcaga                                                    19


<210>  440
<211>  19
<212>  DNA
<213>  Artificial sequence

<220>
<223>  Exemplary siRNA target sequence

<400>  440
gactatggac cacagcaaa                                                    19


<210>  441
<211>  19
<212>  DNA
<213>  Artificial sequence

<220>
<223>  Exemplary siRNA target sequence

<400>  441
gcttctgact gcagttctt                                                    19


<210>  442
<211>  19
<212>  DNA
<213>  Artificial sequence

<220>
<223>  Exemplary siRNA target sequence

<400>  442
gctggattgg tctgaggaa                                                    19

<210>  443
<211>  19
<212>  DNA
<213>  Artificial sequence

<220>
<223>  Exemplary siRNA target sequence

<400>  443
gaggtgggaa gatggatca                                                    19


<210>  444
<211>  19
<212>  DNA
<213>  Artificial sequence

<220>
<223>  Exemplary siRNA target sequence

<400>  444
tctaaacttc tcaaggatt                                                    19


<210>  445
<211>  19
<212>  DNA
<213>  Artificial sequence

<220>
<223>  Exemplary siRNA target sequence

<400>  445
agacatgcgg tgccatcaa                                                    19


<210>  446
<211>  19
<212>  DNA
<213>  Artificial sequence

<220>
<223>  Exemplary siRNA target sequence

<400>  446
cagaccgctg gatgaaata                                                    19


<210>  447
<211>  19
<212>  DNA
<213>  Artificial sequence

<220>
<223>  Exemplary siRNA target sequence

<400>  447
ggaggtggga agatggatc                                                    19


<210>  448

<211>  19
<212>  DNA
<213>  Artificial sequence

<220>
<223>  Exemplary siRNA target sequence

<400>  448
ctctaaactt ctcaaggat                                                    19


<210>  449
<211>  19
<212>  DNA
<213>  Artificial sequence

<220>
<223>  Exemplary siRNA target sequence

<400>  449
cggcaaccca agcccagaa                                                    19


<210>  450
<211>  19
<212>  DNA
<213>  Artificial sequence

<220>
<223>  Exemplary siRNA target sequence

<400>  450
gtgtaagaag gtcagactt                                                    19


<210>  451
<211>  19
<212>  DNA
<213>  Artificial sequence

<220>
<223>  Exemplary siRNA target sequence

<400>  451
taagaaggtc agactttga                                                    19


<210>  452
<211>  19
<212>  DNA
<213>  Artificial sequence

<220>
<223>  Exemplary siRNA target sequence

<400>  452
gaacaattct ggctggagg                                                    19


<210>  453
<211>  19
<212>  DNA
<213>  Artificial sequence

<220>
<223>  Exemplary siRNA target sequence

<400>  453
tgtgtaagaa ggtcagact                                                    19


<210>  454
<211>  19
<212>  DNA
<213>  Artificial sequence

<220>
<223>  Exemplary siRNA target sequence

<400>  454
ggaggaaaag gactggaat                                                    19


<210>  455
<211>  19
<212>  DNA
<213>  Artificial sequence

<220>
<223>  Exemplary siRNA target sequence

<400>  455
gtgggaagat ggatcacct                                                    19


<210>  456
<211>  19
<212>  DNA
<213>  Artificial sequence

<220>
<223>  Exemplary siRNA target sequence

<400>  456
ctaatagctt tgtgcagac                                                    19


<210>  457
<211>  19
<212>  DNA
<213>  Artificial sequence

<220>
<223>  Exemplary siRNA target sequence

<400>  457
gtgcagacat gcggtgcca                                                    19


<210>  458
<211>  19
<212>  DNA
<213>  Artificial sequence

<220>
<223>  Exemplary siRNA target sequence

<400>  458
atgcggtgcc atcaacaaa                                                    19


<210>  459
<211>  19
<212>  DNA
<213>  Artificial sequence

<220>
<223>  Exemplary siRNA target sequence

<400>  459
agcctcaagc tgtgaagtt                                                    19


<210>  460
<211>  19
<212>  DNA
<213>  Artificial sequence

<220>
<223>  Exemplary siRNA target sequence

<400>  460
ctggatgaaa tatggtaac                                                    19


<210>  461
<211>  19
<212>  DNA
<213>  Artificial sequence

<220>
<223>  Exemplary siRNA target sequence

<400>  461
gttattattt ctcagtgga                                                    19


<210>  462
<211>  19
<212>  DNA
<213>  Artificial sequence

<220>
<223>  Exemplary siRNA target sequence

<400>  462
aggaaaagga ctggaattc                                                    19


<210>  463
<211>  19
<212>  DNA
<213>  Artificial sequence

<220>
<223>  Exemplary siRNA target sequence

<400>  463
cagaatgact atggaccac                                                    19

<210>  464
<211>  19
<212>  DNA
<213>  Artificial sequence

<220>
<223>  Exemplary siRNA target sequence

<400>  464
ctcaaggatt tcttctaat                                                    19


<210>  465
<211>  19
<212>  DNA
<213>  Artificial sequence

<220>
<223>  Exemplary siRNA target sequence

<400>  465
aagctgtgaa gttccttta                                                    19


<210>  466
<211>  19
<212>  DNA
<213>  Artificial sequence

<220>
<223>  Exemplary siRNA target sequence

<400>  466
gctgtgaagt tcctttaca                                                    19


<210>  467
<211>  19
<212>  DNA
<213>  Artificial sequence

<220>
<223>  Exemplary siRNA target sequence

<400>  467
tgggtgtgta agaaggtca                                                    19


<210>  468
<211>  19
<212>  DNA
<213>  Artificial sequence

<220>
<223>  Exemplary siRNA target sequence

<400>  468
tggcaatagc tttggggct                                                    19